# EUROPÄISCHE PATENTSCHRIFT

(11) **EP 0 647 637 B1**
(45) Veröffentlichungstag und Bekanntmachung des Hinweises auf die Patenterteilung: **27.01.1999**
(21) Anmeldenummer: 94113566.7
(22) Anmeldetag: 31.08.1994
(51) Int. Cl.: C07D 307/94, C07D 405/12, C07C 69/757, A01N 43/08

(54) **3-Aryl-4-hydroxy-3-dihydrofuranon-Derivate**
3-Aryl-4-hydroxy-3-dihydrofuranone derivatives
Dérivés de 3-aryle-4-hydroxy-3-dihydrofuranone

(30) Priorität: 17.09.1993 DE 4331672; 05.11.1993 DE 4337853
(43) Veröffentlichungstag der Anmeldung: 12.04.1995
(73) Patentinhaber: BAYER AG, 51368 Leverkusen (DE)
(72) Erfinder: Fischer, Dr. Reiner, D-40789 Monheim (DE); Bretschneider, Dr. Thomas, D-53797 Lohmar (DE); Krüger, Dr. Bernd-Wieland, D-51467 Bergisch Gladbach (DE); Santel, Dr. Hans-Joachim, D-51371 Leverkusen (DE); Dollinger, Dr. Markus, D-51381 Leverkusen (DE); Wachendorff-Neumann, Dr. Ulrike, D-40789 Monheim (DE); Erdelen, Dr. Christoph, D-42799 Leichlingen (DE)

(56) Entgegenhaltungen:
- EP-A- 0 528 156
- US-A- 4 525 201

## Beschreibung

Die vorliegende Erfindung betrifft neue 3-Aryl-4-hydroxy-Δ³-dihydro-furanon-Derivate, Verfahren zu ihrer Herstellung und ihre Verwendung als Schädlingsbekämpfungsmittel.

Es ist bekannt, daß bestimmte substituierte Δ³-Dihydrofuran-2-on-Derivate herbizide Eigenschaften besitzen (vgl. DE-A 4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2) ist ebenfalls in DE-A 4 014 420 beschrieben. Ähnlich strukturierte Verbindungen ohne Angabe einer insektiziden und/oder akariziden Wirksamkeit sind aus der Publikation Campbell et al. J. Chem. Soc., Perkin Trans. 1 1985, (8) 1567-76 bekannt. Weiterhin sind 3-Aryl-Δ³-dihydrofuranon-Derivate mit herbiziden, akariziden und insektiziden Eigenschaften aus EP 528 156 bekannt, jedoch ist die dort beschriebene Wirkung nicht immer ausreichend.

Es wurden nun neue 3-Aryl-4-hydroxy-Δ³-dihydrofuranon-Derivate der Formel (I) 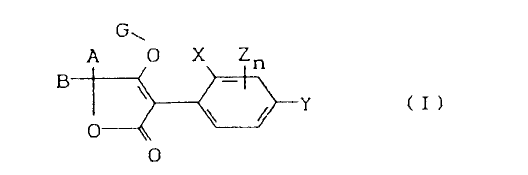 gefunden,
in welcher
- X: für Alkyl, Halogen, Alkoxy oder Halogenalkyl steht,
- Y: für Wasserstoff, Alkyl, Halogen, Alkoxy, Halogenalkyl steht,
- Z: für Alkyl, Halogen, Alkoxy steht,
- n: für eine Zahl von 0-3 steht, oder wobei die Reste X und Z gemeinsam mit dem Phenylrest an den sie gebunden sind, den Naphthalinrest der Formel 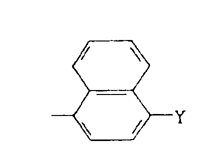 bilden,
in welchem Y die oben angegebene Bedeutung hat,
- G: für Wasserstoff (a) oder für die Gruppen 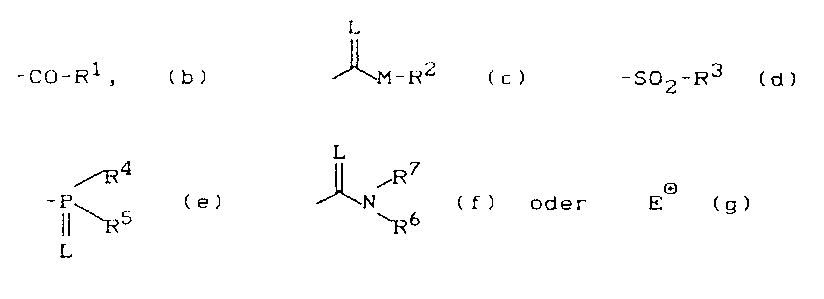 steht,
- A und B: gemeinsam mit dem Kohlenstoffatom, an das sie gebunden sind einen durch Alkoxy, Alkylthio, Alkylsulfoxyl oder Alkylsulfonyl substituierten Cyclus bilden,
- A und B: gemeinsam mit dem Kohlenstoffatom, an das sie gebunden sind für einen Cyclus stehen, bei dem zwei Substituenten gemeinsam mit den Kohlenstoffatomen, an die sie gebunden sind, für einen gegebenenfalls durch Alkyl, Alkoxy oder Halogen substituierten gesättigten oder einfach ungesättigten Cyclus stehen, der durch Sauerstoff oder Schwefel unterbrochen sein kann,
- E^{⊕}: für ein Metallionäquivalent oder ein Ammonium steht,
- L und M: für Sauerstoff und/oder Schwefel steht,
- R¹: für gegebenenfalls durch Halogen substituiertes Alkyl, Alkenyl, Alkoxyalkyl, Alkylthioalkyl, Polyalkoxyalkyl oder Cycloalkyl, das durch Heteroatome unterbrochen sein kann, gegebenenfalls substituiertes Phenyl, gegebenenfalls substituiertes Phenylalkyl, substituiertes Hetaryl, substituiertes Phenoxyalkyl oder substituiertes Hetaryloxyalkyl steht und
- R²: für gegebenenfalls durch Halogen substituiertes Alkyl, Alkenyl, Alkoxyalkyl, Polyalkoxyalkyl oder gegebenenfalls substituiertes Phenyl oder Benzyl steht,
- R³, R⁴ und R⁵: unabhängig voneinander für gegebenenfalls durch Halogen substituiertes Alkyl, Alkoxy, Alkylamino, Dialkylamino, Alkylthio, Alkenylthio, Alkinylthio, Cycloalkylthio und für gegebenenfalls substituiertes Phenyl, Phenoxy oder Phenylthio stehen,
- R⁶ und R⁷: unabhängig voneinander für Wasserstoff, gegebenenfalls durch Halogen substituiertes Alkyl, Alkenyl, Alkoxy, Alkoxyalkyl, für gegebenenfalls substituiertes Phenyl, für gegebenenfalls substituiertes Benzyl stehen
- oder wobei R⁶ und R⁷: zusammen für einen gegebenenfalls durch Sauerstoff oder Schwefel unterbrochenen Alkylenrest stehen,
sowie die stereo- und enantiomerenreinen Formen von Verbindungen der Formel (I).

Unter Einbeziehung der verschiedenen Bedeutungen (a), (b), (c), (d), (e), (f) und (g) der Gruppe G der allgemeinen Formel (I) ergeben sich folgende hauptsächlichen Strukturen (Ia) bis (Ig): 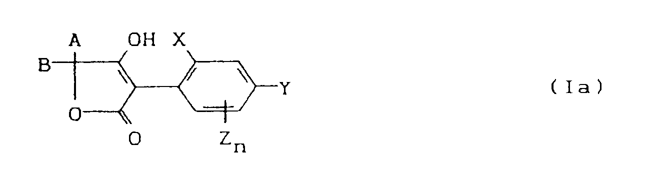 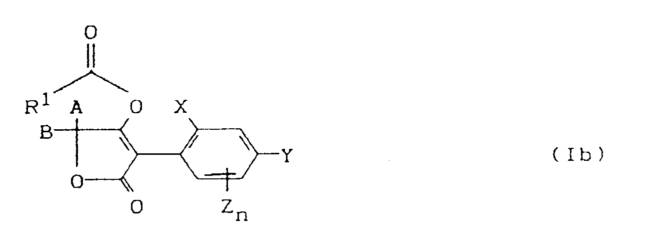 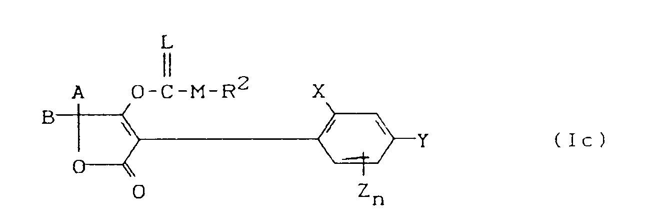 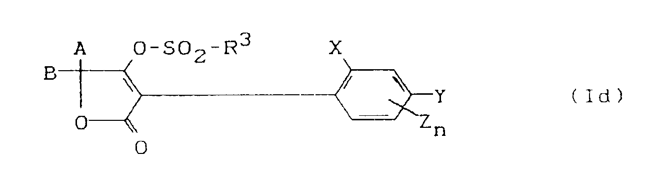 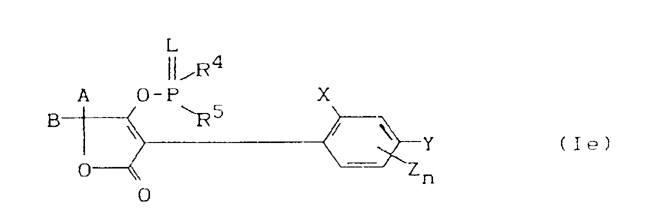 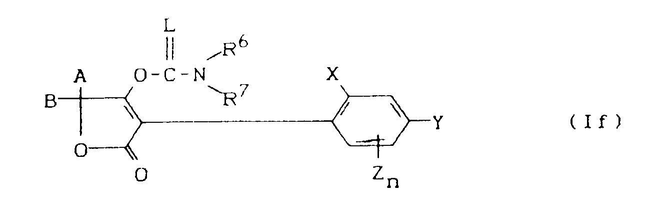 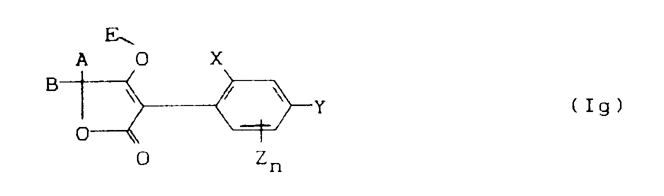 worin
A, B, E, L, M, X, Y, Zₙ, R¹, R², R³, R⁴, R⁵, R⁶, R⁷ und
n die oben angebenenen Bedeutungen besitzen.

Weiterhin wurde gefunden, daß man 3-Aryl-4-hydroxy-Δ³-dihydrofuranon-Derivate der Formel (Ia) 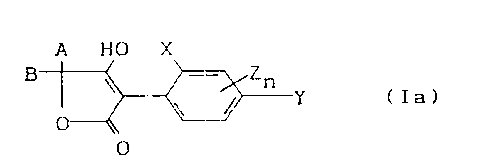 in welcher
A, B, X, Y, Z und n die oben angegebene Bedeutung haben,
erhält, wenn man
(A)
   Carbonsäureester der Formel (II) 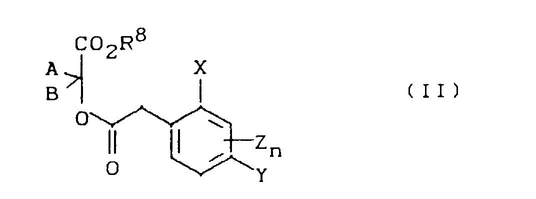 in welcher
   A, B, X, Y, Z und n die oben angegebene Bedeutung haben
   und
   - R⁸: für Alkyl steht,
   in Gegenwart eines Verdünnungsmittels und in Gegenwart einer Base intramolekular kondensiert.
(B)
   Außerdem wurde gefunden, daß man Verbindungen der Formel (Ib) 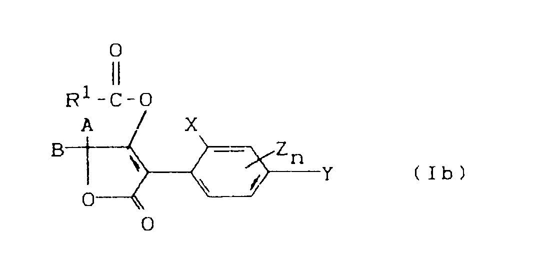 in welcher
   A, B, X, Y, Z, R¹ und n die oben angegebene Bedeutung haben,
   erhält, wenn man Verbindungen der Formel (Ia), 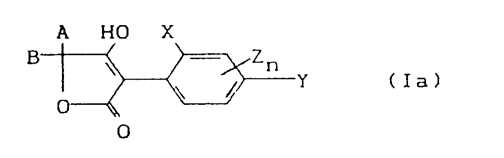 in welcher
   A, B, X, Y, Z und n die oben angegebene Bedeutung haben,
   α) mit Säurehalogeniden der allgemeinen Formel (III) 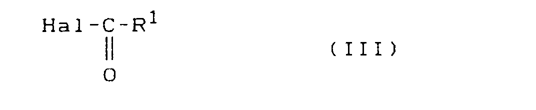 in welcher
      - R¹: die oben angegebene Bedeutung hat
      und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
      umsetzt.
(C)
   Ferner wurde gefunden, daß man Verbindungen der Formel (Ic) 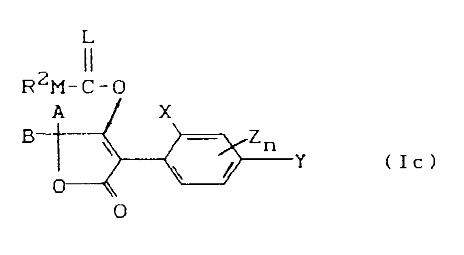 in welcher
   - A, B, X, Y, Z, R² und n: die oben angegebene Bedeutung haben,
   - L: für Sauerstoff
   und
   - M: für Sauerstoff oder Schwefel steht,
   erhält, wenn man Verbindungen der Formel (Ia) 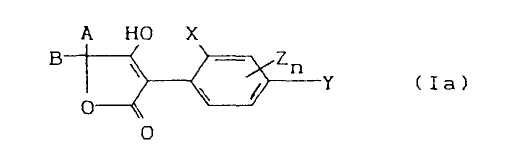 in welcher
   A, B, X, Y, Z und n die oben angegebene Bedeutung haben
   mit Chlorameisensäureester oder Chlorameisensäurethiolester der allgemeinen Formel (V)

   R²-M-CO-Cl (V)

   in welcher
   - R² und M: die oben angegebene Bedeutung haben, gegebenenfals in Gegenwart eines Verdünnungsmittels und gegebenenfalls in Gegenwart eines Säurebindemittels umsetzt.
D) Ferner wurde gefunden, daß man Verbindungen der Formel (Ic) 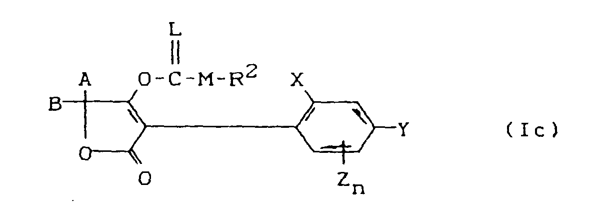 in welcher
   - A, B, R², X, Y, Z und n: die oben angegebene Bedeutung haben,
   - L: für Schwefel
   und
   - M: für Sauerstoff oder Schwefel steht,
   erhält, wenn man Verbindungen der Formel (Ia) 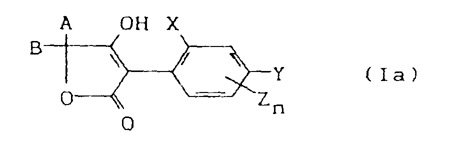 in welcher
   A, B, X, Y, Z und n die oben angegebene Bedeutung haben
   mit Chlormonothioameisensäureestern oder Chlordithioameisensäureestern der allgemeinen Formel (VI) 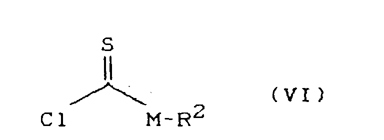 in welcher
   - M und R²: die oben angegebene Bedeutung haben
   gegebenenfalls in Gegenwart eines Verdünnungsmittels und gegebenenfalls in Gegenwart eines Säurebindemittels umsetzt.
E) Außerdem wurde gefunden, daß man Verbindungen der Formel (Id) 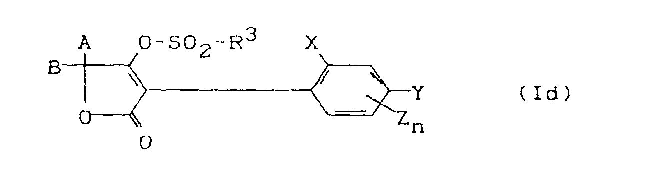 in welcher
   A, B, X, Y, Z, R³ und n die oben angegebene Bedeutung haben,
   erhält, wenn man Verbindungen der Formel (Ia) 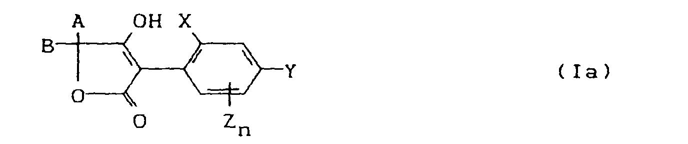 in welcher
   A, B, X, Y, Z und n die oben angegebene Bedeutung haben,
   mit Sulfonsäurechloriden der allgemeinen Formel (VII)

   R³-SO₂-Cl (VII)

   in welcher
   - R³: die oben angegebene Bedeutung hat
   gegebenenfalls in Gegenwart eines Verdünnungsmittels und gegebenenfalls in Gegenwart eines Säurebindemittels,
   umsetzt.
F) Weiterhin wurde gefunden, daß man Verbindungen der Formel (Ie) 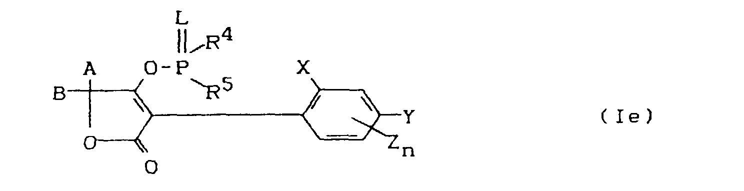 in welcher
   A, B, L, X, Y, Z, R⁴, R⁵ und n die oben angegebene Bedeutung haben,
   erhält, wenn man
   Verbindungen der Formel (Ia) 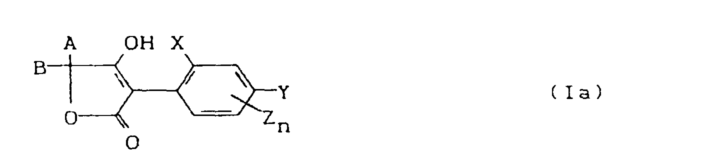 in welcher
   A, B, X, Y, Z und n die oben angegebene Bedeutung haben
   mit Phosphorverbindungen der Formel (IX) 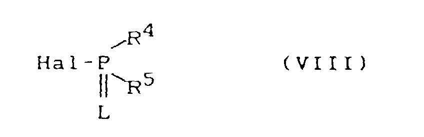 in welcher
   - L, R⁴ und R⁵: die oben angegebene Bedeutung haben
   und
   - Hal: für Halogen, insbesondere Chlor oder Brom steht,
   gegebenenfalls in Gegenwart eines Verdünnungsmittels und gegebenenfalls in Gegenwart eines Säurebindemittels umsetzt.
G) Ferner wurde gefunden, daß man Verbindungen der Formel (If) 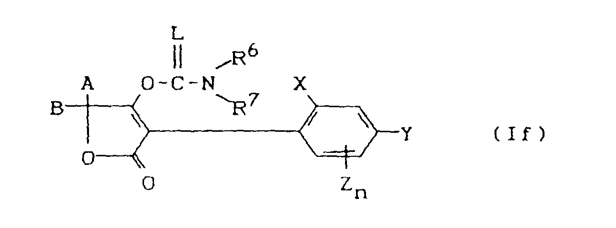 in welcher
   A, B, L, X, Y, Z, R⁶, R⁷ und n die oben angegebene Bedeutung haben,
   erhält, wenn man Verbindungen der Formel (Ia), 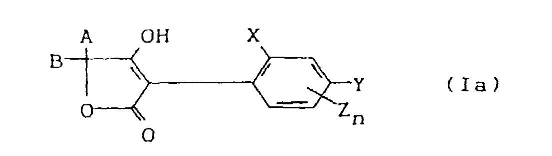 in welcher
   A, B, X, Y, Z und n die oben angegebene Bedeutung haben
   α) mit Isocyanaten der allgemeinen Formel (IX)

      R⁶-N=C=L (IX)

      in welcher
      L und R⁶ die oben angegebene Bedeutung hat gegebenenfalls in Gegenwart eines Verdünnungsmittels und gegebenenfalls in Gegenwart eines Katalysators umsetzt,
      oder
   B) mit Carbamidsäurechloriden oder Thiocarbamidsäurechloriden der allgemeinen Formel (X) 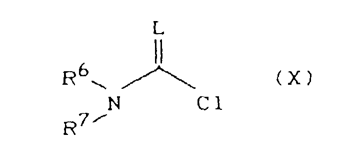 in welcher
      L, R⁶ und R⁷ die oben angegebene Bedeutung haben, gegebenenfalls in Gegenwart eines Verdünnungsmittels und gegebenenfalls in Gegenwart eines Säurebindemittels,
      umsetzt.
H) Weiterhin wurde gefunden, daß man Verbindungen der Formel (Ig) 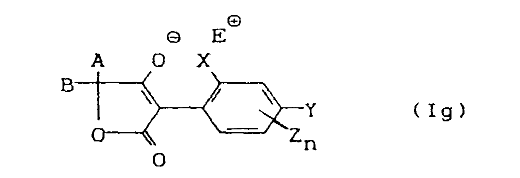 in welcher
   X, Y, Z, A, B und n die oben angegebene Bedeutung haben,
   und E^{⊕} für ein Metallionäquivalent oder für ein Ammoniumion steht,
   erhält, wenn man Verbindungen der Formel (Ia) 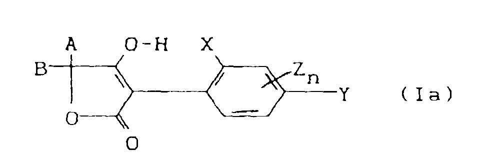 in welcher
   X, Y, Z, A, B und n die oben angegebene Bedeutung haben,
   mit Metallhydroxiden oder Aminen der allgemeinen Formeln (XI) und (XII) 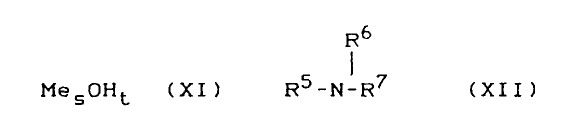 in welchen
   - Me: für ein- oder zweiwertige Metallionen
   - s und t: für die Zahl 1 oder 2 und
   - R⁵, R⁶ und R⁷: unabhängig voneinander für Wasserstoff und Alkyl
   stehen,
   gegebenenfalls in Gegenwart eines Verdünnungsmittels, umsetzt.

Weiterhin wurde gefunden, daß sich die neuen 3-Aryl-4-hydroxy-Δ³-dihydrofuranon-Derivate der Formel (I) durch hervorragende akarizide, insektizide und herbizide Wirkungen auszeichnen und somit als Schädlingsbekämpfungsmittel verwendet werden können.

Bevorzugt sind Verbindungen der Formel (I)
in welcher
- X: für C₁-C₆-Alkyl, Halogen, C₁-C₆-Alkoxy oder C₁-C₃-Halogenalkyl steht,
- Y: für Wasserstoff, C₁-C₆-Alkyl, Halogen, C₁-C₆-Alkoxy, C₁-C₃-Halogenalkyl steht,
- Z: für C₁-C₆-Alkyl, Halogen, C₁-C₆-Alkoxy steht,
- n: für eine Zahl von 0 bis 3 steht,
oder wobei die Reste X und Z gemeinsam mit dem Phenylrest an den sie gebunden sind, den Naphthalinrest der Formel 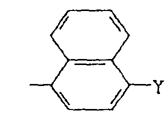 bilden, in welchem Y die oben angegebene Bedeutung hat,
oder worin
- A und B: gemeinsam mit dem Kohlenstoffatom, an das sie gebunden sind, einen gesättigten oder ungesättigten, durch C₁-C₆-Alkoxy, C₁-C₄-Alkylthio, C₁-C₄-Alkylsulfoxyl oder C₁-C₄-Alkylsulfonyl substituierten 3- bis 8-gliedrigen Ring bilden,
- A und B: gemeinsam mit dem Kohlenstoff, an das sie gebunden sind, für einen C₃-C₈-gliedrigen Ring stehen, bei dem zwei Substituenten gemeinsam mit den Kohlenstoffatomen, an die sie gebunden sind, für einen gegebenenfalls durch Alkyl, Alkoxy oder Halogen substituierten gesättigten oder einfach ungesättigten C₅-C₇-Ring stehen, der durch Sauerstoff oder Schwefel unterbrochen sein kann.
- G: für Wasserstoff (a) oder für die Gruppen 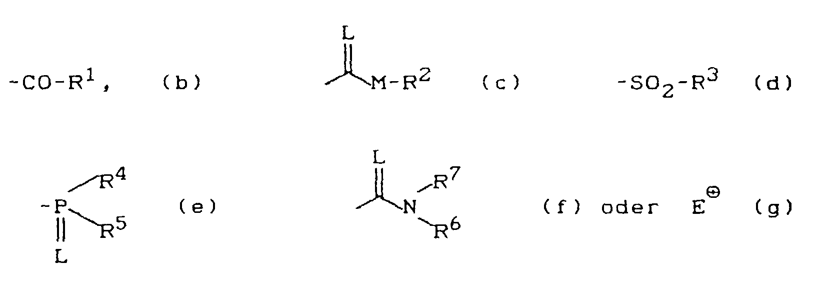 steht,
in welchen
- E^{⊕}: für ein Metallionäquivalent oder ein Ammoniumion steht,
- L und M: für Sauerstoff und/oder Schwefel steht,
- R¹: für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy-substituiertes Phenyl steht;
für gegebenenfalls durch Halogen, C₁-C₆-Alkyl, C₁-C₆-Alkoxy, C₁-C₆-Halogenalkyl, C₁-C₆-Halogenalkoxy-substituiertes Phenyl-C₁-C₆-alkyl steht,
für gegebenenfalls durch Halogen und/oder C₁-C₆-Alkyl substituiertes Hetaryl steht,
für gegebenenfalls durch Halogen und C₁-C₆-Alkyl-substituiertes Phenoxy-C₁-C₆-alkyl steht,
für gegebenenfalls durch Halogen, Amino und C₁-C₆-Alkyl-substituiertes Hetaryloxy-C₁-C₆-Alkyl steht,
- R²: für gegebenenfalls durch Halogen substituiertes C₁-C₂₀-Alkyl, C₃-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl-substituiertes Phenyl oder Benzyl steht,
- R³, R⁴ und R⁵: unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₃-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff oder Schwefel unterbrochenen C₄-C₆-Alkylenring stehen,
sowie die stereo- und enantiomerenreinen Formen von Verbindungen der Formel (I).

Besonders bevorzugt sind Verbindungen der Formel (I), in welcher
- X: für C₁-C₆-Alkyl, Halogen, C₁-C₆-Alkoxy oder C₁-C₂-Halogenalkyl steht,
- Y: für Wasserstoff, C₁-C₆-Alkyl, Halogen, C₁-C₆-Alkoxy, C₁-C₂-Halogenalkyl steht,
- Z: für C₁-C₄-Alkyl, Halogen, C₁-C₄-Alkoxy steht,
- n: für eine Zahl von 0 bis 3 steht,
oder wobei die Reste X und Z gemeinsam mit dem Phenylrest an den sie gebunden sind, den Naphthalinrest der Formel 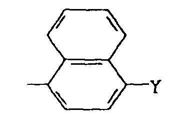 bilden,
in welchem Y die oben angegebene Bedeutung hat,
- A und B: gemeinsam mit dem Kohlenstoffatom, an das sie gebunden sind, einen gesättigten oder ungesättigten, durch C₁-C₅-Alkoxy, C₁-C₃-Alkylthio, C₁-C₃-Alkylsulfoxyl oder C₁-C₃-Alkylsulfonyl substituierten 5- bis 7-gliedrigen Ring bilden,
- A und B: gemeinsam mit dem Kohlenstoff, an das sie gebunden sind, für einen C₄-C₇-gliedrigen Ring stehen, bei dem zwei Substituenten gemeinsam mit den Kohlenstoffatomen, an die sie gebunden sind, für einen gegebenenfalls durch C₁-C₃-Alkyl, C₁-C₃-Alkoxy, Fluor oder Chlor substituierten gesättigten oder einfach ungesättigten C₅-C₆-Ring stehen, der durch Sauerstoff oder Schwefel unterbrochen sein kann,
- G: für Wasserstoff (a) oder für die Gruppen 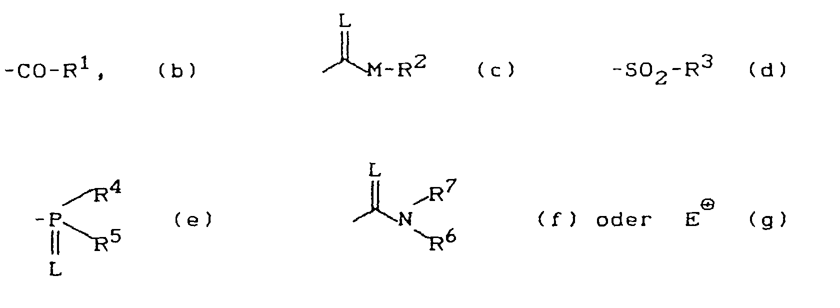
steht,
in welchen
- E^{⊕}: für ein Metallionäquivalent oder ein Ammoniumion steht
- L und M: jeweils für Sauerstoff und/oder Schwefel steht,
- R¹: für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff- und/oder Schwefelatome unterbrochen sein kann steht,
für gegebenenfalls durch Halogen-, Nitro, C₁-C₄-Alkyl-, C₁-C₄-Alkoxy-, C₁-C₃-Halogenalkyl-, C₁-C₃-Halogenalkoxy-substituierets Phenyl steht,
für gegebenenfalls durch Halogen-, C₁-C₄-Alkyl-, C₁-C₄-Alkoxy-, C₁-C₃-Halogenalkyl-, C₁-C₃-Halogenalkoxy-substituiertes Phenyl-C₁-C₄-alkyl steht,
für gegebenenfalls durch Halogen- und C₁-C₆-Alkyl-substituiertes Hetaryl steht,
für gegebenenfalls durch Halogen- und C₁-C₄-Alkyl-substituiertes Phenoxy-C₁-C₅-alkyl steht,
für gegebenenfalls durch Halogen, Amino und C₁-C₄-Alkyl substituiertes Hetaryloxy-C₁-C₅-alkyl steht,
- R²: für gegebenenfalls durch Halogen substituiertes: C₁-C₁₆-Alkyl, C₃-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro-, C₁-C₄-Alkyl, C₁-C₃-Alkoxy-, C₁-C₃-Halogenalkyl-substituiertes Phenyl oder Benzyl steht,
- R³, R⁴ und R⁵: unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substiutiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₃-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht oder zusammen für einen gegebenenfalls durch Sauerstoff oder Schwefel unterbrochenen C₄-C₆-Alkylenring stehen,
sowie die stereo- und enantiomerenreinen Formen von Verbindungen der Formel (I).

Ganz besonders bevorzugt sind Verbindungen der Formel (I), in welcher
- X: Methyl, Ethyl, Propyl, i-Propyl, Fluor, Chlor, Brom, Methoxy, Ethoxy und Trifluormethyl steht,
- Y: für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
- Z: für Methyl, Ethyl, 1-Propyl, Butyl, i-Butyl, tert.-Butyl, Fluor, Chlor, Brom, Methoxy und Ethoxy steht,
- n: für eine Zahl von 0 bis 3 steht,
oder wobei die Reste X und Z gemeinsam mit dem Phenylrest an den sie gebunden sind, den Rest der Formel 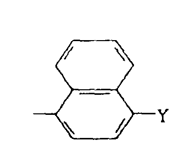 bilden,
in welchem Y die oben angegebene Bedeutung hat,
- A und B: gemeinsam mit dem Kohlenstoffatom, an das sie gebunden sind, einen gesättigten oder ungesättigten, durch C₁-C₄-Alkoxy, C₁-C₂-Alkylthio, C₁-C₂-Alkylsulfoxyl oder C₁-C₂-Alkylsulfonyl substituierten 5- bis 6-gliedrigen Ring bilden,
- A und B: gemeinsam mit dem Kohlenstoff, an das sie gebunden sind, für einen C₄-C₆-gliedrigen Ring stehen, bei dem zwei Substituenten gemeinsam mit den Kohlenstoffatomen, an die sie gebunden sind, für einen gegebenenfalls Methyl, Ethyl, Methoxy, Ethoxy, Fluor oder Chlor substituierten gesättigten oder einfach ungesättigten C₅-C₆-Ring stehen, der durch Sauerstoff oder Schwefel unterbrochen sein kann,
- G: für Wasserstoff (a) oder für die Gruppen 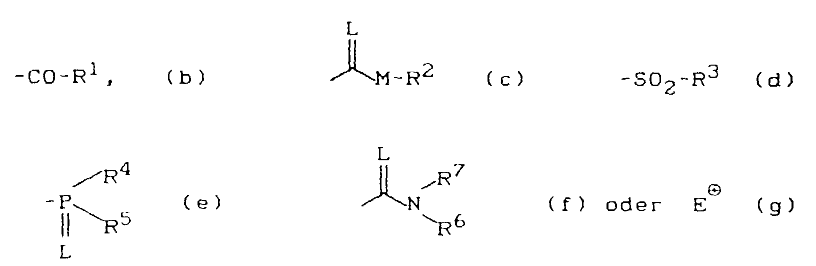
steht,
in welchen
- E^{⊕}: für ein Metallionäquivalent oder ein Ammoniumion steht,
- L und M: jeweils für Sauerstoff oder Schwefel steht,
- R¹: für jeweils gegebenenfalls durch Fluor und/oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl, oder für gegebenenfalls durch Fluor, Chlor, Methyl oder Ethyl substituiertes C₃-C₆-Cycloalkyl, das durch 1-2 Sauerstoff-und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-C₁-C₃-alkyl steht,
für jeweils gegebenenfalls durch Fluor, Chlor, Brom, Methyl, Ethyl substituiertes Furanyl, Thienyl, Pyridyl, Pyrimidyl, Thiazolyl oder Pyrazolyl steht,
für gegebenenfalls durch Fluor, Chlor, Methyl, Ethyl-substituiertes Phenoxy-C₁-C₄-alkyl steht,
für gegebenenfalls durch Fluor, Chlor, Amino, Methyl-, Ethyl, substituiertes Pyridyloxy-C₁-C₄-alkyl, Pyrimidyloxy-C₁-C₄-alkyl und Thiazolyloxy-C₁-C₄-alkyl steht,
- R²: für gegebenenfalls durch Fluor oder Chlor substituiertes C₁-C₁₄-Alkyl, C₃-C₁₄-Alkenyl, C₁-C₄-C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
- R³, R⁴ und R⁵: unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
- R⁶ und R⁷: unabhängig voneinander für gegebenenfalls durch Fluor, Chlor, Brom substituiertes C₁-C₁₀-Alkyl, C₃-C₆-Alkenyl, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht oder zusammen für einen gegebenenfalls durch Sauerstoff oder Schwefel unterbrochenen C₄-C₆-Alkylenring stehen,
sowie die stereo- und enantiomerenreinen Formen von Verbindungen der Formel (I).

Im einzelnen seien außer den bei den Herstellungsbeispielen genannten Verbindungen die folgenden 3-Aryl-4-hydroxy-Δ³-dihydrofuran-Derivate der Formel (Ia) genannt:

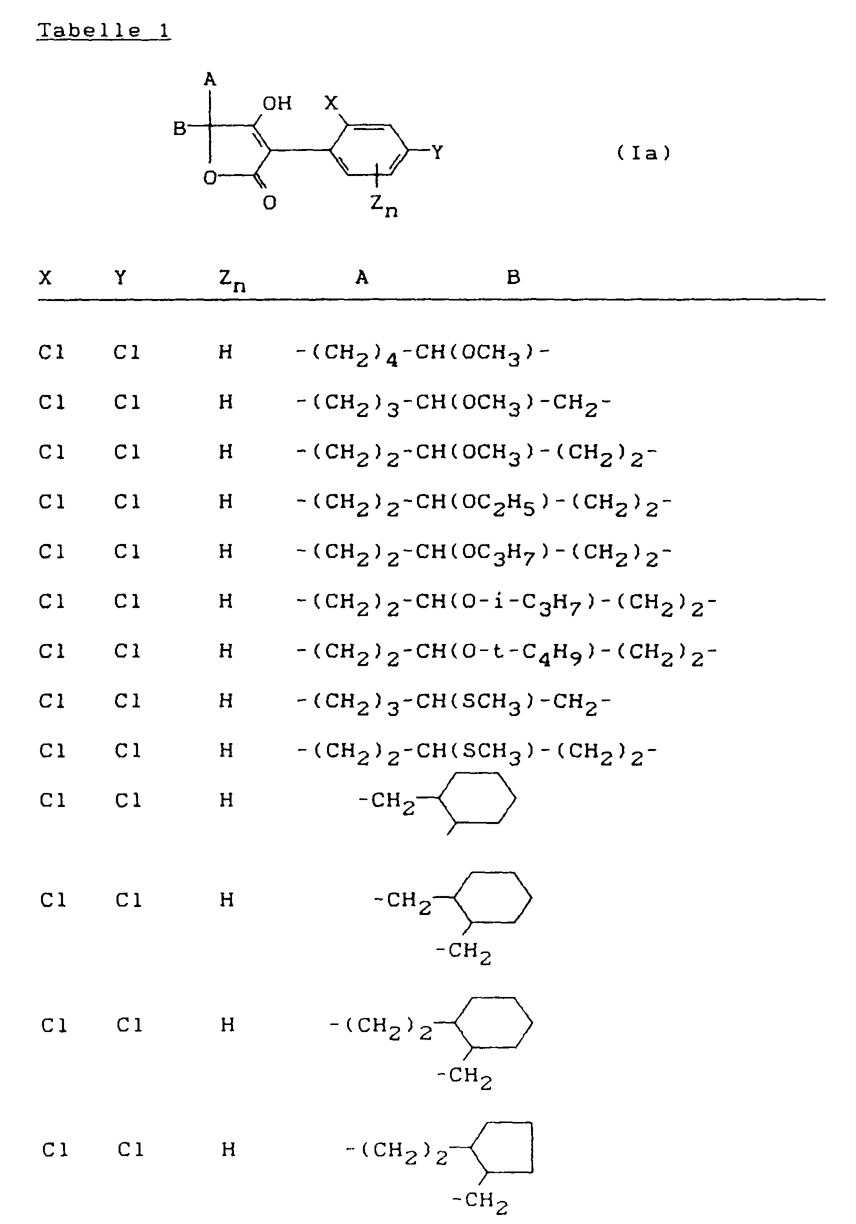

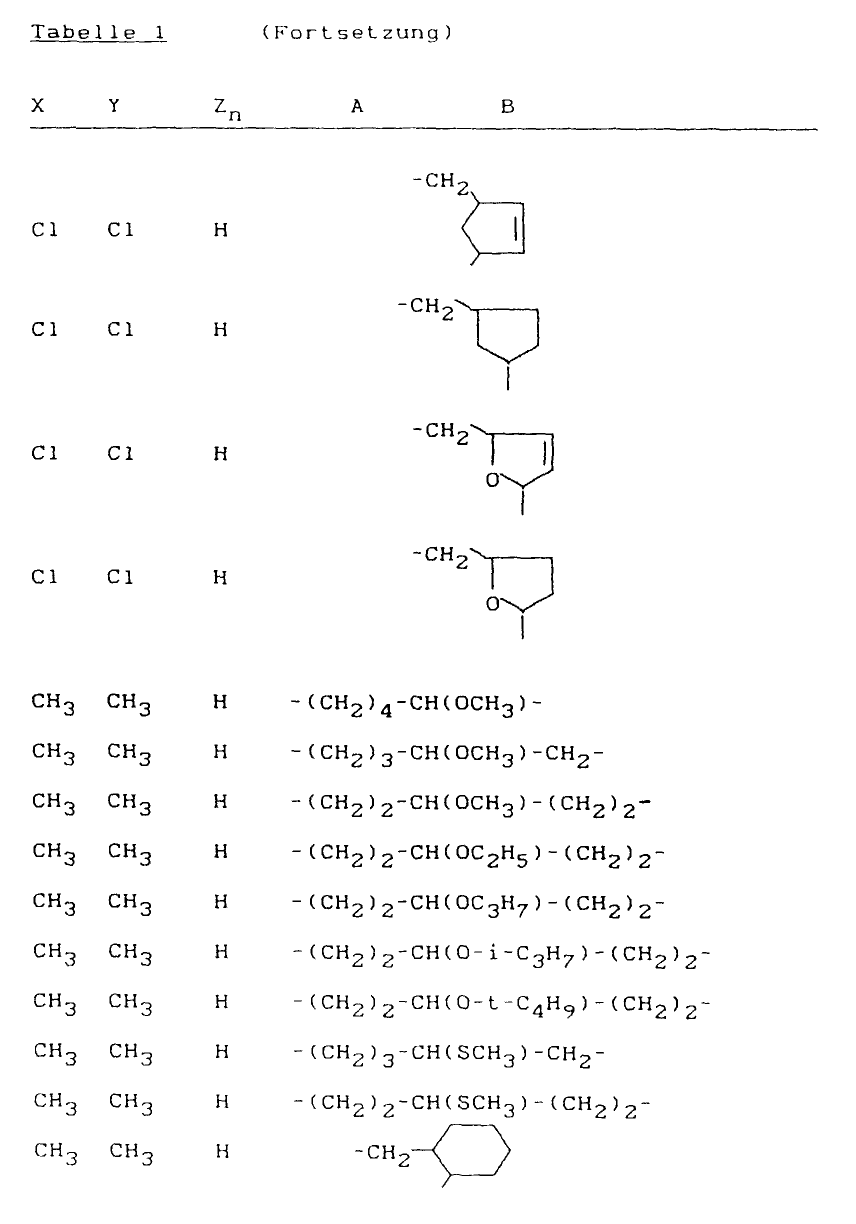

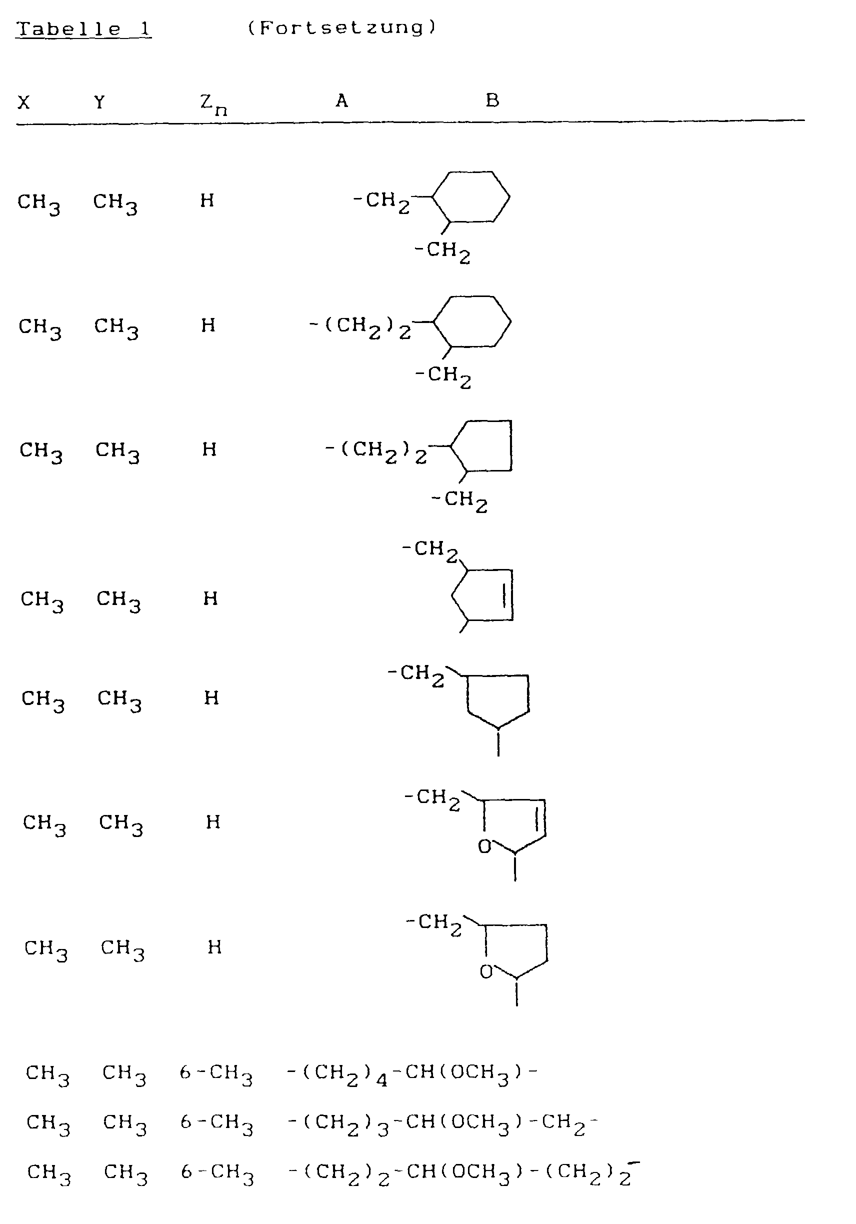

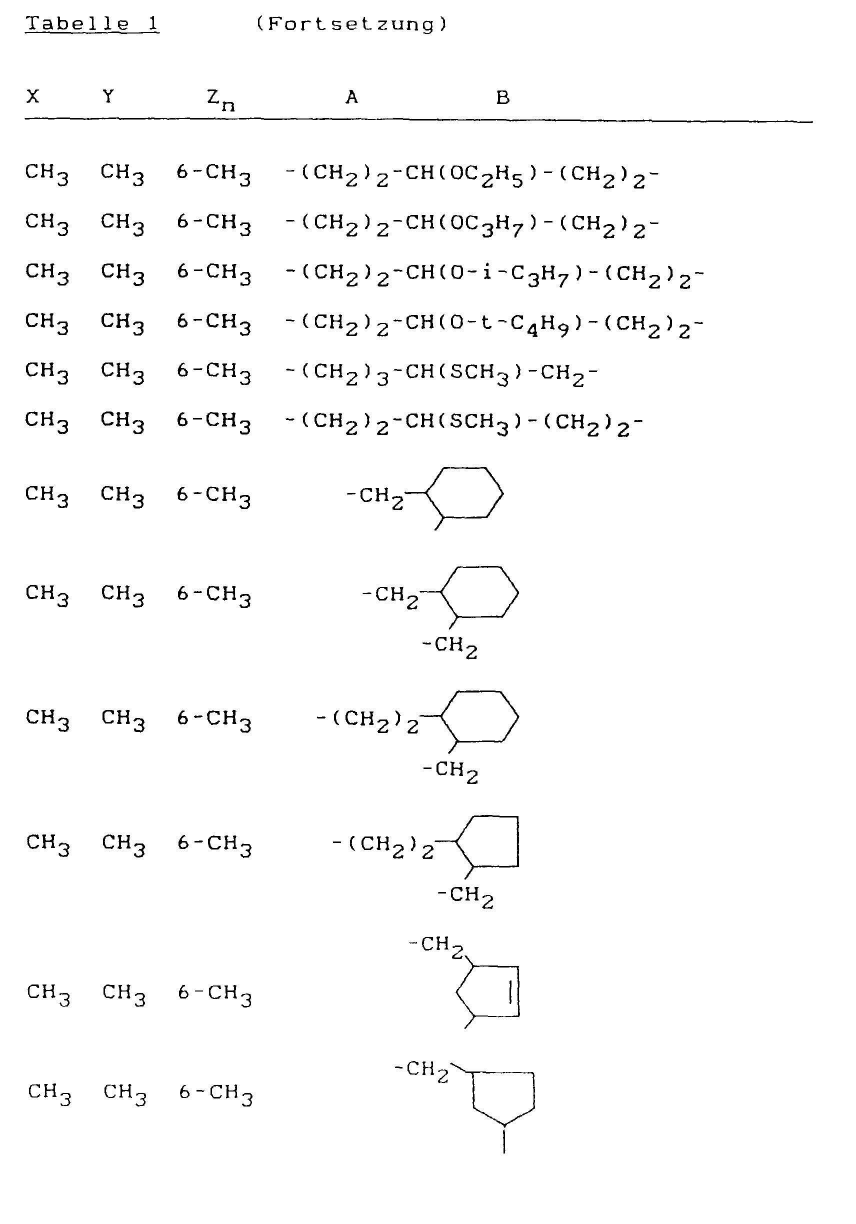

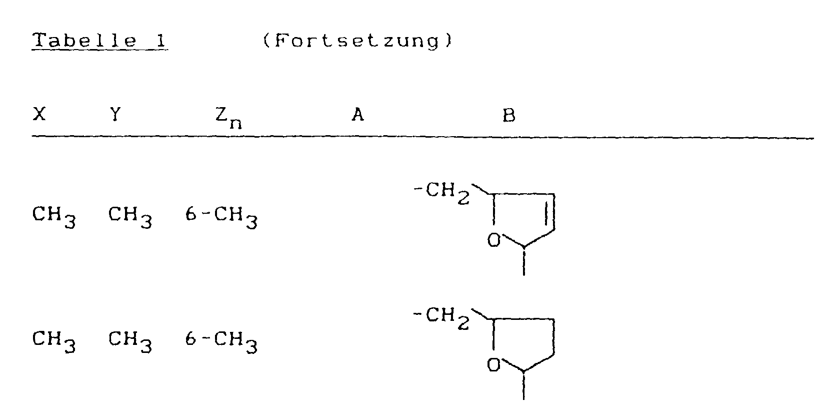

Im einzelnen seien außer den bei den Herstellungsbeispielen genannten Verbindungen die folgenden 3-Aryl-4-hydroxy-Δ³-dihydrofuran-Derivate der Formel (Ib) genannt:

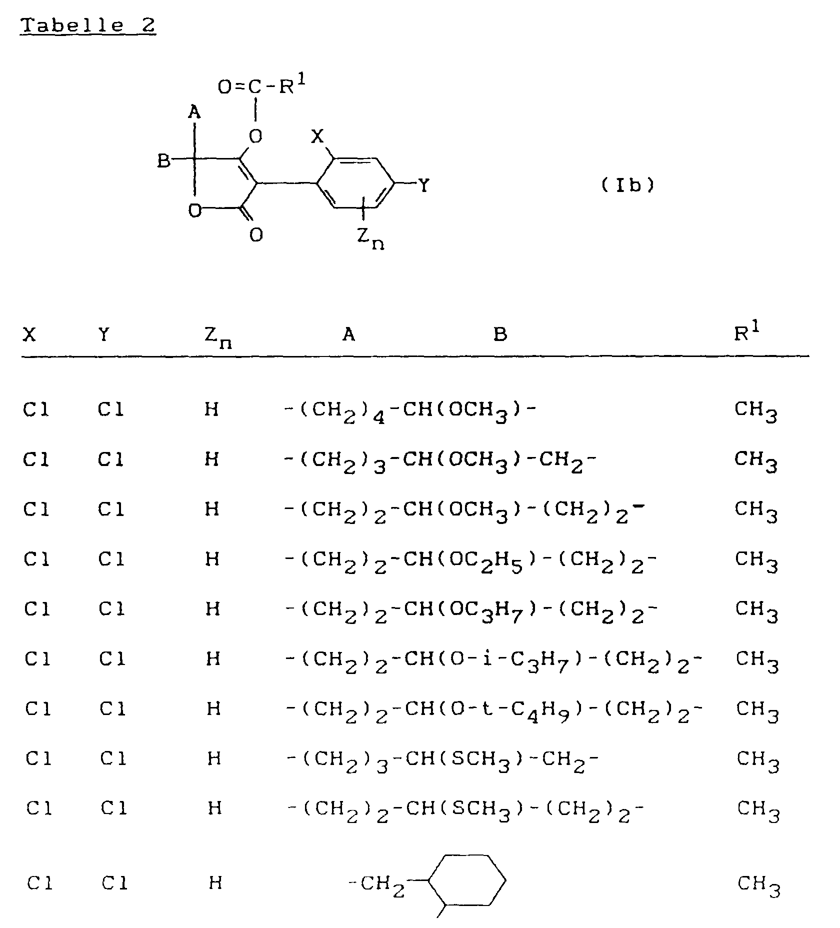

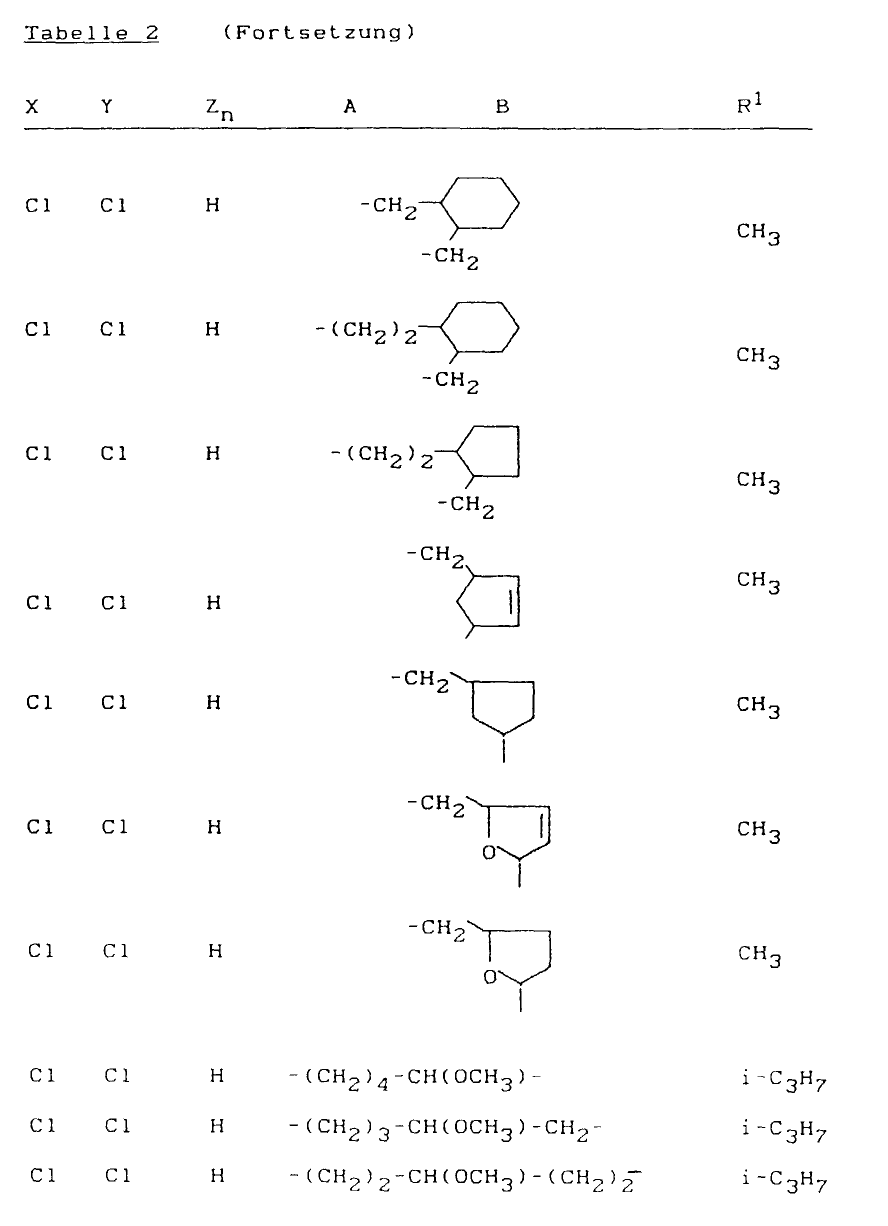

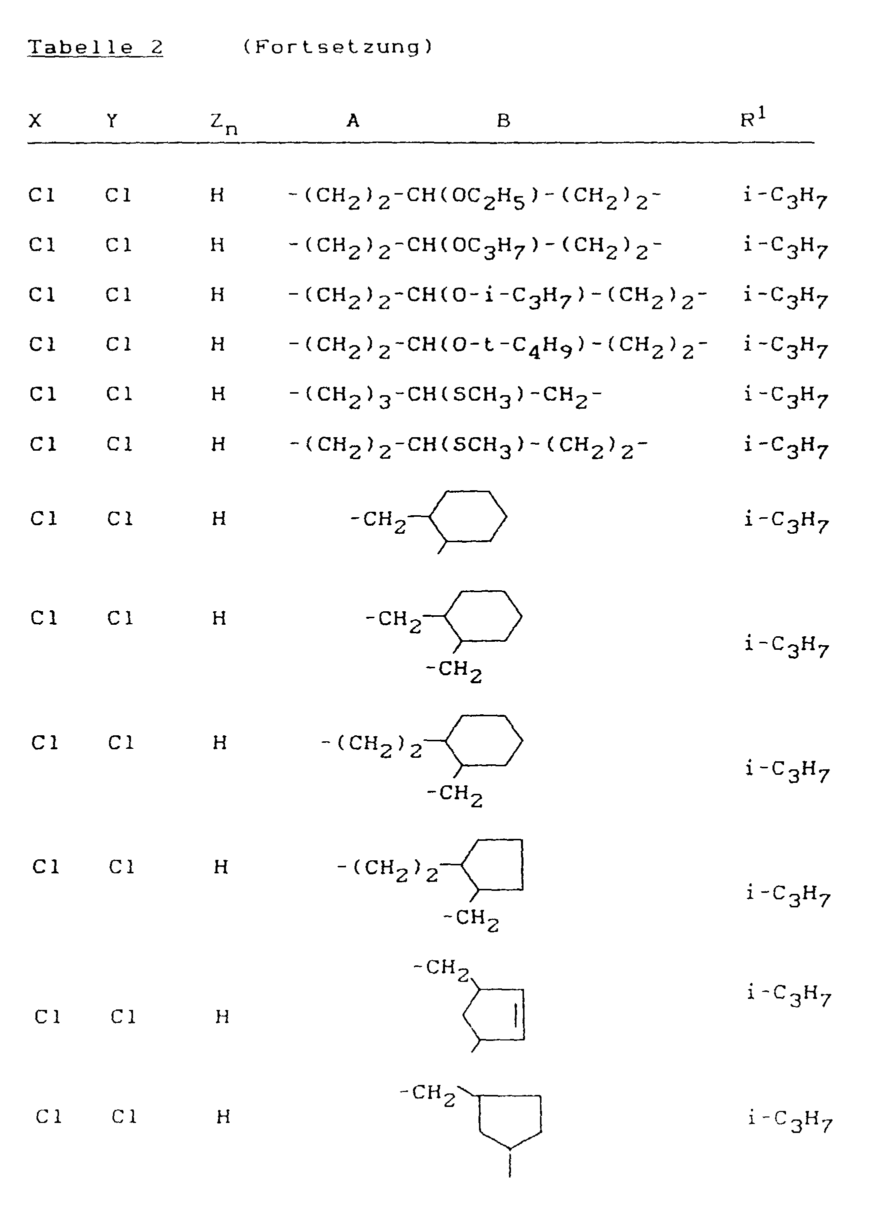

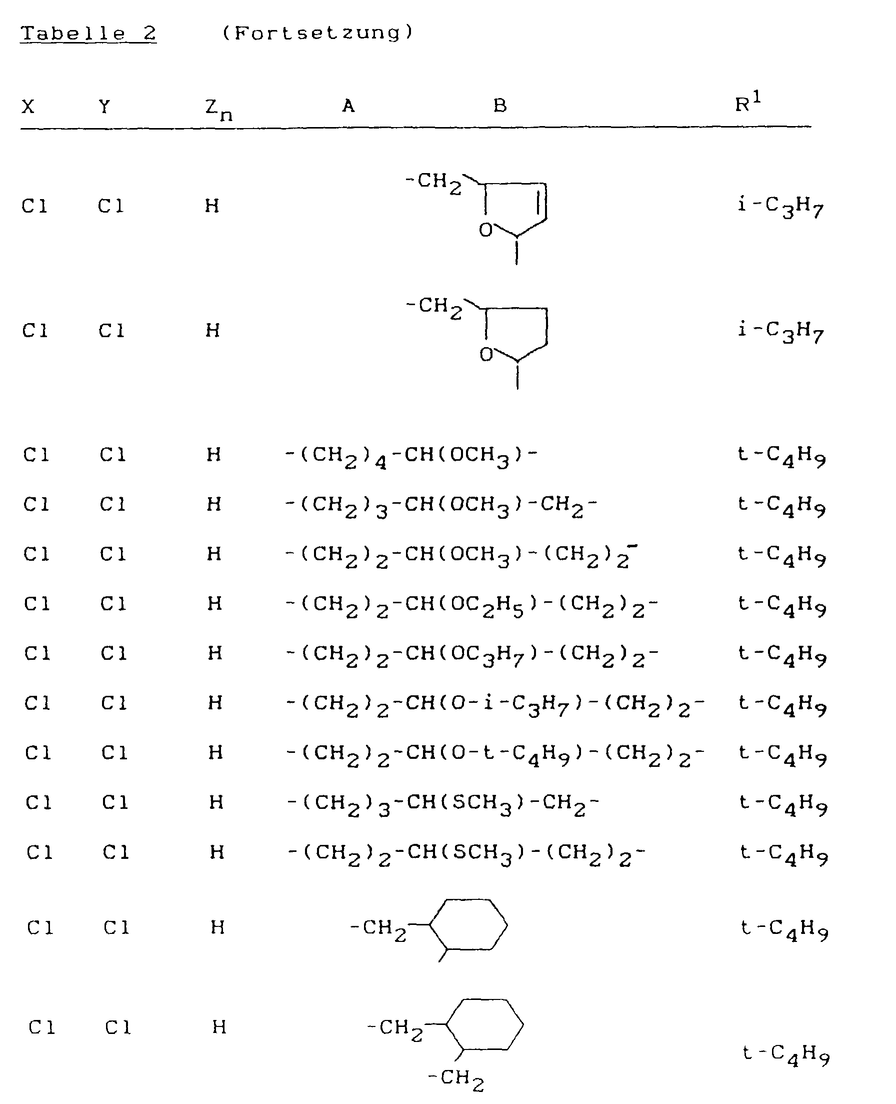

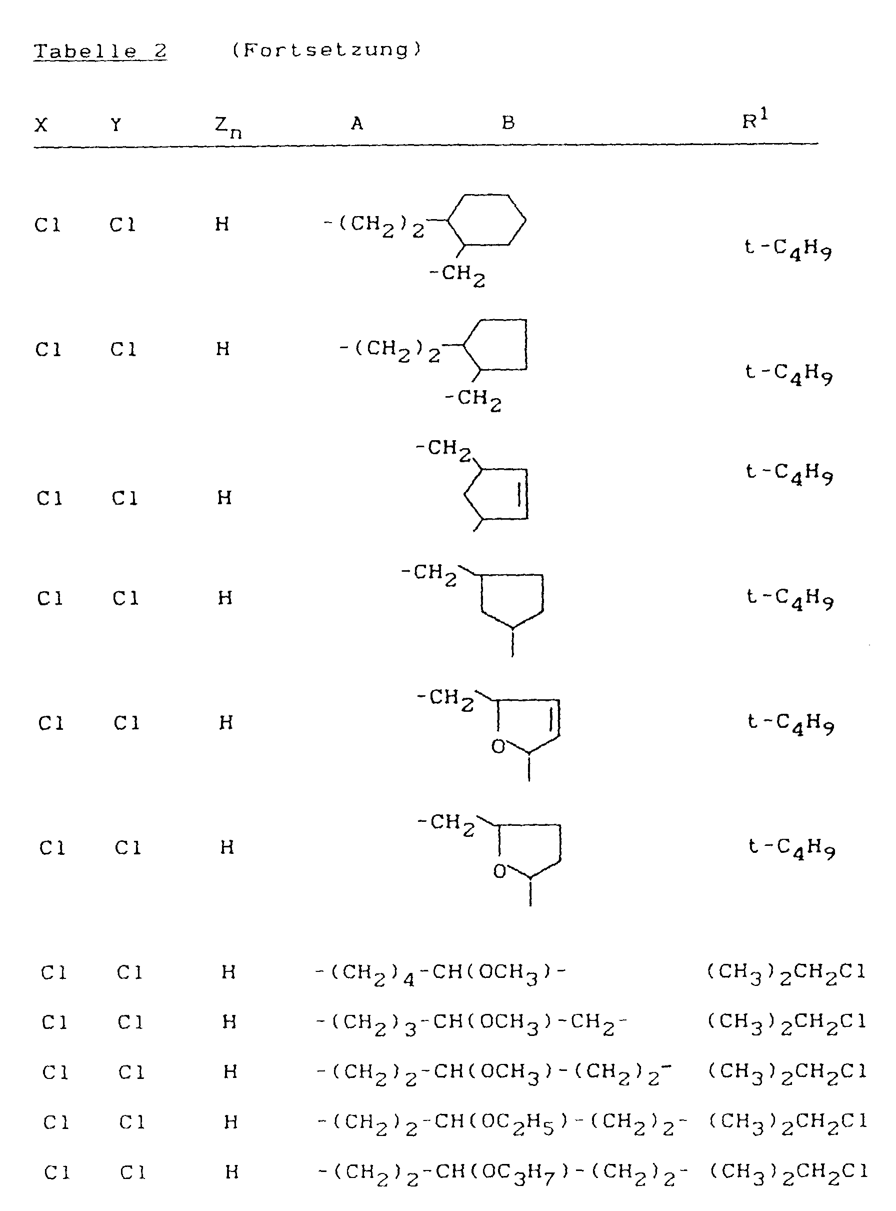

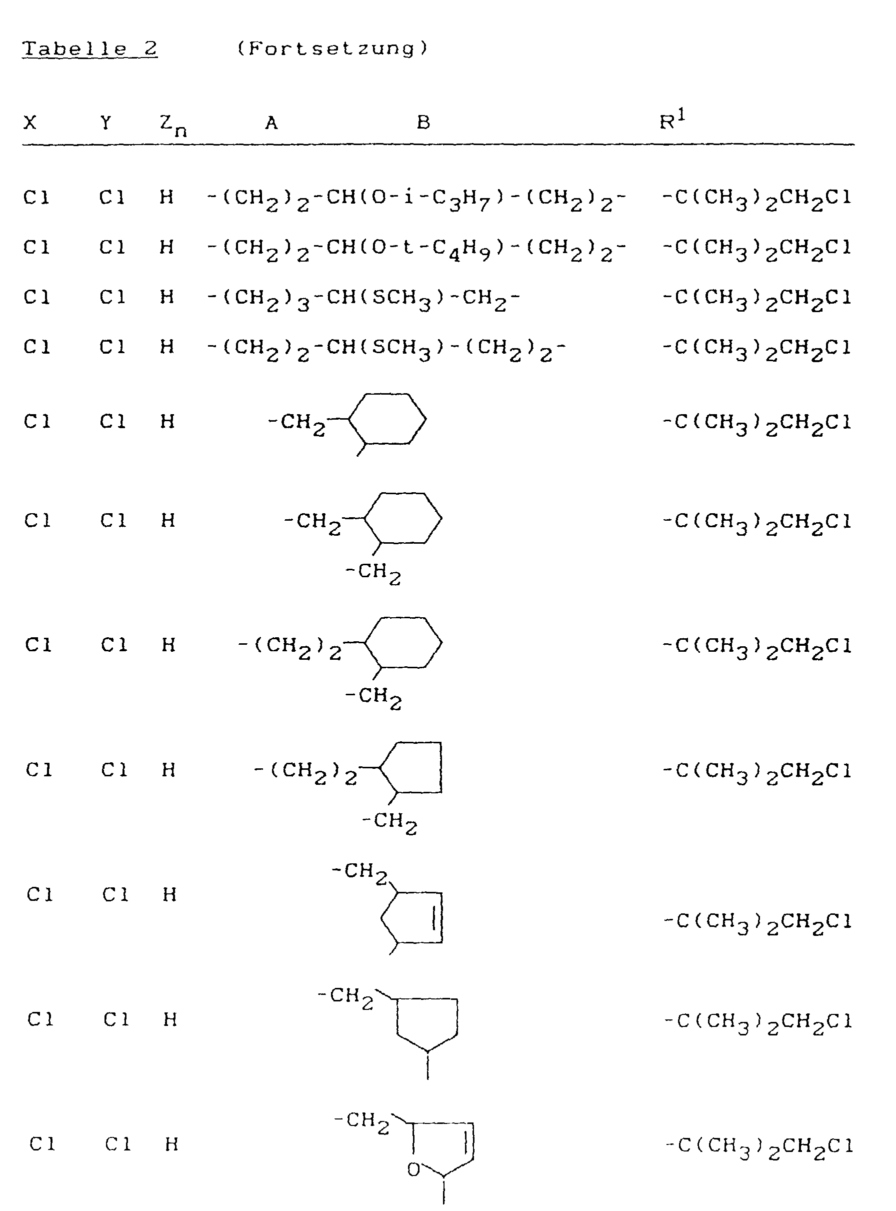

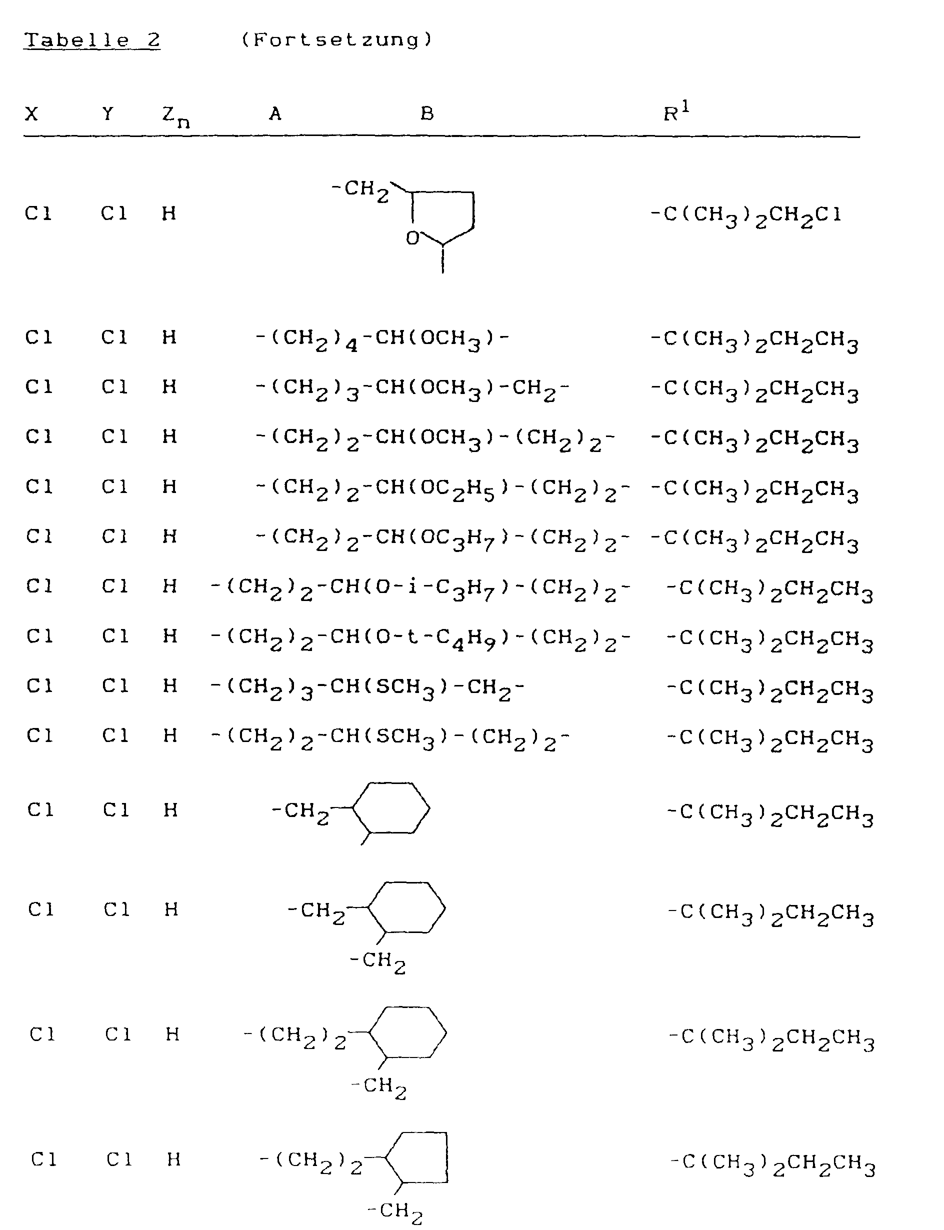

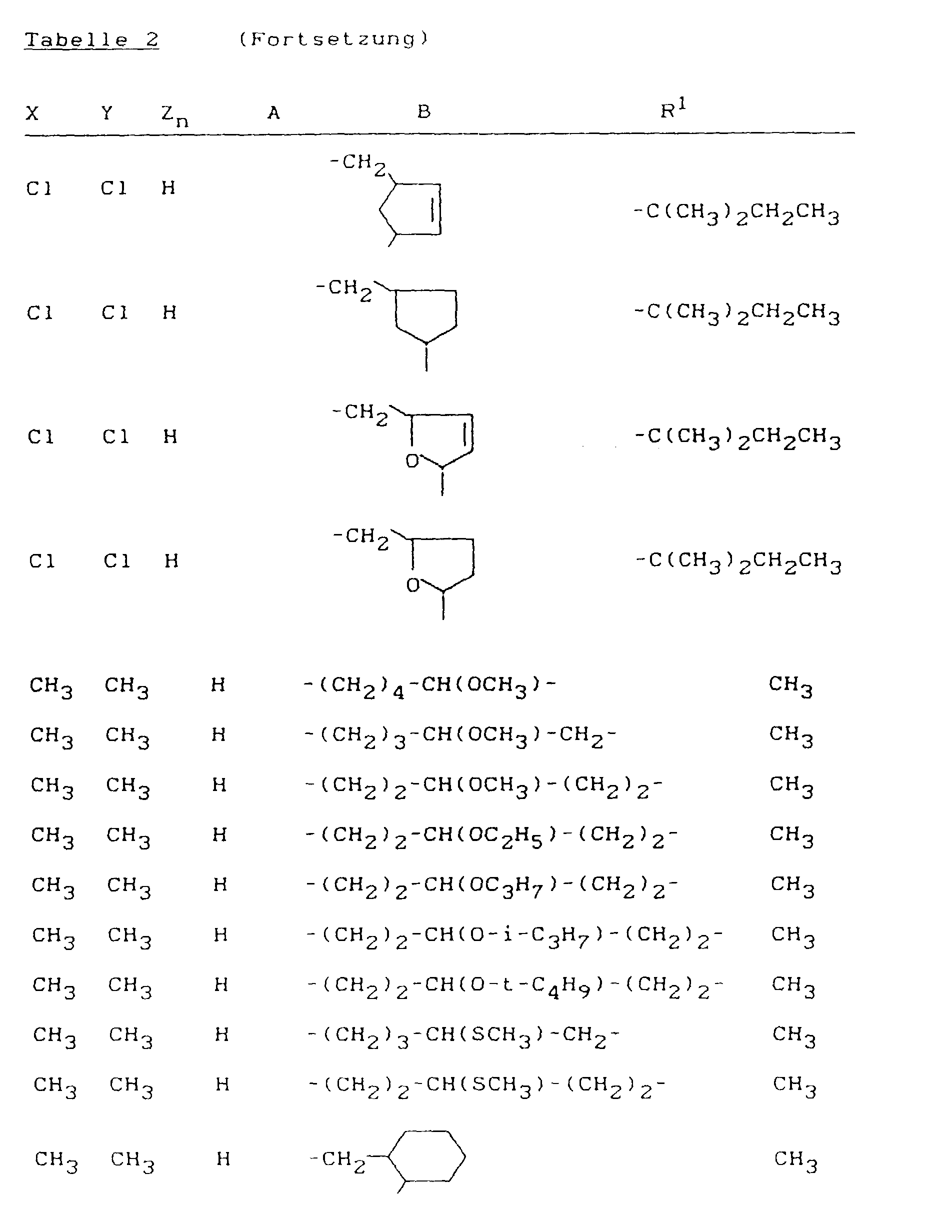

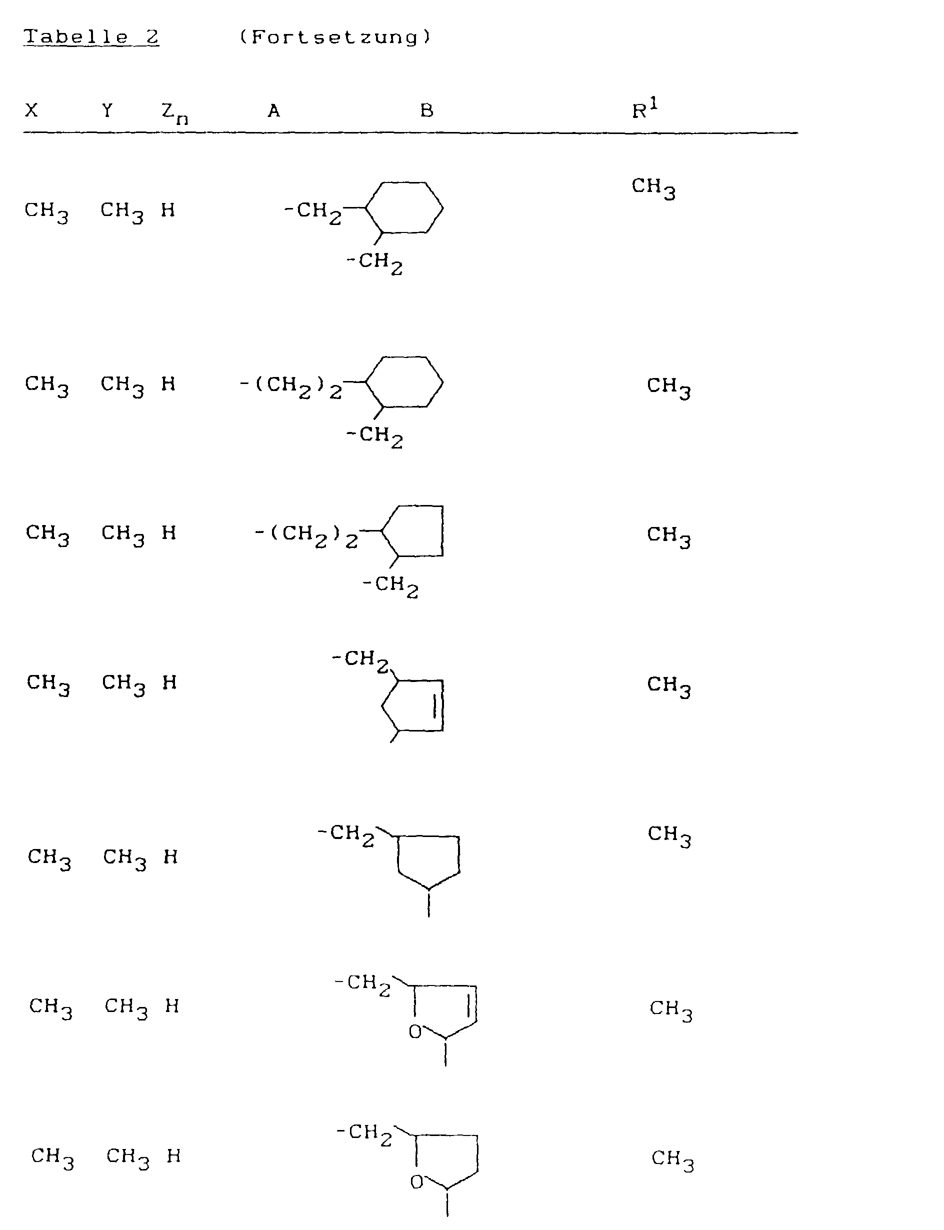

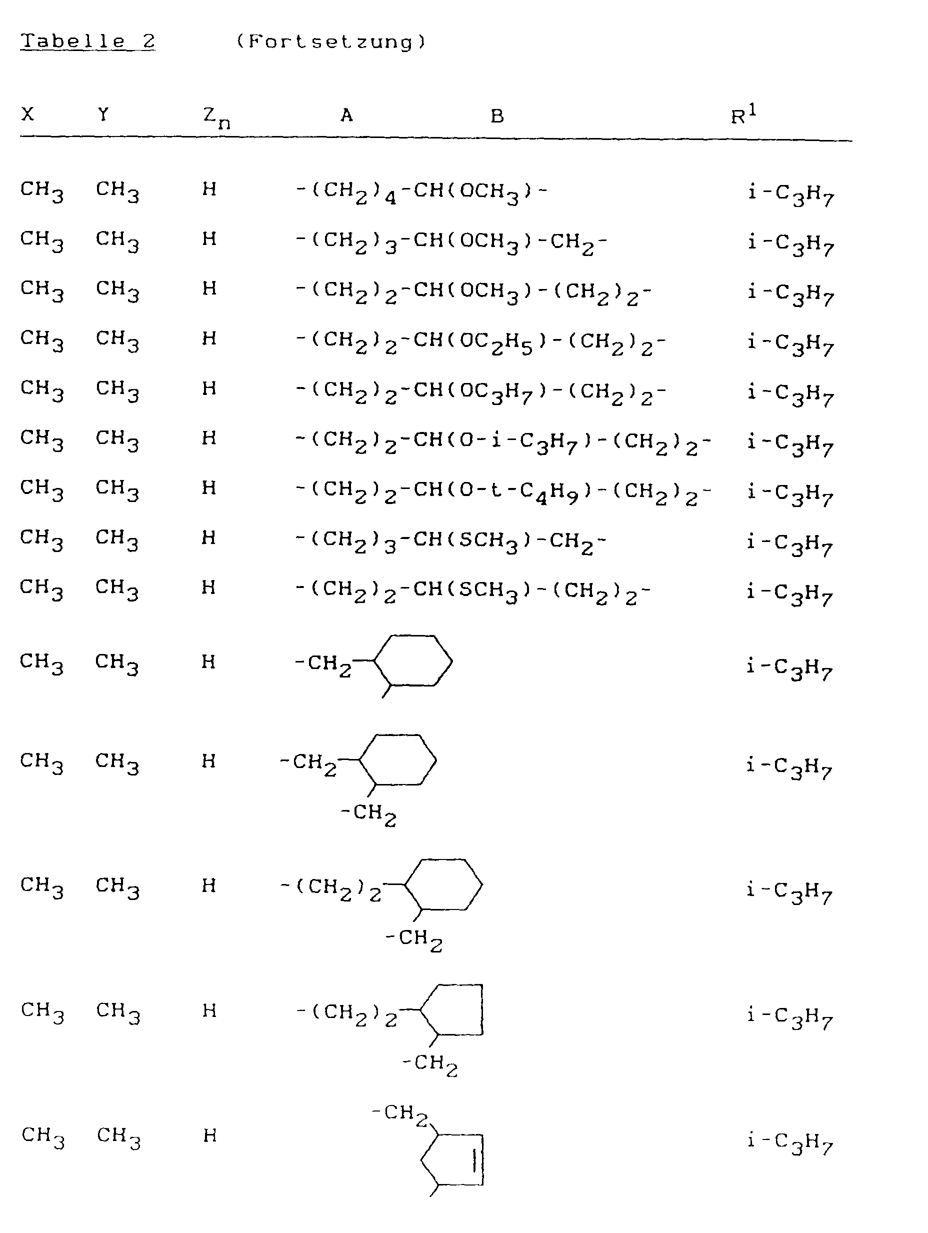

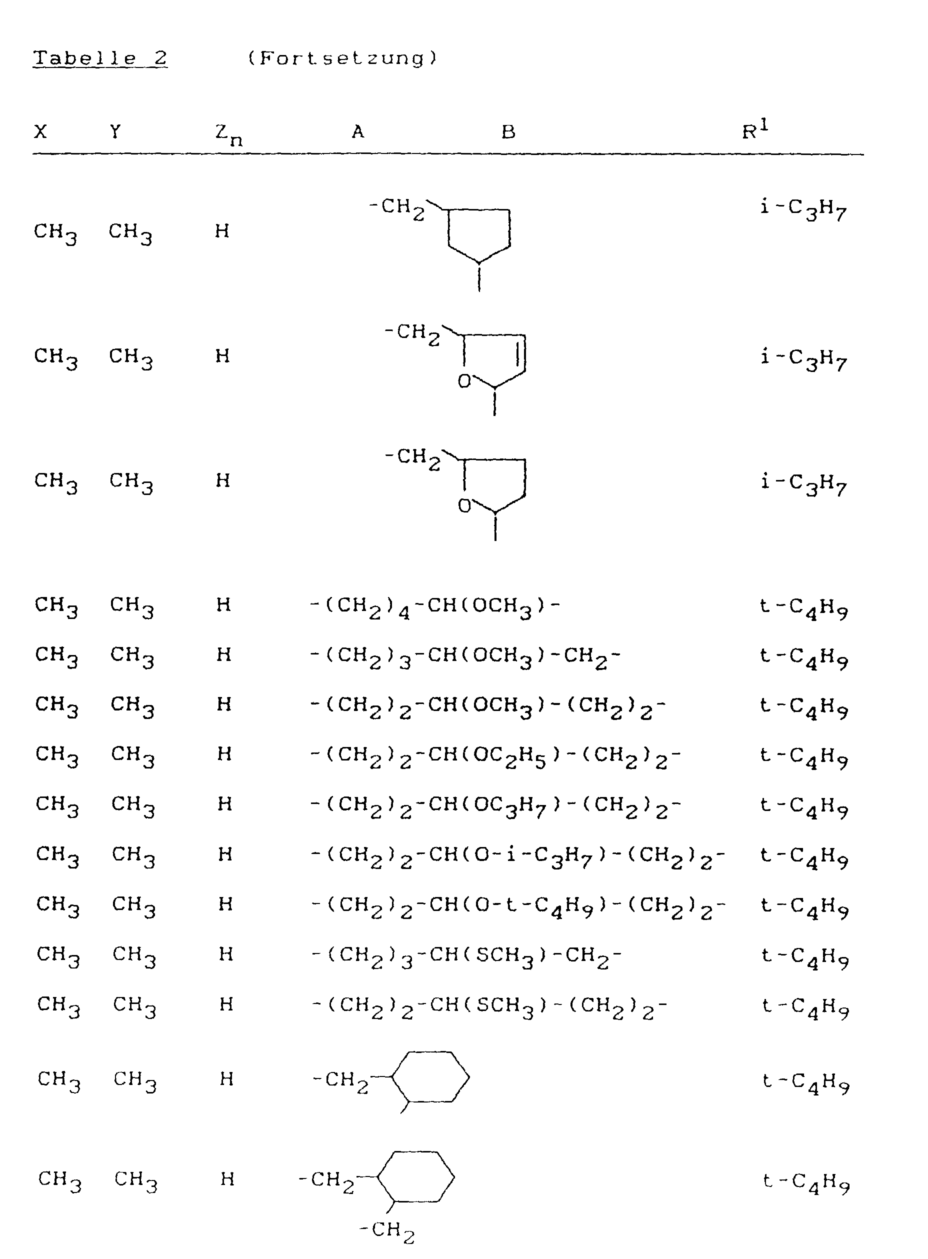

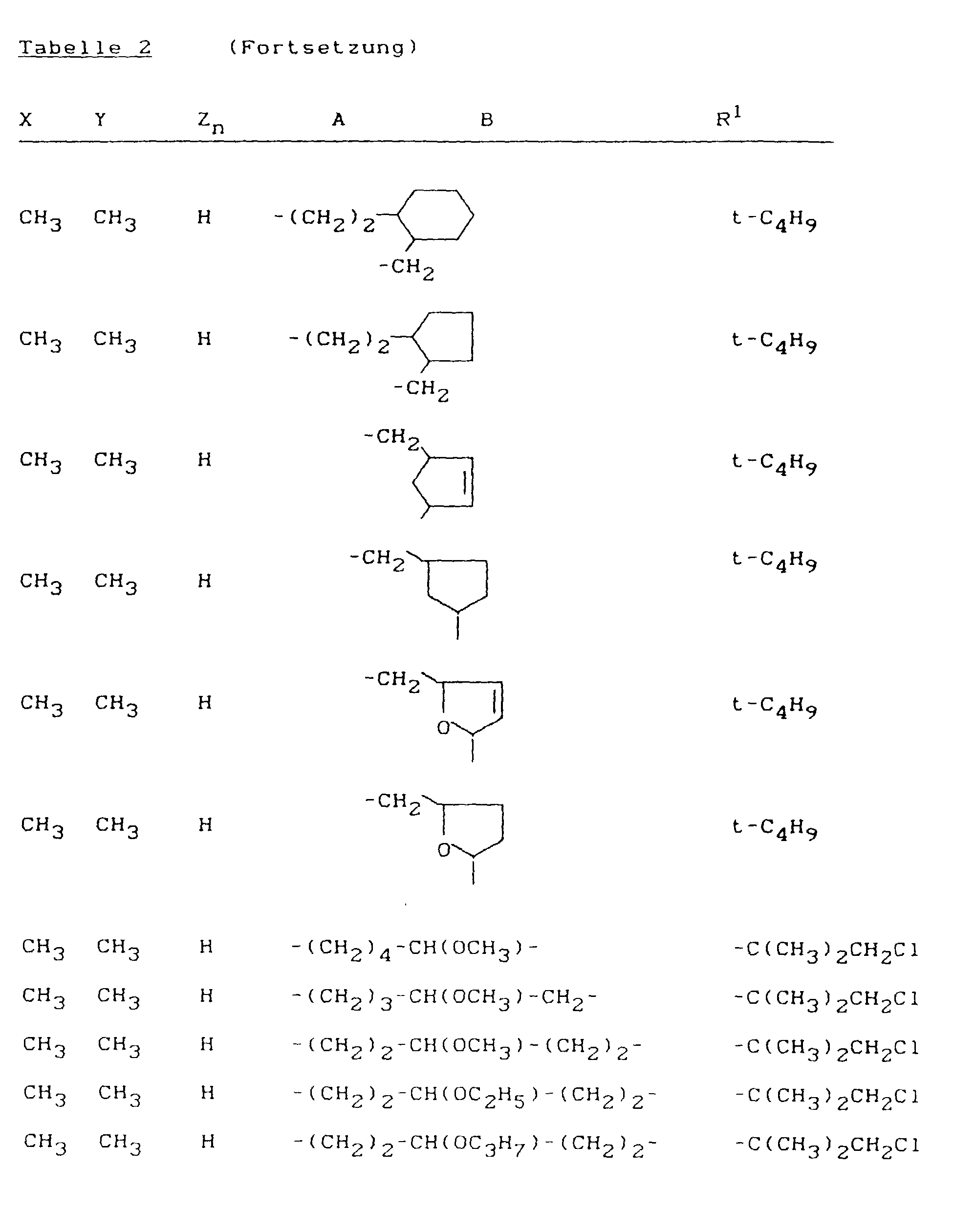

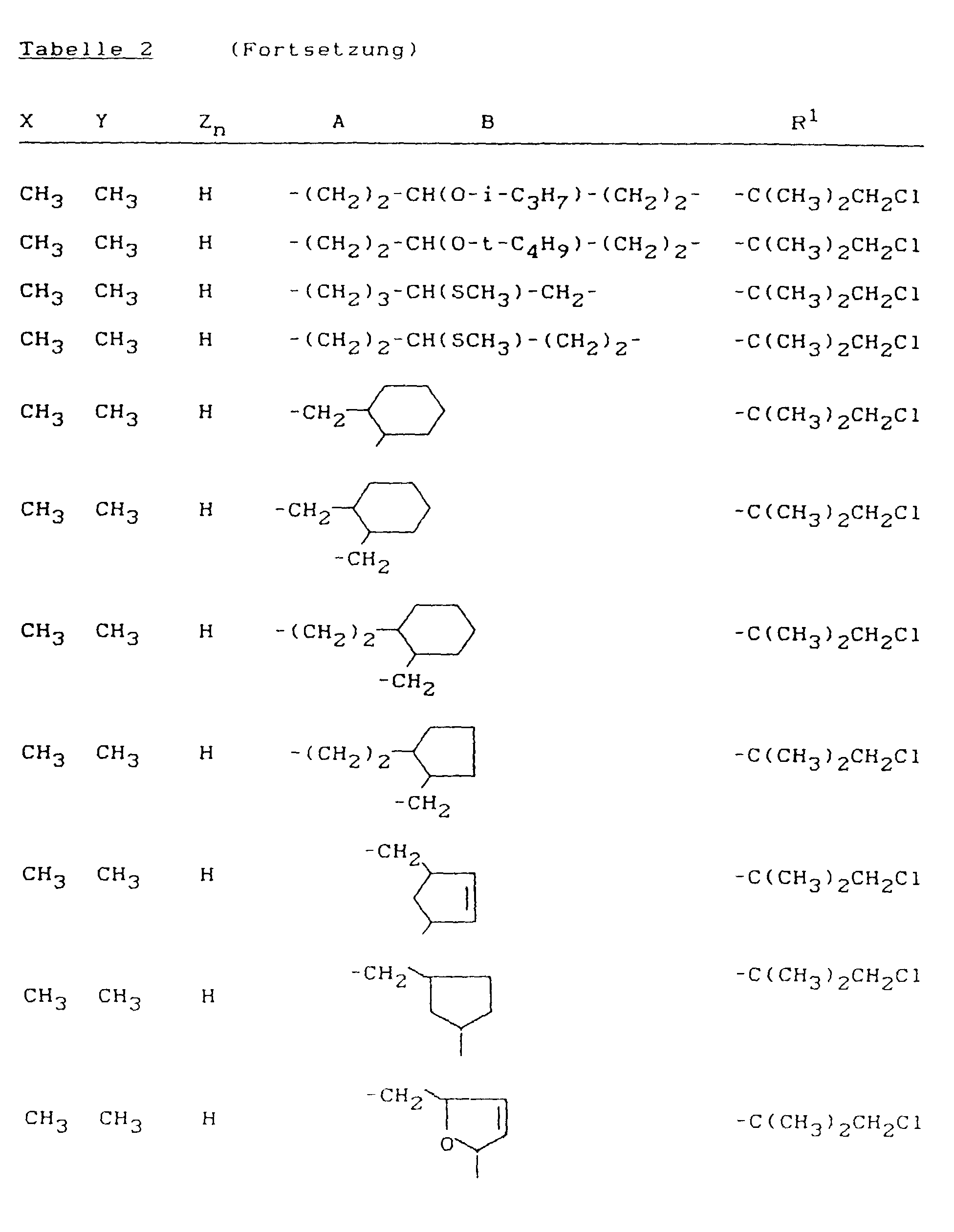

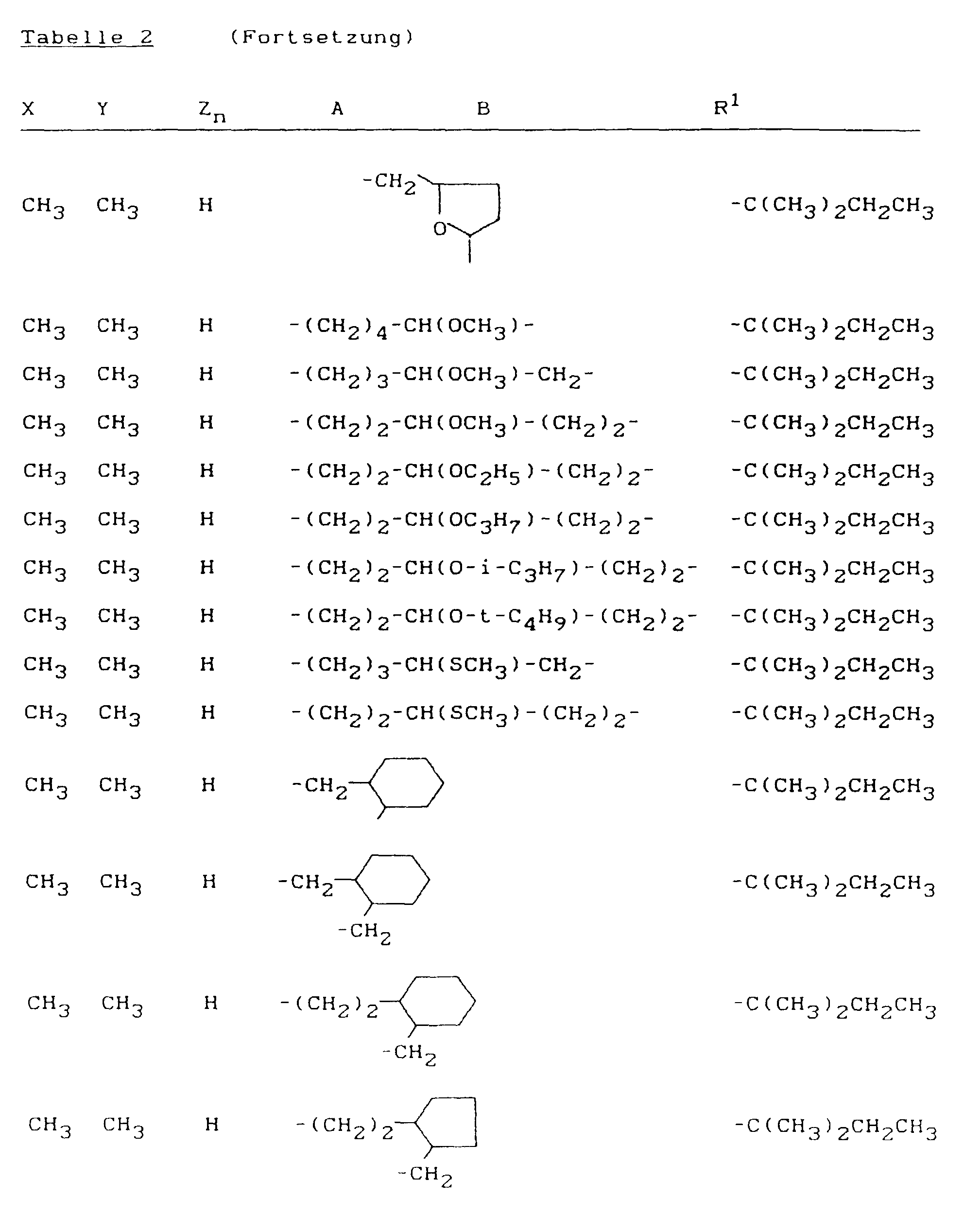

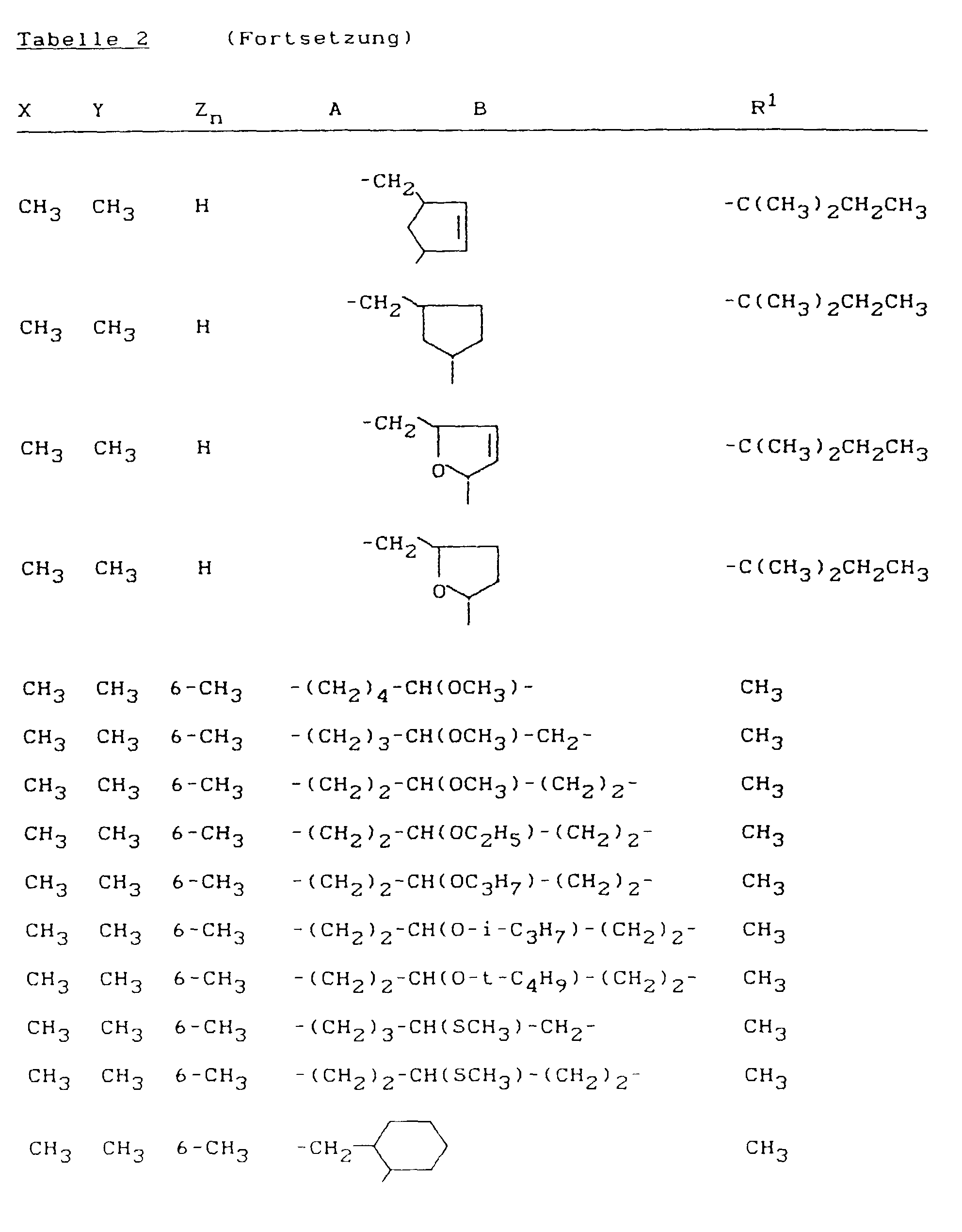

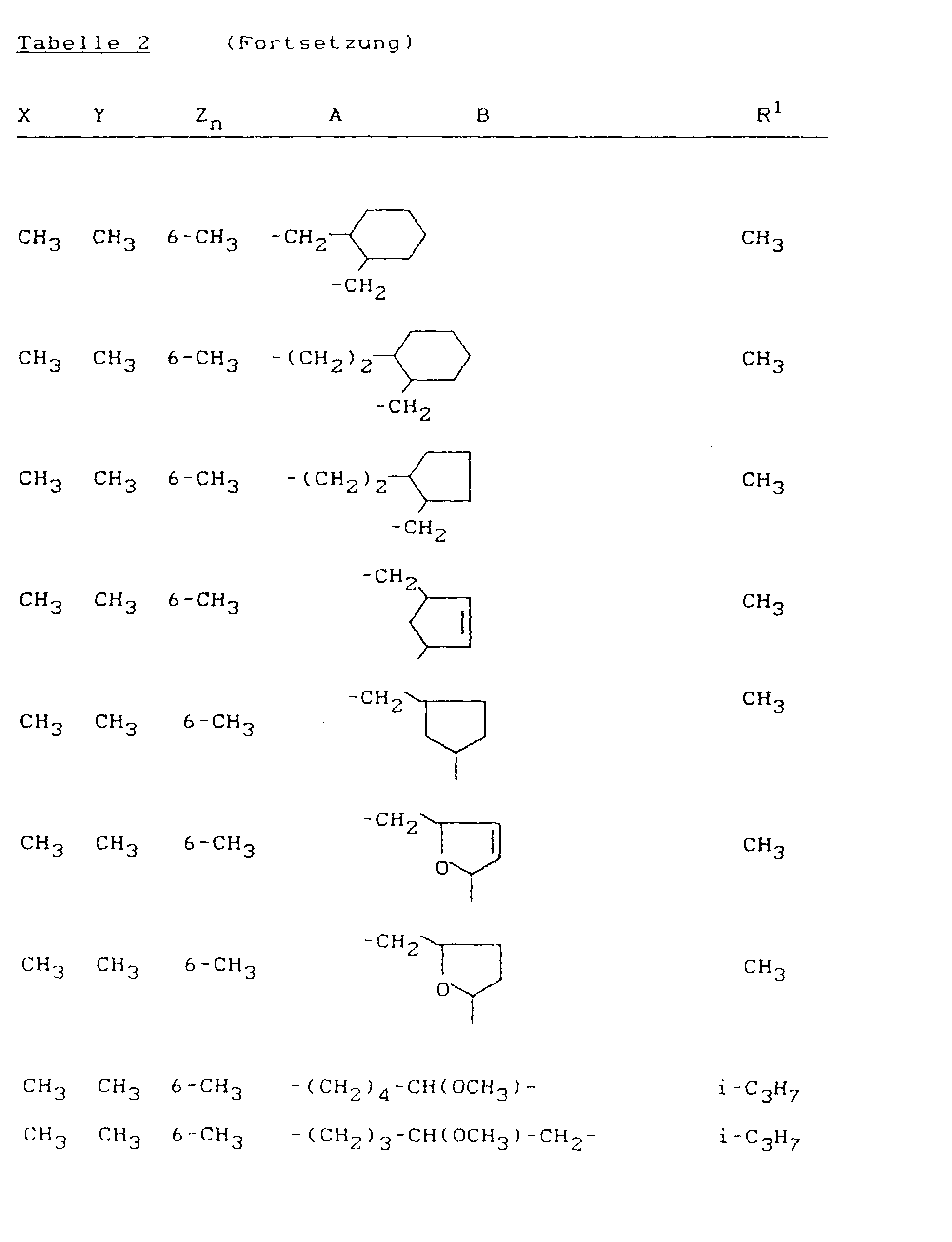

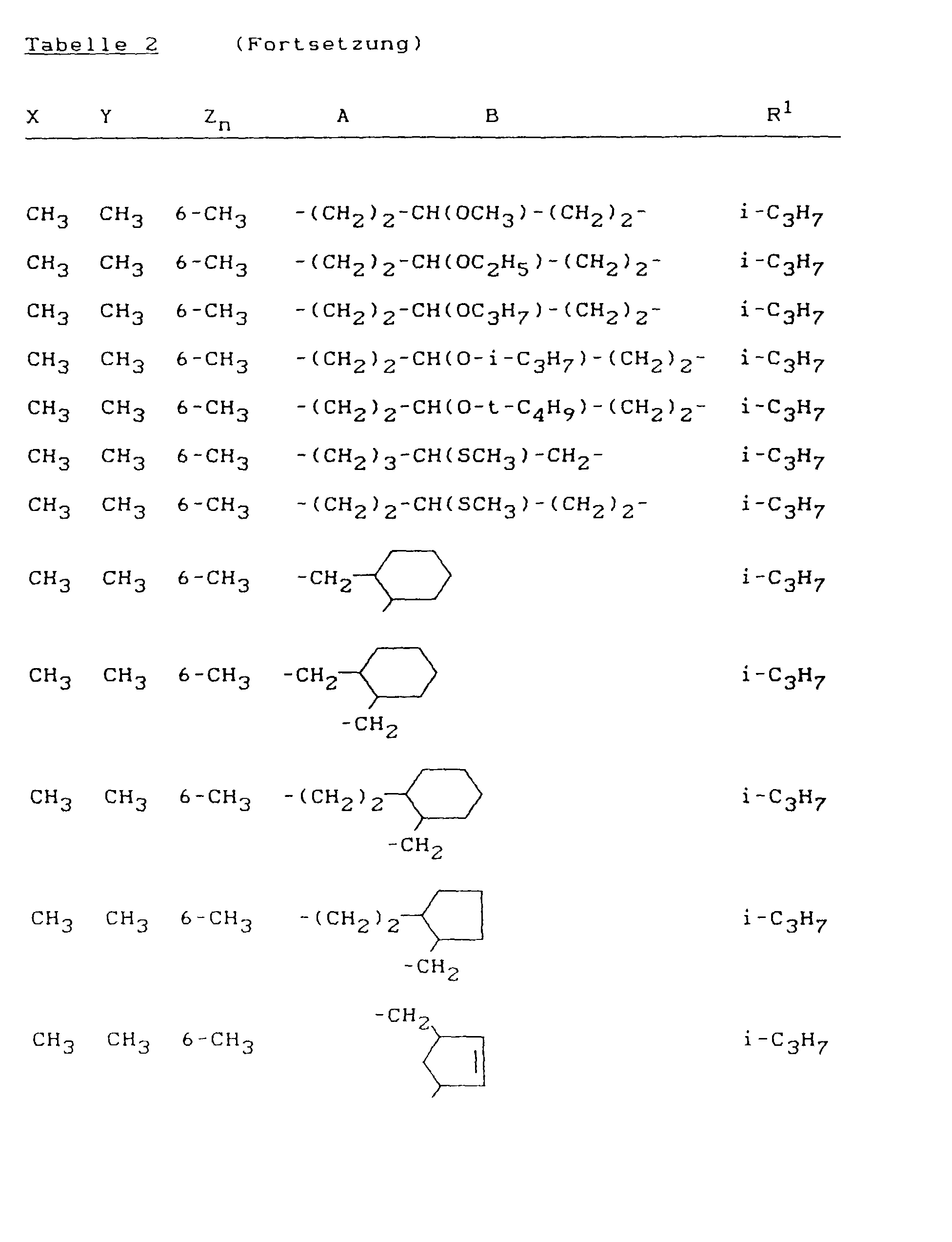

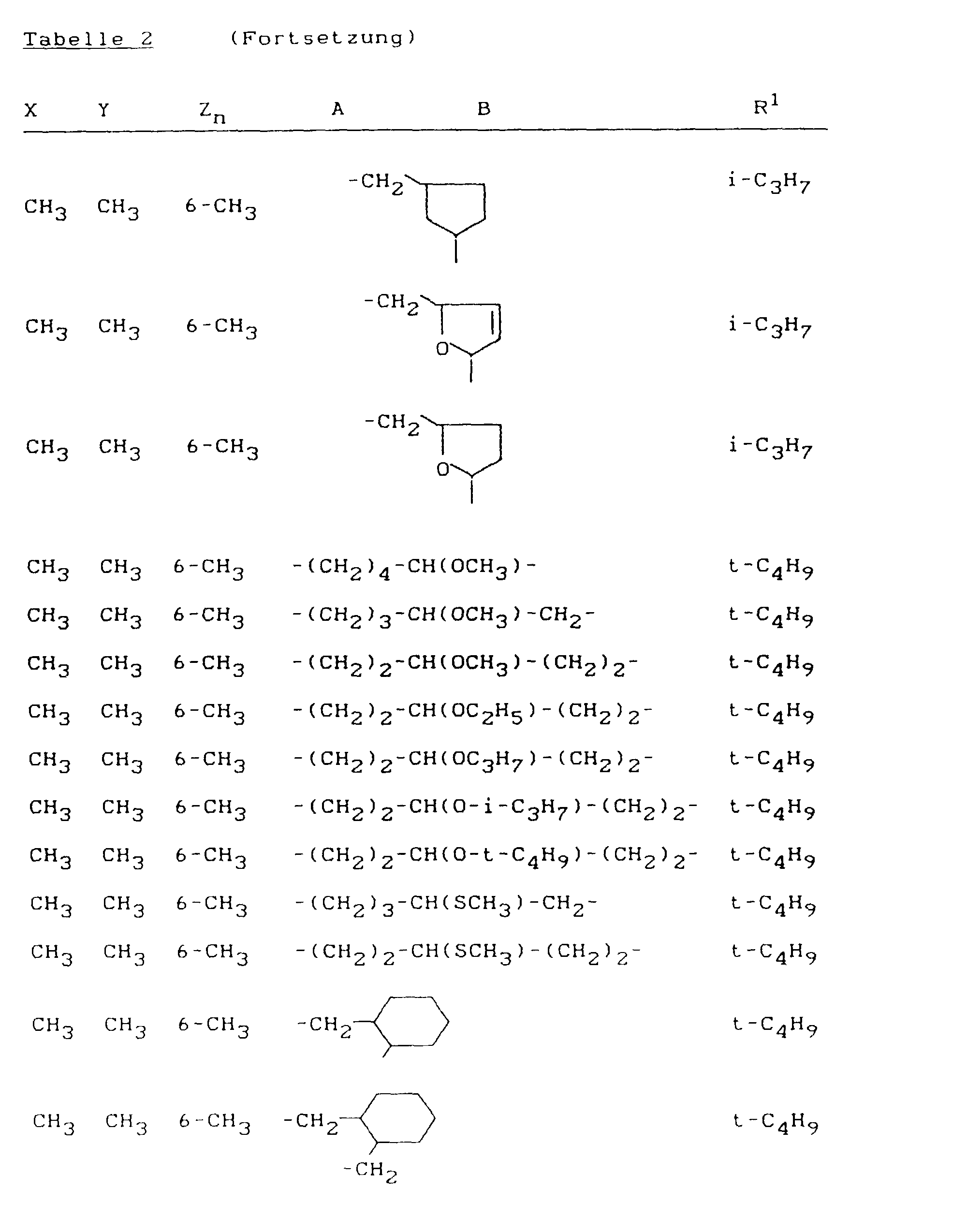

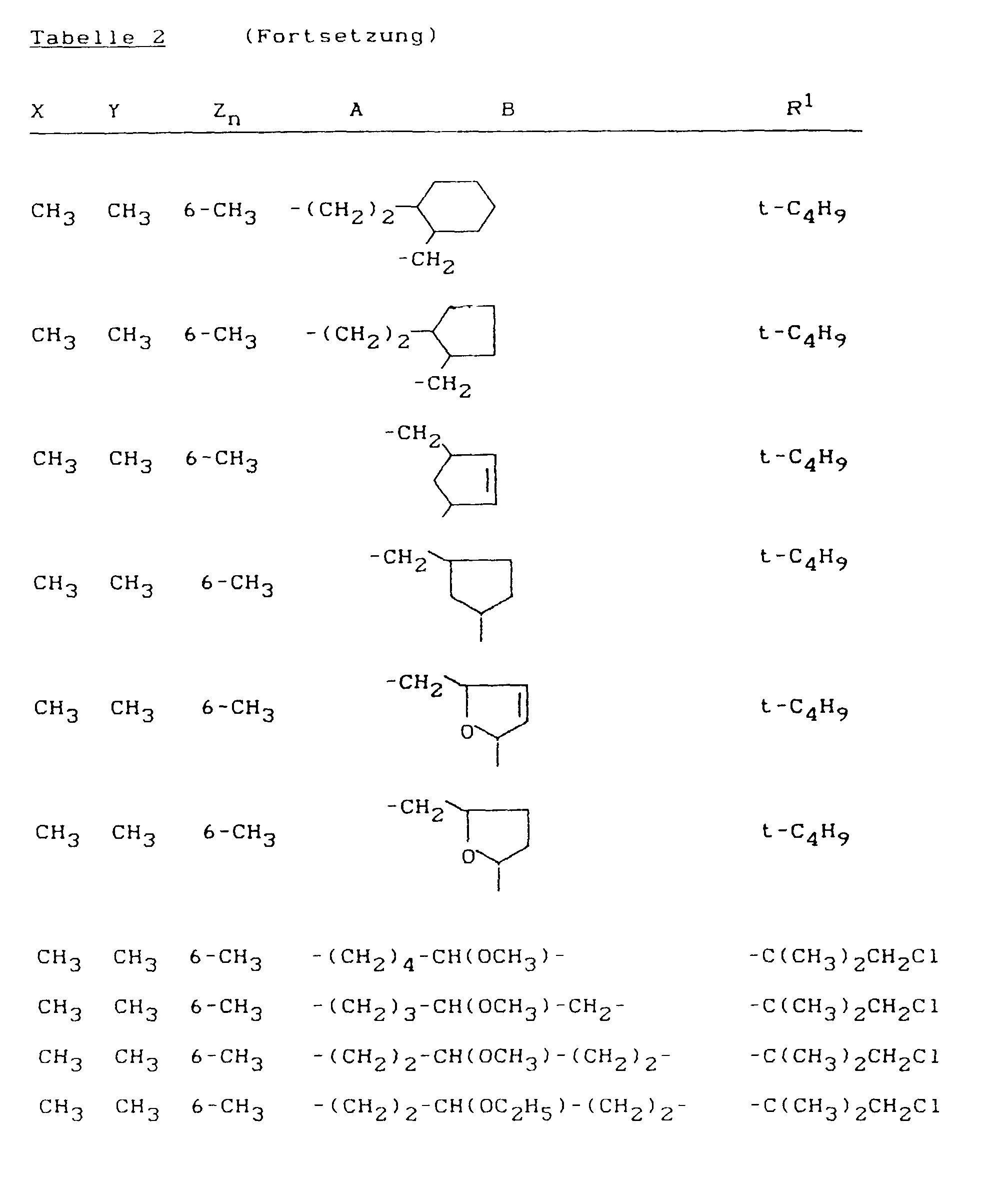

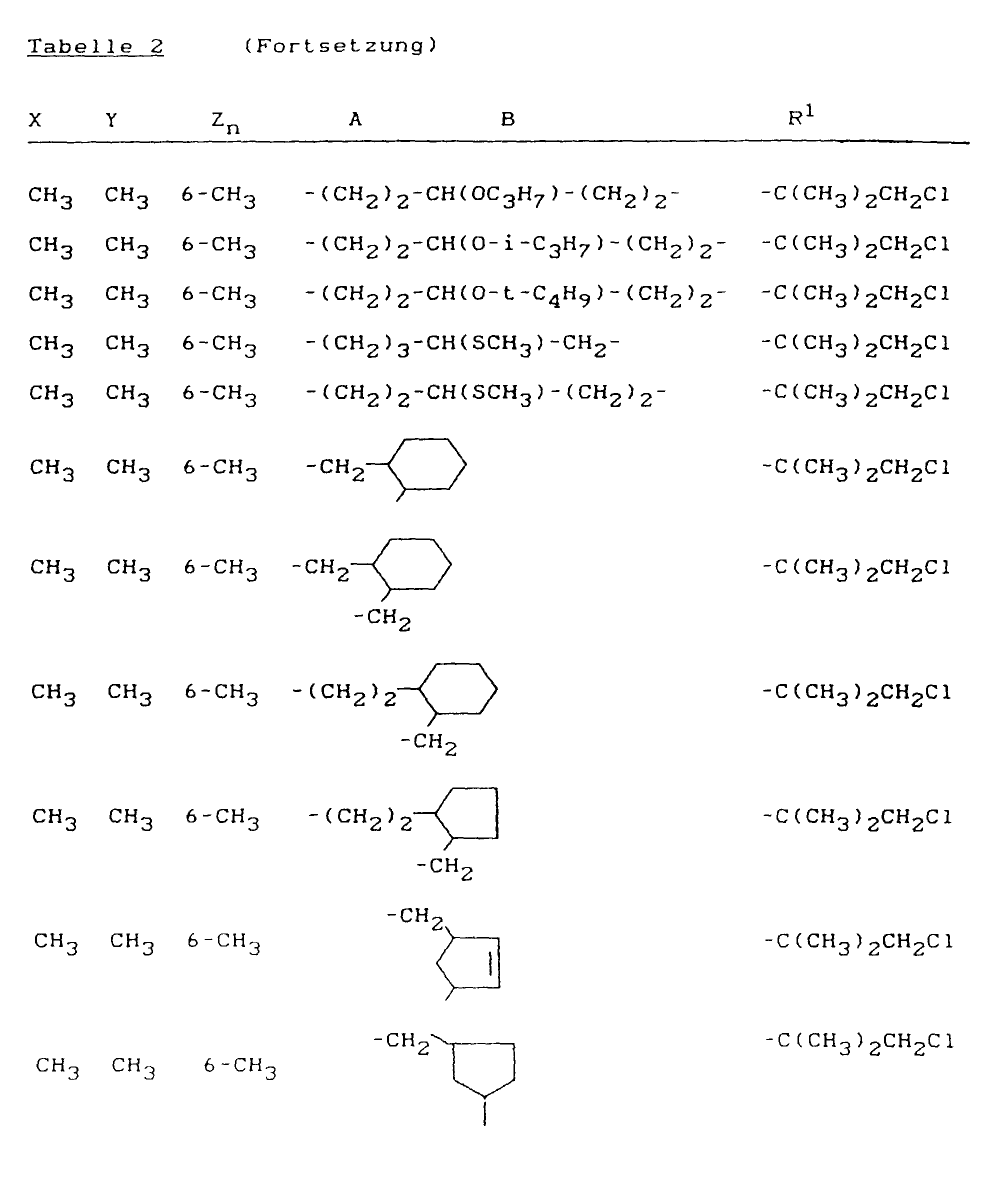

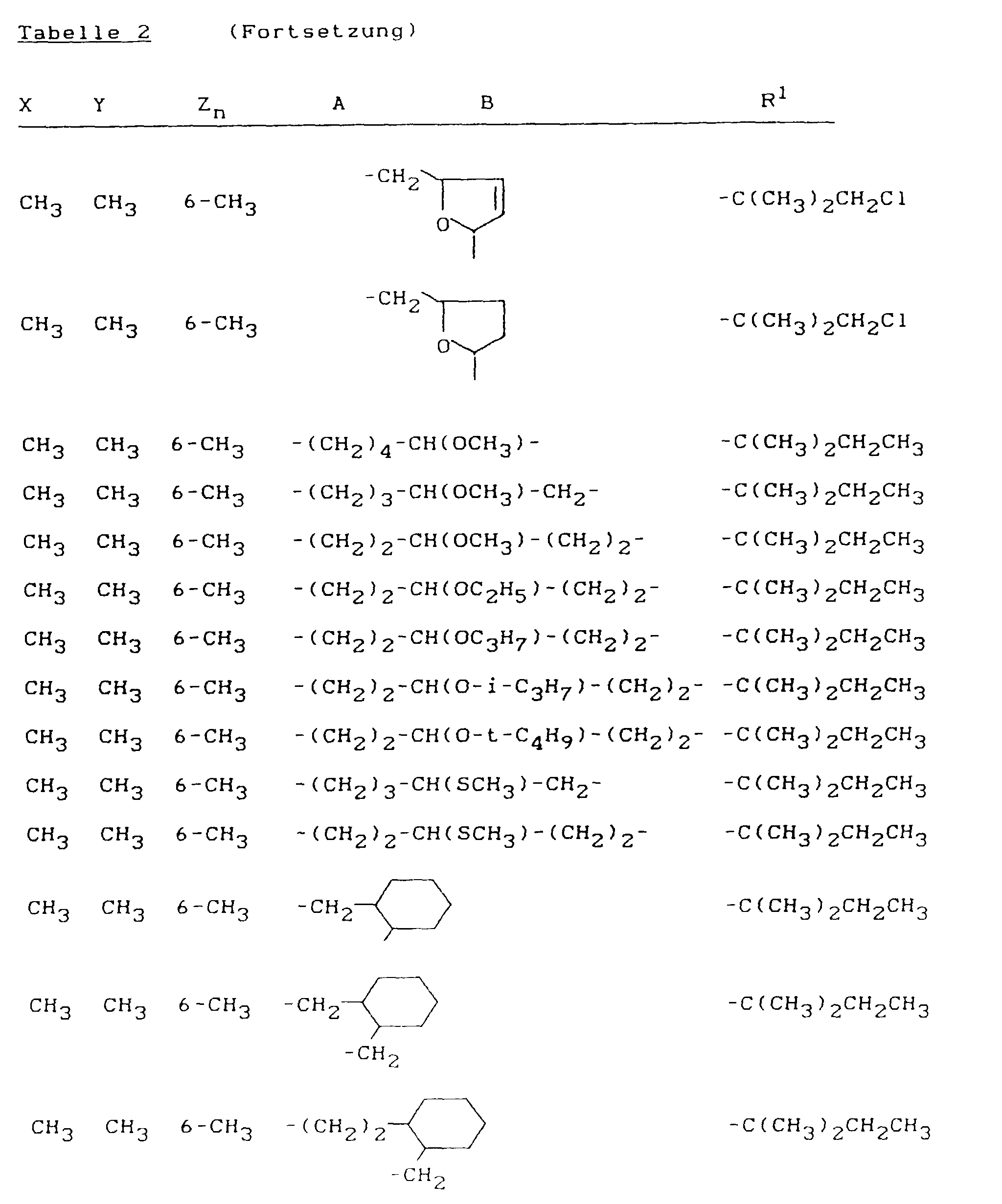

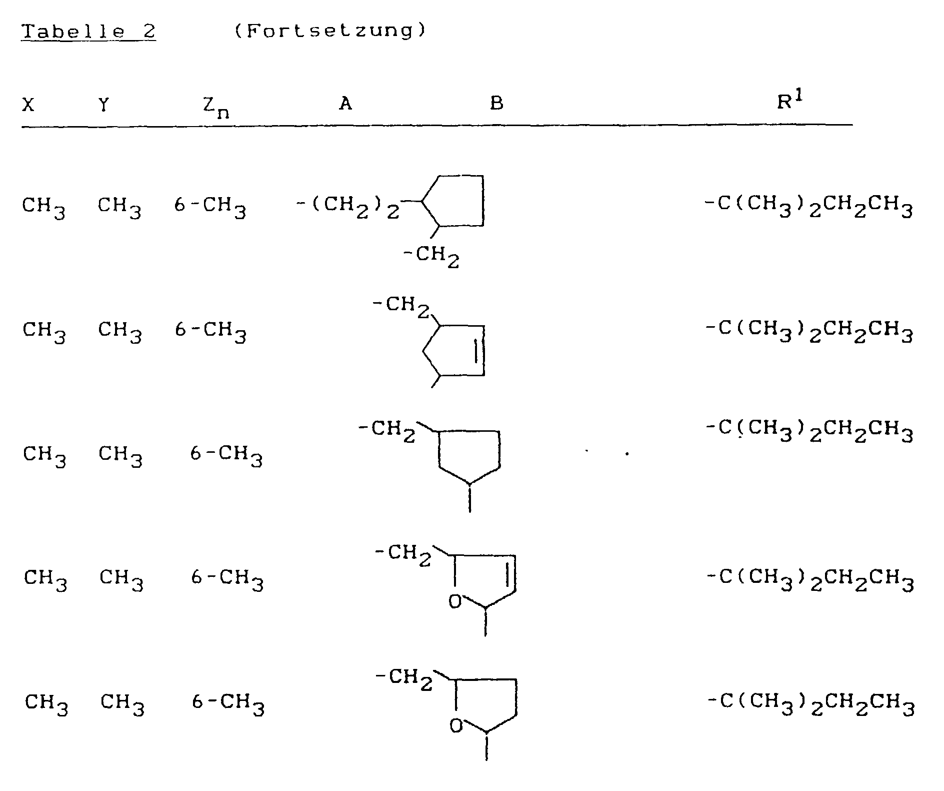

Im einzelnen seien außer den bei den Herstellungsbeispielen genannten Verbindungen die folgenden 3-Aryl-4-hydroxy-Δ³-dihydrofuran-Derivate der Formel (Ic) genannt:

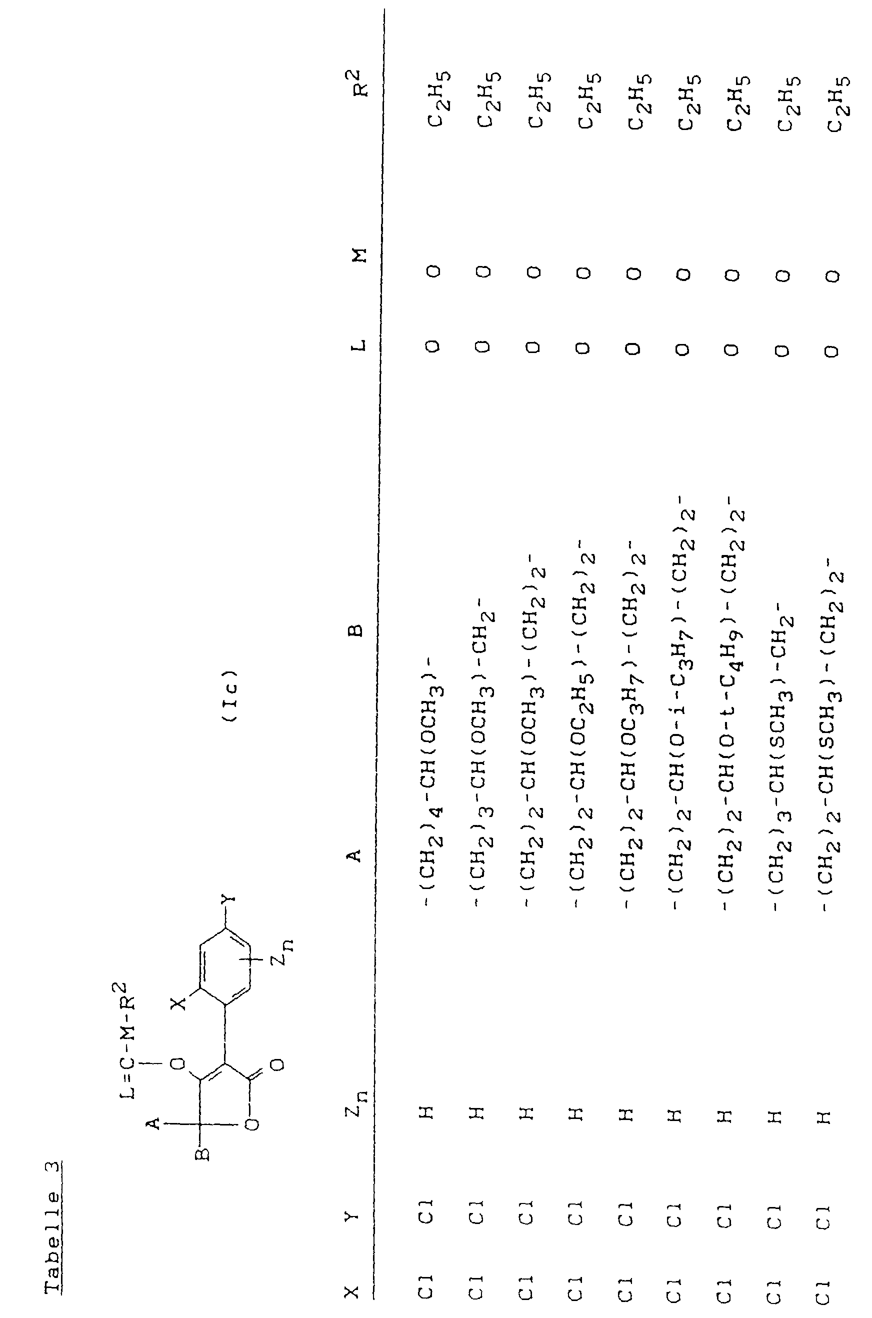

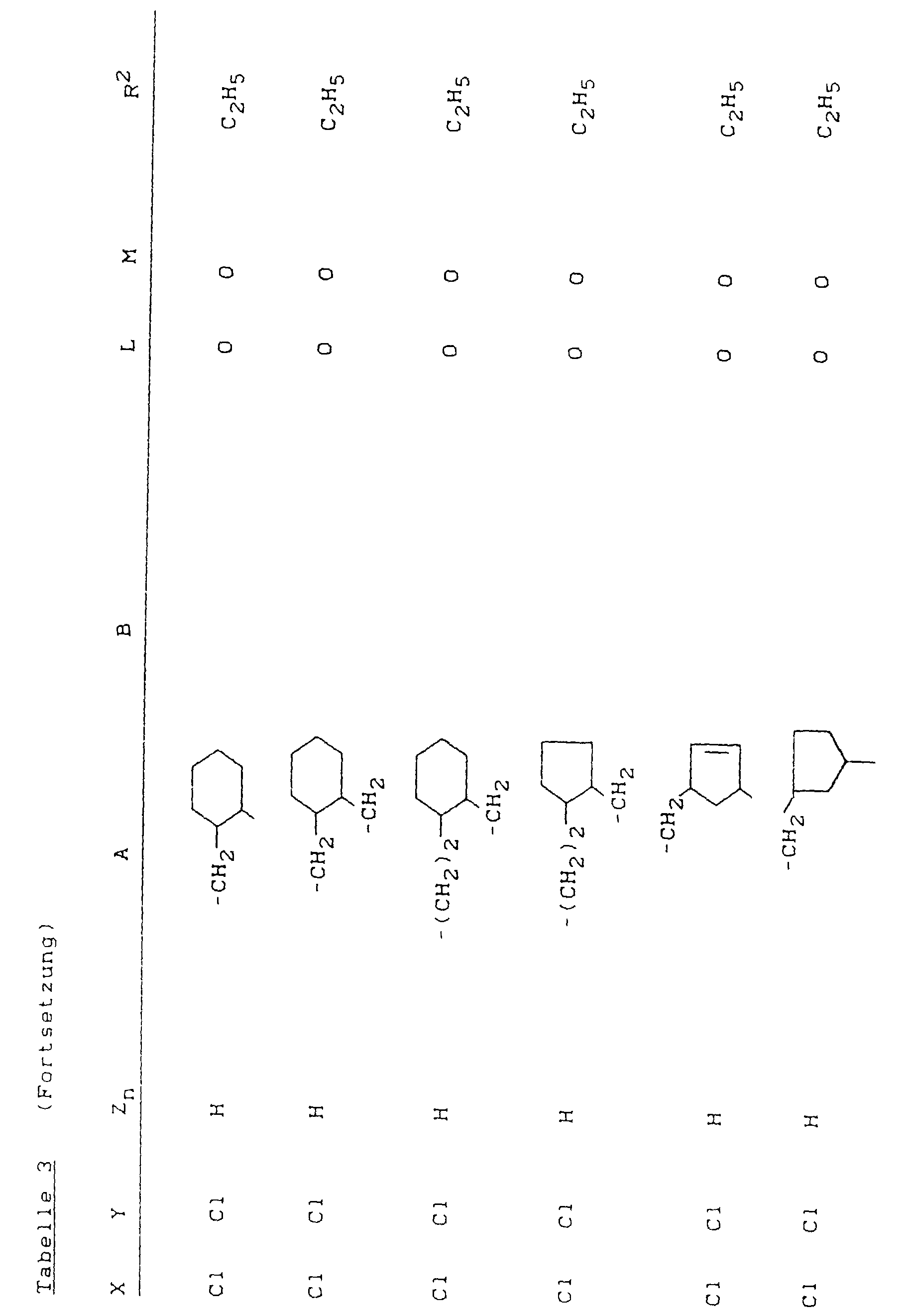

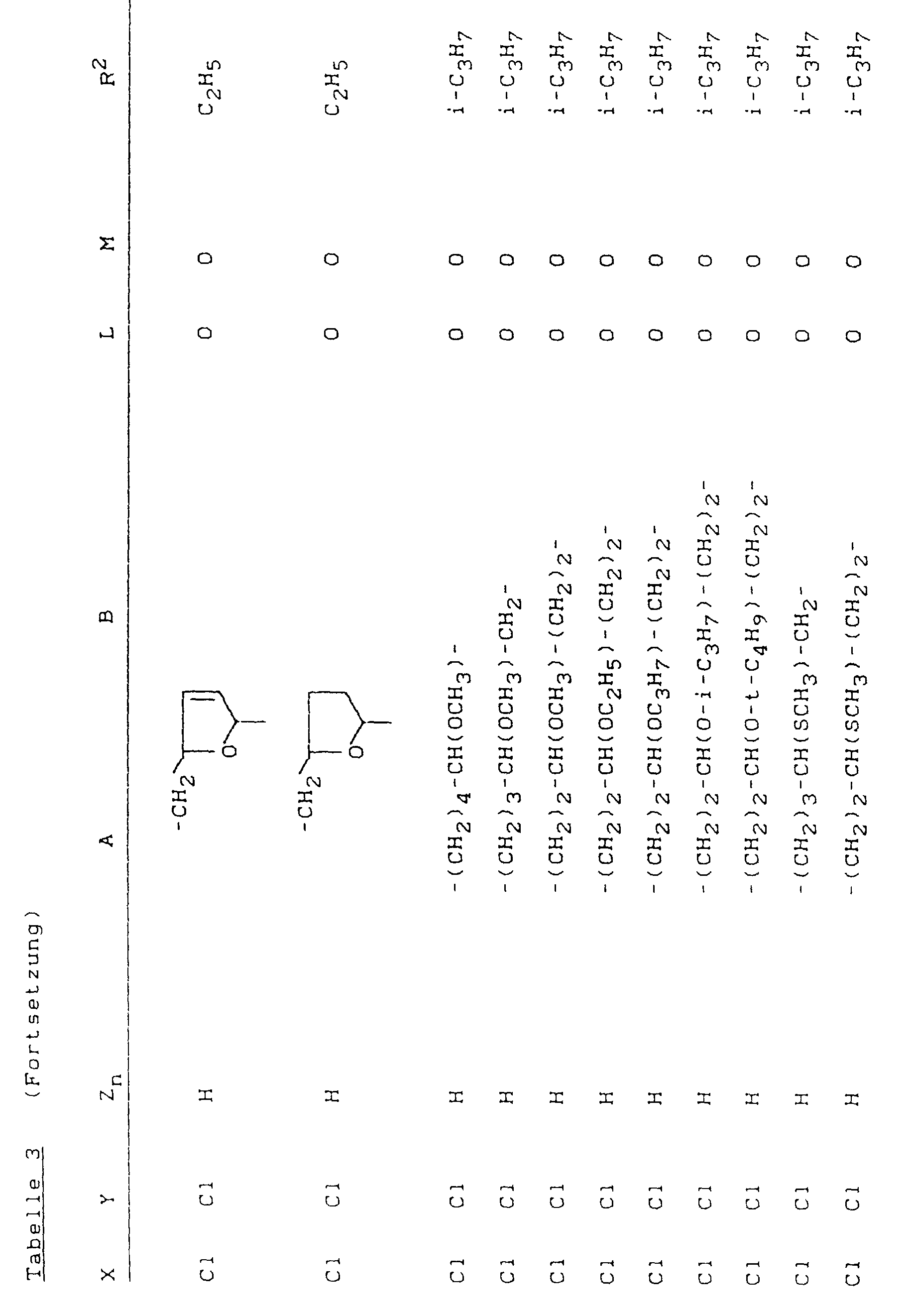

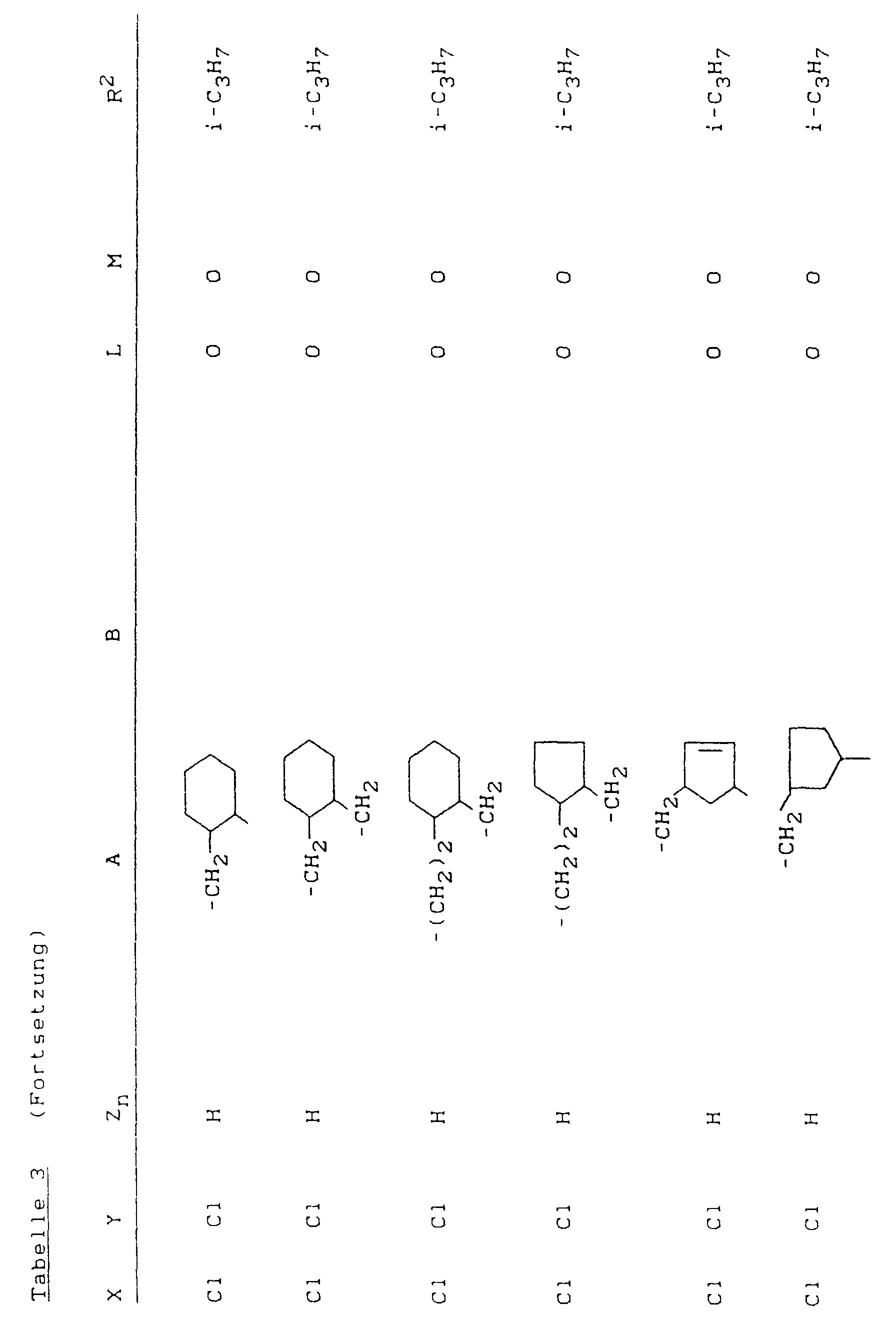

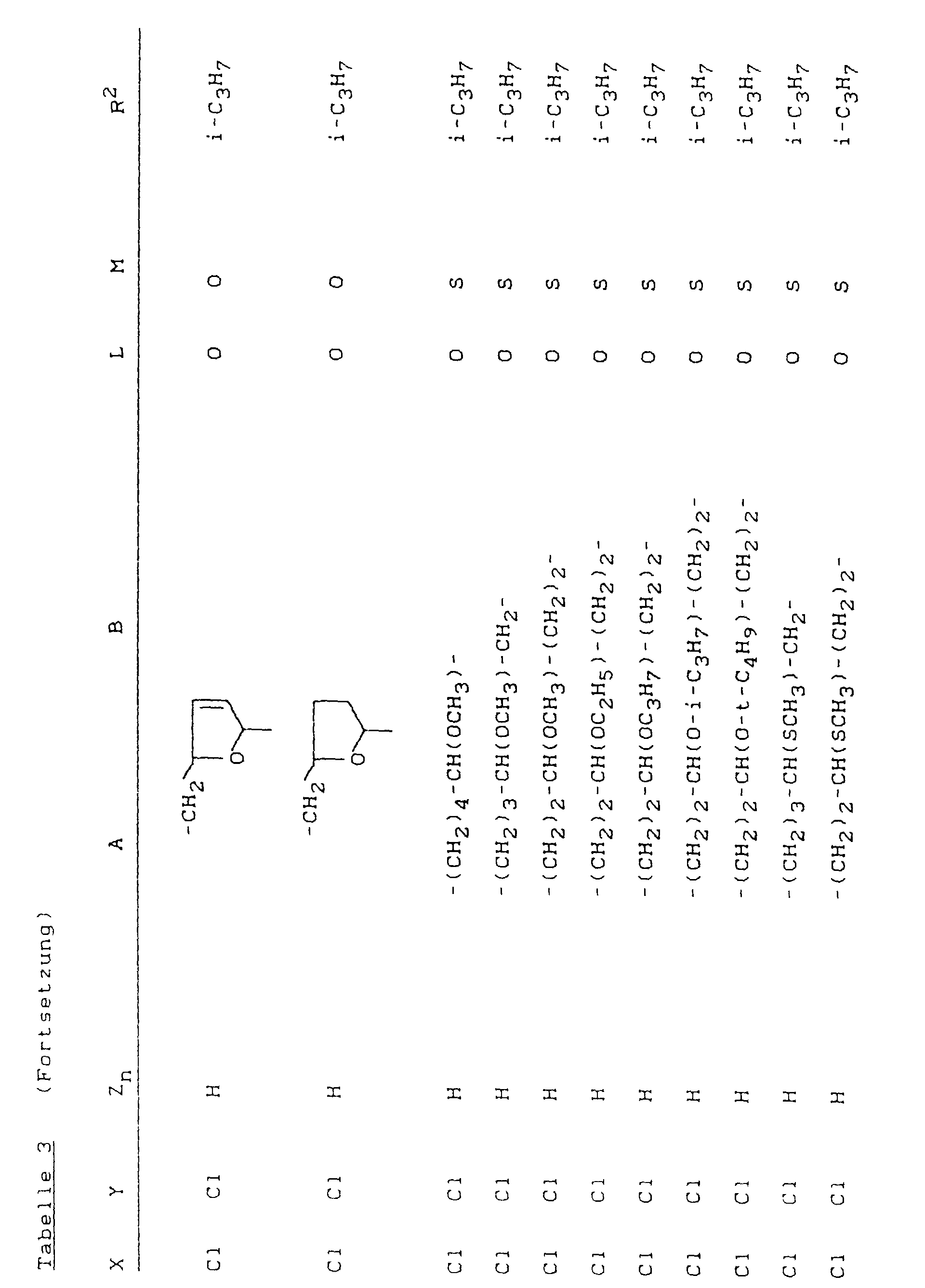

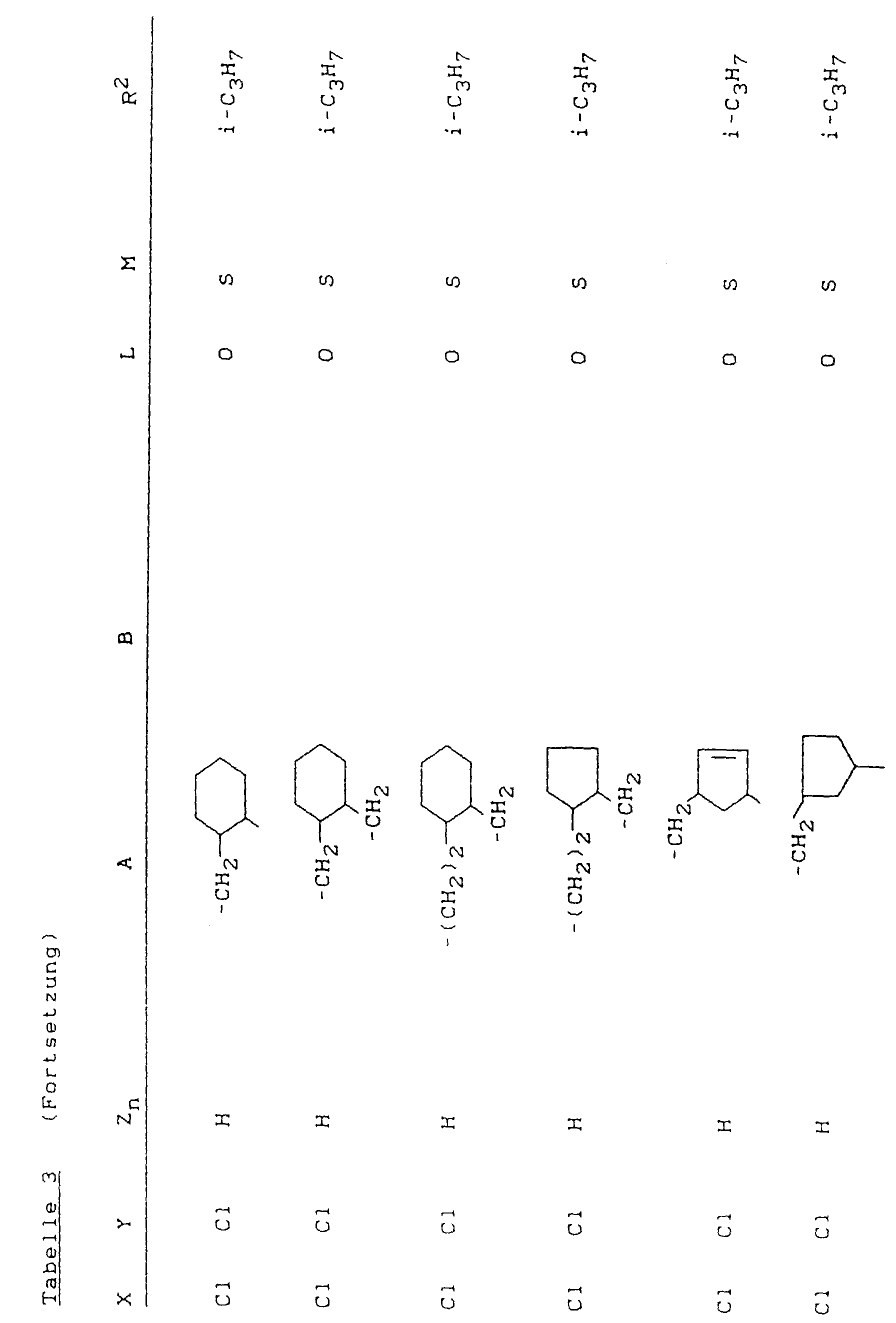

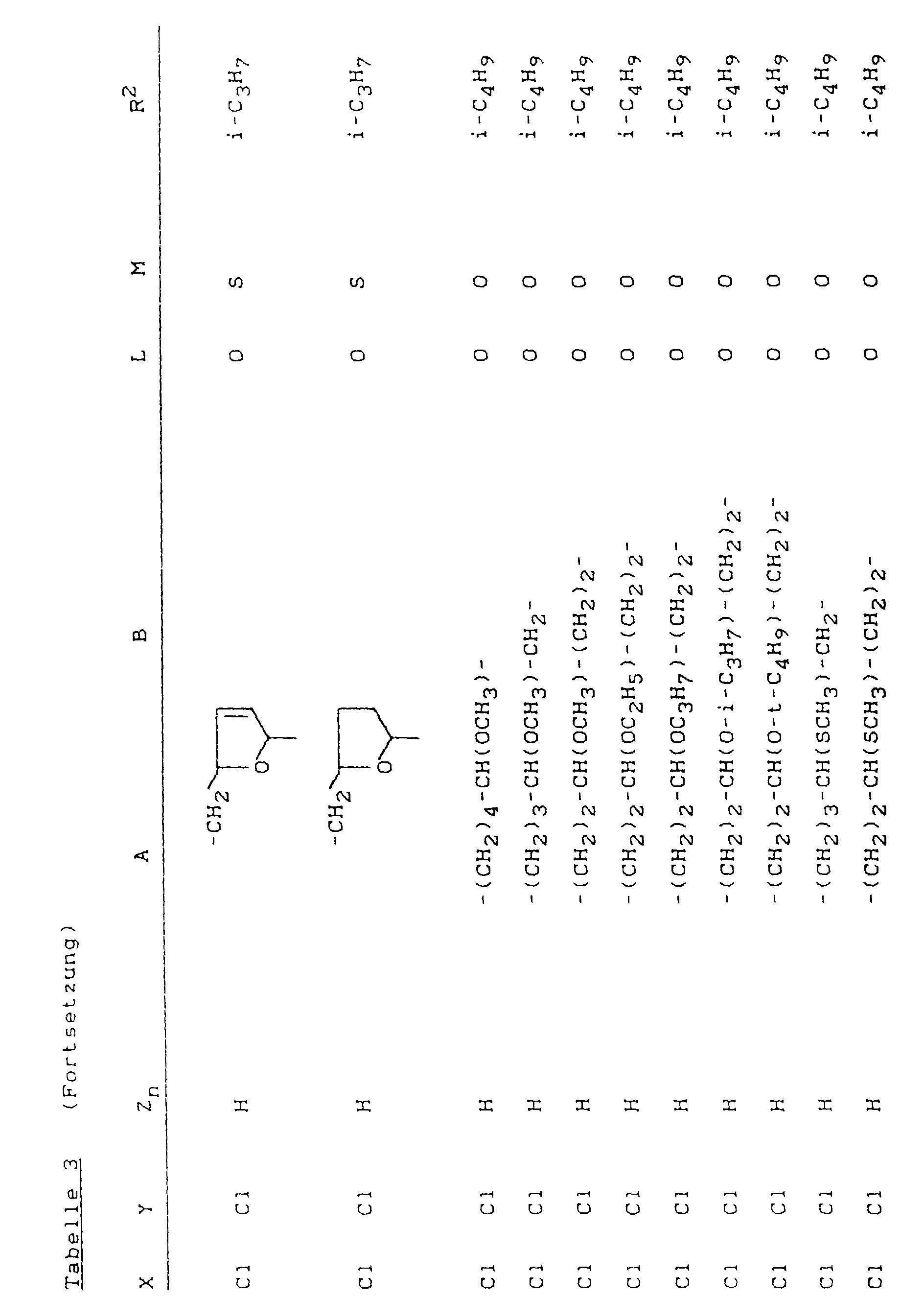

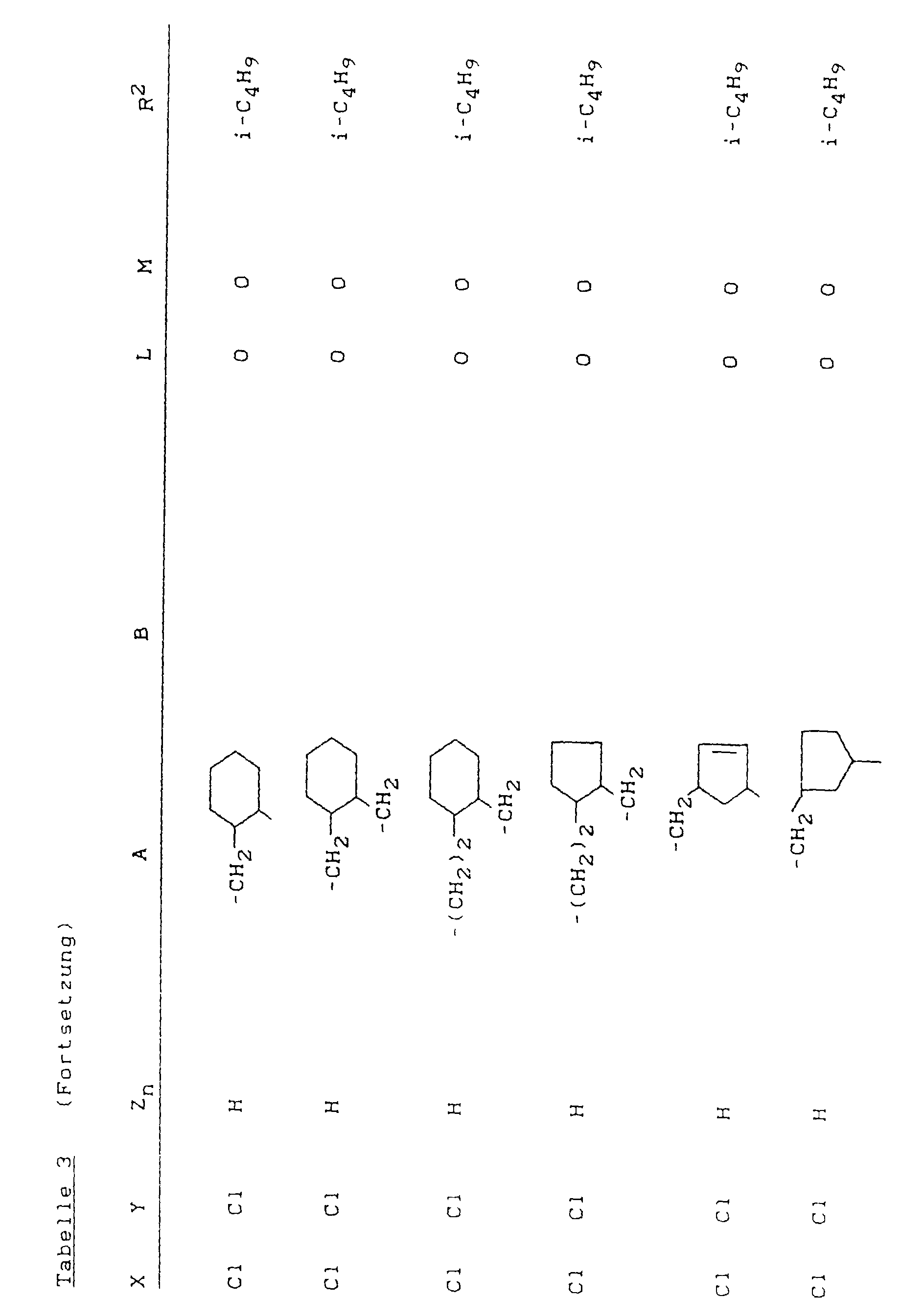

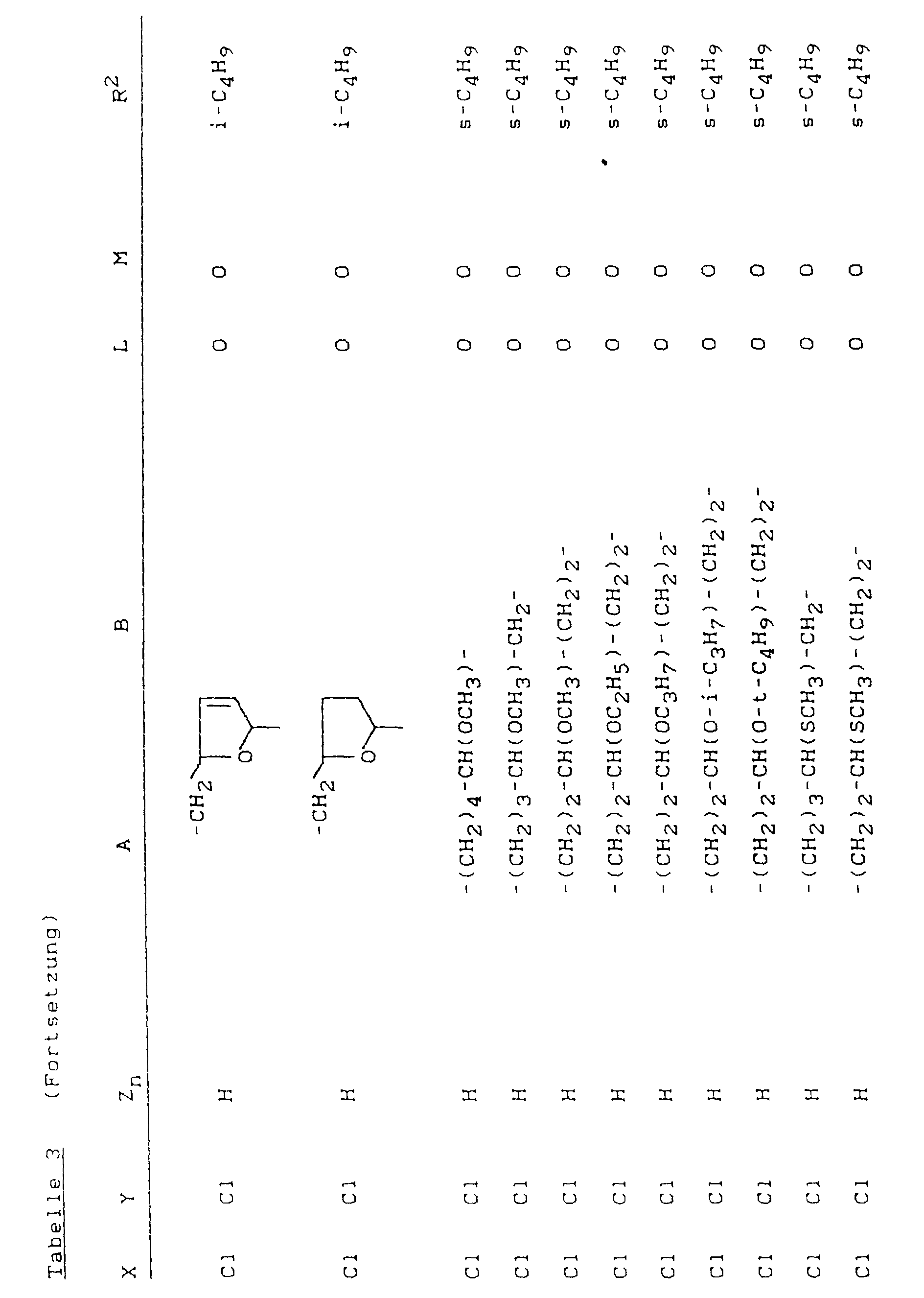

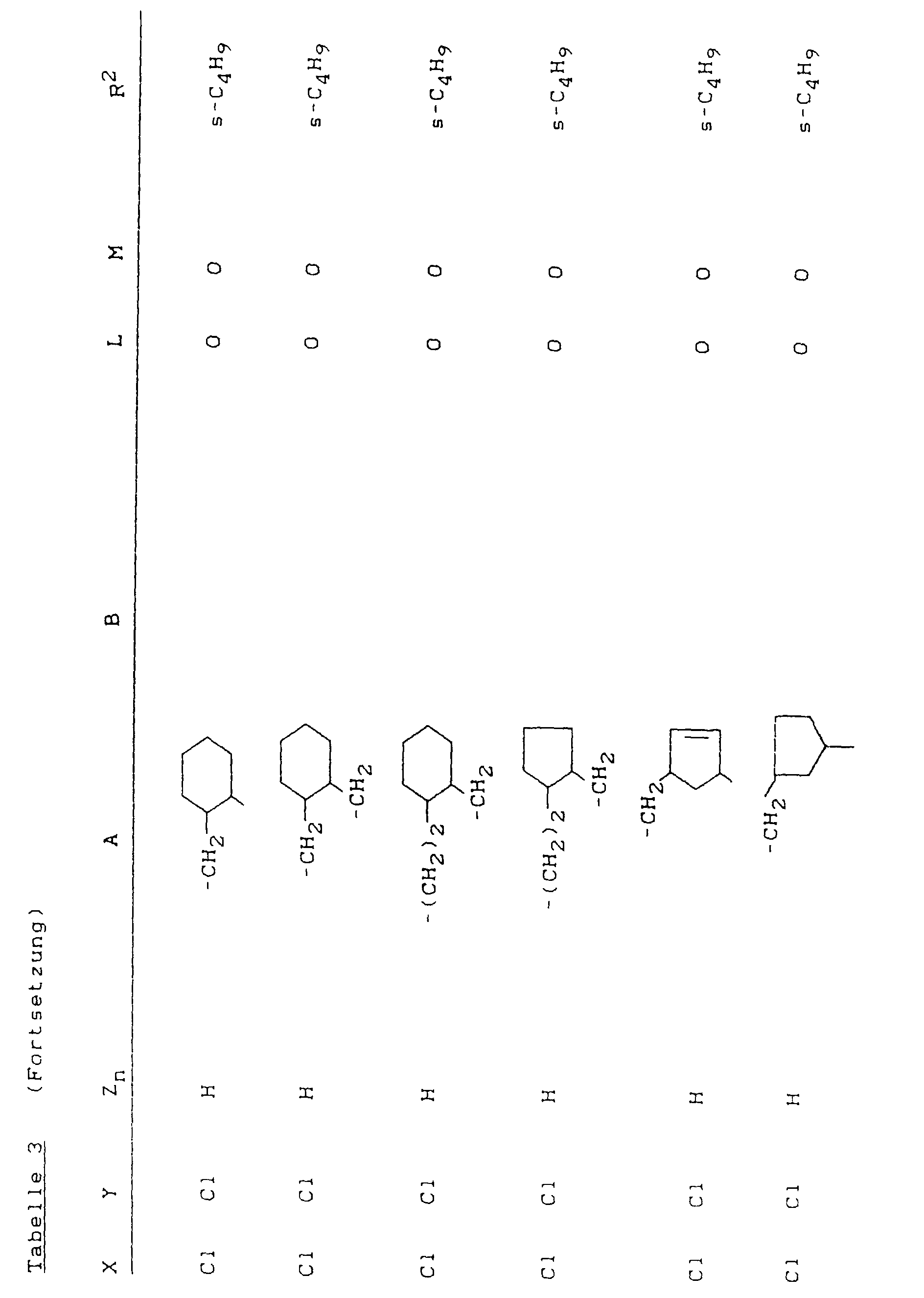

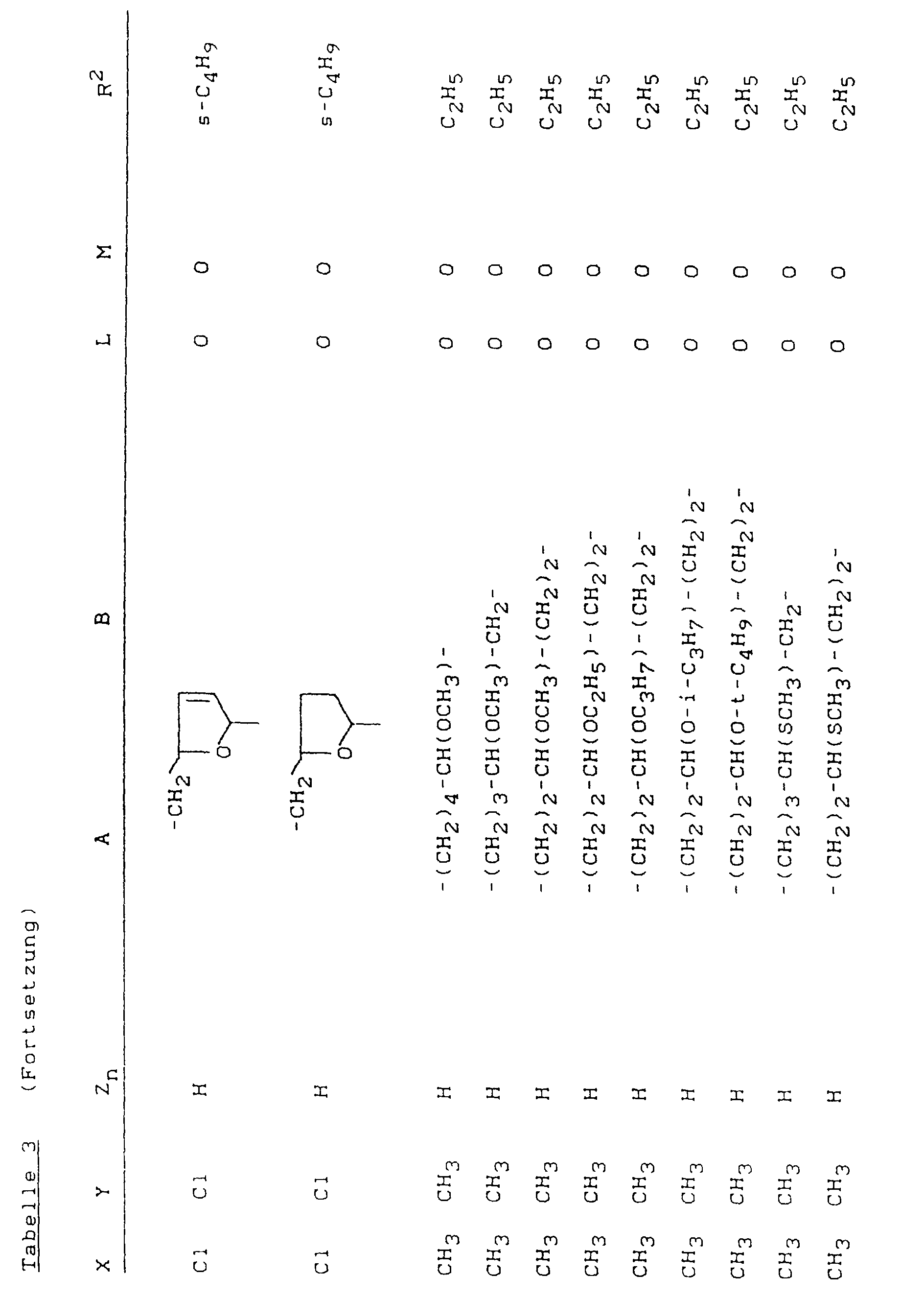

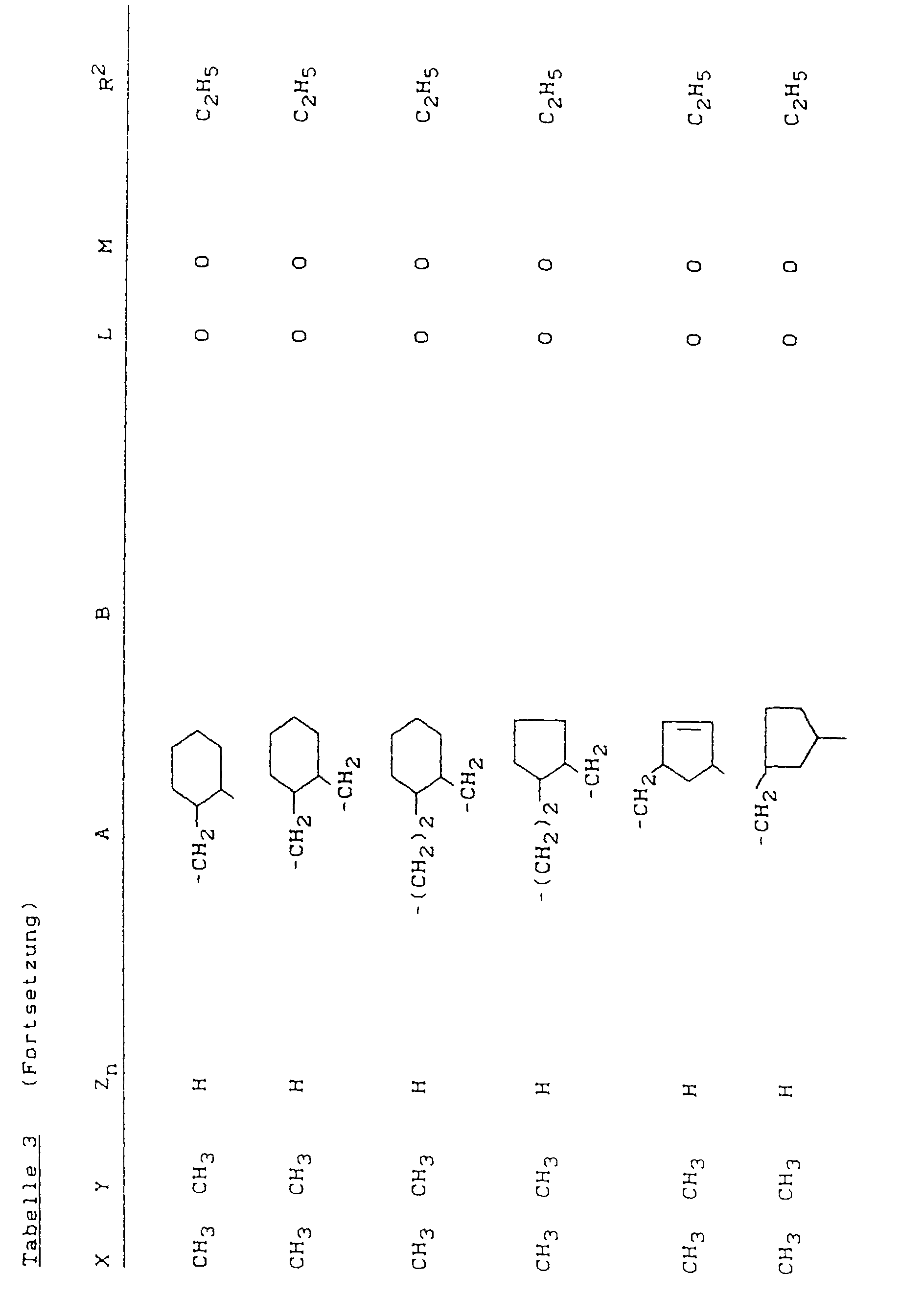

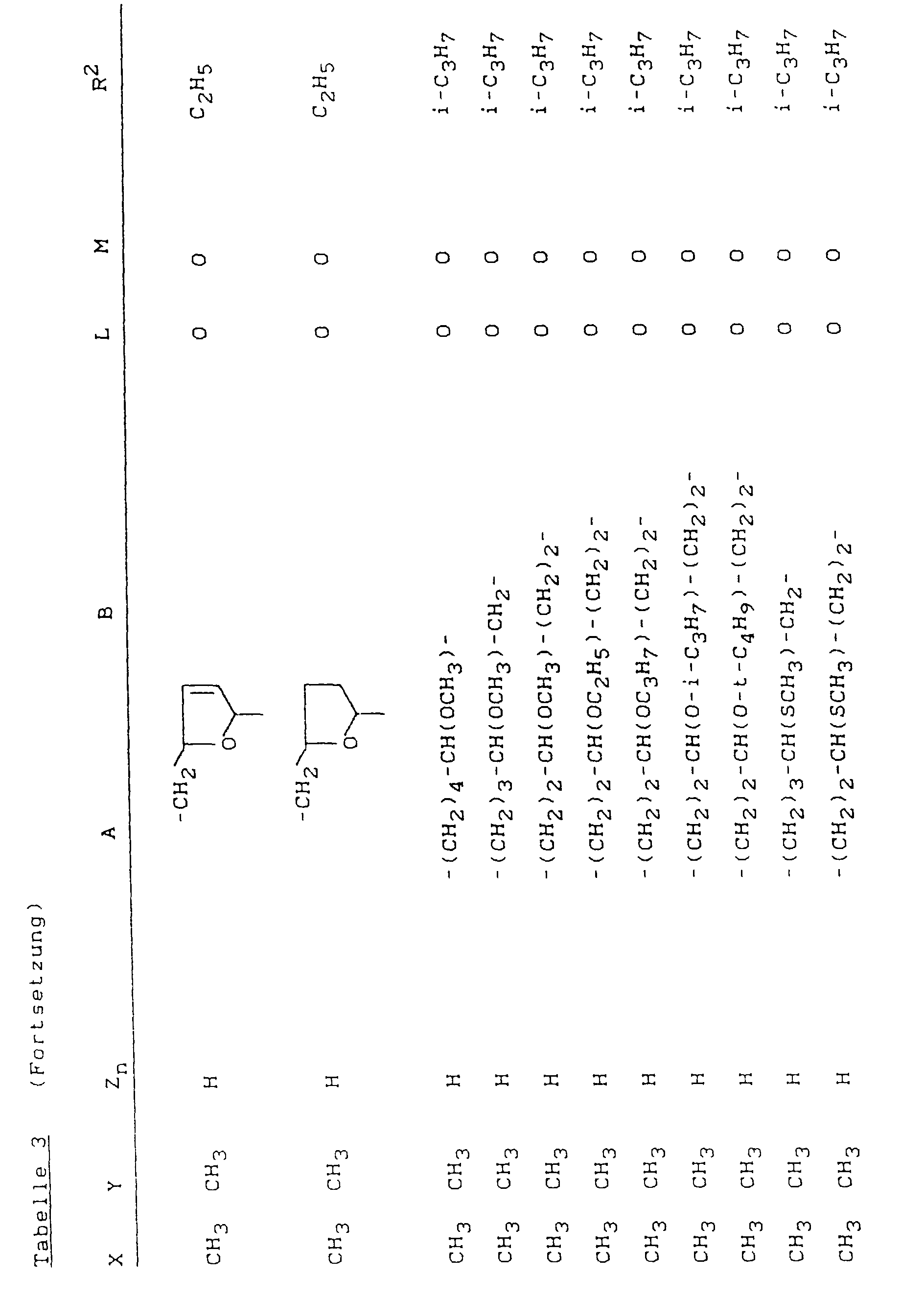

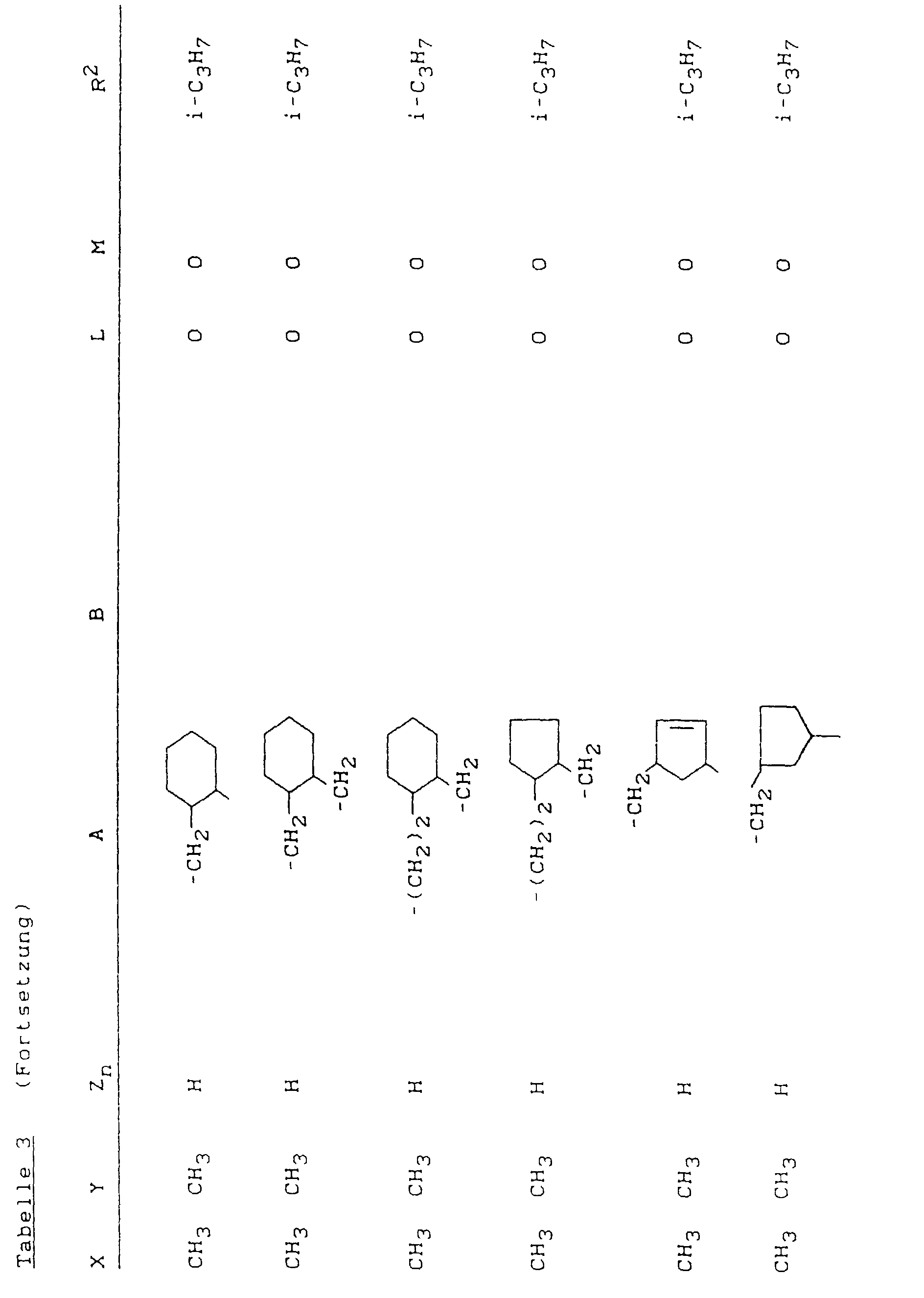

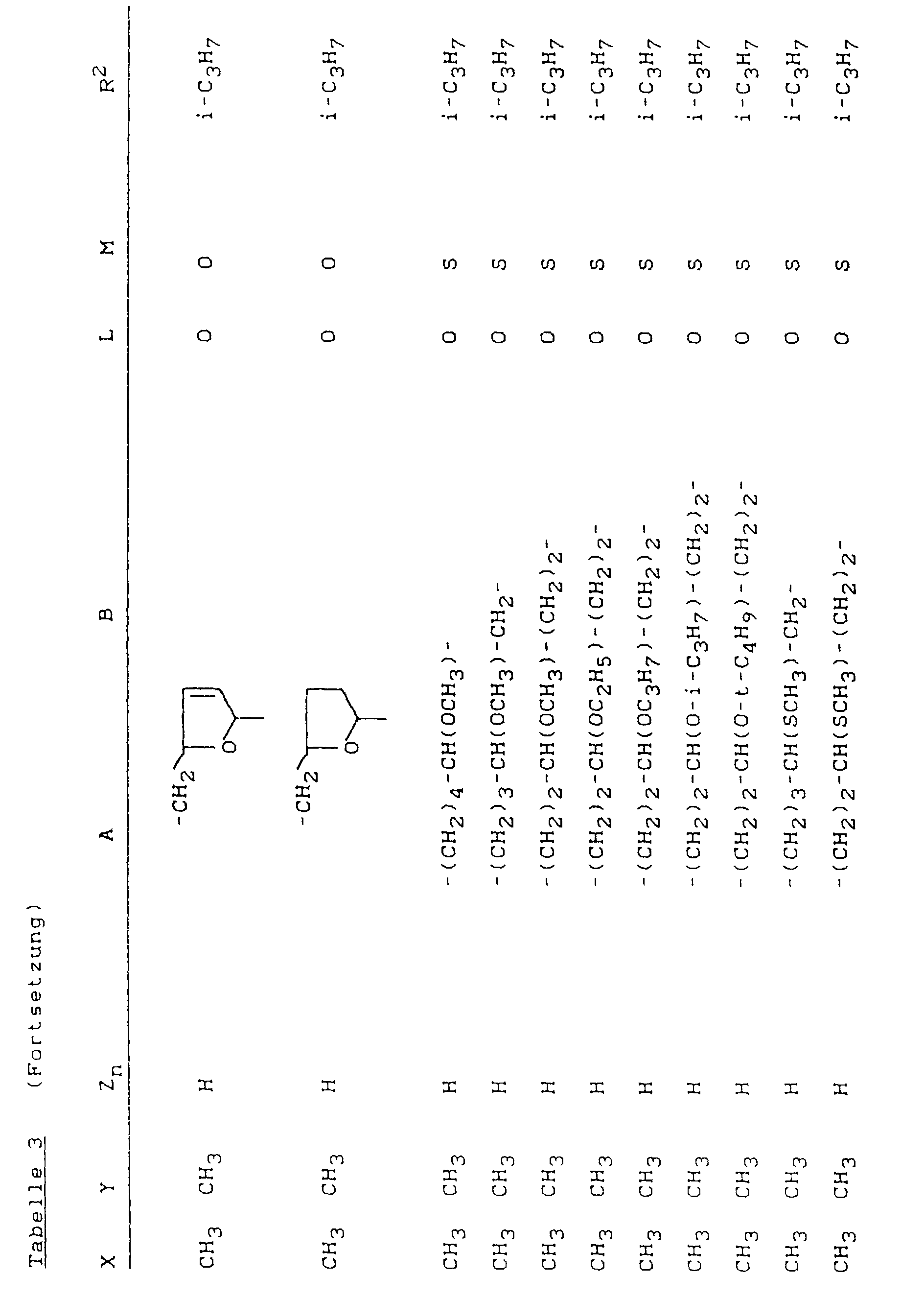

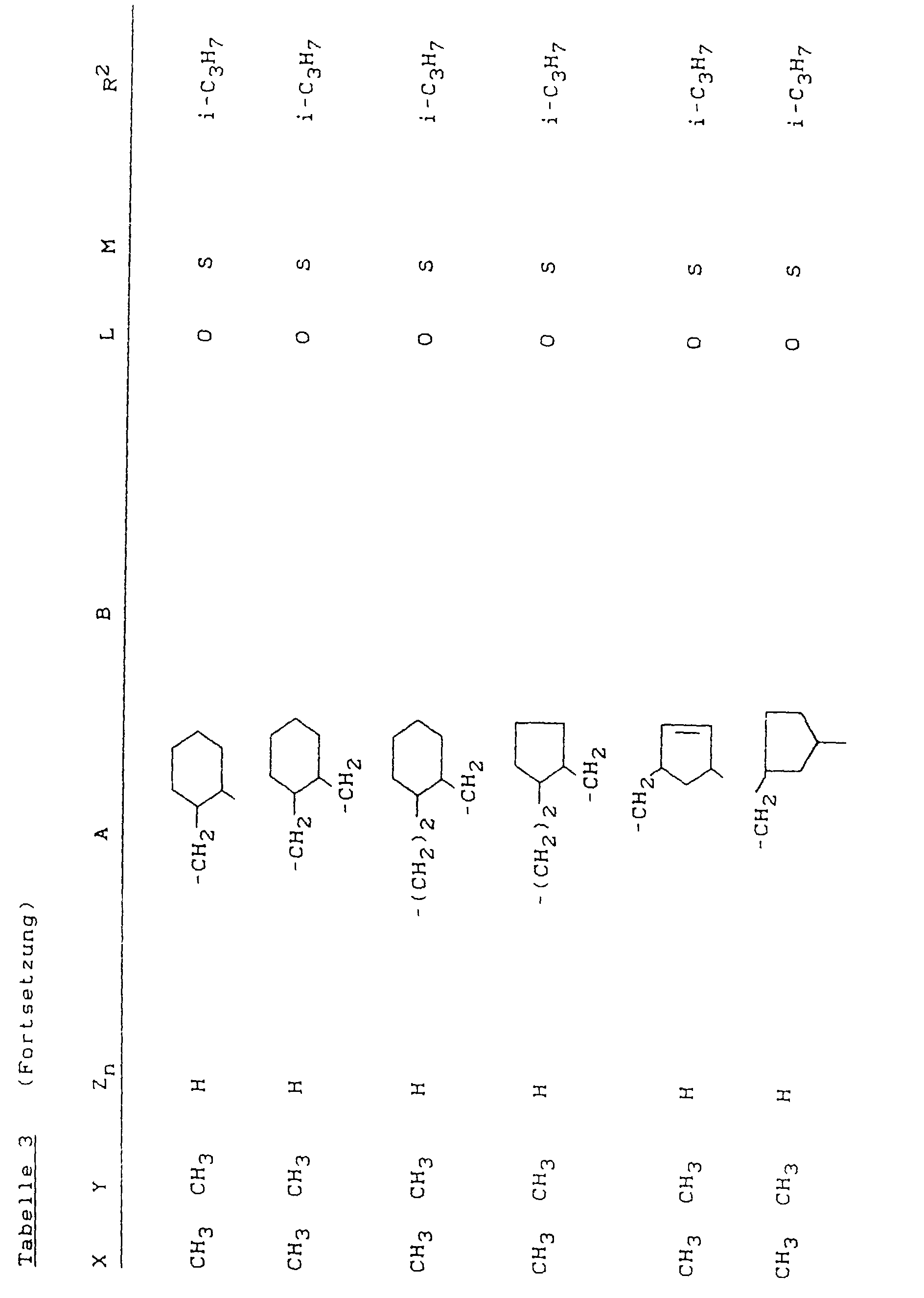

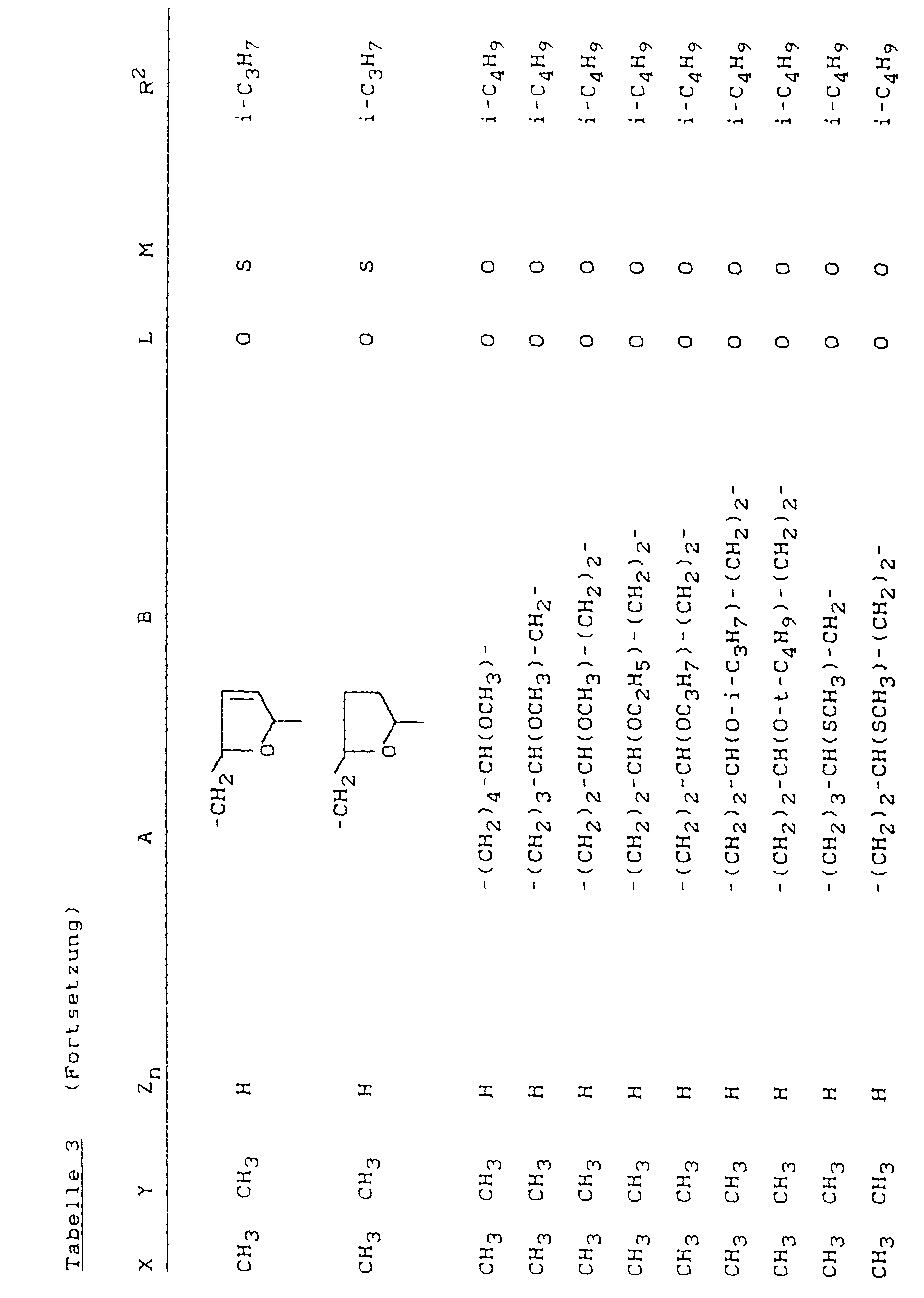

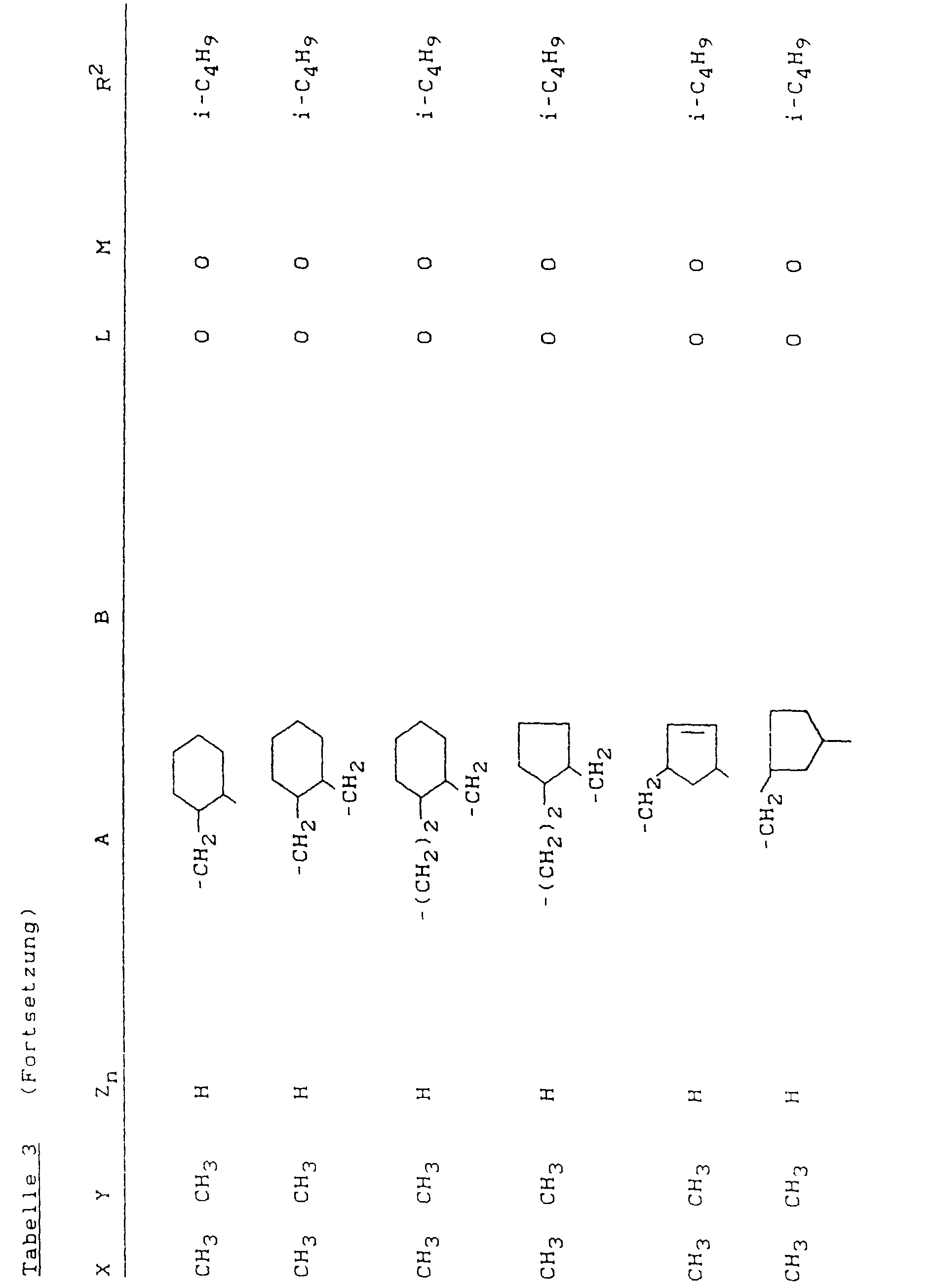

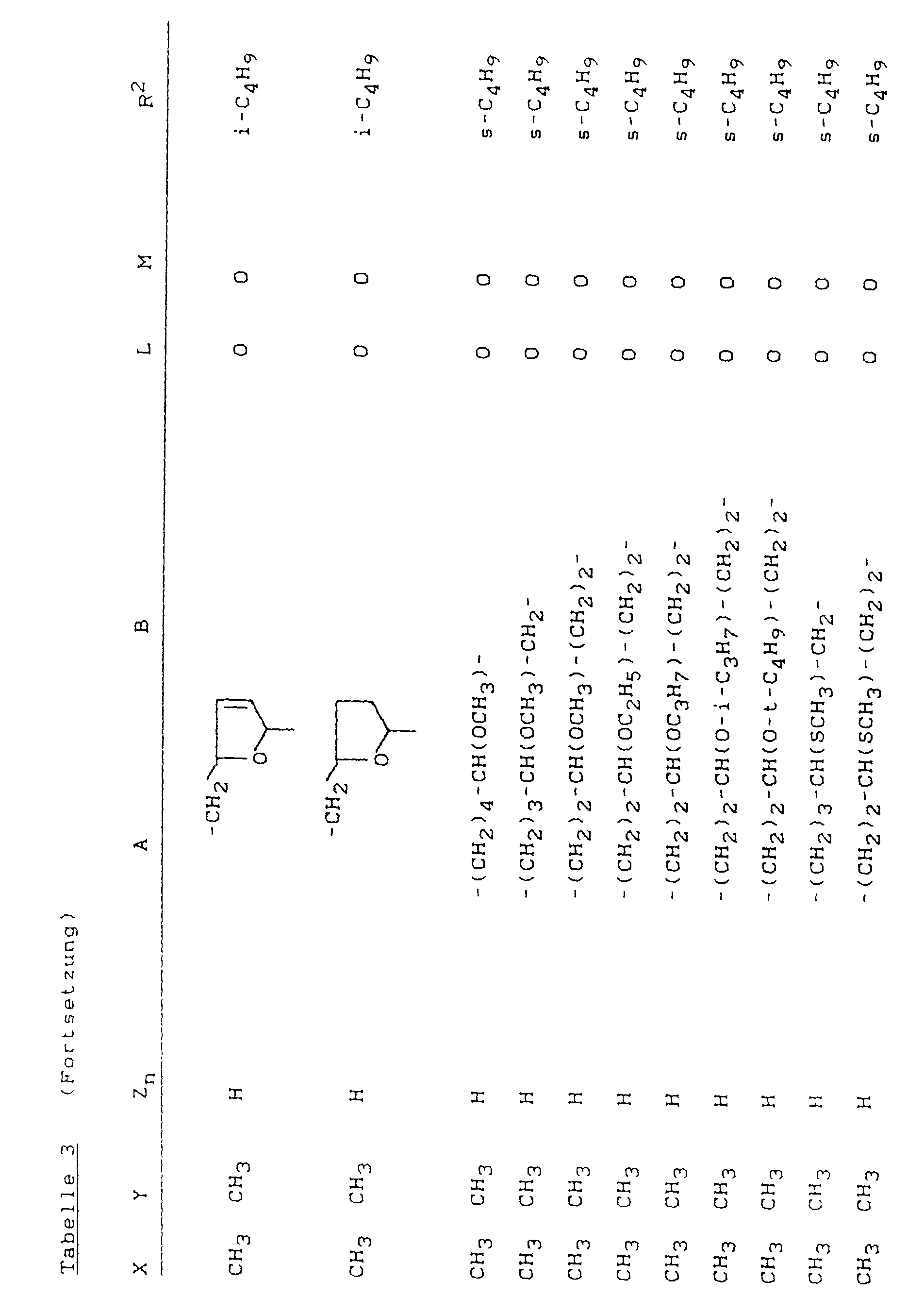

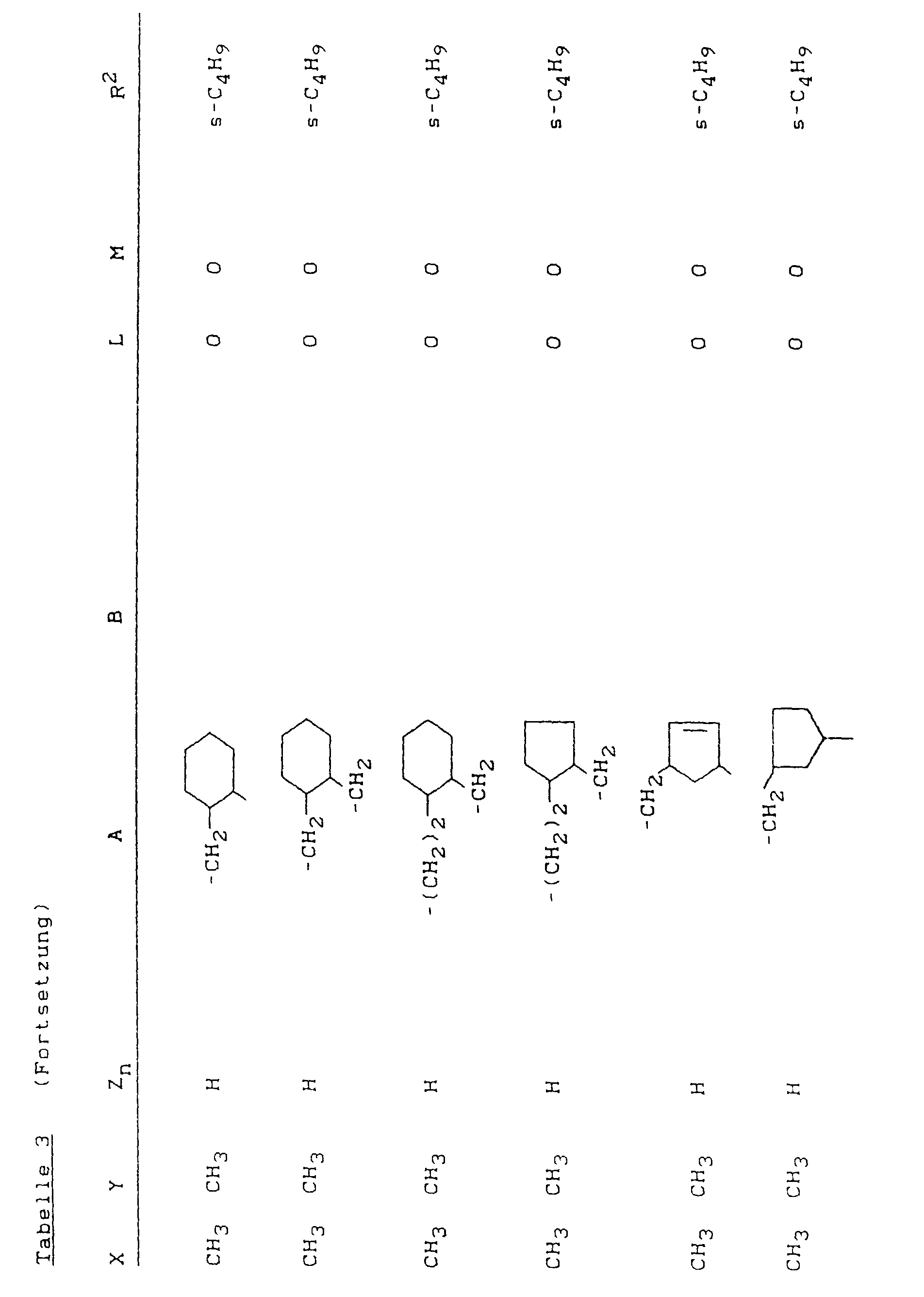

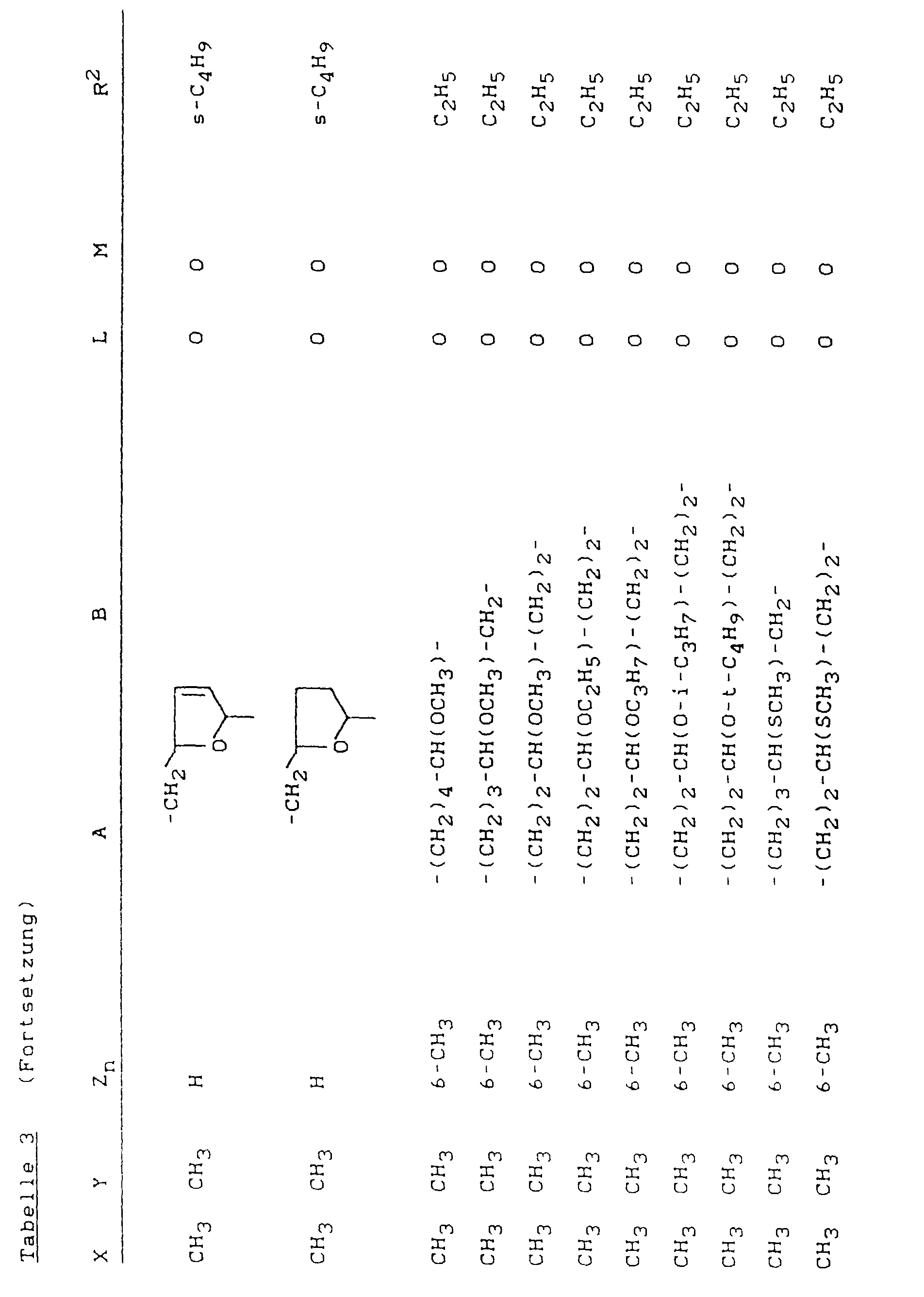

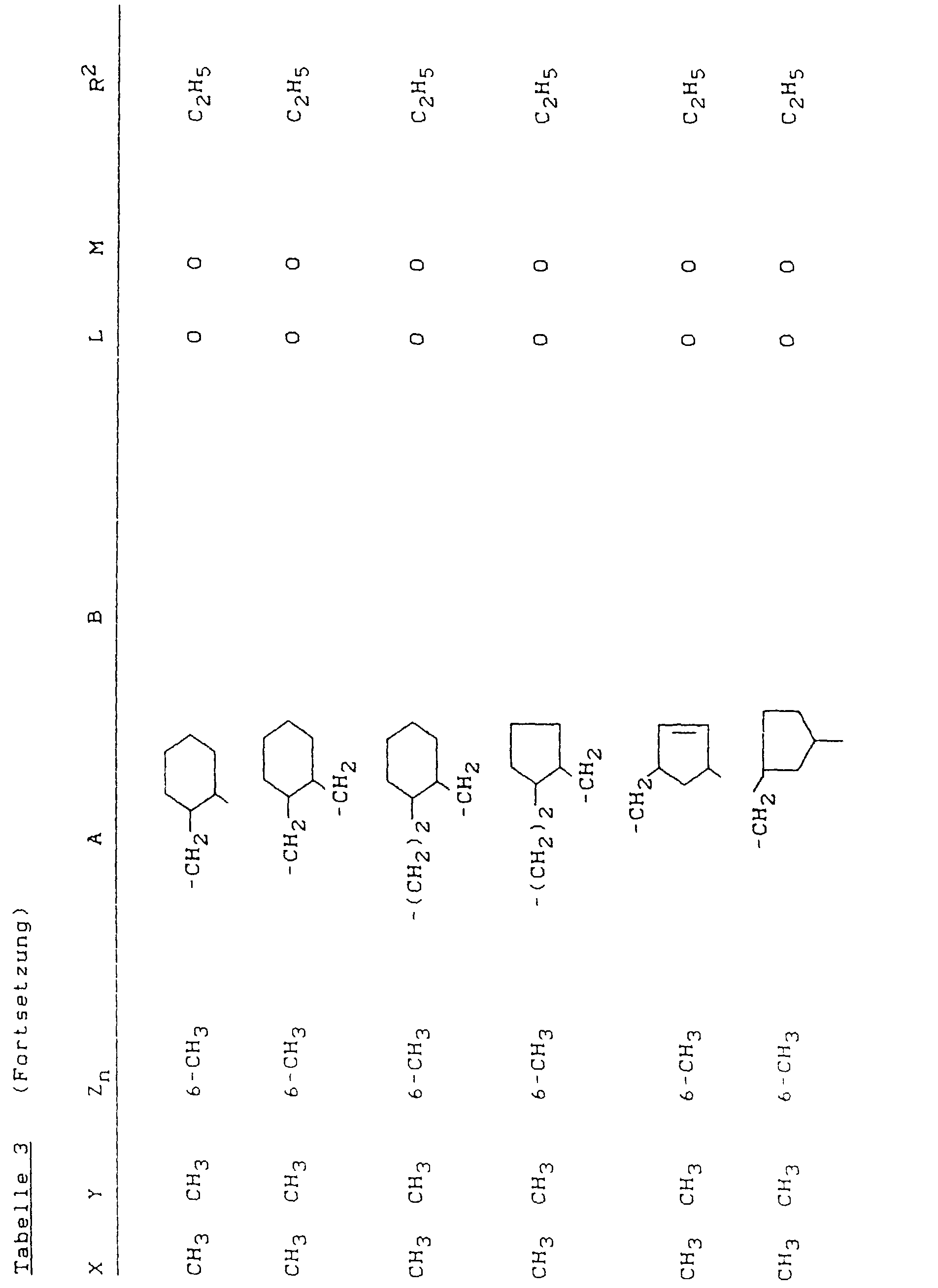

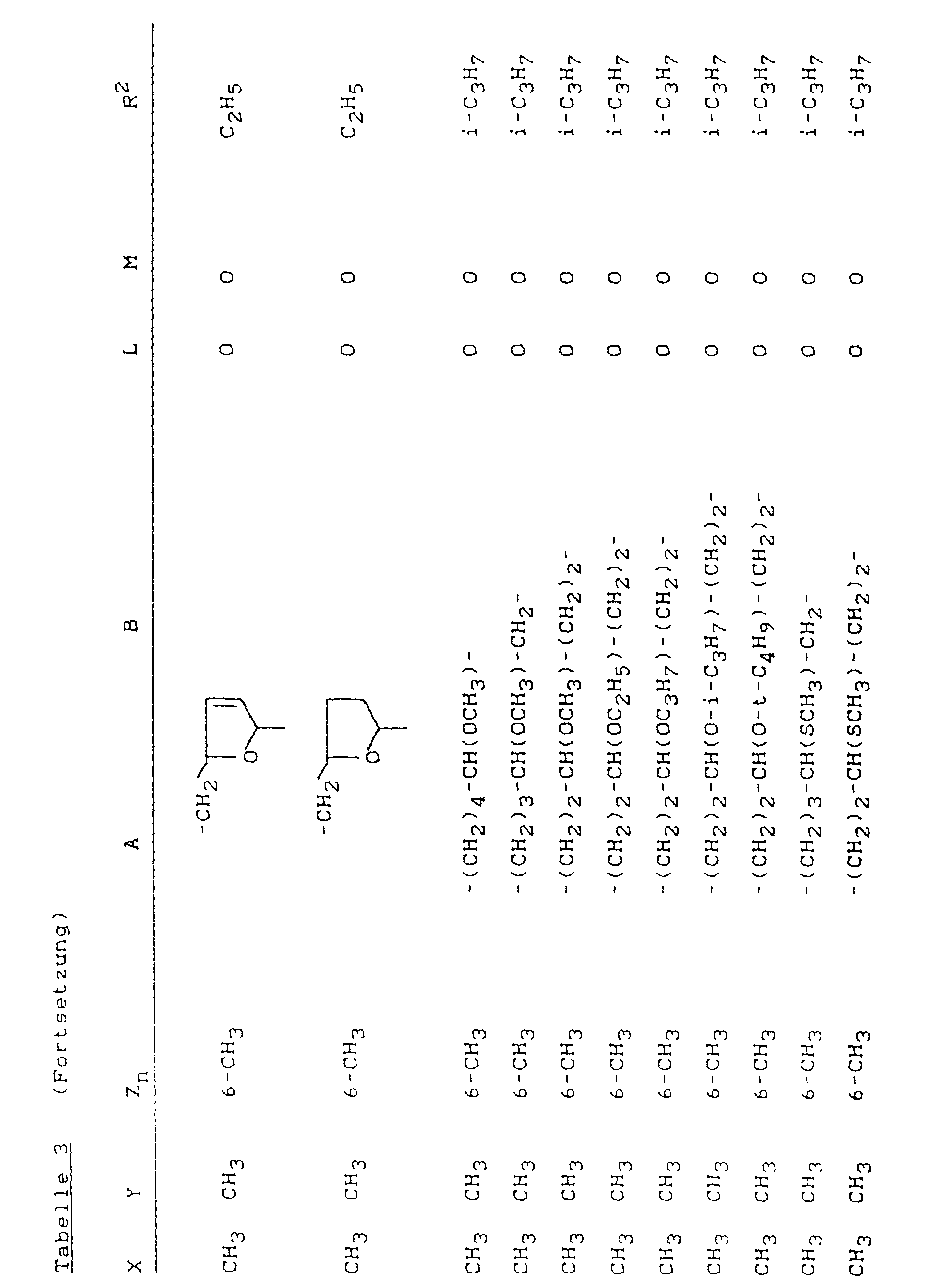

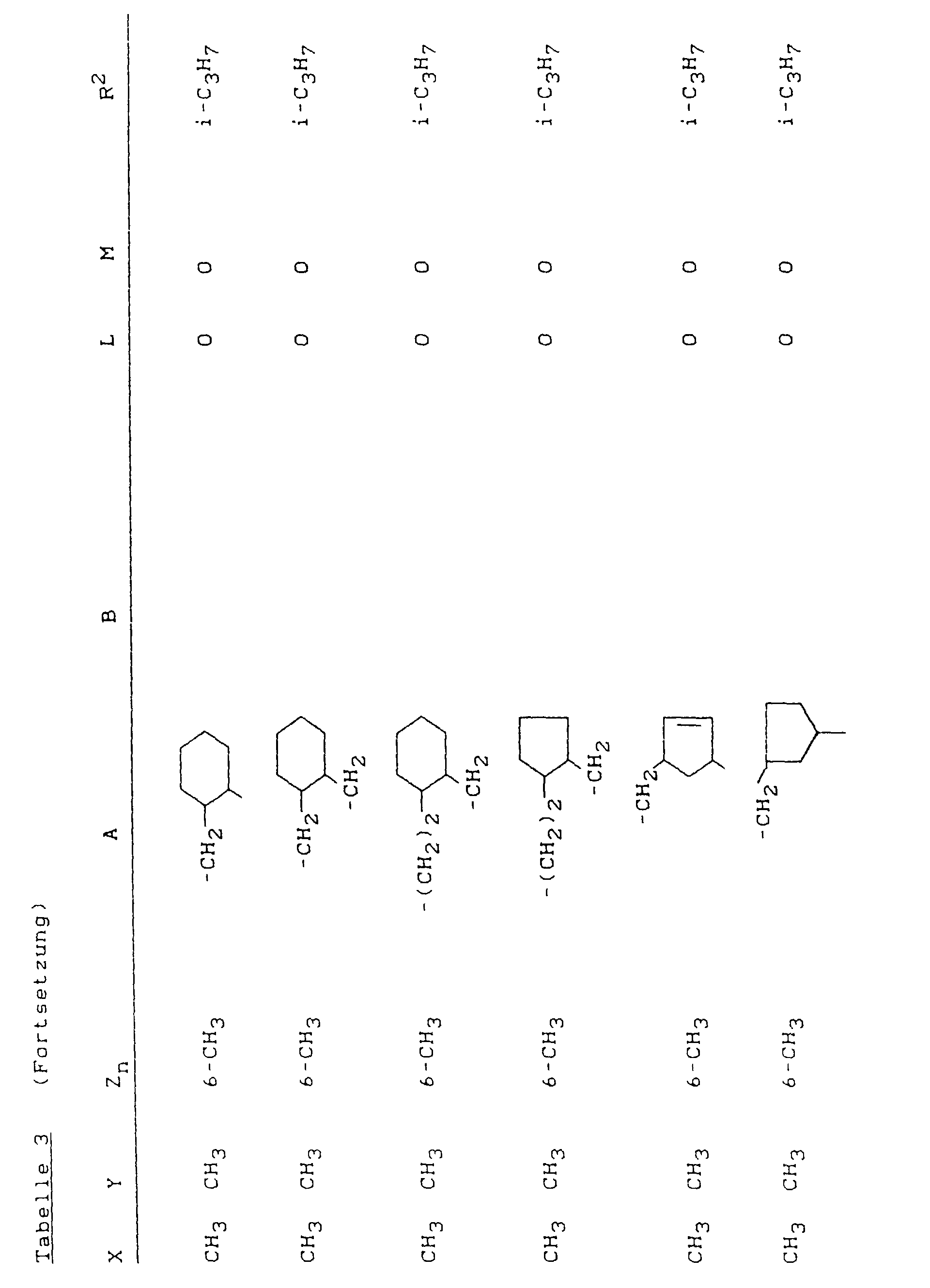

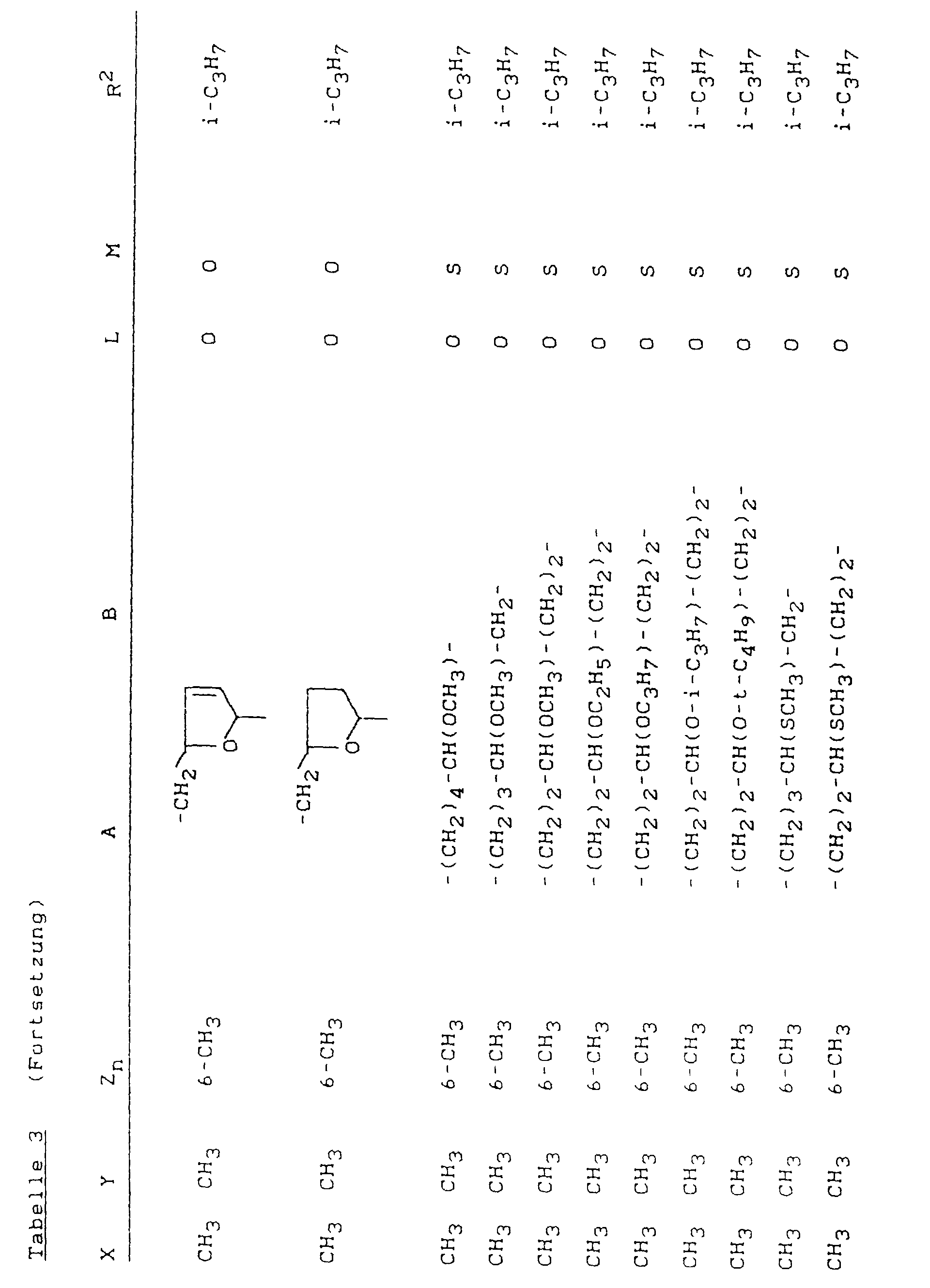

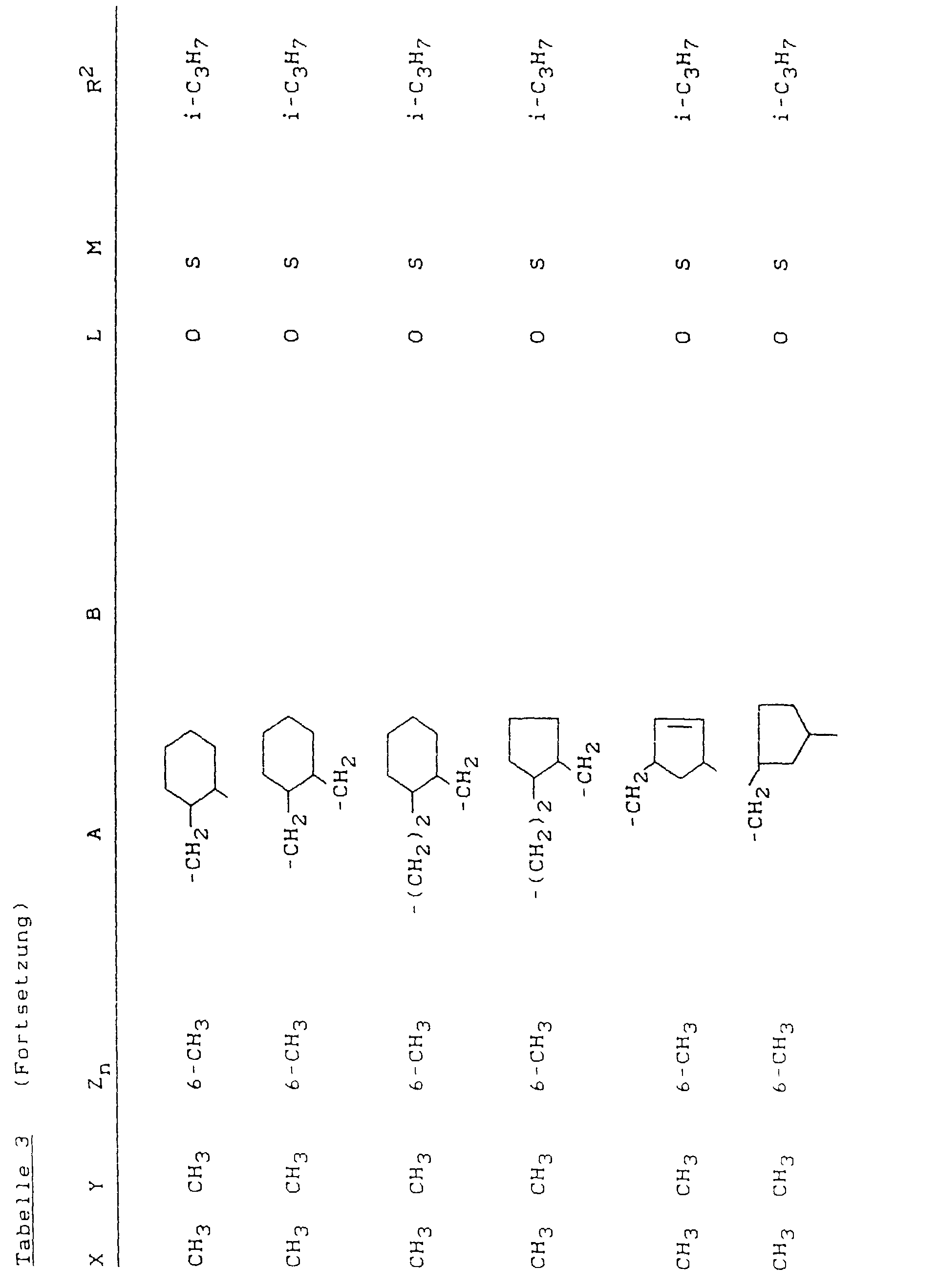

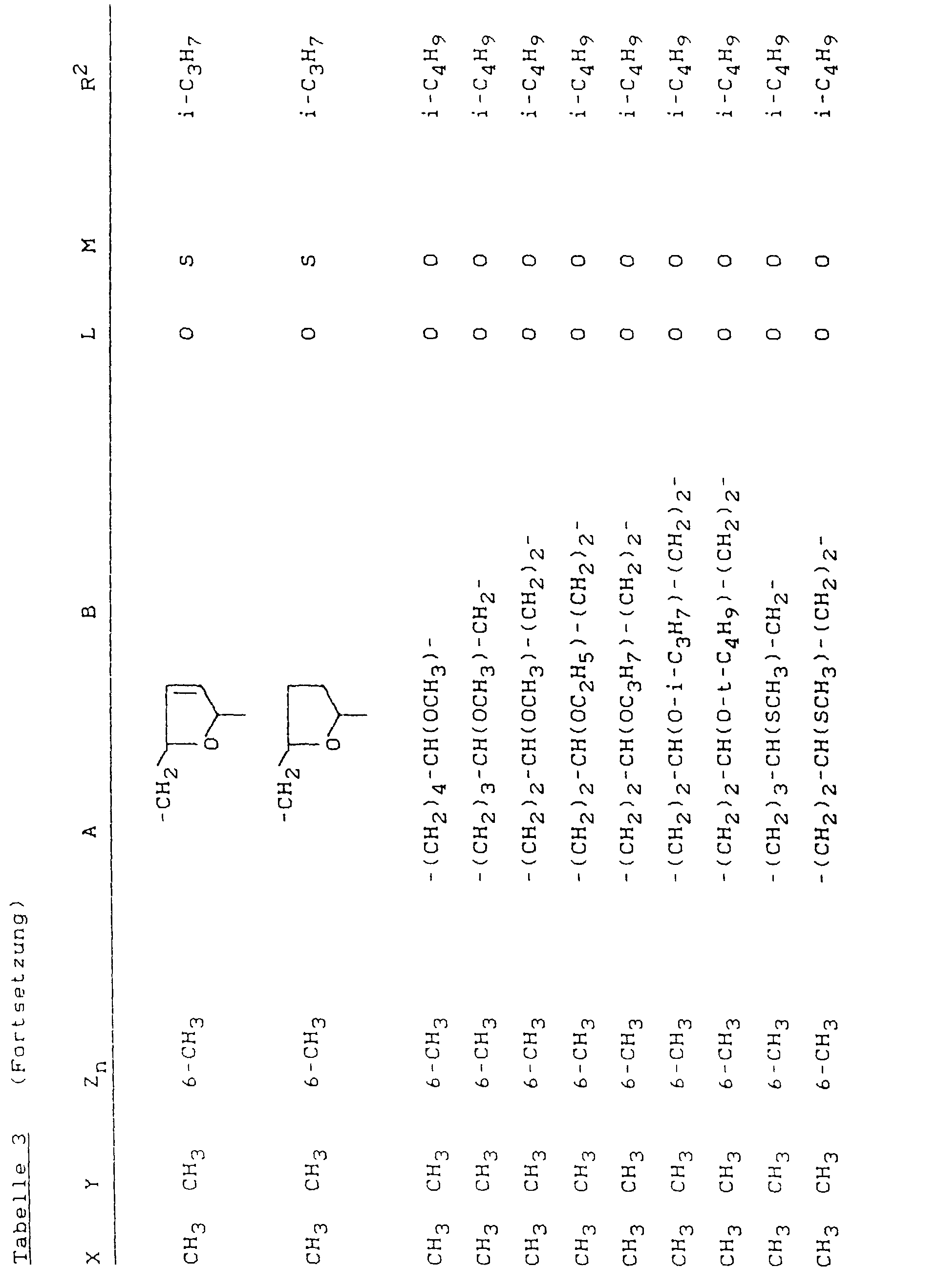

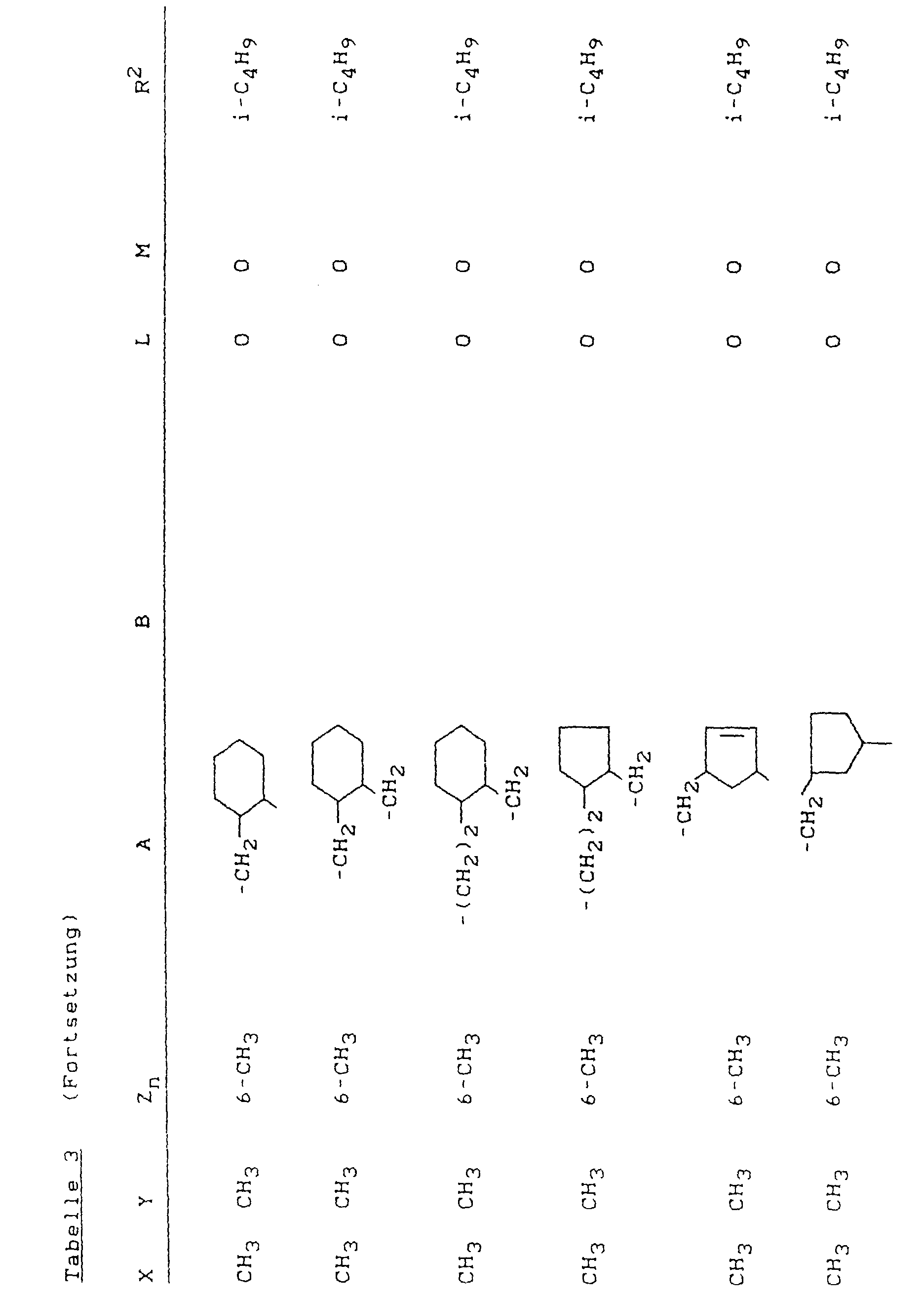

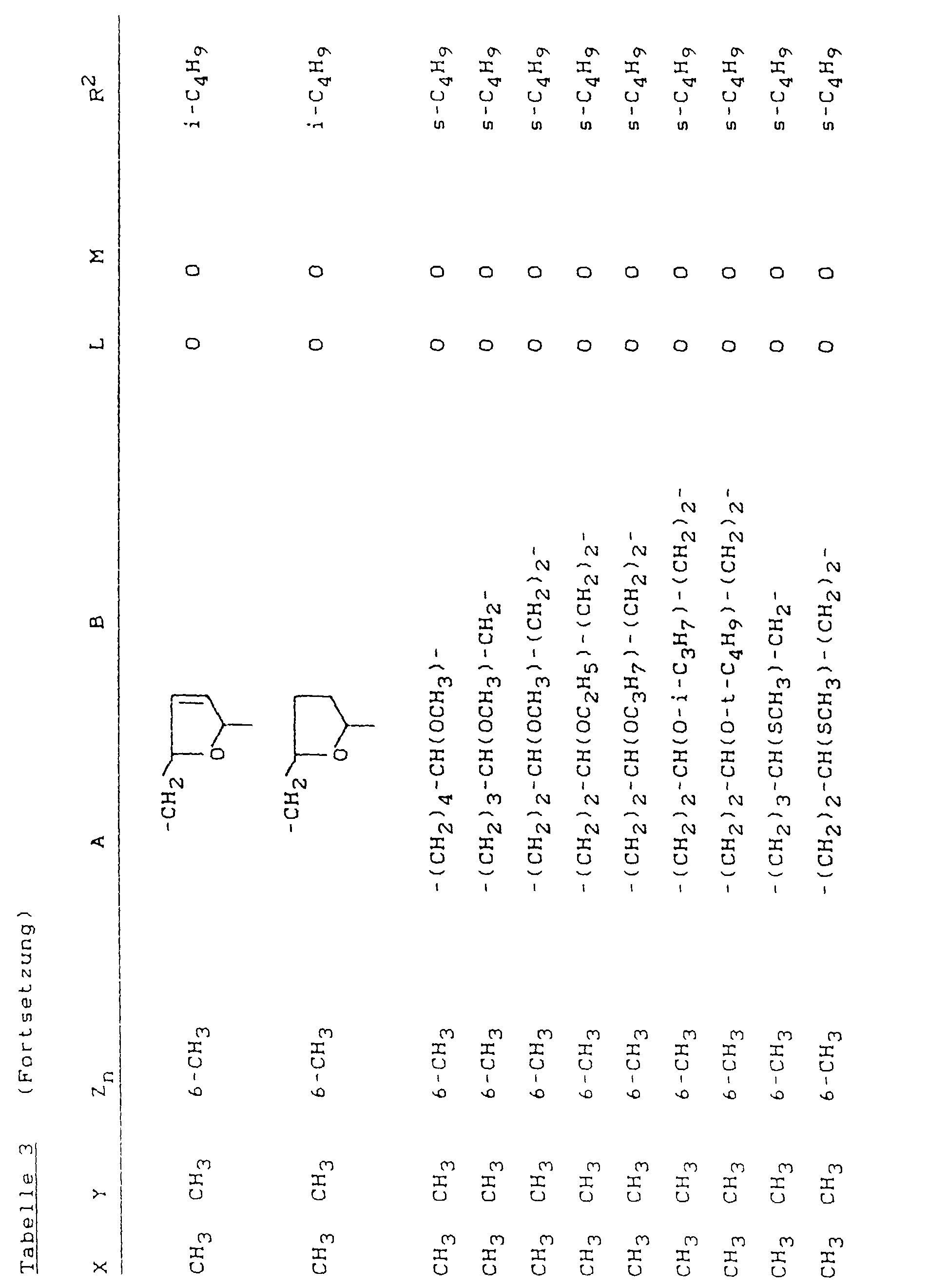

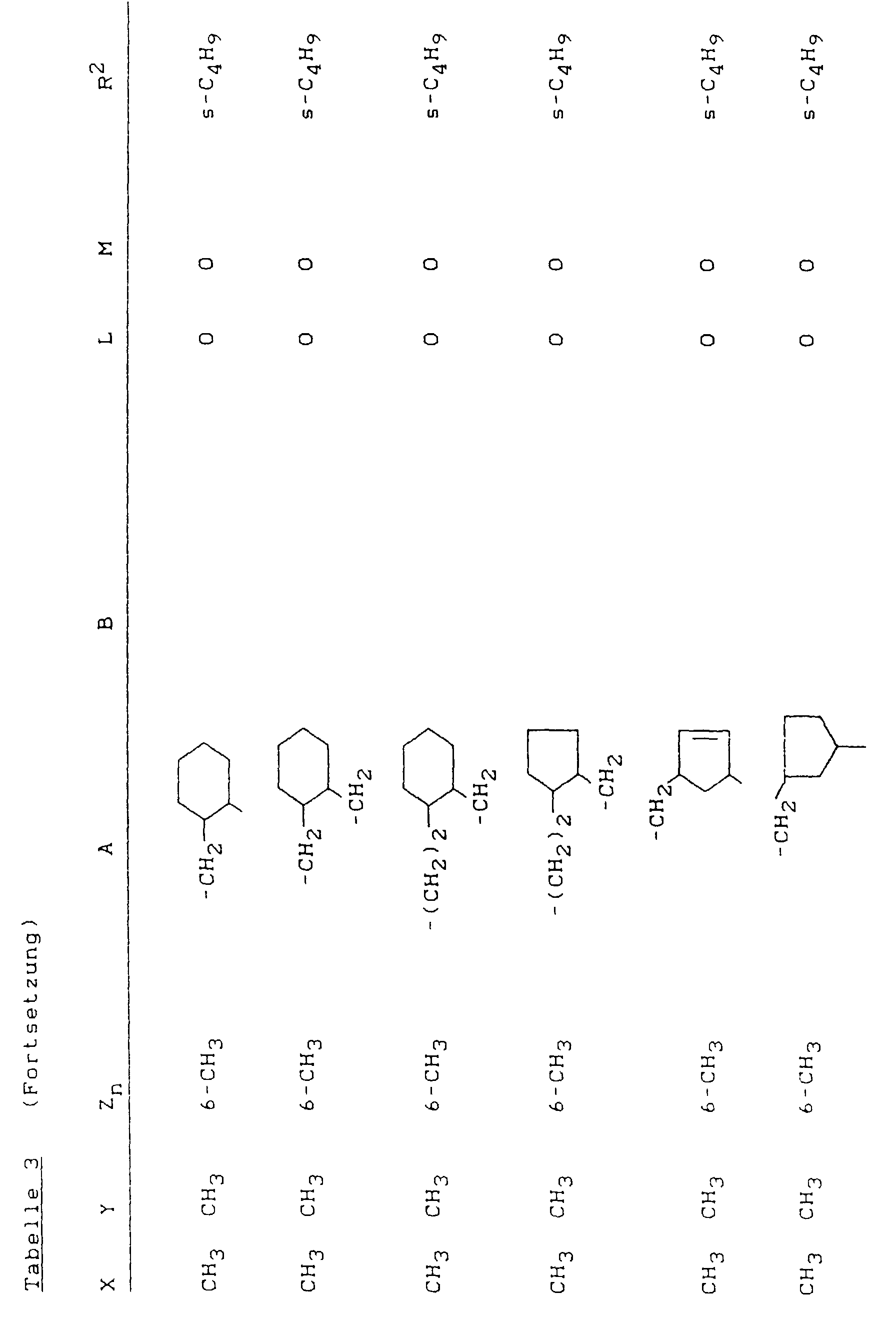

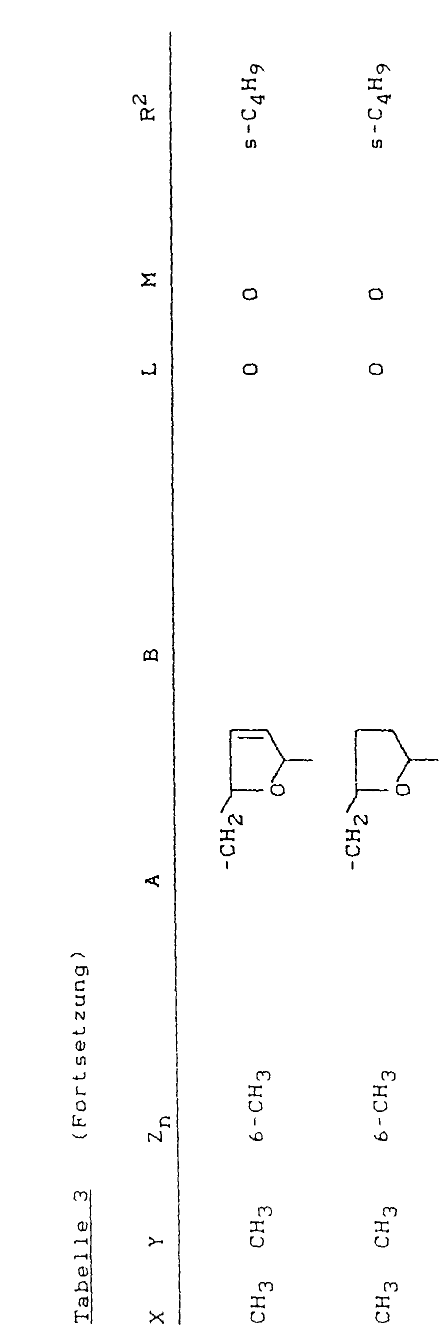

Im einzelnen seien außer den bei den Herstellungsbeispielen genannten Verbindungen die folgenden 3-Aryl-4-hydroxy-Δ³-dihydrofuran-Derivate der Formel (Ig) genannt:

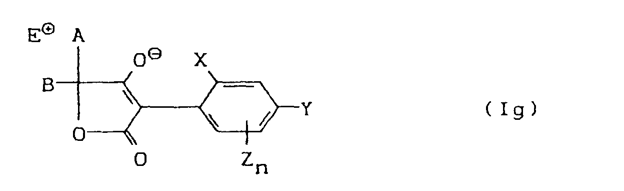

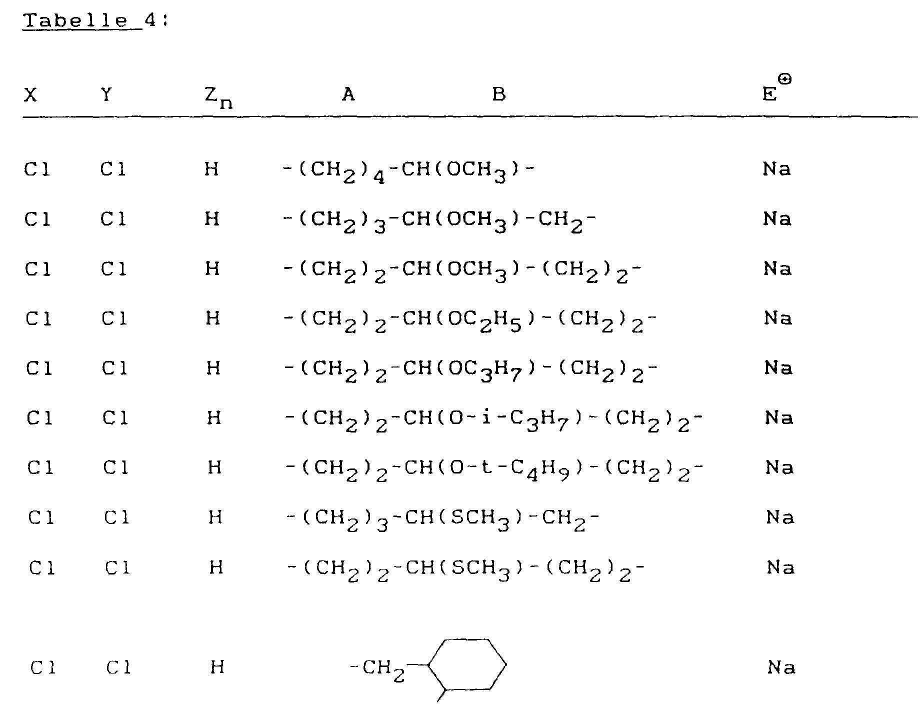

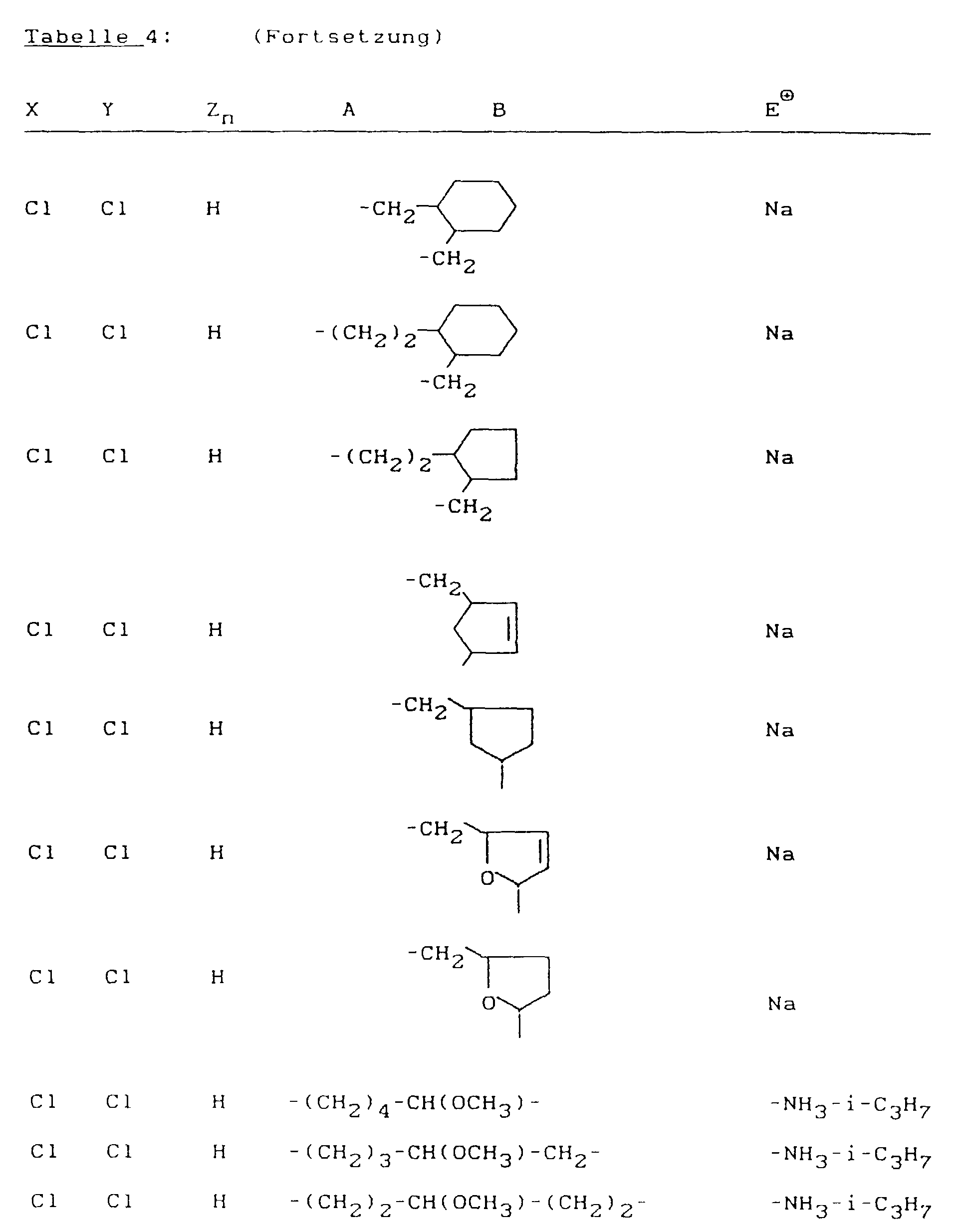

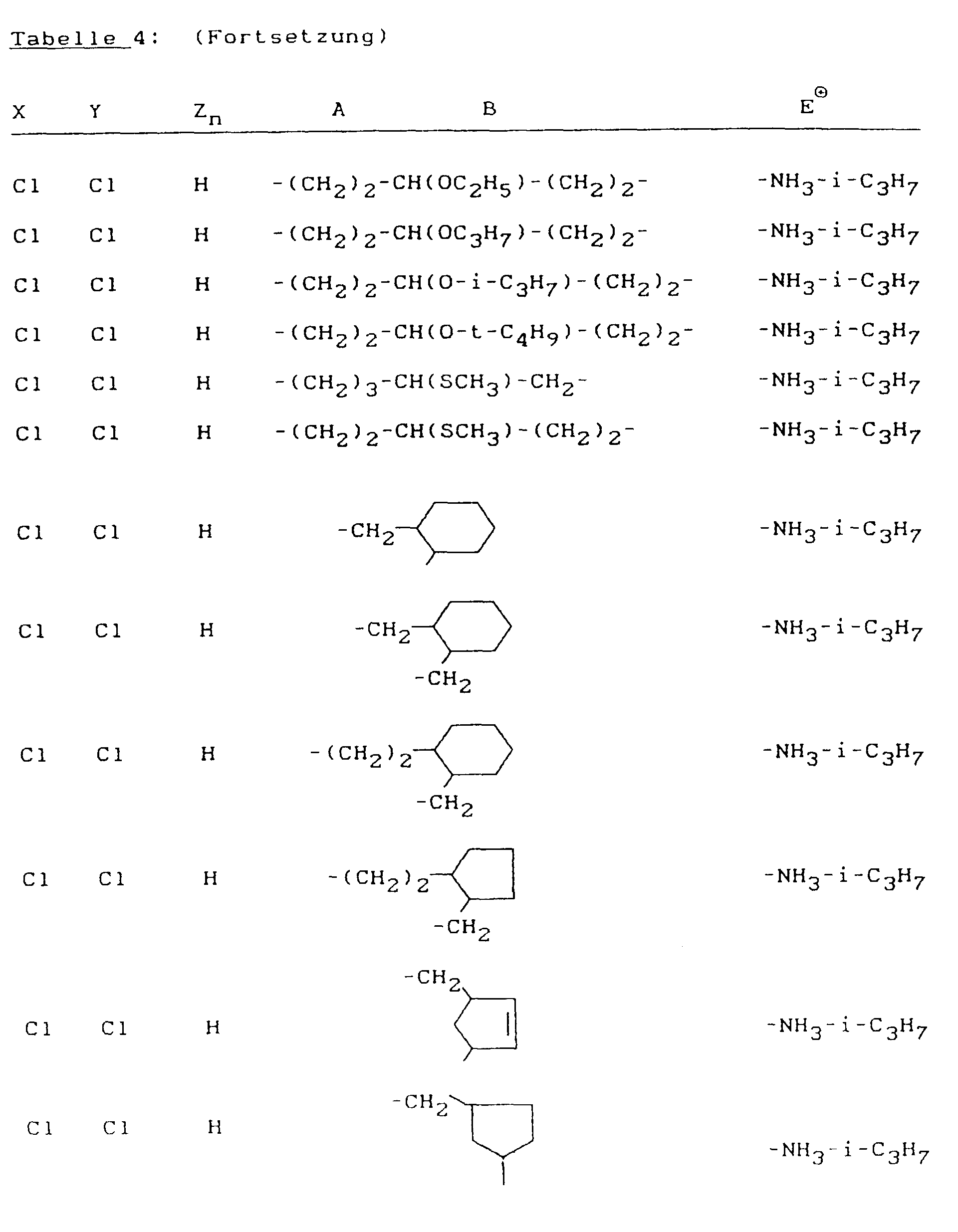

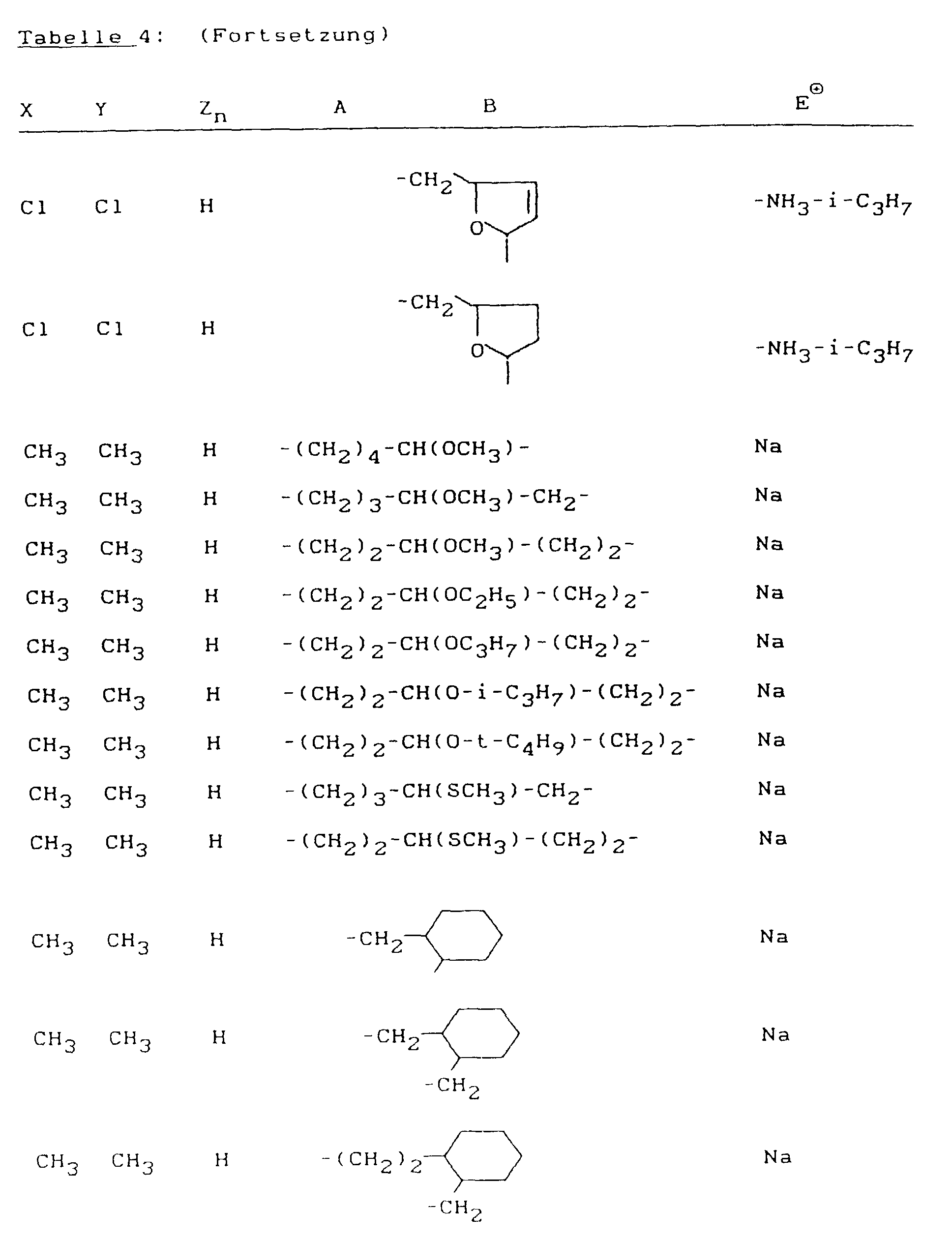

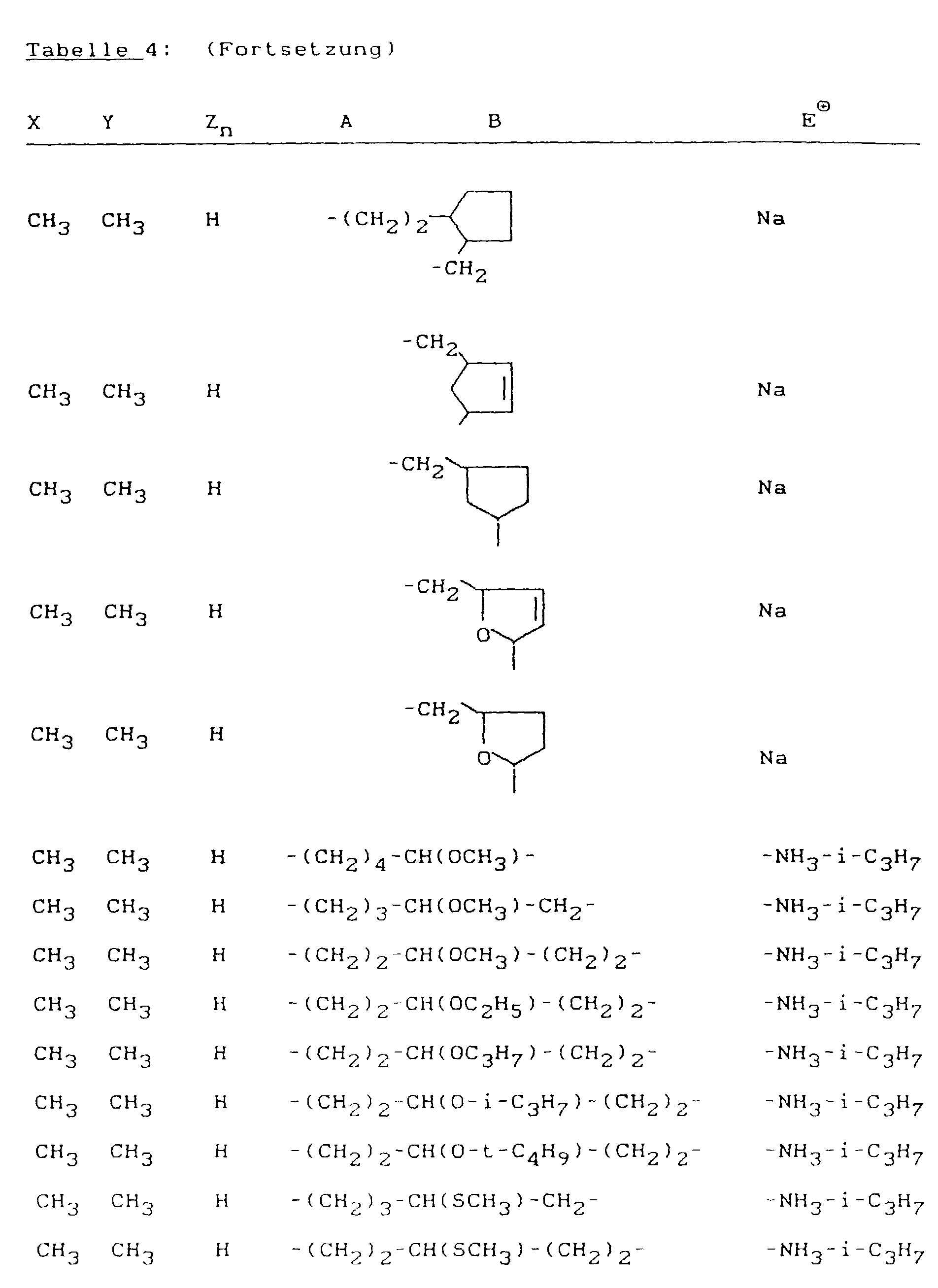

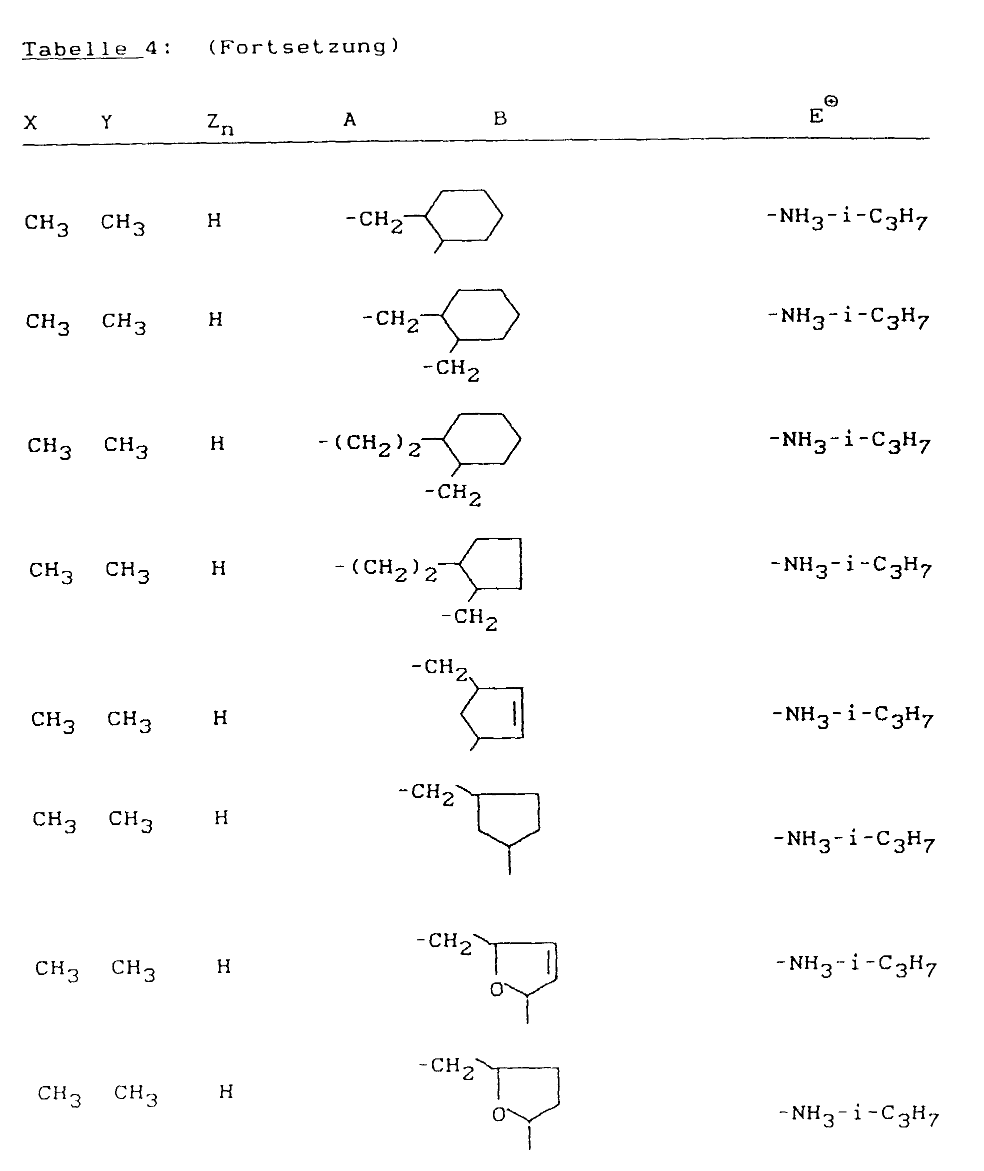

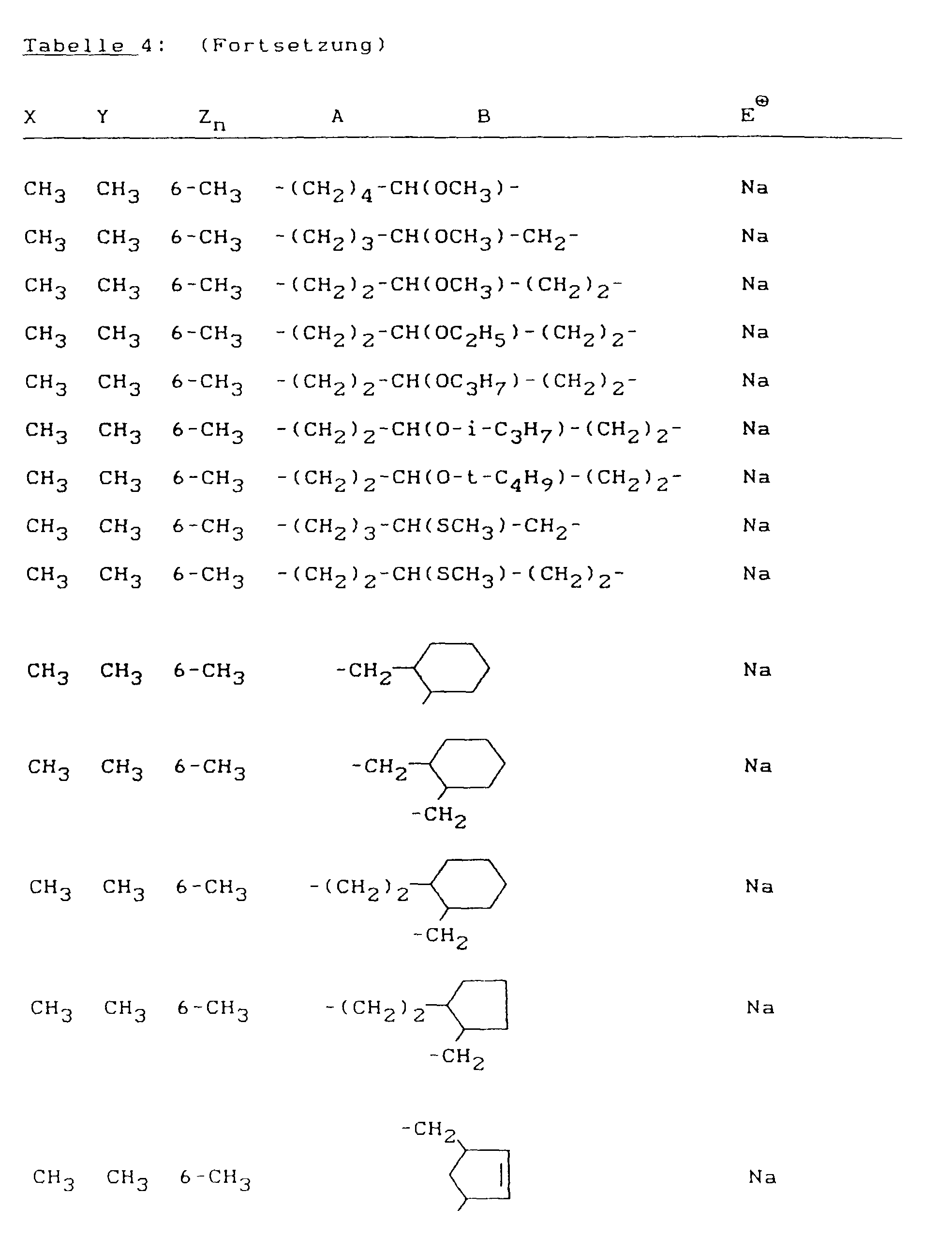

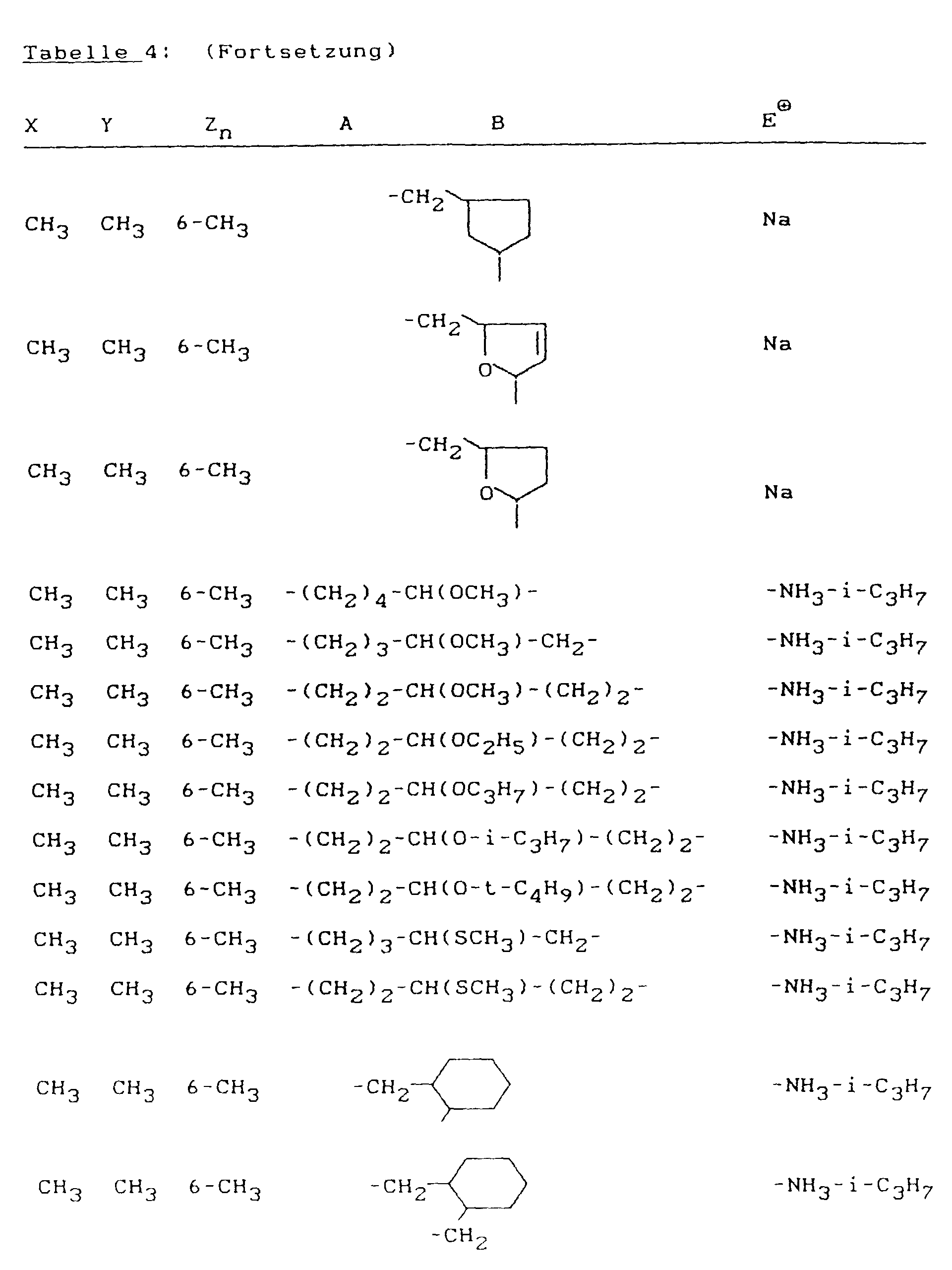

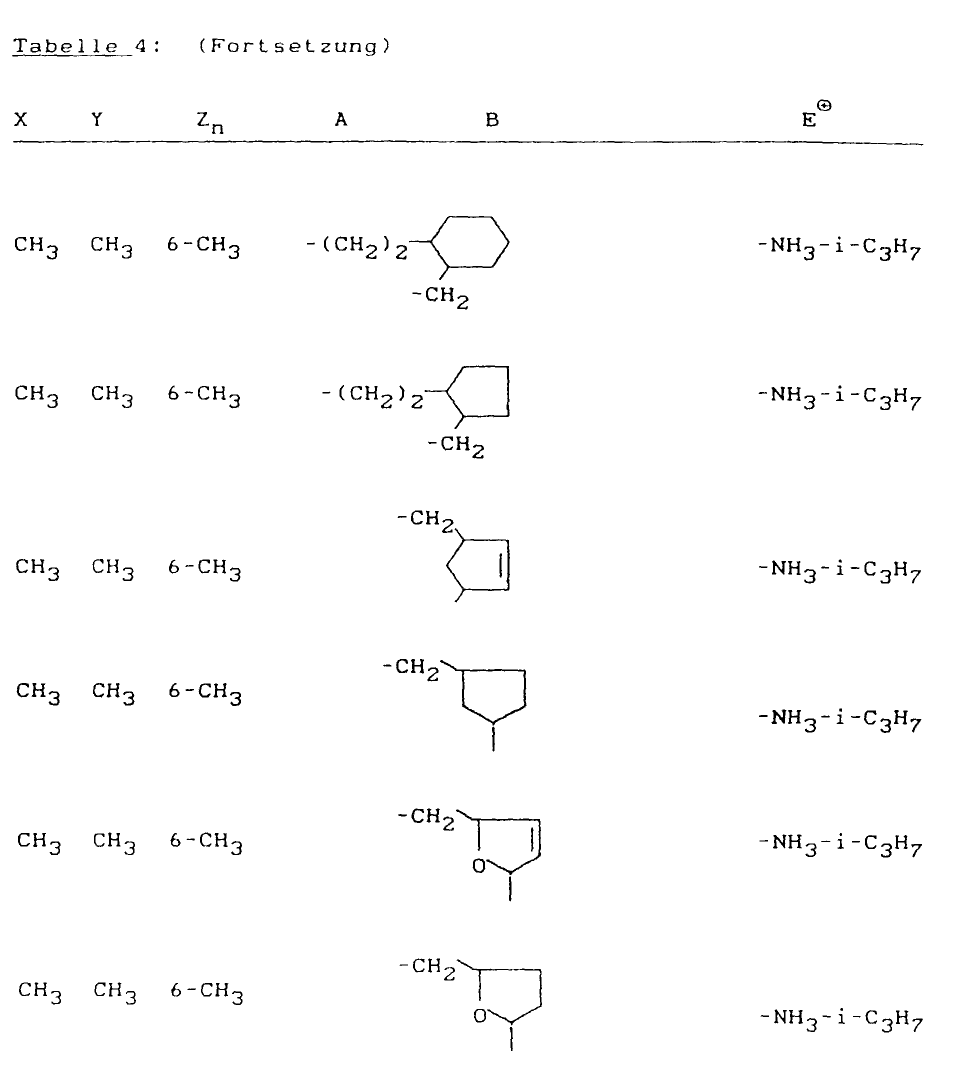

Verwendet man gemäß Verfahren (A) 1-(2,6-Dichlorphenylacetyloxy)-4-isopropoxy-cyclohexancarbonsäureethylester, so kann der Verlauf des erfindungsgemäßen Verfahrens durch folgendes Reaktionsschema wiedergegeben werden:

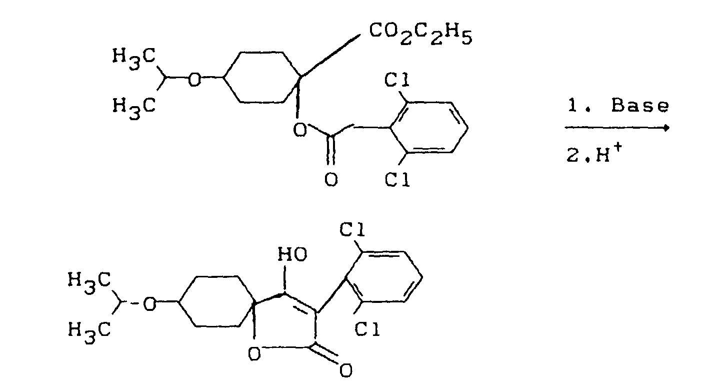

Verwendet man gemäß Verfahren (B) (Variante α) 3-(2,4,6 Trimethylphenyl)-4-hydroxy-5,5-(3-ethoxy)-pentamethylen-Δ³-dihydrofuran-2-on und Pivaloylchlorid als Ausgangsstoff, so kann der Verlauf des erfindungsgemäßen Verfahrens durch folgendes Reaktionsschema wiedergegeben werden.

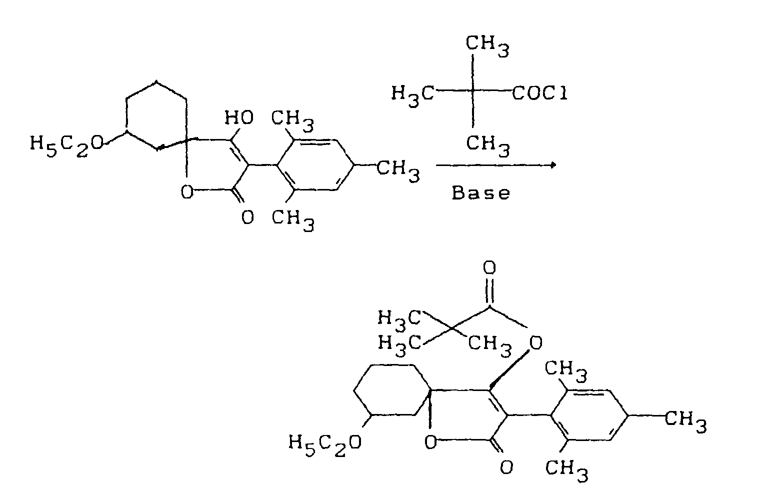

Verwendet man gemäß Verfahren B (Variante β) 3-(2,4,5-Trimethylphenyl)-4-hydroxy-5,5-(3-methoxy)-tetramethylen-Δ³-dihydrofuran-2-on und Acetanhydrid als Ausgangsverbindungen, so kann der Verlauf des erfindungsgemäßen Verfahrens durch folgendes Reaktionsschema wiedergegeben werden.

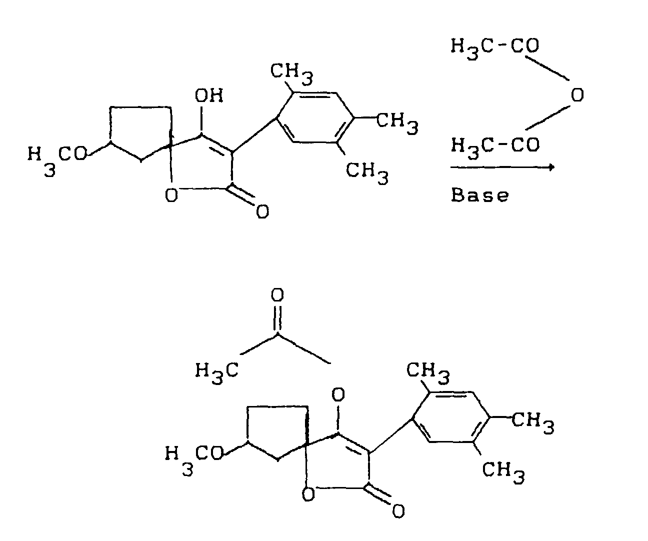

Verwendet man gemäß Verfahren C 3-(2,4-Dichlorphenyl)-4-hydroxy-5,5-(3-propoxy)-pentamethylen-Δ³-dihydrofuran-2-on und Chlorameisensäureethoxyethylester als Ausgangsverbindungen, so kann der Verlauf des erfindungsgemäßen Verfahrens durch folgendes Reaktionsschema wiedergegeben werden.

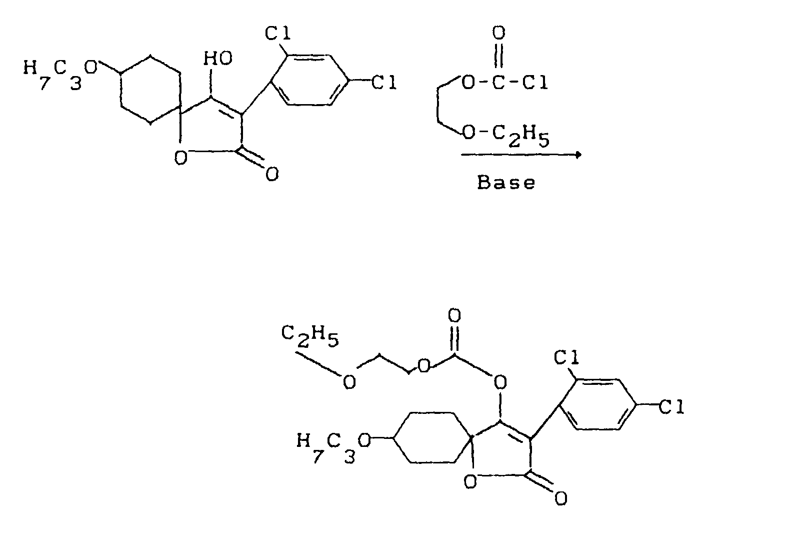

Verwendet man gemäß Verfahren (D_{α}) 3-(2,4,6-Trimethylphenyl)-4-hydroxy-5,5-(4-tert.-butoxy)-pentamethylen-Δ³-dihydrofuran-2-on und Chlormonothioameisensäuremethylester als Ausgangsprodukte, so kann der Reaktionsverlauf wie folgt wiedergegeben werden:

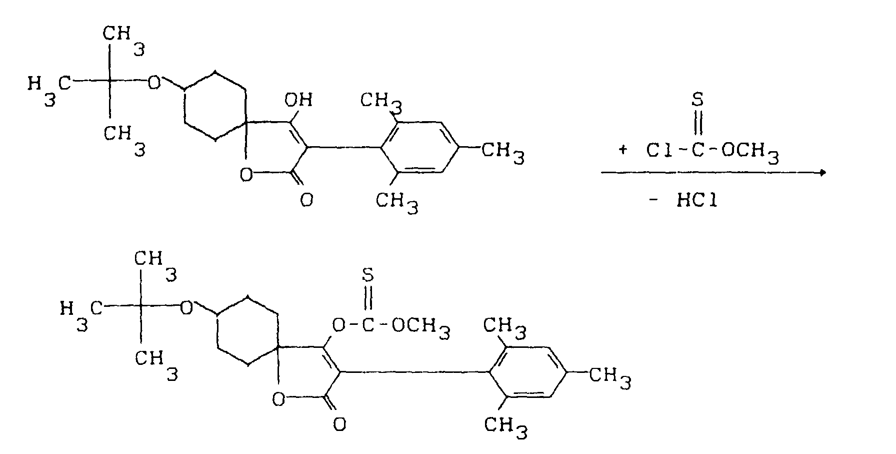

Verwendet man gemäß Verfahren (E) 3-(2,4,6-Trimethylphenyl)-4-hydroxy-5,5-(2,3-tetramethylen)-trimethylen-Δ³-dihydrofuran-2-on und Methansulfonsäurechlorid als Ausgangsprodukt, so kann der Reaktionsverlauf durch folgendes Reaktionsschema wiedergegeben werden:

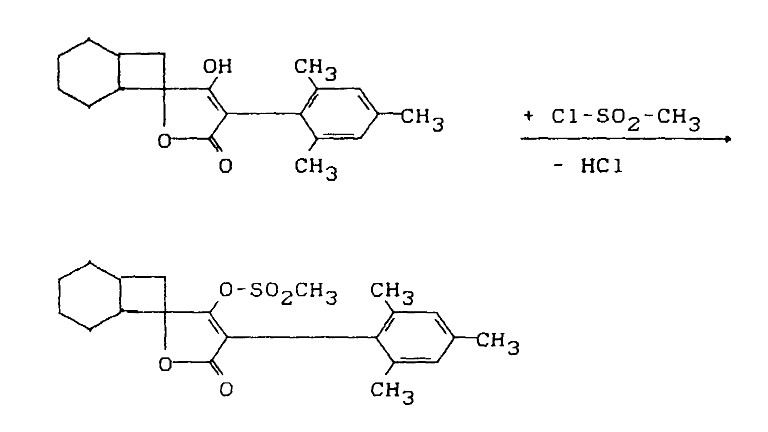

Verwendet man gemäß Verfahren (F) 3-(2,4,6-Trimethylphenyl)-4-hydroxy-5,5-(2,5-oxy)-pentamethylen-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

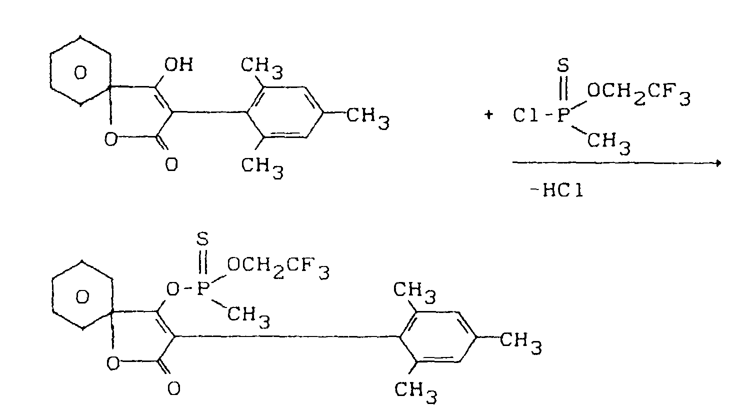

Verwendet man gemäß Verfahren (G_{α}) 3-(2,4,6-Trimethylphenyl)-4-hydroxy-5,5-(3,4-tetramethylen)-tetramethylen-Δ³-dihydrofuran-2-on und Ethylisocyanat als Ausgangsprodukte, so kann der Reaktionsverlauf durch folgendes Reaktionsschema wiedergegeben werden:

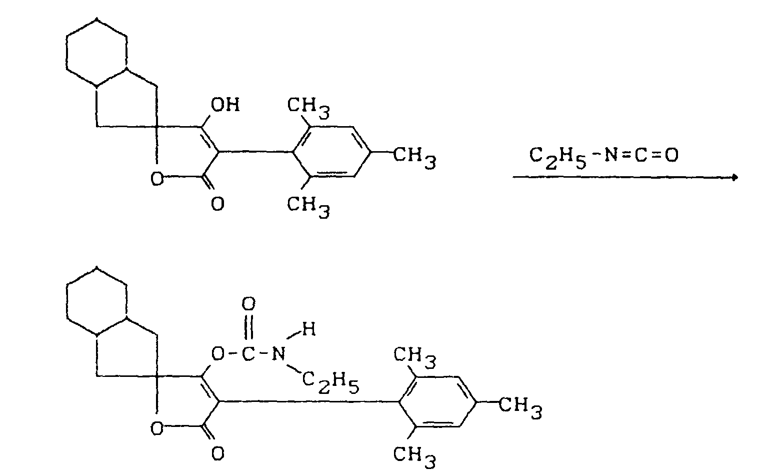

Verwendet man gemäß Verfahren (G_{β}) 3-(2,4,6-Trimethylphenyl)-4-hydroxy-5,5-(3-methylmercapto)-pentamethylen-Δ³-dihydrofuran-2-on und Dimethylcarbamidsäurechlorid als Ausgangsprodukte, so kann der Reaktionsverlauf durch folgendes Schema wiedergegeben werden:

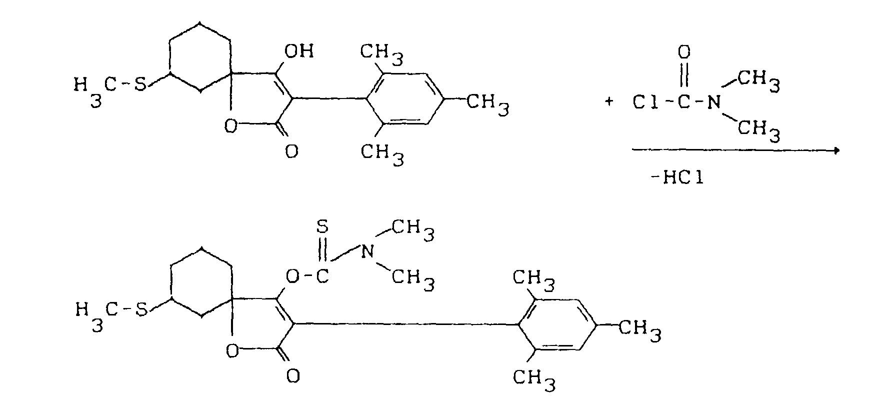

Verwendet man gemäß Verfahren (H) 3-(2,4,6-Trimethylphenyl)-4-hydroxy-5,5-(2-methoxy)-pentamethylen-Δ³-dihydrofuran-2-on und NaOH als Komponenten, so kann der Verlauf des erfindungsgemäßen Verfahrens durch folgendes Reaktionsschema wiedergegeben werden:

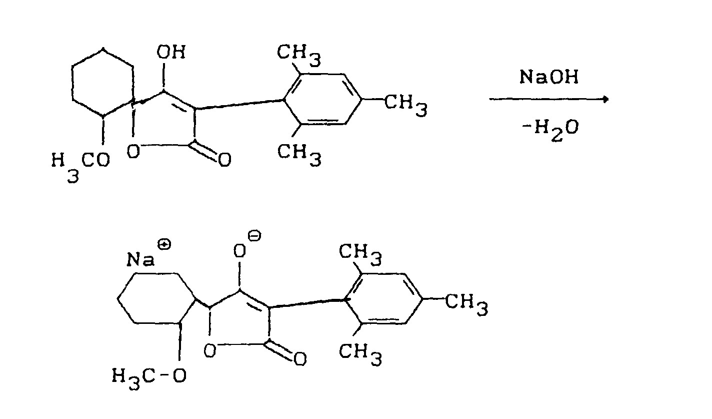

Die bei dem obigen Verfahren (A) als Ausgangsstoffe benötigten Verbindungen der Formel (II) 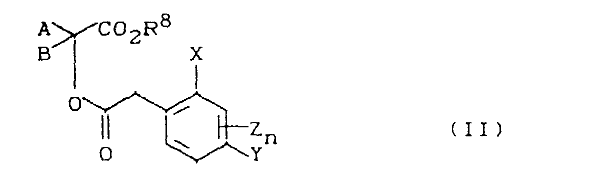 in welcher
A, B, X, Y, Z, n und R⁸ die oben angegebene Bedeutung haben sind neu, lassen sich aber nach im Prinzip bekannten Methoden in einfacher Weise herstellen. So erhält man z.B. O-Acyl-α-hydroxycarbonsäureester der Formel (II), wenn man
a) 2-Hydroxycarbonsäure-(ester) der Formel (XIII) 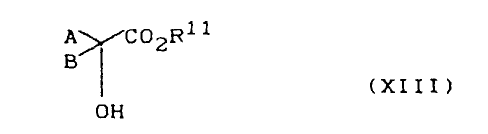 in welcher
   - R¹¹: für Wasserstoff (XIIIa) oder Alkyl (XIIIb) steht
   und
   A und B die oben angegebene Bedeutung haben, mit Phenylessigsäurehalogeniden der Formel (XIV) 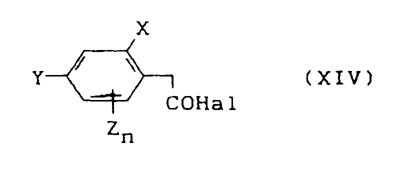
   in welcher
   - X, Y, Z und n: die oben angegebene Bedeutung haben und
   - Hal: für Chlor oder Brom steht, acycliert (Chem. Reviews 52 237-416 (1953));
   oder wenn man Hydroxycarbonsäuren der Formel (IIa), 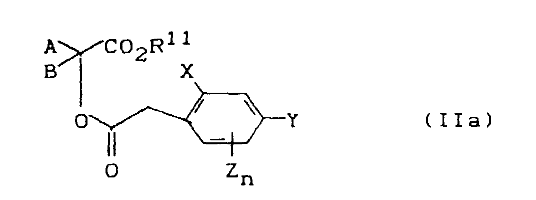
   in welcher
   - A, B, X, Y, Z und n: die oben angegebene Bedeutung haben
   und
   - R¹¹: für Wasserstoff steht,
   verestert (Chem. Ind. (London) 1568 (1968).

Verbindungen der Formel (IIa) sind beispielsweise aus den Phenylessigsäurehalogeniden der Formel (XIII) und Hydroxycarbonsäuren der Formel XIIIa) erhältlich (Chem. Reviews 52 237-416 (1953).

Beispielhaft seien folgende Verbindungen der Formel II genannt:
1-(2,4-Dichlorphenyl-acetyloxy)-2-methoxy-cyclohexan-carbonsäureethylester
1-(2,6-Dichlorphenyl-acetyloxy)-2-methoxy-cyclohexancarbonsäureethylester
1-(2-Chlor-6-fluorphenyl-acetyloxy)-2-methoxy-cyclohexancarbonsäureethylester
1-(2,4-Dimethylphenyl-acetyloxy)-2-methoxy-cyclohexancarbonsäureethylester
1-(2,6-Dimethylphenyl-acetyloxy)-2-methoxy-cyclohexancarbonsäureethylester
1-(2,4,6-Trimethylphenyl-acetoxy)-2-methoxy-cyclohexan carbonsäureethylester
1-(2,6-Dichlor-4-trifluormethylphenyl-acetoxy)-2-methoxy-cyclohexancarbonsäureethylester
1-(2,4-Dichlorphenyl-acetyloxy)-3-methoxy-cyclohexancarbonsäureethylester
1-(2,6-Dichlorphenyl-acetyloxy)-3-methoxy-cyclohexancarbonsäureethylester
1-(2-Chlor-6-fluorphenyl-acetyloxy)-3-methoxy-cyclohexancarbonsäureethylester
1-(2,4-Dimethylphenyl-acetyloxy)-3-methoxy-cyclohexancarbonsäureethylester
1-(2,6-Dimethylphenyl-acetyloxy)-3-methoxy-cyclohexancarbonsäureethylester
1-(2,4,6-Trimethylphenyl-acetoxy)-3-methoxy-cyclohexan carbonsäureethylester
1-(2,6-Dichlor-4-trifluormethylphenyl-acetoxy)-3-methoxy-cyclohexancarbonsäureethylester
1-(2,4-Dichlorphenyl-acetyloxy)-4-methoxy-cyclohexancarbonsäureethylester
1-(2,6-Dichlorphenyl-acetyloxy)-4-methoxy-cyclohexancarbonsäureethylester
1-(2-Chlor-6-fluorphenyl-acetyloxy)-4-methoxy-cyclohexancarbonsäureethylester
1-(2,4-Dimethylphenyl-acetyloxy)-4-methoxy-cyclohexancarbonsäureethylester
1-(2,6-Dimethylphenyl-acetyloxy)-4-methoxy-cyclohexancarbonsäureethylester
1-(2,4,6-Trimethylphenyl-acetoxy)-4-methoxy-cyclohexan carbonsäureethylester
1-(2,6-Dichlor-4-trifluormethylphenyl-acetoxy)-4-methoxy-cyclohexancarbonsäureethylester
1-(2,4-Dichlorphenyl-acetyloxy)-4-ethoxy-cyclohexancarbonsäureethylester
1-(2,6-Dichlorphenyl-acetyloxy)-4-ethoxy-cyclohexancarbonsäureethylester
1-(2-Chlor-6-fluorphenyl-acetyloxy)-4-ethoxy-cyclohexancarbonsäureethylester
1-(2,4-Dimethylphenyl-acetyloxy)-4-ethoxy-cyclohexancarbonsäureethylester
1-(2,6-Dimethylphenyl-acetyloxy)-4-ethoxy-cyclohexancarbonsäureethylester
1-(2,4,6-Trimethylphenyl-acetoxy)-4-ethoxy-cyclohexan carbonsäureethylester
1-(2,6-Dichlor-4-trifluormethylphenyl-acetoxy)-4-ethoxycyclohexancarbonsäureethylester
1-(2,4-Dichlorphenyl-acetyloxy)-4-isopropoxy-cyclohexancarbonsäureethylester
1-(2,6-Dichlorphenyl-acetyloxy)-4-isopropoxy-cyclohexancarbonsäureethylester
1-(2-Chlor-6-fluorphenyl-acetyloxy)-4-isopropoxy-cyclohexancarbonsäureethylester
1-(2,4-Dimethylphenyl-acetyloxy)-4-isopropoxy-cyclohexancarbonsäureethylester
1-(2,6-Dimethylphenyl-acetyloxy)-4-isopropoxy-cyclohexancarbonsäureethylester
1-(2,4,6-Trimethylphenyl-acetoxy)-4-isopropoxy-cyclohexan-carbonsäureethylester
1-(2,6-Dichlor-4-trifluormethylphenyl-acetoxy)-4-isopropoxy-cyclohexancarbonsäureethylester
1-(2,4-Dichlorphenyl-acetyloxy)-4-t-butoxy-cyclohexancarbonsäureethylester
1-(2,6-Dichlorphenyl-acetyloxy)-4-t-butoxy-cyclohexancarbonsäureethylester
1-(2-Chlor-6-fluorphenyl-acetyloxy)-4-t-butoxy-cyclohexancarbonsäureethylester
1-(2,4-Dimethylphenyl-acetyloxy)-4-t-butoxy-cyclohexancarbonsäureethylester
1-(2,6-Dimethylphenyl-acetyloxy)-4-t-butoxy-cyclohexancarbonsäureethylester
1-(2,4,6-Trimethylphenyl-acetoxy)-4-t-butoxy-cyclohexan carbonsäureethylester
1-(2,6-Dichlor-4-trifluormethylphenyl-acetoxy)-4-t-butoxy-cyclohexancarbonsäureethylester
1-(2,4-Dichlorphenyl-acetyloxy)-3,4-trimethylen-cyclohexancarbonsäureethylester
1-(2,6-Dichlorphenyl-acetyloxy)-3,4-trimethylen-cyclohexancarbonsäureethylester
1-(2-Chlor-6-fluorphenyl-acetyloxy)-3,4-trimethylencyclohexancarbonsäureethylester
1-(2,4-Dimethylphenyl-acetyloxy)-3,4-trimethylen-cyclohexancarbonsäureethylester
1-(2,6-Dimethylphenyl-acetyloxy)-3,4-trimethylen-cyclohexancarbonsäureethylester
1-(2,4,6-Trimethylphenyl-acetoxy)-3,4-trimethylencyclohexan-carbonsäureethylester
1-(2,6-Dichlor-4-trifluormethylphenyl-acetoxy)-3,4-trimehylen-cyclohexancarbonsäureethylester
1-(2,4-Dichlorphenyl-acetyloxy)-2,5-methylen-cyclohexancarbonsäureethylester
1-(2,6-Dichlorphenyl-acetyloxy)-2,5-methylen-cyclohexancarbonsäureethylester
1-(2-Chlor-6-fluorphenyl-acetyloxy)-2,5-methylen-cyclohexancarbonsäureethylester
1-(2,4-Dimethylphenyl-acetyloxy)-2,5-methylen-cyclohexancarbonsäureethylester
1-(2,6-Dimethylphenyl-acetyloxy)-2,5-methylen-cyclohexancarbonsäureethylester
1-(2,4,6-Trimethylphenyl-acetoxy)-2,5-methylen-cyclohexancarbonsäureethylester
1-(2,6-Dichlor-4-trifluormethylphenyl-acetoxy)-2,5-methylen-cyclohexancarbonsäureethylester

Das Verfahren (A) ist dadurch gekennzeichnet, daß Verbindungen der Formel (II) in welcher A, B, X, Y, Z, n und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Weiterhin können Alkalimetalle wie Natrium oder Kalium verwendet werden. Ferner sind Alkalimetall- und Erd-alkalimetallamide und -hydride, wie Natriumamid, Natriumhydrid und Calciumhydrid, und außerdem auch Alkalimetall-alkoholate, wie Natrium-methylat, Natriumethylat und Kalium-tert.-butylat einsetzbar.
Adogen 464 = Methyltrialkyl(C₈-C₁₀)ammoniumchlorid
TDA 1 = Tris-(methoxyethoxyethyl)-amin

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Uberschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hüning-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren D setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren E) setzt man pro Mol Ausgangsverbindung der Formel (Ia) ca. 1 Mol Sulfonsäurechlorid (VII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weitern Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (VIII) bei Temperaturen zwischen -40°C und 150°C, vorzugsweise zwischen -10 und 110°C.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren G_{α} setzt man pro Mol Ausgangsverbindung der Formel Ia ca. 1 Mol Isocyanat der Formel (IX) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren G_{β} setzt man pro Mol Ausgangsverbindung der Formel (Ia) ca. 1 Mol Carbamidsäurechlorid bzw. Thiocarbamidsäurechlorid der Formel (X) bei 0 bis 150°C, vorzugsweise bei 20 bis 70°C um.

Als gegebenenfalls zugesetzte Verdünnungsmittel kommen aller inerten polaren organischen Lösungsmittel in Frage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallverbindungen (XII) oder Aminen (XII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formel (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Die erfindungsgemäßen Verbindungen der Formel I können zur Schädlingsbekämpfung eingesetzt werden. Schädlinge sind unerwünschte tierische Schädlinge, insbesondere Insekten, Milben und Nematoden, welche Pflanzen oder höhere Tiere schädigen. Zu den Schädlingen gehören jedoch auch unerwünschte Pflanzen.

Die erfindungsgemäßen Wirkstoffe eignen sich bei guter Pflanzenverträglichkeit und günstiger Wärmeblütertoxizität zur Bekämpfung von tierischen Schädlingen, vorzugsweise von Arthropoden, insbesondere von Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp.,
Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp.,
Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Daneben besitzen die erfindungsgemäßen Wirkstoffe der Formel (I) auch eine gute fungizide Wirksamkeit und lassen sich zur Bekämpfung von Pflanzenkrankheiten wie beispielsweise gegen den Erreger der Reisfleckenkrankheit (Pyricularia oryzae) einsetzen.

Die erfindungsgemäßen Wirkstoffe können zur Verwendung als Insektizide, Akarizide und Nematizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Verbindungen eignen sich auch in besonderer Weise zur Behandlung von vegativem und generativem Vermehrungsmaterial, wie z.B. von Saatgut von Getreide, Mais, Gemüse u.s.w. oder von Zwiebeln, Stecklingen u.s.w.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe können auch als Herbizide, vorzugsweise als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in dikotylen Kulturen sowohl im Vorauflauf- als auch im Nachauflauf-Verfahren.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streu en.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Zur Herstellung der Schädlingsbekämpfungsmittel können die erfindungsgemäßen Wirkstoffe in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmneben-Formulierungen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B, gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Mittel enthalten bevorzugt neben wenigstens einer verbindung der allgmeeinen Formel (I) und gegebenenfalls neben erheblichen Streck- und Hilfsmitteln wenigstens einen oberflächenaktiven Stoff.

Die Herstellung der erfindungsgemäßen Verbindungen der allgemeinen Formel (I) soll durch die folgenden Herstellungsbeispiele und die biologische Wirksamkeit durch die folgenden biologischen Beispiele erläutert werden.

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. die Verbindungen der Herstellungsbeispiele 46, 47 und 65 bei einer beispielhaften Konzentration von 0,1 % nach 7 Tagen eine Abtötung von 100 %.

### Beispiel B

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die Verbindungen der Herstellungsbeispiele 38, 46, 52, 53, 61, 81 und 88 bei einer beispielhaften Wirkstoffkonzentration von 0,1 % nach 7 Tagen eine Abtötung von 100 %.

### Beispiel C

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigten z.B. die Verbindungen der Herstellungsbeispiele 3, 31, 32, 34, 35, 37, 51, 52, 56, 58, 62, 65, 68, 69, 71, 74, 84 und 85 bei einer beispielhaften Wirkstoffkonzentration von 0,1 % nach 6 Tagen eine Abtötung von 100 %.

### Beispiel D

### Aphis-Test (systemische Wirkung)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. die Verbindungen der Herstellungsbeispiele 32 und 38 bei einer beispielhaften Wirkstoffkonzentration von 0,1 % eine Abtötung von 100 %.

### Beispiel E

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. die Verbindungen der Herstellungsbeispiele 3, 31, 84, 85 und 88 bei einer beispielhaften Wirkstoffkonzentration von 0,01 % nach 7 Tagen eine Abtötung von 100 %.

### Beispiel E

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. die Verbindungen der Herstellungsbeispiele 3, 31, 84, 85 und 88 bei einer beispielhaften Wirkstoffkonzentration von 0,01 % nach 7 Tagen eine Abtötung von 100 %.

### Beispiel F

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Bei diesem Test wurden mit einer beispielhaften Aufwandmenge von 500 g/ha bei einer guten bis sehr guten Verträglichkeit durch Soja die folgenden Ergebnisse erhalten:

| Pflanze | % Wirkung | Verbindung des Herstellungsbeispieles Nr. |
|---|---|---|
| Digitaria | 90-100 | 31, 32, 33, 34, 35, 38, 46, 52, 53, 60, 62, 63, 68, 69, 81, 84, 88 |
| | | |
| Echinochloa | 70-100 | 31, 32, 33, 34, 34, 37, 46, 38, 52, 53, 56, 63, 68, 69, 81, 84 |
| | | |
| Panicum | 95-100 | 31, 32, 33, 34, 35, 37, 38, 46, 52, 53, 56, 60, 62, 63, 68, 69, 81, 84, 88 |
| | | |
| Setaria | 70-100 | 31, 32, 33, 34, 35, 37, 38, 52, 53, 56, 60, 63, 68, 69, 81, 84, 88 |

### Beispiel Ia-1

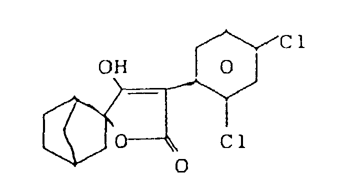

16,83 g (0,15 mol) Kalium-tert.-butylat werden in 100 ml abs. DMF vorgelegt, bei 0-10°C eine Lösung von 37,10 g (0,10 mol) 2-O-(2,4-Dichlorphenyl-acetyl)-norbornan-2-carbonsäureethylester in 100 ml abs. DMF zugetropft und 16 h bei Raumtemperatur gerührt.

Zur Aufarbeitung tropft man das Reaktionsgemisch in 500 ml 1N Salzsäure ein, saugt das ausgefallene Produkt ab und trocknet im Vakuumtrockenschrank.
Ausbeute: 29,79 g (92 % der Theorie) eines weißen Feststoffs vom Fp. 227°C.

In Analogie wurden die in Tabelle 5 beschriebenen Verbindungen hergestellt.

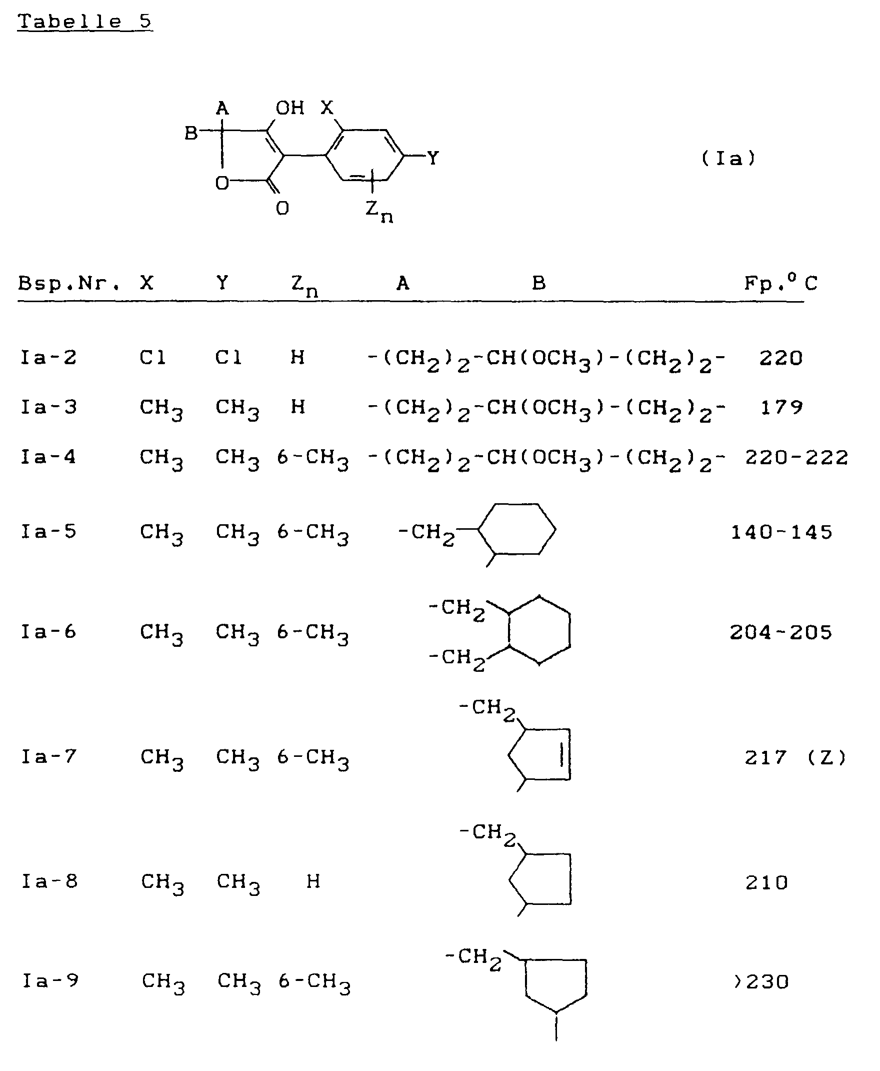

### Beispiel Ib-1

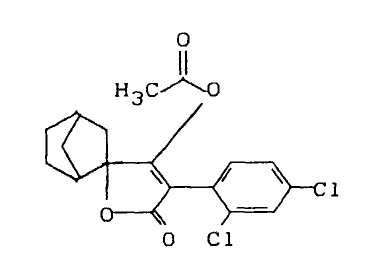

3,25 g (10 mmol) der Verbindung Ia-1 werden in 40 ml abs. Methylenchlorid vorgelegt, 1,42 g (14 mmol) Triethylamin und eine Spatelspitze DMAP zugegeben, eine Lösung von 0,94 g (12 mmol) Acetylchlorid in 20 ml Methylenchlorid zugetropft und 16 h bei Raumtemperatur gerührt.

Zur Aufarbeitung wäscht man das Reaktionsgemisch mit wäßriger Citronensäure, NaHCO₃-Lösung und NaCl-Lösung, trocknet und rotiert ein. Die weitere Reinigung erfolgt durch Flash-Chromatographie an Kieselgel mit Cyclohexan/Essigester 3:1.
Ausbeute: 1,70 g (46 % der Theorie) eines Feststoffs vom Fp. 126°C.

In Analogie wurden die in Tabelle 6 aufgeführten Verbindungen hergestellt.

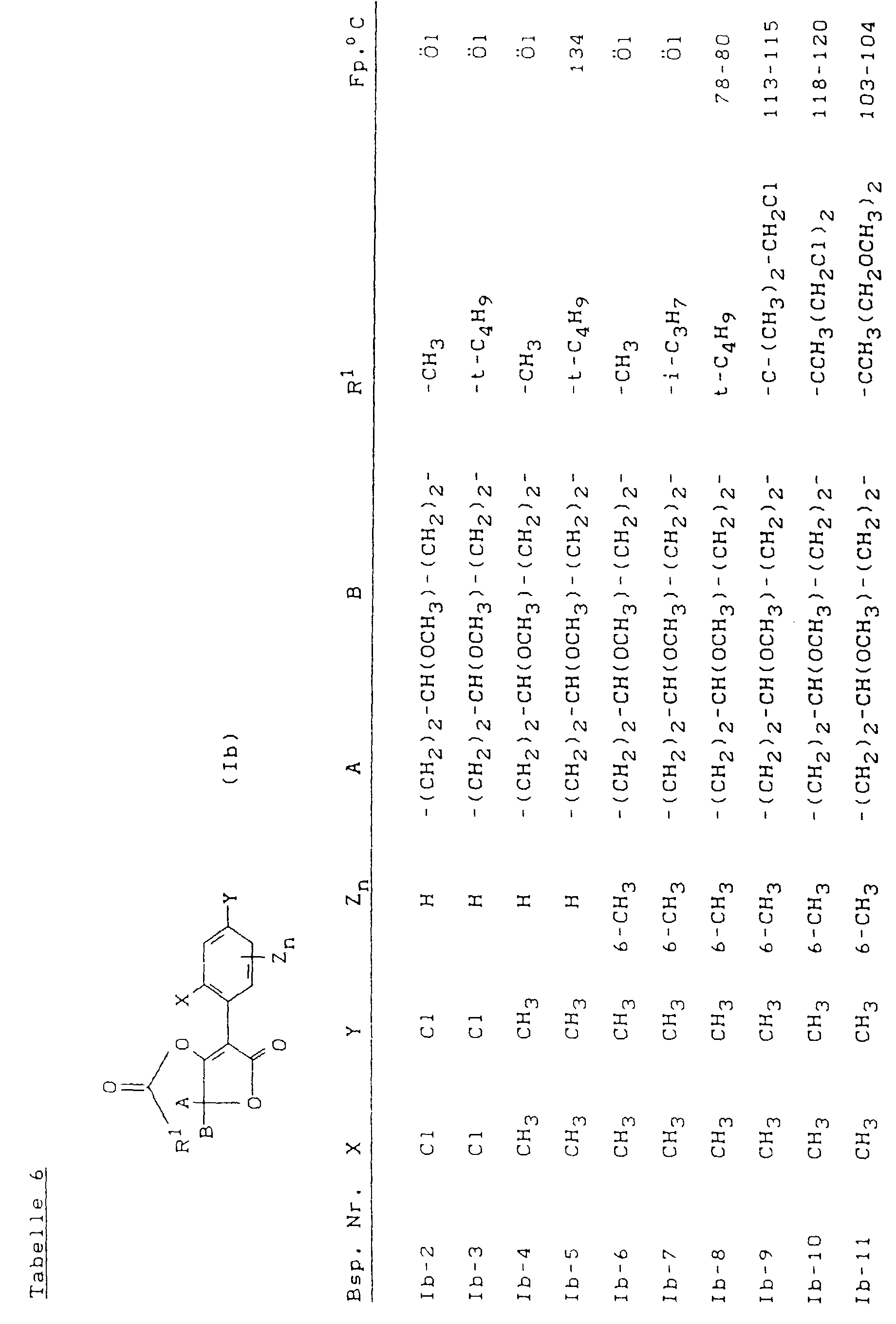

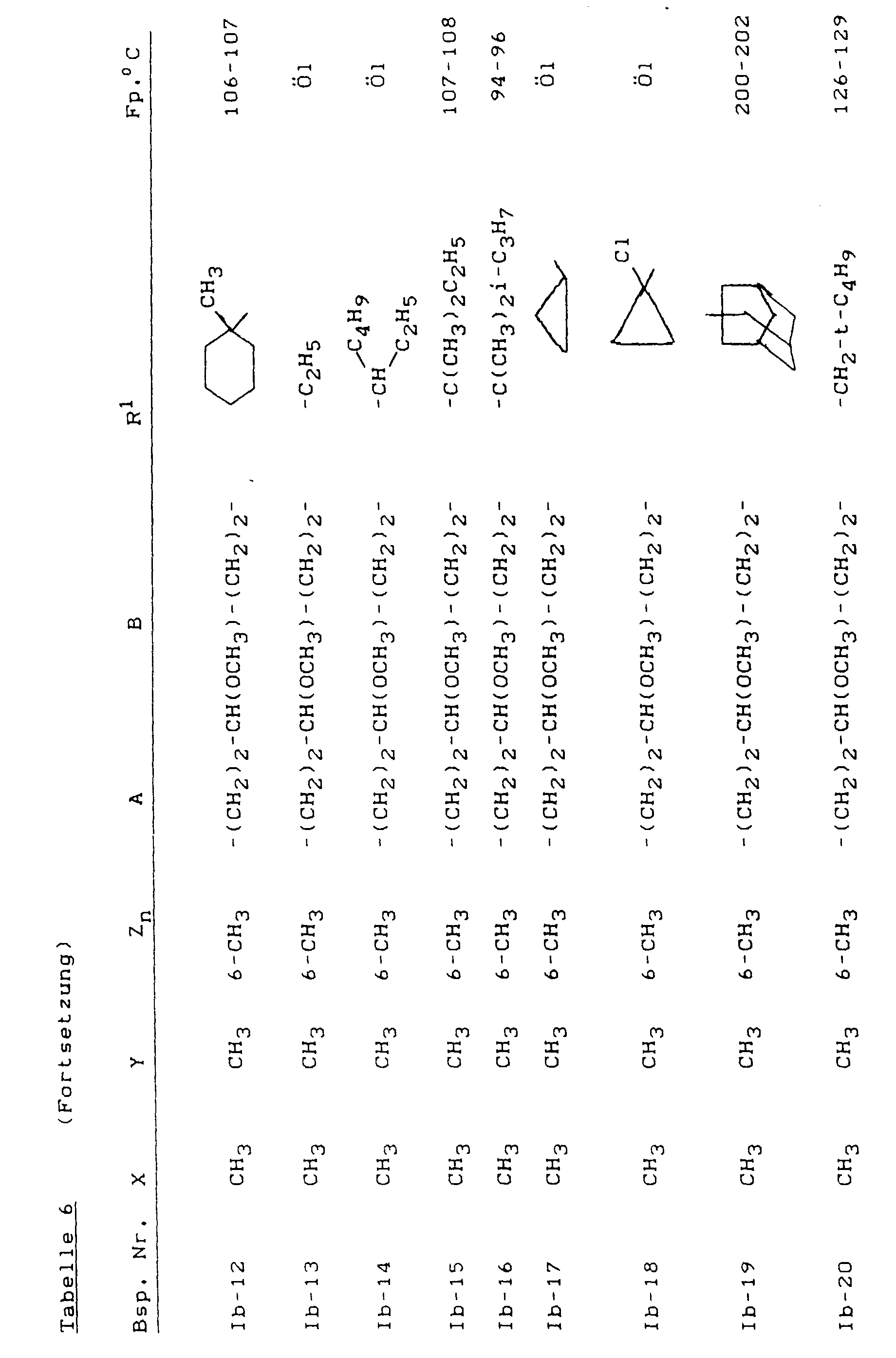

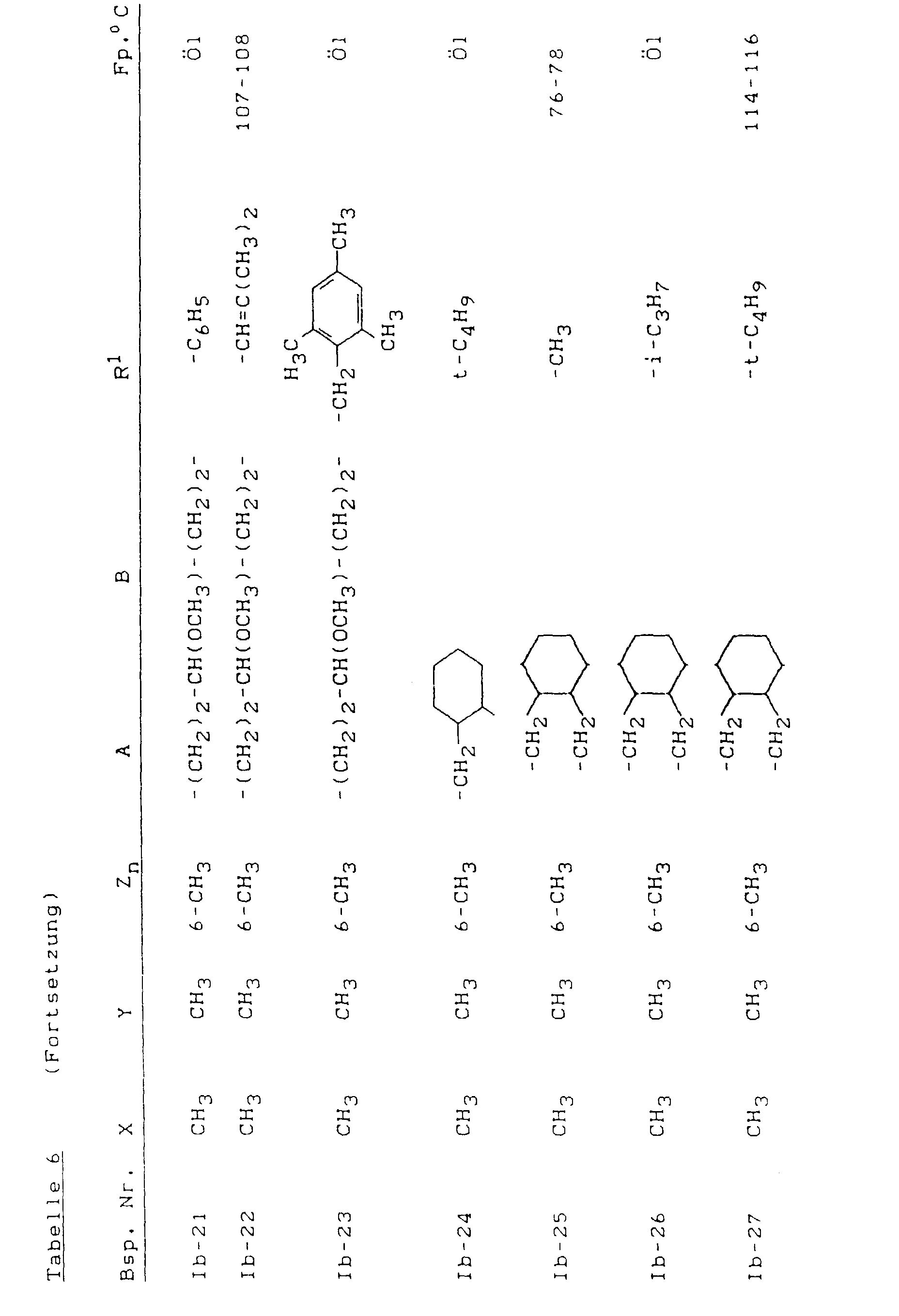

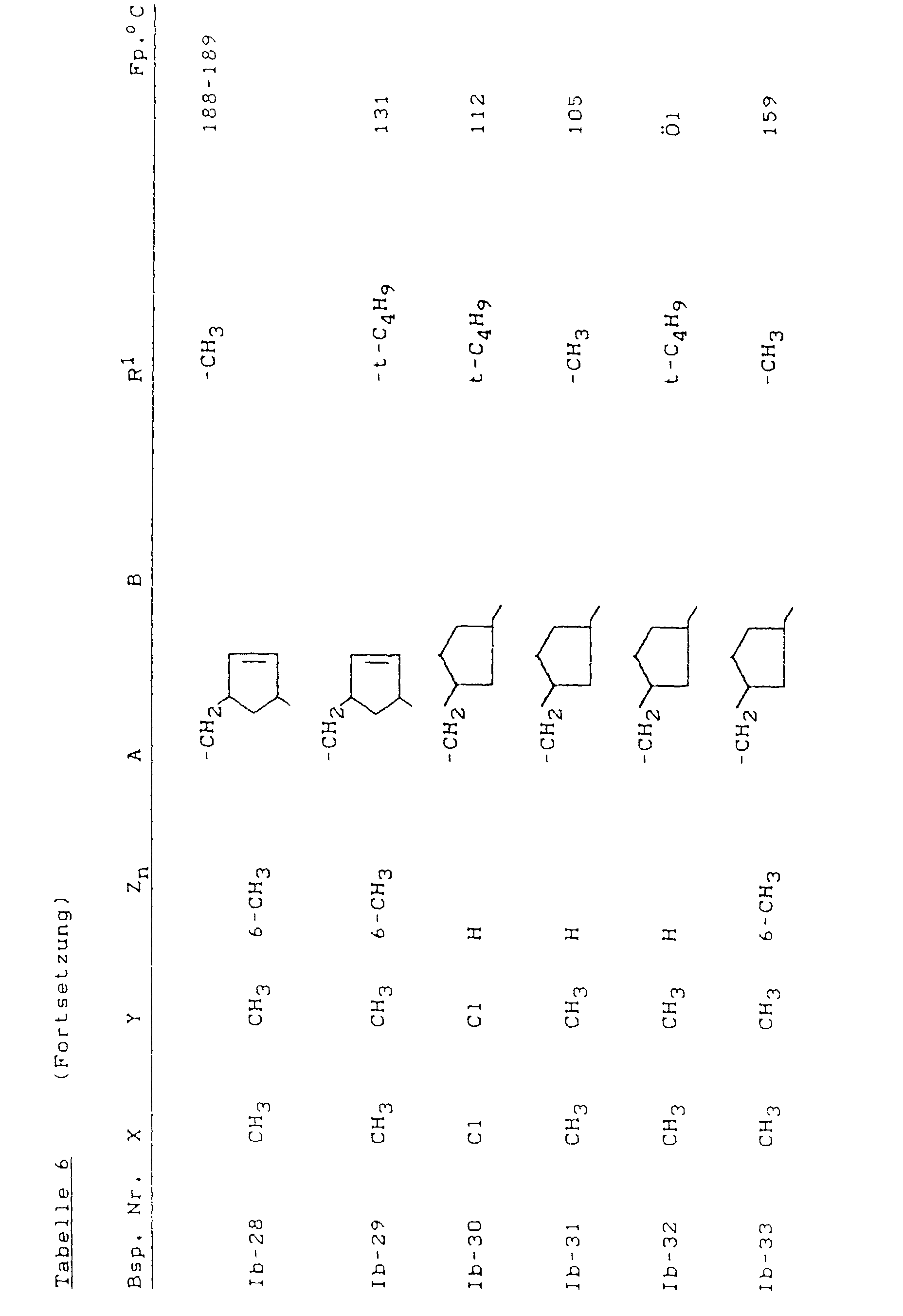

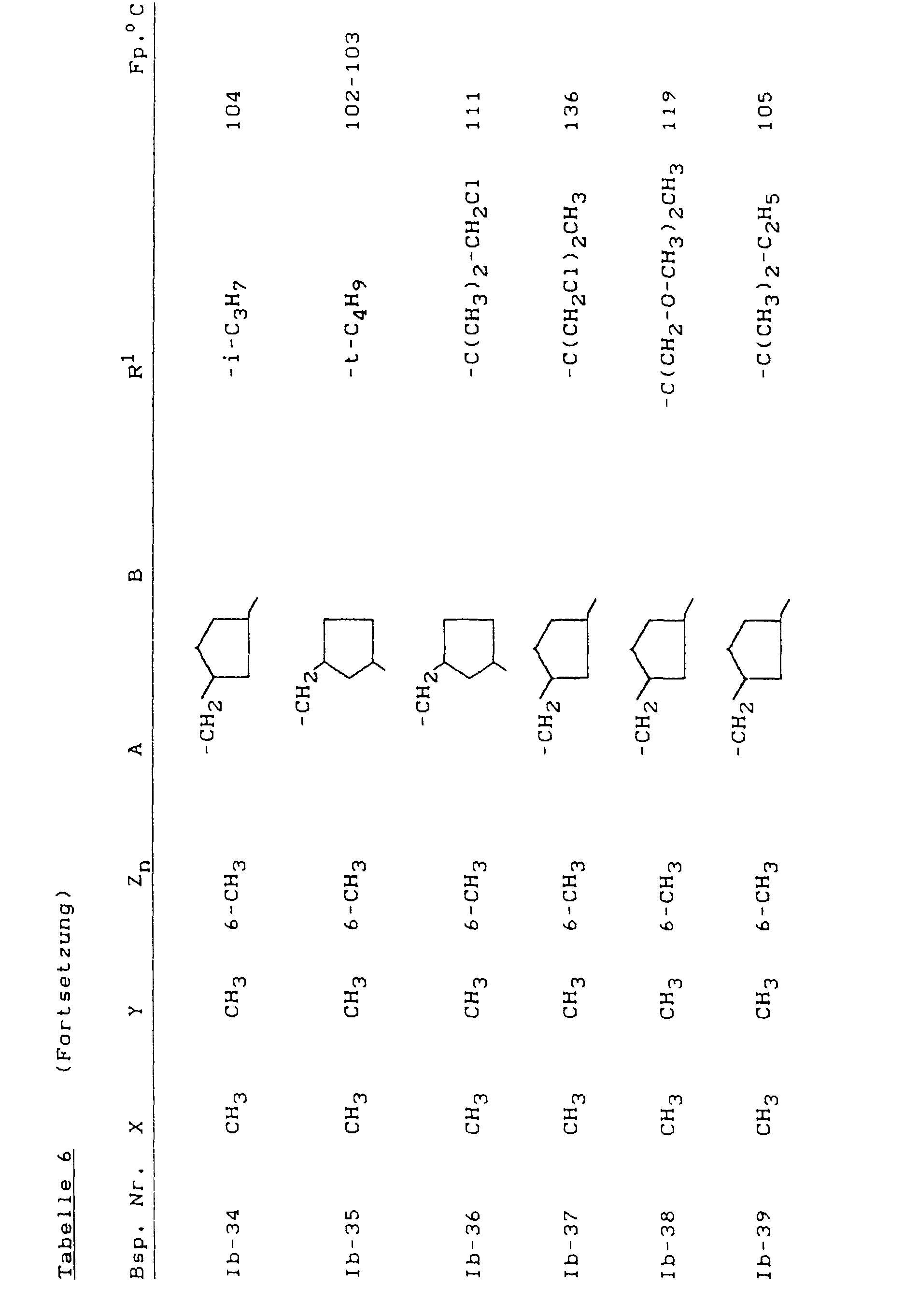

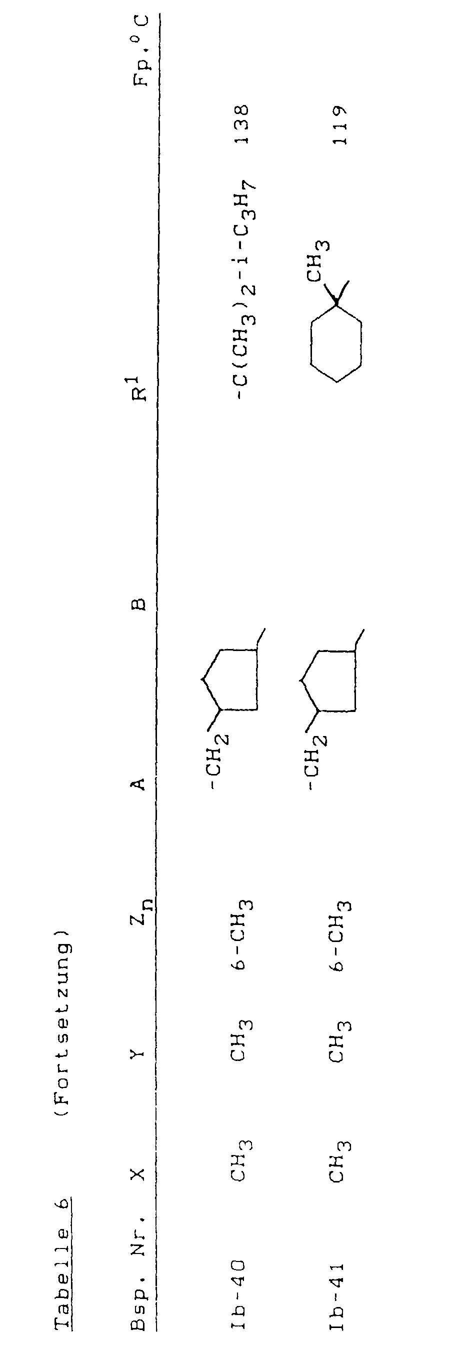

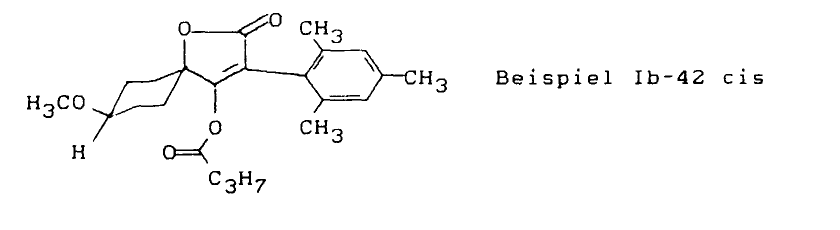

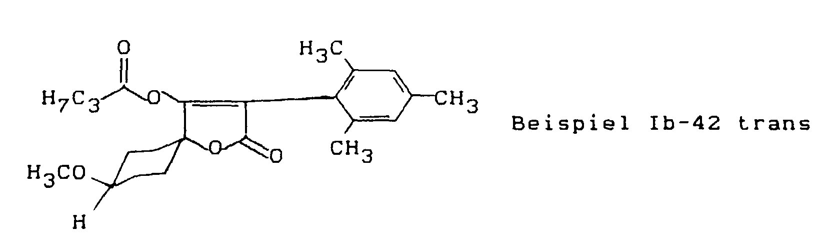

3,16 g (10 mmol) der Verbindung Ia-4 werden in 40 ml abs. Methylenchlorid vorgelegt, 1,42 g (14 mmol) Triethylamin und eine Spatelspitze DMAP zugegeben, eine Lösung von 1,38 g (13 mmol) Buttersäurechlorid in 20 ml Methylenchlorid zugetropft und 16 h bei Raumtemperatur gerührt.

Zur Aufarbeitung wäscht man das Reaktionsgemisch mit wäßriger Citronensäure, NaHCO₃-Lösung und NaCl-Lösung, trocknet und rotiert ein. Die Auftrennung in die Isomeren erfolgt durch Flash-Chromatographie an Kieselgel mit Cyclohexan/Essigester 6:1.
Ausbeute: 0,92 g (24 % der Theorie) des unpolaren trans-Isomeren vom Fp. 85-87°C und 0,21 g (5 % der Theorie) des polaren cis-Isomeren vom Fp. 125-126°C.

### Beispiel Ic-1

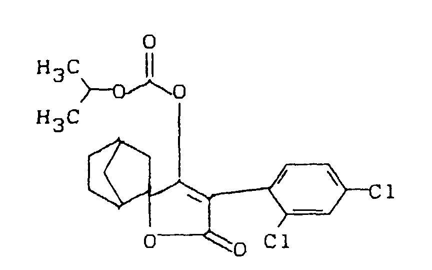

3,25 g (10 mmol) der Verbindung Ia-1 werden in 40 ml abs. Methylenchlorid vorgelegt, 1,42 g (14 mmol) Triethylamin und eine Spatelspitze DMAP zugegeben, eine Lösung von 1,47 g (12 mmol) Chlorameisensäureisopropylester in 20 ml Methylenchlorid zugetropft und 16 h bei Raumtemperatur gerührt.

Zur Aufarbeitung wäscht man das Reaktionsgemisch mit wäßriger Citronensäure, NaHCO₃-Lösung und NaCl-Lösung, trocknet und rotiert ein. Die weitere Reinigung erfolgt durch Umkristallisation aus MTB-Ether/n-Hexan.
Ausbeute:2,84 g (69 % der Theorie) eines Feststoffs vom Fp. 136°C.

In Analogie wurden die in Tabelle 7 aufgeführten Verbindungen hergestellt:

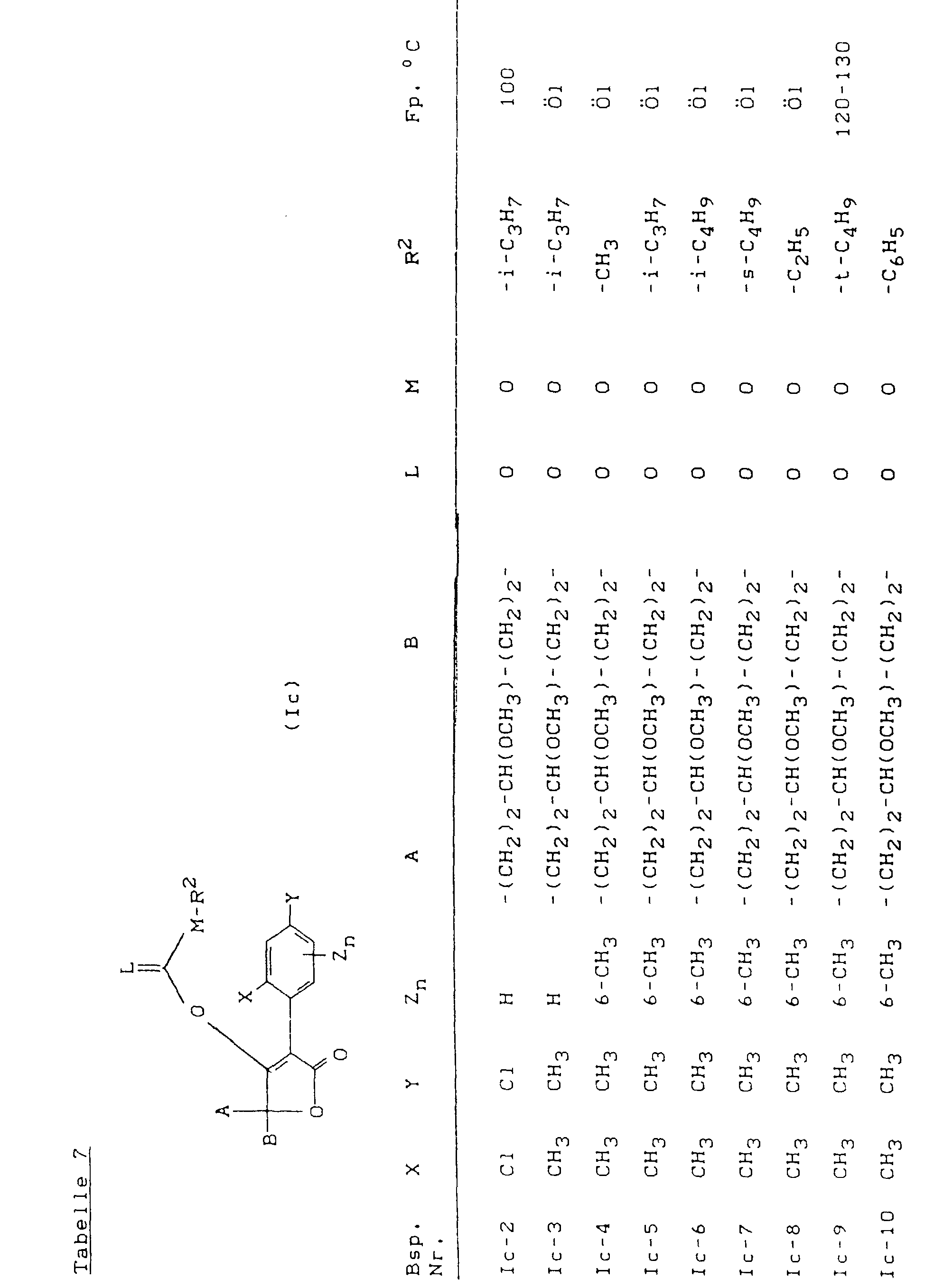

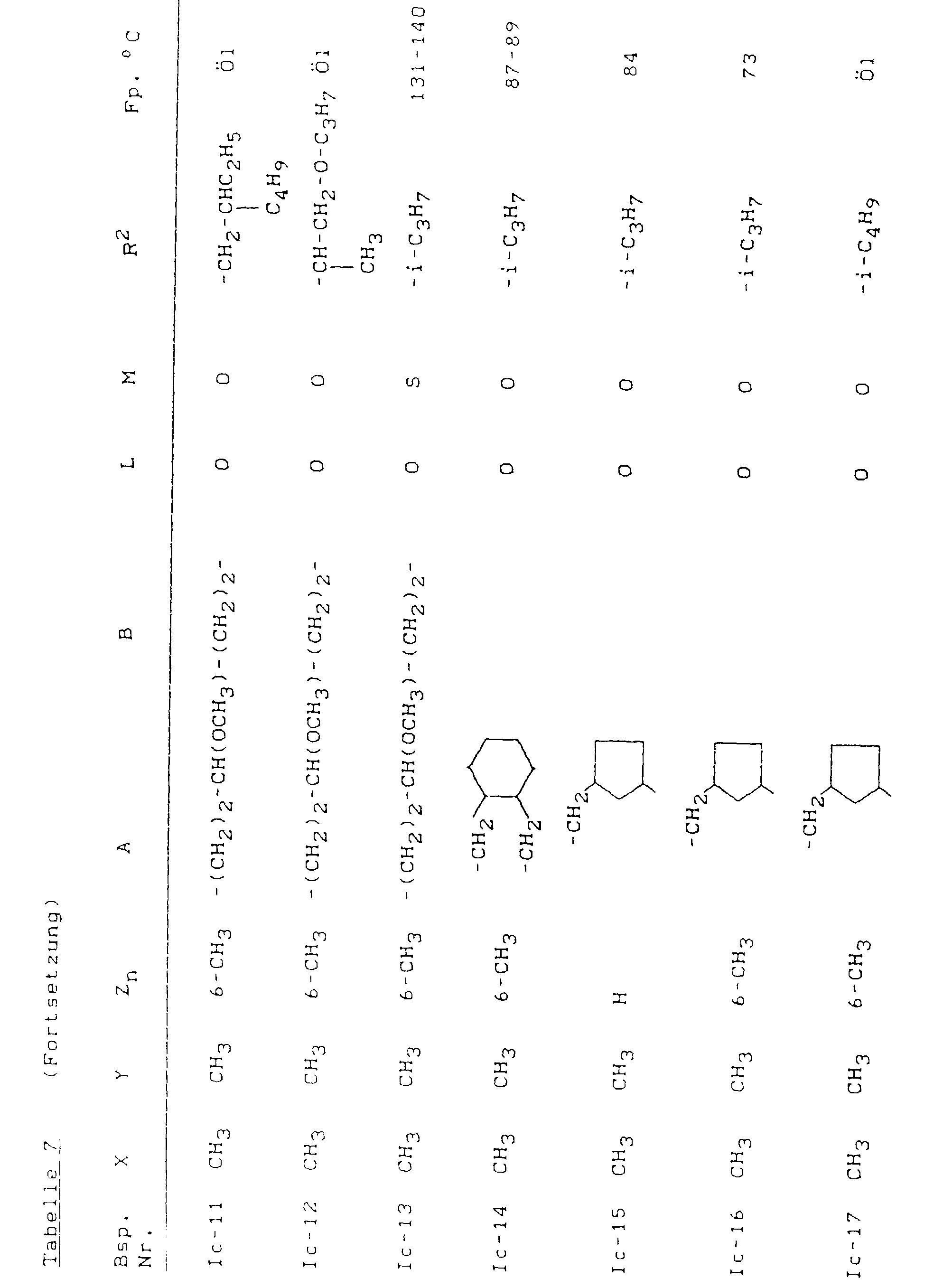

### Beispiel II-1

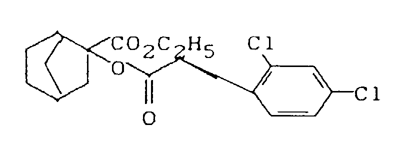

### Beispiel II-2

18,40 g (0,10 mol) 2-Hydroxy-norbornan-2-carbonsäureethylester und 24,59 g (0,11 mol) 2,4-Dichlorphenylessigsäurechlorid werden 16 h in 100 ml abs. Toluol refluxiert und einrotiert. Man erhält die obengezeigte Verbindung in einer Ausbeute von 37,10 g (quant) in Form eines Öls.

In Analogie wurden die in Tabelle 8 aufgeführten Verbindungen hergestellt.

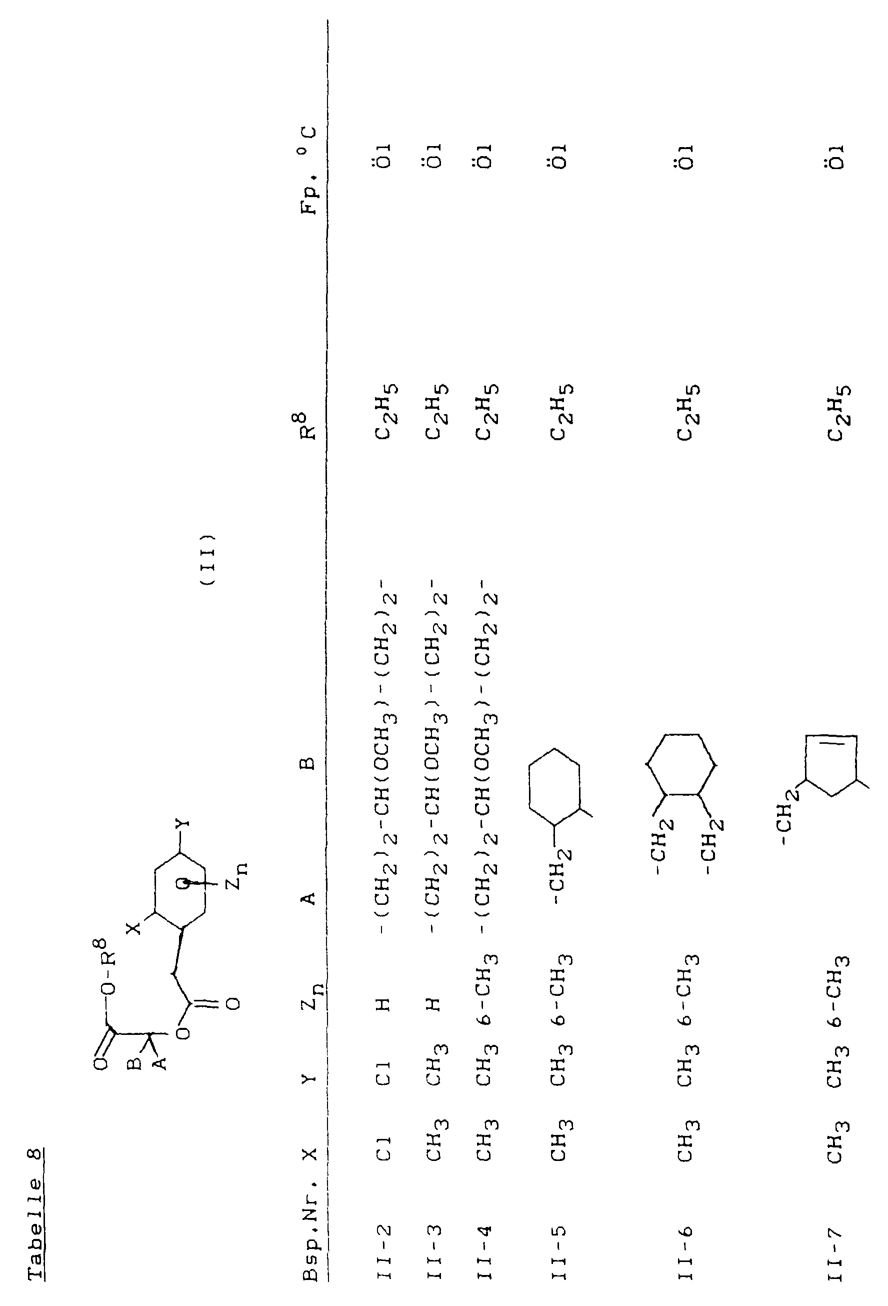

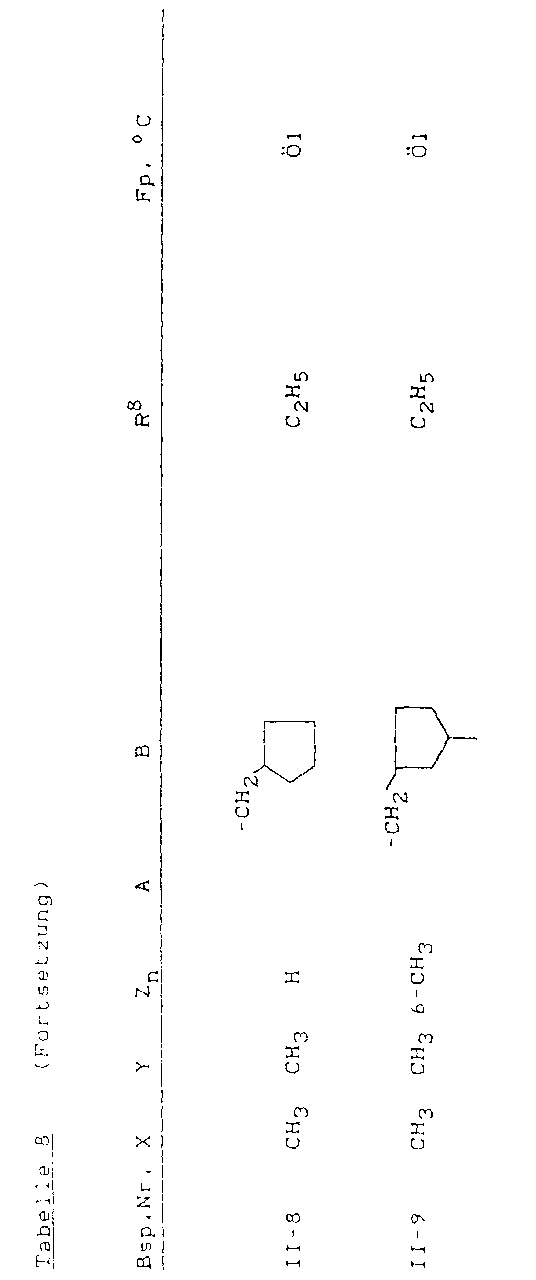

## Patentansprüche

1. 3-Aryl-4-hydroxy-Δ³-dihydrofuranon-Derivate der allgemeinen Formel (I) 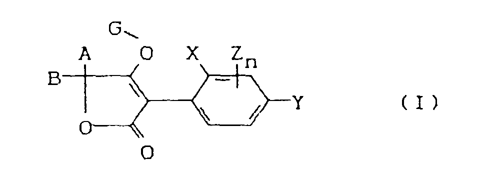 in welcher
X für Alkyl, Halogen, Alkoxy oder Halogenalkyl steht,
Y für Wasserstoff, Alkyl, Halogen, Alkoxy, Halogenalkyl steht,
Z für Alkyl, Halogen, Alkoxy steht,
n für eine Zahl von 0-3 steht, oder wobei die Reste X und Z gemeinsam mit dem Phenylrest an den sie gebunden sind, den Naphthalinrest der Formel 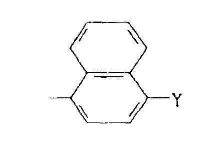 bilden,
in welchem Y die oben angegebene Bedeutung hat,
G für Wasserstoff (a) oder für die Gruppen 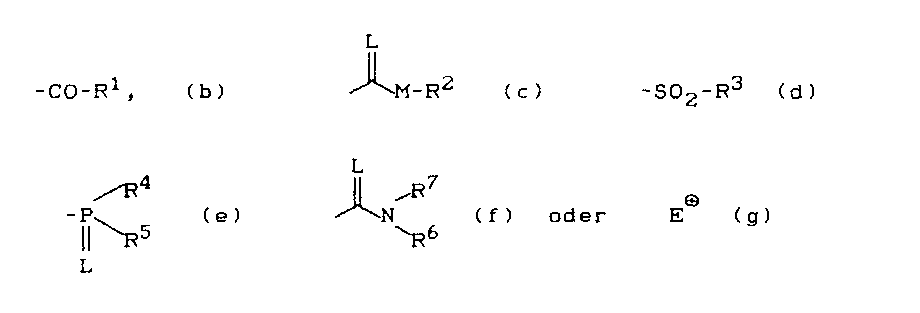 steht,
A und B gemeinsam mit dem Kohlenstoffatom, an das sie gebunden sind einen durch Alkoxy, Alkylthio, Alkylsulfoxyl oder Alkylsulfonyl substituierten Cyclus bilden, oder
A und B gemeinsam mit dem Kohlenstoffatom, an das sie gebunden sind für einen Cyclus stehen, bei dem zwei Substituenten gemeinsam mit den Kohlenstoffatomen, an die sie gebunden sind für einen gegebenenfalls durch Alkyl, Alkoxy oder Halogen substituierten gesättigten oder einfach ungesättigten Cyclus stehen, der durch Sauerstoff oder Schwefel unterbrochen sein kann,
E^{⊕} für ein Metallionäquivalent oder ein Ammonium steht,
L und M für Sauerstoff und/oder Schwefel steht,
R¹ für gegebenenfalls durch Halogen substituiertes Alkyl, Alkenyl, Alkoxyalkyl, Alkylthioalkyl, Polyalkoxyalkyl oder Cycloalkyl, das durch Heteroatome unterbrochen sein kann, gegebenenfalls substituiertes Phenyl, gegebenenfalls substituiertes Phenylalkyl, substituiertes Hetaryl, substituiertes Phenoxyalkyl oder substituiertes Hetaryloxyalkyl steht und
R² für gegebenenfalls durch Halogen substituiertes Alkyl, Alkenyl, Alkoxyalkyl, Polyalkoxyalkyl oder gegebenenfalls substituiertes Phenyl oder Benzyl steht,
R³, R⁴ und R⁵ unabhängig voneinander für gegebenenfalls durch Halogen substituiertes Alkyl, Alkoxy, Alkylamino, Dialkylamino, Alkylthio, Alkenylthio, Alkinylthio, Cycloalkylthio und für gegebenenfalls substituiertes Phenyl, Phenoxy oder Phenylthio stehen,
R⁶ und R⁷ unabhängig voneinander für Wasserstoff, gegebenenfalls durch Halogen substituiertes Alkyl, Alkenyl, Alkoxy, Alkoxyalkyl, für gegebenenfalls substituiertes Phenyl, für gegebenenfalls substituiertes Benzyl stehen
oder wobei R⁶ und R⁷ zusammen für einen gegebenenfalls durch Sauerstoff unterbrochenen Alkylenrest stehen, sowie die stereo- und enantiomerenreinen Formen und deren Gemische.

2. Verfahren zur Herstellung der 3-Aryl-4-hydroxy-Δ³-dihydrofuranon-Derivate der Formel I gemäß Anspruch 1 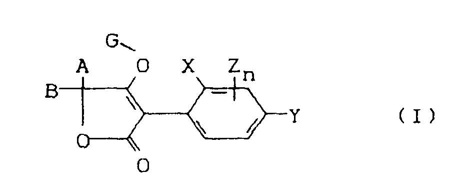 in welcher
X für Alkyl, Halogen, Alkoxy oder Halogenalkyl steht,
Y für Wasserstoff, Alkyl, Halogen, Alkoxy, Halogenalkyl steht,
Z für Alkyl, Halogen, Alkoxy steht,
n für eine Zahl von 0-3 steht, oder wobei die Reste X und Z gemeinsam mit dem Phenylrest an den sie gebunden sind, den Naphthalinrest der Formel 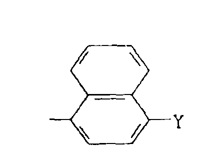 bilden,
in welchem Y die oben angegebene Bedeutung hat,
G für Wasserstoff (a) oder für die Gruppen 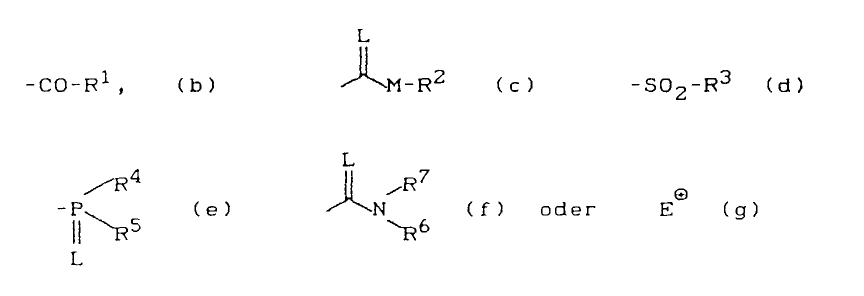 steht,
A und B gemeinsam mit dem Kohlenstoffatom, an das sie gebunden sind einen durch Alkoxy, Alkoxythio oder Alkylsulfonyl substituierten Cyclus bilden, oder
A und B gemeinsam mit dem Kohlenstoffatom, an das sie gebunden sind für einen Cyclus stehen, bei dem zwei Substituenten gemeinsam mit den Kohlenstoffatomen, an die sie gebunden sind für einen gegebenenfalls durch Alkyl, Alkoxy oder Halogen substituierten gesättigten oder einfach ungesättigten Cyclus stehen, der durch Sauerstoff oder Schwefel unterbrochen sein kann,
E^{⊕} für ein Metallionäquivalent oder ein Ammonium steht,
L und M für Sauerstoff und/oder Schwefel steht,
R¹ für gegebenenfalls durch Halogen substituiertes Alkyl, Alkenyl, Alkoxyalkyl, Alkylthioalkyl, Polyalkoxyalkyl oder Cycloalkyl, das durch Heteroatome unterbrochen sein kann, gegebenenfalls substituiertes Phenyl, gegebenenfalls substituiertes Phenylalkyl, substituiertes Hetaryl, substituiertes Phenoxyalkyl oder substituiertes Hetaryloxyalkyl steht und
R² für gegebenenfalls durch Halogen substituiertes Alkyl, Alkenyl, Alkoxyalkyl, Polyalkoxyalkyl oder gegebenenfalls substituiertes Phenyl oder Benzyl steht,
R³, R⁴ und R⁵ unabhängig voneinander für gegebenenfalls durch Halogen substituiertes Alkyl, Alkoxy, Alkylamino, Dialkylamino, Alkylthio, Alkenylthio, Alkinylthio, Cycloalkylthio und für gegebenenfalls substituiertes Phenyl, Phenoxy oder Phenylthio stehen,
R⁶ und R⁷ unabhängig voneinander für Wasserstoff, gegebenenfalls durch Halogen substituiertes Alkyl, Alkenyl, Alkoxy, Alkoxyalkyl, für gegebenenfalls substituiertes Phenyl, für gegebenenfalls substituiertes Benzyl stehen
oder wobei R⁶ und R⁷ zusammen für einen gegebenenfalls durch Sauerstoff unterbrochenen Alkylenrest stehen,
dadurch gekennzeichnet, daß man
(A)
zur Herstellung von Verbindungen der Formel Ia 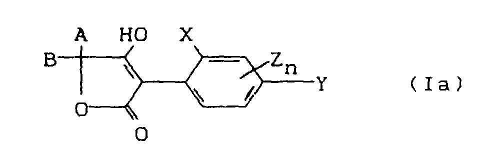 in welcher
A, B, X, Y, Z und n die oben angegebene Bedeutung haben,
Carbonsäureester der Formel (II) 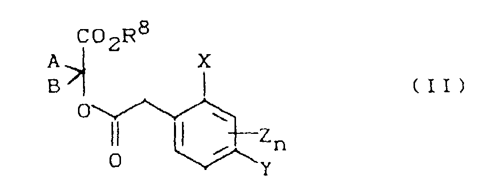 in welcher
A, B, X, Y, Z und n die oben angegebene Bedeutung haben
und
R⁸ für Alkyl steht,
in Gegenwart eines Verdünnungsmittels und in Gegenwart einer Base intramolekular kondensiert oder
(B)
zur Herstellung von Verbindungen der Formel (Ib) 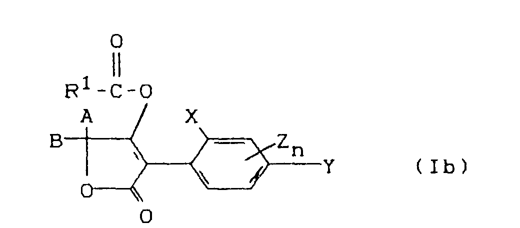 in welcher
A, B, X, Y, Z, R¹ und n die oben angegebene Bedeutung haben,
Verbindungen der Formel (Ia), 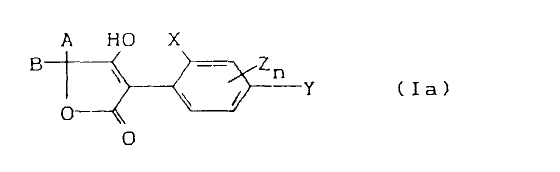 in welcher
A, B, X, Y, Z und n die oben angegebene Bedeutung haben,
α) mit Säurehalogeniden der allgemeinen Formel (III) 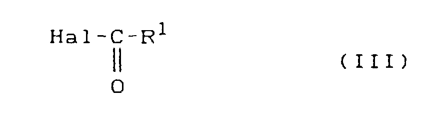 in welcher
R¹ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt, oder
(C)
zur Herstellung von Verbindungen der Formel (Ic) 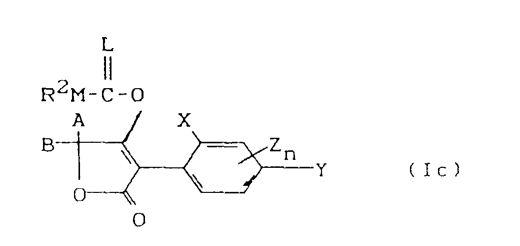 in welcher
A, B, X, Y, Z, R² und n die oben angegebene Bedeutung haben,
L für Sauerstoff
und
M für Sauerstoff oder Schwefel steht,
Verbindungen der Formel (Ia) 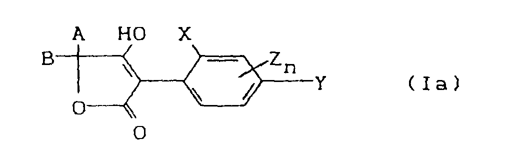 in welcher
A, B, X, Y, Z und n die oben angegebene Bedeutung haben
mit Chlorameisensäureester oder Chlorameisensäurethiolester der allgemeinen Formel (V)
R²-M-CO-Cl (V)
in welcher
R² und M die oben angegebene Bedeutung haben, gegebenenfals in Gegenwart eines Verdünnungsmittels und gegebenenfalls in Gegenwart eines Säurebindemittels umsetzt oder
(D)
zur Herstellung von Verbindungen der Formel (Ic) 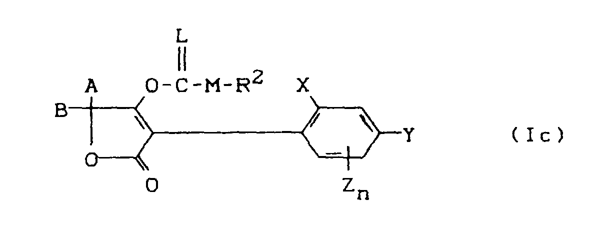 in welcher
A, B, R², X, Y, Z und n die oben angegebene Bedeutung haben,
L für Schwefel
und
M für Sauerstoff oder Schwefel steht,
Verbindungen der Formel (Ia) 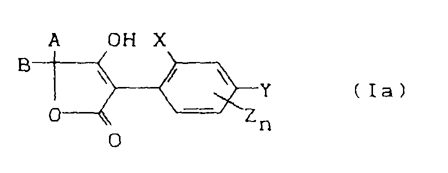 in welcher
A, B, X, Y, Z und n die oben angegebene Bedeutung haben
mit Chlormonothioameisensäureestern oder Chlordithioameisensäureestern der allgemeinen Formel (VI) 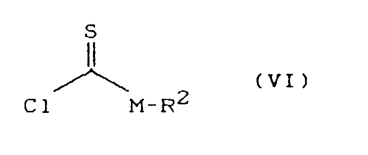 in welcher
M und R² die oben angegebene Bedeutung haben
gegebenenfalls in Gegenwart eines Verdünnungsmittels und gegebenenfalls in Gegenwart eines Säurebindemittels umsetzt oder
(E)
zur Herstellung von Verbindungen der Formel (Id) 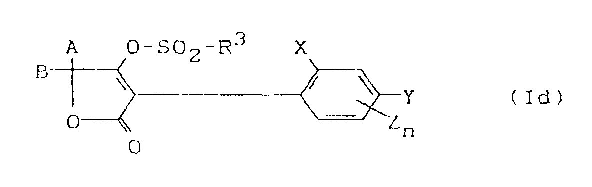 in welcher
A, B, X, Y, Z, R³ und n die oben angegebene Bedeutung haben,
Verbindungen der Formel (Ia) 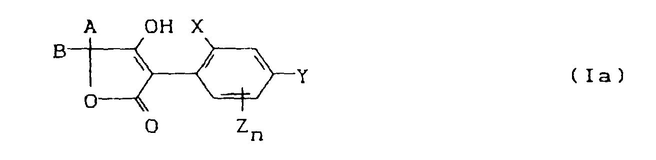 in welcher
A, B, X, Y, Z und n die oben angegebene Bedeutung haben,
mit Sulfonsäurechloriden der allgemeinen Formel (VII)
R³-SO₂-Cl (VII)
in welcher
R³ die oben angegebene Bedeutung hat
gegebenenfalls in Gegenwart eines Verdünnungsmittels und gegebenenfalls in Gegenwart eines Säurebindemittels,
umsetzt, oder
(F)
zur Herstellung von Verbindungen der Formel (Ie) 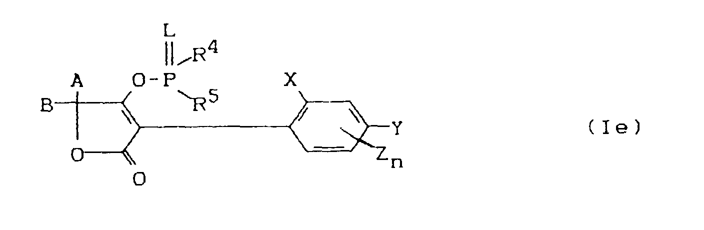 in welcher A, B, L, X, Y, Z, R⁴, R⁵ und n die oben angegebene Bedeutung haben,
Verbindungen der Formel (Ia) 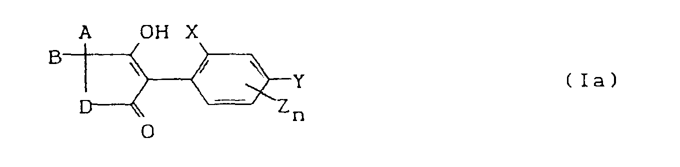 in welcher
A, B, X, Y, Z und n die oben angegebene Bedeutung haben
mit Phosphorverbindungen der allgemeinen Formel (IX) 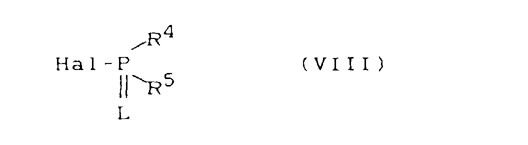 in welcher
L, R⁴ und R⁵ die oben angegebene Bedeutung haben
und
Hal für Halogen, insbesondere Chlor und Brom steht,
gegebenenfalls in Gegenwart eines Verdünnungsmittels und gegebenenfalls in Gegenwart eines Säurebindemittels umsetzt oder
(G)
zur Herstellung von Verbindungen der Formel (If) 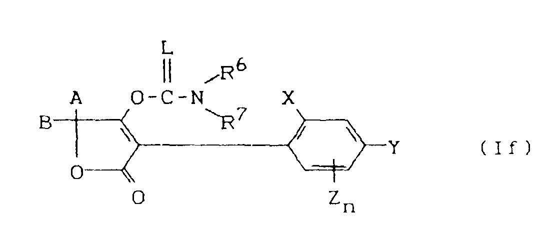 in welcher
A, B, L, X, Y, Z, R⁶, H⁷ und n die oben angegeben Bedeutung haben,
Verbindungen der Formel (Ia), 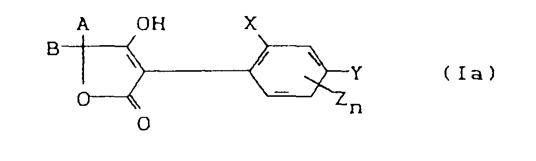 in welcher
A, B, X, Y, Z und n die oben angegebene Bedeutung haben
α) mit Isocyanaten der allgemeinen Formel (IX)
R⁶-N=C=L (IX)
in welcher
L und R⁶ die oben angegebene Bedeutung hat gegebenenfalls in Gegenwart eines Verdünnungsmittels und gegebenenfalls in Gegenwart eines Katalysators umsetzt,
oder
β) mit Carbamidsäurechloriden oder Thiocarbamidsäurechloriden der allgemeinen Formel (X) 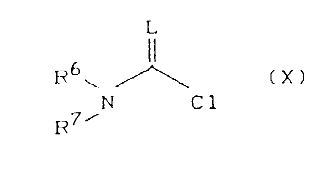 in welcher
L, R⁶ und R⁷ die oben angegebene Bedeutung haben,
gegebenenfalls in Gegenwart eines Verdünnungsmittels und gegebenenfalls in Gegenwart eines Säurebindemittels, umsetzt oder
(H)
zur Herstellung von Verbindungen der Formel (Ig) 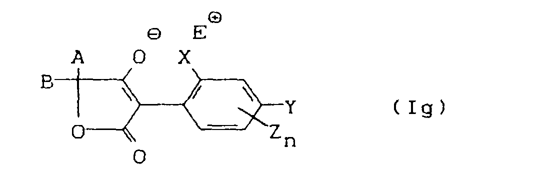 in welcher
X, Y, Z, A, B und n die oben angegebene Bedeutung haben,
und E^{⊕} für ein Metallionäquivalent oder für ein Ammoniumion steht,
Verbindungen der Formel (Ia) 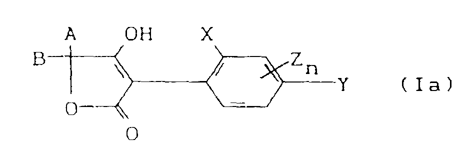 in welcher
X, Y, Z, A, B und n die oben angegebene Bedeutung haben,
mit Metallhydroxiden oder Aminen der allgemeinen Formeln (XI) und (XII) 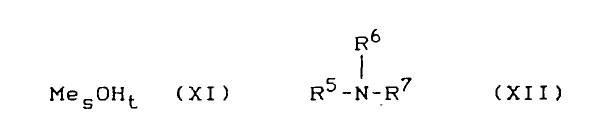 in welchen
Me für ein- oder zweiwertige Metallionen
s und t für die Zahl 1 oder 2 und
R⁵, R⁶ und R⁷ unabhängig voneinander für Wasserstoff und Alkyl
stehen,
gegebenenfalls in Gegenwart eines Verdünnungsmittels, umsetzt.

3. 3-Aryl-4-hydroxy-Δ³-dihydrofuranon-Derivate der allgemeinen Formel (I) gemäß Anspruch 1 welche unter Einbeziehung der verschiedenen Bedeutungen von (a), (b), (c), (d), (e), (f) und (g) der Gruppe G folgende Strukturen (Ia) bis (Ig) besitzen: 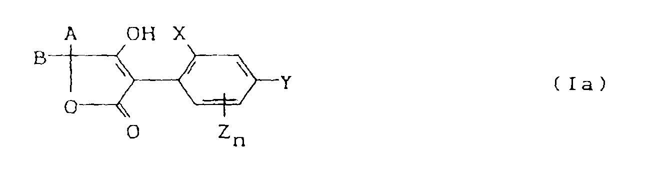 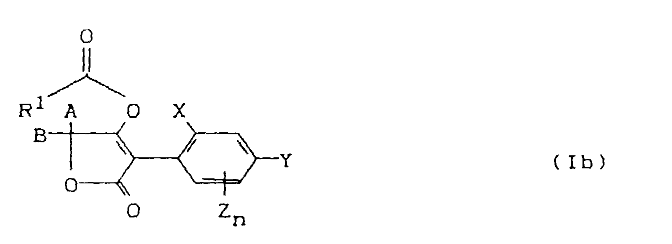 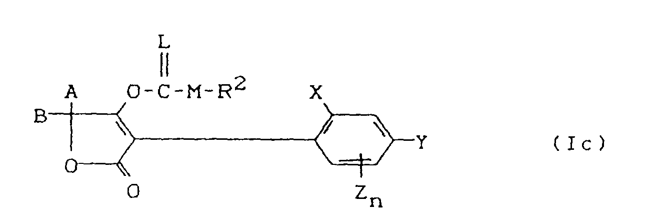 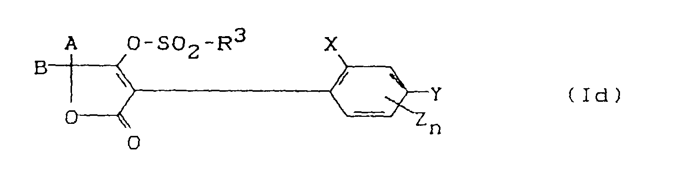 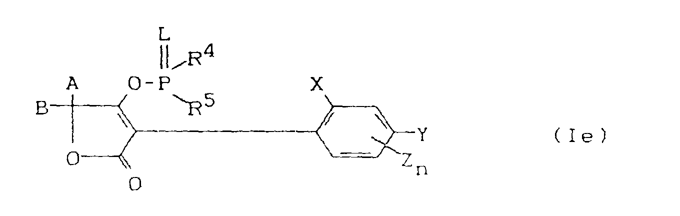 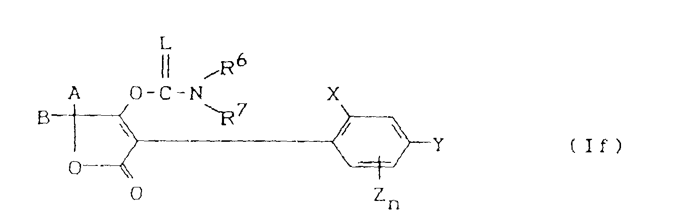 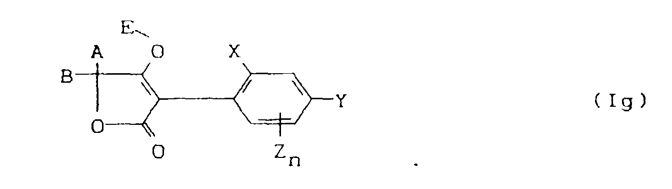 worin
A, B, E, L, M, X, Y, Zₙ, R¹, R², R³, R⁴, R⁵, R⁶ und R⁷ die in Anspruch 1 angegebenen Bedeutungen besitzen.

4. 3-Aryl-4-hydroxy-Δ³-dihydrofuranon-Derivate der allgemeinen Formel (I) gemäß Anspruch 1
in welcher
X für C₁-C₆-Alkyl, Halogen, C₁-C₆-Alkoxy oder C₁-C₃-Halogenalkyl steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0 bis 3 steht,
oder wobei die Reste X und Z gemeinsam mit dem Phenylrest an den sie gebunden sind, den Naphthalinrest der Formel 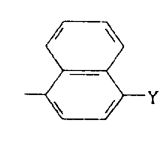 bilden,
in welchem Y die oben angegebene Bedeutung hat,
oder worin
A und B gemeinsam mit dem Kohlenstoffatom, an das sie gebunden sind, einen gesättigten oder ungesättigten, durch C₁-C₆-Alkoxy, C₁-C₄-Alkylthio, C₁-C₄-Alkylsulfoxyl oder C₁-C₄-Alkylsulfonyl substituierten 3- bis 8-gliedrigen Ring bilden, oder
A und B gemeinsam mit dem Kohlenstoff, an das sie gebunden sind, für einen C₃-C₈-gliedrigen Ring stehen, bei dem zwei Substituenten gemeinsam mit den Kohlenstoffatomen, an die sie gebunden sind, für einen gegebenenfalls durch Alkyl, Alkoxy oder Halogen substituierten gesättigten oder einfach ungesättigten C₅-C₇-Ring stehen, der durch Sauerstoff oder Schwefel unterbrochen sein kann,
G für Wasserstoff (a) oder für die Gruppen 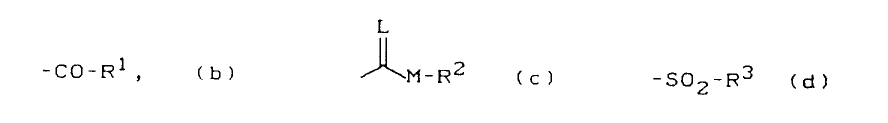 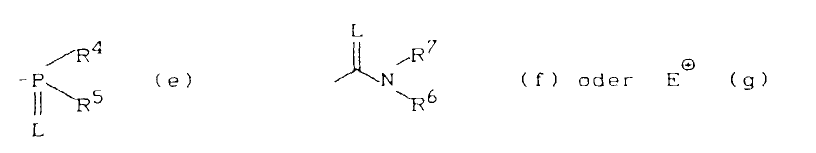 in welchen
E^{⊕} für ein Metallionäquivalent oder ein Ammoniumion steht,
L und M für Sauerstoff und/oder Schwefel steht,
R¹ für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy-substituiertes Phenyl steht;
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Halogen und/oder C₁-C₆-Alkyl substituiertes Hetaryl steht,
für gegebenenfalls durch Halogen und C₁-C₆-Alkyl-substituiertes Phenoxy-C₁-C₆-alkyl steht,
für gegebenenfalls durch Halogen, Amino und C₁-C₆-Alkyl-substituiertes Hetaryloxy-C₁-C₆-Alkyl steht,
R² für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl-substituiertes Phenyl oder Benzyl steht,
R³, R⁴ und R⁵ unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₃-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff oder Schwefel unterbrochenen C₄-C₆-Alkylenring stehen,
sowie die stereo- und enantiomerenreinen Formen dieser Verbindungen und ihre Gemische.

5. 3-Aryl-4-hydroxy-Δ³-dihydrofuranon-Derivate der Formel (I) gemäß Anspruch 1 in welcher
X für C₁-C₆-Alkyl, Halogen, C₁-C₆-Alkoxy oder C₁-C₃-Halogenalkyl steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0 bis 3 steht,
oder wobei die Reste X und Z gemeinsam mit dem Phenylrest an den sie gebunden sind, den Naphthalinrest der Formel 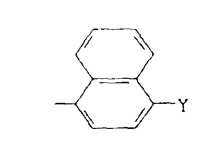 bilden,
in welchem Y die oben angegebene Bedeutung hat,
A und B gemeinsam mit dem Kohlenstoffatom, an das sie gebunden sind, einen gesättigten oder ungesättigten, durch C₁-C₅-Alkoxy, C₁-C₃-Alkylthio, C₁-C₃-Alkylsulfoxyl oder C₁-C₃-Alkylsulfonyl substituierten 5- bis 7-gliedrigen Ring bilden, oder
A und B gemeinsam mit dem Kohlenstoff, an das sie gebunden sind, für einen C₄-C₇-gliedrigen Ring stehen, bei dem zwei Substituenten gemeinsam mit den Kohlenstoffatomen, an die sie gebunden sind, für einen gegebenenfalls durch C₁-C₃-Alkyl, C₁-C₃-Alkoxy, Fluor oder Chlor substituierten gesättigten oder einfach ungesättigten C₅-C₆-Ring stehen, der durch Sauerstoff oder Schwefel unterbrochen sein kann,
G für Wasserstoff (a) oder für die Gruppen 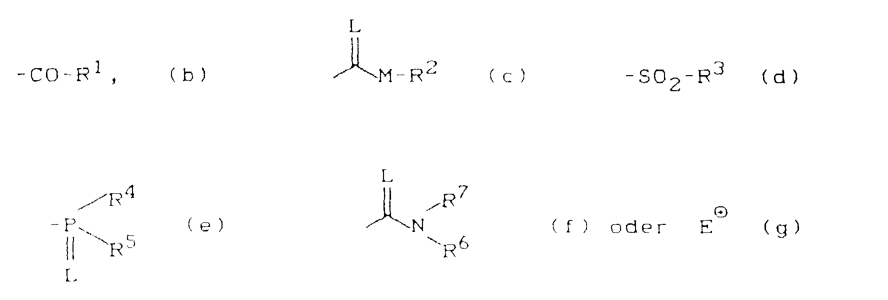 steht,
in welchen
E^{⊕} für ein Metallionäquivalent oder ein Ammoniumion steht;
L und M jeweils für Sauerstoff und/oder Schwefel steht,
R¹ für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁.C₆-Polyalkoxy-C₂-C₆-alkyl und Cycloalkyl mit 3-7 Ringatomen, das durch 1-2 Sauerstoff- und/oder Schwefelatome unterbrochen sein kann steht,
für gegebenenfalls durch Halogen- Nitro, C₁-C₄-Alkyl-, C₁-C₄-Alkoxy-, C₁-C₃-Halogenalkyl-, C₁-C₃-Halogenalkoxy-substituierets Phenyl steht,
für gegebenenfalls durch Halogen- C₁-C₄-Alkyl-, C₁-C₄-Alkoxy-, C₁-C₃-Halogenalkyl-, C₁-C₃-Halogenalkoxy-substituiertes Phenyl-C₁-C₄-alkyl steht,
für gegebenenfalls durch Halogen- und C₁-C₆-Alkyl-substituiertes Hetaryl steht,
für gegebenenfalls durch Halogen- und C₁-C₄-Alkyl-substituiertes Phenoxy-C₁-C₅-alkyl steht,
für gegebenenfalls durch Halogen, Amino und C₁-C₄-Alkyl substituiertes Hetaryloxy-C₁-C₅-alkyl steht,
R² für gegebenenfalls durch Halogen substituiertes: C₁-C₁₆-Alkyl, C₃-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro-, C₁-C₄-Alkyl, C₁-C₃-Alkoxy-, C₁-C₃-Halogenalkyl- substituiertes Phenyl oder Benzyl steht,
R³, R⁴ und R⁵ unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substiutiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₃-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht oder zusammen für einen gegebenenfalls durch Sauerstoff oder Schwefel unterbrochenen C₄-C₆-Alkylenring stehen,
sowie ihre stereo- und enantiomerenreinen Formen und deren Gemische.

6. 3-Aryl-4-hydroxy-Δ³-dihydrofuranon-Derivate der Formel (I) gemäß Anspruch 1, in welcher
X Methyl, Ethyl, Propyl, i-Propyl, Fluor, Chlor, Brom, Methoxy, Ethoxy und Trifluormethyl steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0 bis 3 steht,
oder wobei die Reste X und Z gemeinsam mit dem Phenylrest an den sie gebunden sind, den Rest der Formel 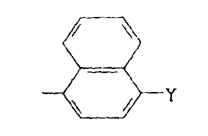 bilden,
in welchem Y die oben angegebene Bedeutung hat,
A und B gemeinsam mit dem Kohlenstoffatom, an das sie gebunden sind, einen gesättigten oder ungesättigten, durch C₁-C₄-Alkoxy, C₁-C₂-Alkylthio, C₁-C₂-Alkylsulfoxyl oder C₁-C₂-Alkylsulfonyl substituierten 5- bis 6-gliedrigen Ring bilden, oder
A und B gemeinsam mit dem Kohlenstoff, an das sie gebunden sind, für einen C₄-C₆-gliedrigen Ring stehen, bei dem zwei Substituenten gemeinsam mit den Kohlenstoffatomen, an die sie gebunden sind, für einen gegebenenfalls Methyl, Ethyl, Methoxy, Ethoxy, Fluor oder Chlor substituierten gesättigten oder einfach ungesättigten C₅-C₆-Ring stehen, der durch Sauerstoff oder Schwefel unterbrochen sein kann,
G für Wasserstoff (a) oder für die Gruppen 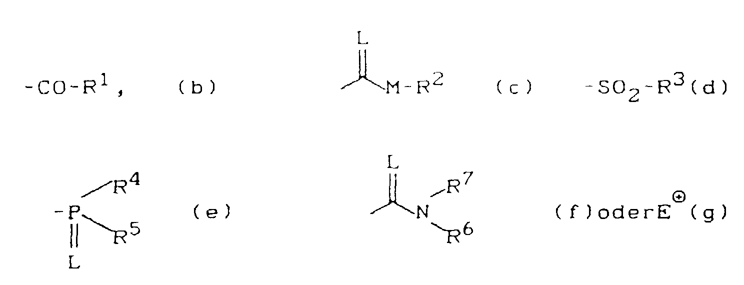 steht,
in welchen
E^{⊕} für ein Metallionäquivalent oder ein Ammoniumion steht,
L und M für Sauerstoff und/oder Schwefel steht,
R¹ für gegebenenfalls durch Fluor oder Chlor substituiertes C₁-C₁₄-Alkyl, C₂-C₁₄-Alkenyl, C₁-C₄- Alkoxy-C₂-C₆-alkyl, C₁-C₄-Alkylthio-C₂-C₆-alkyl, C₁-C₄-Polyalkoxy-C₂-C₄-alkyl und Cycloalkyl mit 3-6 Ringatomen, das durch 1-2 Sauerstoff- und/oder Schwefelatome unterbrochen sein kann, steht, für gegebenenfalls durch Fluor, Chlor, Brom, Methyl, Ethyl, Propyl, i-Propyl, Methoxy, Ethoxy, Trifluormethyl, Trifluormethoxy, Nitro-substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-C₁-C₃-alkyl steht,
für gegebenenfalls durch Fluor, Chlor, Brom, Methyl, Ethyl-substituiertes Pyridyl, Pyrimidyl, Thiazolyl und Pyrazolyl steht,
für gegebenenfalls durch Fluor, Chlor, Methyl, Ethyl-substituiertes Phenoxy-C₁-C₄-alkyl steht,
für gegebenenfalls durch Fluor, Chlor, Amino, Methyl-, Ethyl, substituiertes Pyridyloxy-C₁-C₄-alkyl, Pyrimidyloxy-C₁-C₄-alkyl und Thiazolyloxy- C₁-C₄-alkyl steht,
R² für gegebenenfalls durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₃-C₆-Alkenyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht oder zusammen einen gegebenenfalls durch Sauerstoff oder Schwefel unterbrochenen C₄-C₆-Alkylenring stehen,
sowie ihre stereo- und enantiomerenreinen Formen und deren Gemische.

7. Verbindungen der Formel (II) 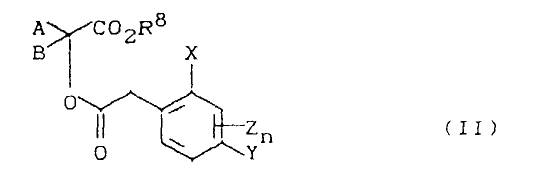 in welcher
A, B, X, Y, Z, n und R⁸ die in Anspruch 1 angegebene Bedeutung haben.

8. Verfahren zur Herstellung der O-Acyl-α-hydroxycarbonsäureester der Formel (II) gemäß Anspruch 7, dadurch gekennzeichnet, daß man
a) 2-Hydroxycarbonsäure-(ester) der Formel (XIII) 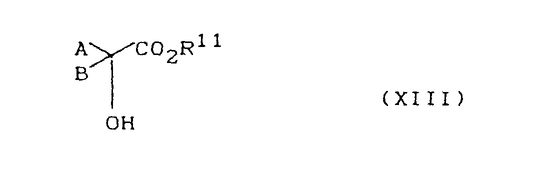 in welcher
R¹¹ für Wasserstoff im Fall von Verbindungen der Formel (XIIIa) oder Alkyl im Fall von Verbindungen der Formel (XIIIb) steht
und
A und B die in Anspruch 1 angegebene Bedeutung haben,
mit Phenylessigsäurehalogeniden der Formel (XIV) 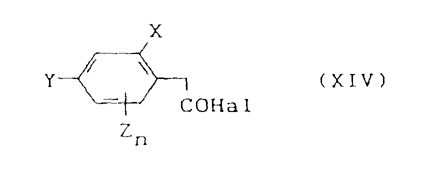 in welcher
X, Y, Z und n die oben angegebene Bedeutung haben und
Hal für Chlor oder Brom steht,
acycliert
oder wenn man Hydroxycarbonsäuren der Formel (IIa), 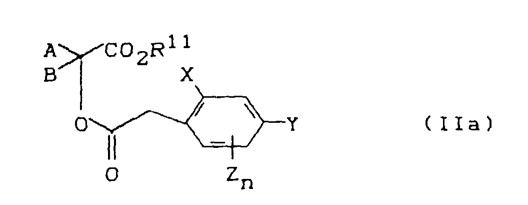 in welcher
A, B, X, Y, Z und n die oben angegebene Bedeutung
haben
und
R¹¹ für Wasserstoff steht,
verestert.

9. Schädlingsbekämpfungsmittel, gekennzeichnet durch einen Gehalt an mindestens einem 3-Aryl-4-hydroxy-Δ³-dihydrofuranon-Derivat der Formel (I) gemäß Anspruch 1.

10. Verwendung von 3-Aryl-4-hydroxy-Δ³-dihydrofuranon-Derivate der Formel (I) gemäß Anspruch 1 zur Bekämpfung von Schädlingen.

11. Verfahren zur Herstellung von Schädlingsbekämpfungsmitteln, dadurch gekennzeichnet, daß man 3-Aryl-4-hydroxy-Δ³-dihydrofuranon-Derivate der Formel (I) gemäß Anspruch 1 mit Streckmitteln und/oder oberflächenaktiven Mitteln vermischt.

## Claims

1. 3-Aryl-4-hydroxy-Δ³-dihydrofuranone derivatives of the general formula (I) 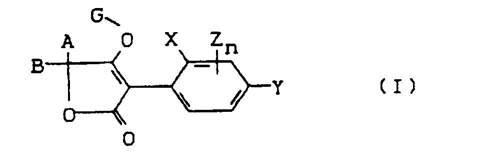 in which
X represents alkyl, halogen, alkoxy or halogenoalkyl,
Y represents hydrogen, alkyl, halogen, alkoxy or halogenoalkyl,
Z represents alkyl, halogen or alkoxy,
n represents a number from 0-3, or in which the radicals X and Z together with the phenyl radical to which they are bonded form the naphthalene radical of the formula 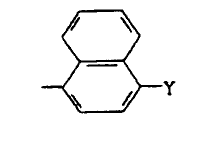 in which Y has the abovementioned meaning,
G represents hydrogen (a) or the groups 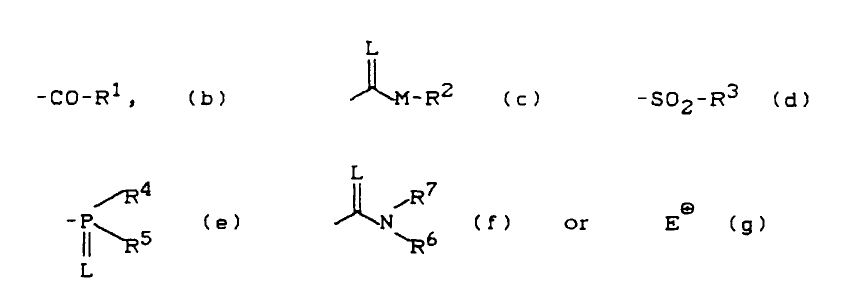
A and B together with the carbon atom to which they are bonded form a cycle which is substituted by alkoxy, alkylthio, alkylsulphoxyl or alkylsulphonyl, or
A and B together with the carbon atom to which they are bonded represent a cycle in which two substituents together with the carbon atoms to which they are bonded represent a saturated or monounsaturated cycle which is optionally substituted by alkyl, alkoxy or halogen and which can be interrupted by oxygen or sulphur,
E^{⊕} represents a metal ion equivalent or an ammonium ion,
L and M represent oxygen and/or sulphur,
R¹ represents optionally halogen-substituted alkyl, alkenyl, alkoxyalkyl, alkylthioalkyl, polyalkoxyalkyl or cycloalkyl which can be interrupted by hetero atoms, optionally substituted phenyl, optionally substituted phenylalkyl, substituted hetaryl, substituted phenoxyalkyl or substituted hetaryloxyalkyl and
R² represents alkyl, alkenyl, alkoxyalkyl or polyalkoxyalkyl, each of which is optionally substituted by halogen, or represents optionally substituted phenyl or benzyl,
R³, R⁴ and R⁵ independently of one another represent alkyl, alkoxy, alkylamino, dialkylamino, alkylthio, alkenylthio, alkinylthio or cycloalkylthio, each of which is optionally substituted by halogen, and optionally substituted phenyl, phenoxy or phenylthio,
R⁶ and R⁷ independently of one another represent hydrogen, or represent alkyl, alkenyl, alkoxy or alkoxyalkyl, each of which is optionally substituted by halogen, optionally substituted phenyl, optionally substituted benzyl,
or in which R⁶ and R⁷ together represent an alkylene radical which is optionally interrupted by oxygen or sulphur,
and the stereomerically and enantiomerically pure forms and their mixtures.

2. Process for the preparation of 3-aryl-4-hydroxy-Δ³-dihydrofuranone derivatives of the formula I according to Claim 1 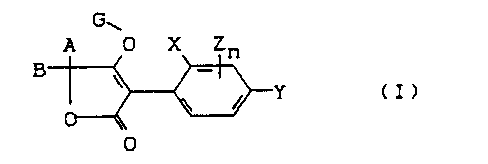 in which
X represents alkyl, halogen, alkoxy or halogenoalkyl,
Y represents hydrogen, alkyl, halogen, alkoxy or halogenoalkyl,
Z represents alkyl, halogen or alkoxy,
n represents a number from 0-3, or in which the radicals X and Z together with the phenyl radical to which they are bonded form the naphthalene radical of the formula 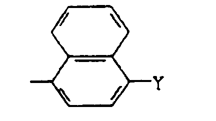 in which Y has the abovementioned meaning,
G represents hydrogen (a) or the groups 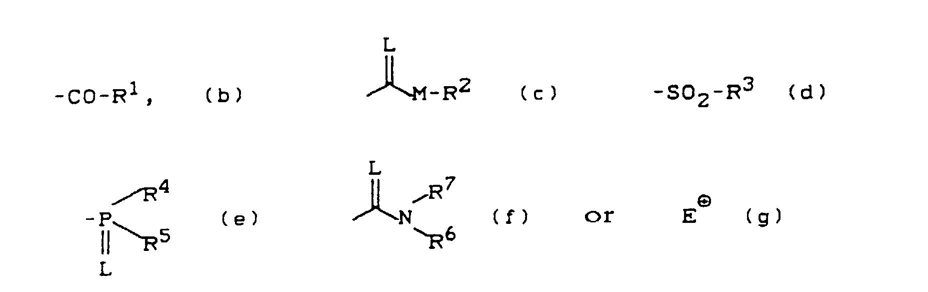
A and B together with the carbon atom to which they are bonded form a cycle which is substituted by alkoxy, alkoxythio or alkylsulphonyl, or
A and B together with the carbon atom to which they are bonded represent a cycle in which two substituents together with the carbon atoms to which they are bonded represent a saturated or monounsaturated cycle which is optionally substituted by alkyl, alkoxy or halogen and which can be interrupted by oxygen or sulphur,
E^{⊕} represents a metal ion equivalent or an ammonium ion,
L and M represent oxygen and/or sulphur,
R¹ represents optionally halogen-substituted alkyl, alkenyl, alkoxyalkyl, alkylthioalkyl, polyalkoxyalkyl or cycloalkyl which can be interrupted by hetero atoms, optionally substituted phenyl, optionally substituted phenylalkyl, substituted hetaryl, substituted phenoxyalkyl or substituted hetaryloxyalkyl and
R² represents alkyl, alkenyl, alkoxyalkyl or polyalkoxyalkyl, each of which is optionally substituted by halogen, or represents optionally substituted phenyl or benzyl,
R³ , R⁴ and R⁵ independently of one another represent alkyl, alkoxy, alkylamino, dialkylamino, alkylthio, alkenylthio, alkinylthio or cycloalkylthio, each of which is optionally substituted by halogen, and optionally substituted phenyl, phenoxy or phenylthio,
R⁶ and R⁷ independently of one another represent hydrogen, or represent alkyl, alkenyl, alkoxy or alkoxyalkyl, each of which is optionally substituted by halogen, optionally substituted phenyl, optionally substituted benzyl,
or in which R⁶ and R⁷ together represent an alkylene radical which is optionally interrupted by oxygen,
characterized in that
(A)
to prepare compounds of the formula Ia 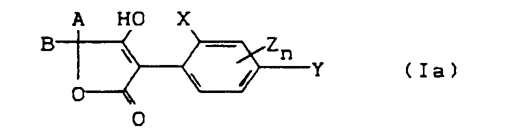 in which
A, B, X, Y, Z and n have the abovementioned meaning,
carboxylic esters of the formula (II) 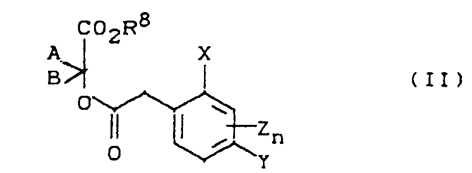 in which
A, B, X, Y, Z and n have the abovementioned meaning and
R⁸ represents alkyl,
are subjected to an intramolecular condensation reaction in the presence of a diluent and in the presence of a base, or
(B)
to prepare compounds of the formula (Ib) 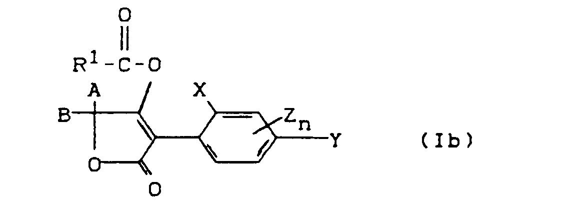 in which
A, B, X, Y, Z, R¹ and n have the abovementioned meaning,
compounds of the formula (Ia) 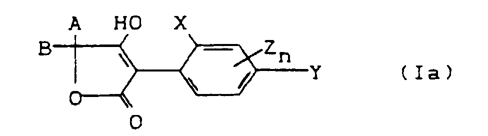 in which
A, B, X, Y, Z and n have the abovementioned meaning,
α) are reacted with acid halides of the general formula (III) 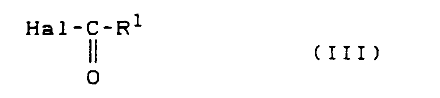 in which
R¹ has the abovementioned meaning
and
Hal represents halogen, in particular chlorine and bromine,
if appropriate in the presence of a diluent and if appropriate in the presence of an acid-binding agent, or
β) are reacted with carboxylic anhydrides of the general formula (IV)
R¹-CO-O-CO-R¹ (IV)
in which
R¹ has the abovementioned meaning
if appropriate in the presence of a diluent and if appropriate in the presence of an acid-binding agent, or
(C)
to prepare compounds of the formula (Ic) 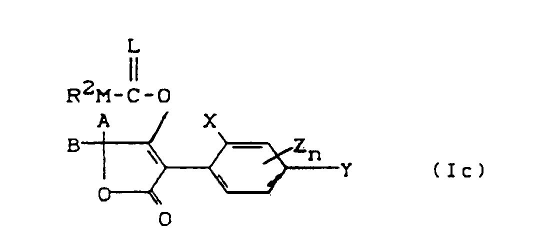 in which
A, B, X, Y, Z, R² and n have the abovementioned meaning
L represents oxygen
and
M represents oxygen or sulphur,
compounds of the formula (Ia) 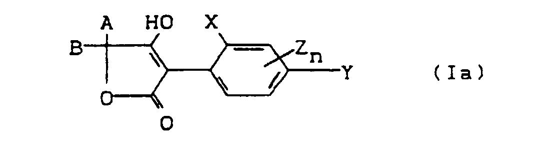 in which
A, B, X, Y, Z and n have the abovementioned meaning
are reacted with chloroformic esters or chloroformic thioesters of the general formula (V)
R²-M-CO-Cl (V)
in which
R² and M have the abovementioned meaning,
if appropriate in the presence of a diluent and if appropriate in the presence of an acid-binding agent, or
(D)
to prepare compounds of the formula (Ic) 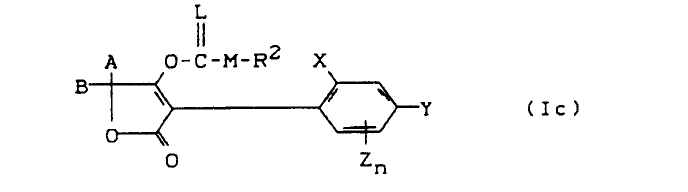 in which
A, B, R², X, Y, Z and n have the abovementioned meaning,
L represents sulphur
and
M represents oxygen or sulphur,
compounds of the formula (Ia) 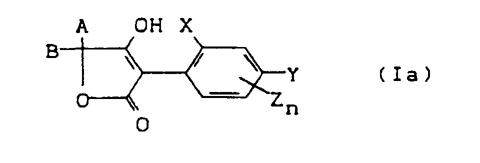 in which
A, B, X, Y, Z and n have the abovementioned meaning
are reacted with chloromonothioformic esters or chlorodithioformic esters of the general formula (VI) 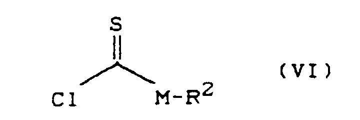 in which
M and R² have the abovementioned meaning,
if appropriate in the presence of a diluent and if appropriate in the presence of an acid-binding agent, or
(E)
to prepare compounds of the formula (Id) 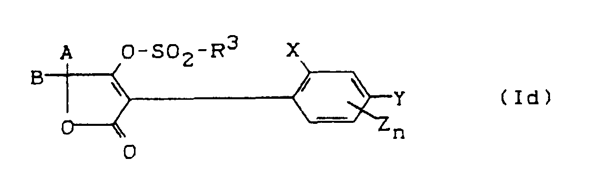 in which
A, B, X, Y, Z, R³ and n have the abovementioned meaning,
compounds of the formula (Ia) 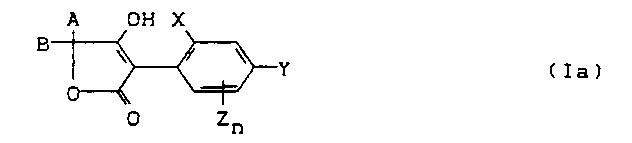 in which
A, B, X, Y, Z and n have the abovementioned meaning,
are reacted with sulphonyl chlorides of the general formula (VII)
R³-SO₂-Cl (VII)
in which
R³ has the abovementioned meaning,
if appropriate in the presence of a diluent and if appropriate in the presence of an acid-binding agent,
or
(F)
to prepare compounds of the formula (Ie) 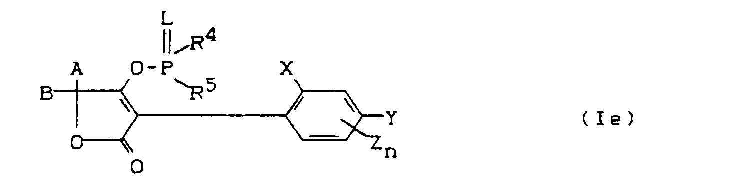 in which
A, B, L, X, Y, Z, R⁴, R⁵ and n have the abovementioned meaning,
compounds of the formula (Ia) 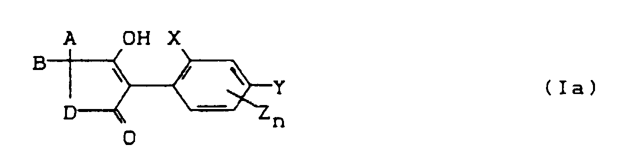 in which
A, B, X, Y, Z and n have the abovementioned meaning
are reacted with phosphorus compounds of the general formula (IX) 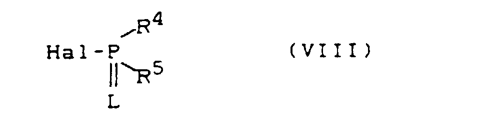 in which
L, R⁴ and R⁵ have the abovementioned meaning
and
Hal represents halogen, in particular chlorine and bromine,
if appropriate in the presence of a diluent and if appropriate in the presence of an acid-binding agent, or
(G)
to prepare compounds of the formula (If) 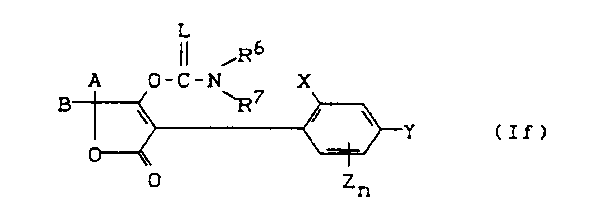 in which
A, B, L, X, Y, Z, R⁶, R⁷ and n have the abovementioned meaning,
compounds of the formula (Ia), 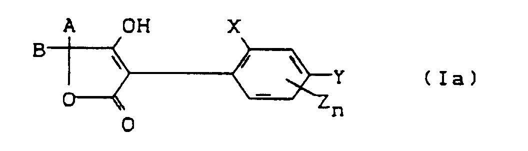 in which
A, B, X, Y, Z and n have the abovementioned meaning,
α) are reacted with isocyanates of the general formula (IX)
R⁶-N=C=L (IX)
in which
L and R⁶ have the abovementioned meaning,
if appropriate in the presence of a diluent and if appropriate in the presence of a catalyst,
or
β) are reacted with carbamoyl chlorides or thiocarbamoyl chlorides of the general formula (X) 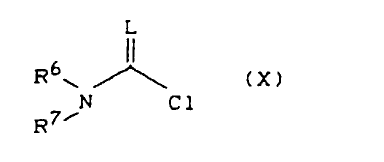 in which
L, R⁶ and R⁷ have the abovementioned meaning,
if appropriate in the presence of a diluent and if appropriate in the presence of an acid-binding agent, or
(H)
to prepare compounds of the formula (Ig) 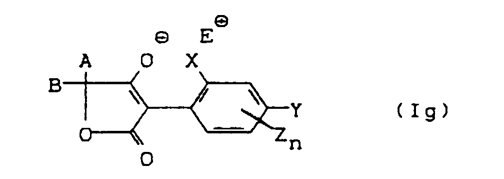 in which
X, Y, Z, A, B and n have the abovementioned meaning,
and E^{⊕} represents a metal ion equivalent or an ammonium ion,
compounds of the formula (Ia) 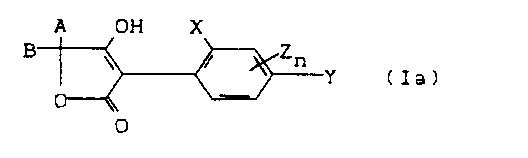 in which
X, Y, Z, A, B and n have the abovementioned meaning,
are reacted with metal hydroxides or amines of the general formulae (XI) and (XII) 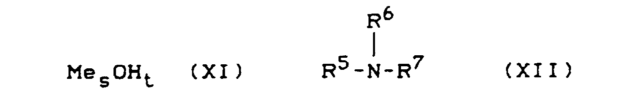 in which
Me represents mono- or divalent metal ions,
s and t represent the number 1 or 2 and
R⁵, R⁶ and R⁷ independently of one another represent hydrogen or alkyl,
if appropriate in the presence of a diluent.

3. 3-Aryl-4-hydroxy-Δ³-dihydrofuranone derivatives of the general formula (I) according to Claim 1 which, taking into consideration the various meanings of (a), (b), (c), (d), (e), (f) and (g) of group G have the following structures (Ia) to (Ig): 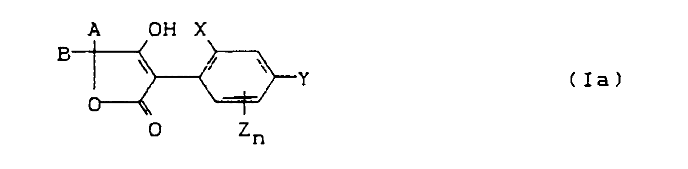 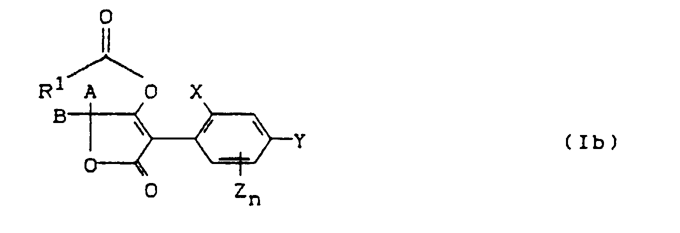 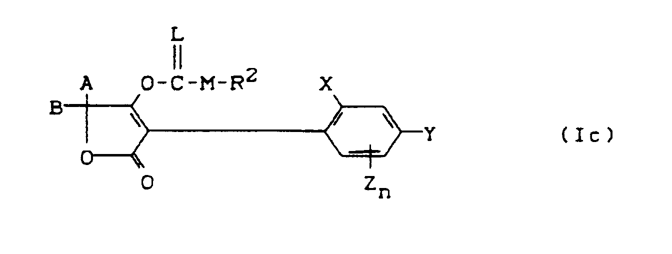 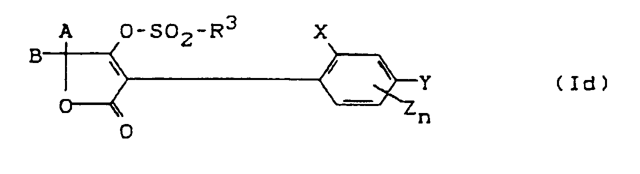 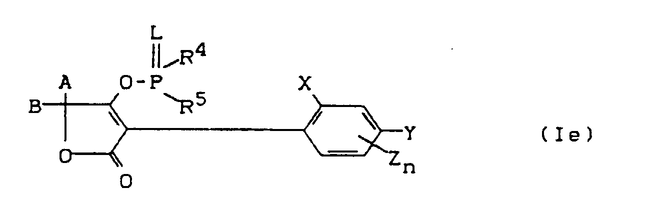 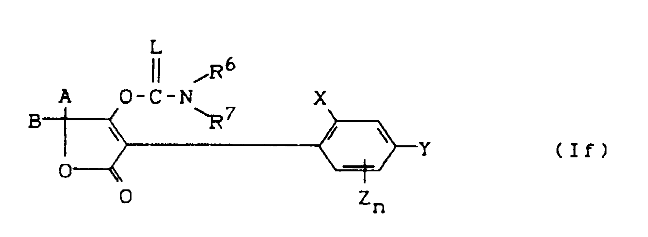 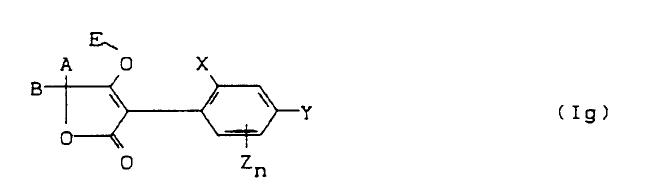 in which
A, B, E, L, M, X, Y, Zₙ, R¹, R², R³, R⁴, R⁵, R⁶ and R⁷ have the meaning given in Claim 1.

4. 3-Aryl-4-hydroxy-Δ³-dihydrofuranone derivatives of the general formula (I) according to Claim 1,
in which
X represents C₁-C₆-alkyl, halogen, C₁-C₆-alkoxy or C₁-C₃-halogenoalkyl,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0 to 3,
or in which the radicals X and Z together with the phenyl radical to which they are bonded form the naphthalene radical of the formula 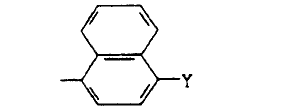 in which Y has the abovementioned meaning,
or in which
A and B together with the carbon atom to which they are bonded form a saturated or unsaturated 3- to 8-membered ring which is substituted by C₁-C₆-alkoxy, C₁-C₄-alkylthio, C₁-C₄-alkylsulphoxyl or C₁-C₄-alkylsulphonyl, or
A and B together with the carbon atom to which they are bonded represent a C₃-C₈-membered ring in which two substituents together with the carbon atoms to which they are bonded represent a saturated or monounsaturated C₅-C₇-ring which is optionally substituted by alkyl, alkoxy or halogen and which can be interrupted by oxygen or sulphur,
G represents hydrogen (a) or the groups 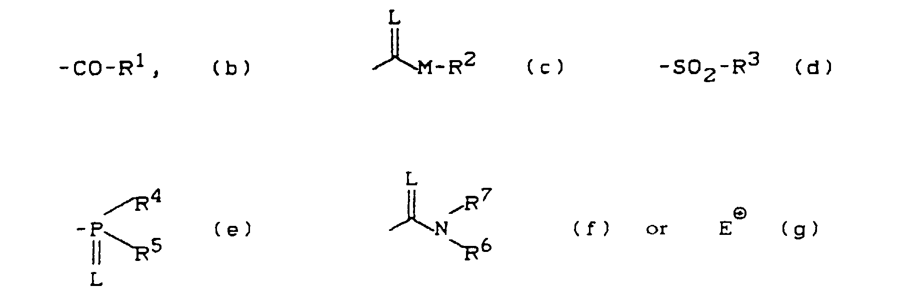 in which
E^{⊕} represents a metal ion equivalent or an ammonium ion,
L and M represent oxygen and/or sulphur,
R¹ represents optionally halogen-substituted: C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl or cycloalkyl having 3-8 ring atoms which can be interrupted by oxygen and/or sulphur atoms,
phenyl which is optionally substituted by halogen, nitro, C₁-C₆-alkyl, C₁-C₆-alkoxy, C₁-C₆-halogenoalkyl or C₁-C₆-halogenoalkoxy;
phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy
hetaryl which is optionally substituted by halogen and/or C₁-C₆-alkyl,
phenoxy-C₁-C₆-alkyl which is optionally substituted by halogen and C₁-C₆-alkyl,
hetaryloxy-C₁-C₆-alkyl which is optionally substituted by halogen, amino and C₁-C₆-alkyl,
R² represents C₁-C₂₀-alkyl, C₃-C₂₀-alkenyl, C₁-C₈-alkoxy-C₂-C₈-alkyl or C₁-C₈-polyalkoxy-C₂-C₈-alkyl, each of which is optionally substituted by halogen,
phenyl or benzyl, each of which is optionally substituted by halogen, nitro, C₁-C₆-alkyl, C₁-C₆-alkoxy or C₁-C₆-halogenoalkyl,
R³, R⁴ and R⁵ independently of one another represent optionally halogen-substituted C₁-C₈-alkyl, C₁-C₈-alkoxy, C₁-C₈-alkylamino, di-(C₁-C₈)-alkylamino, C₁-C₈-alkylthio, C₂-C₅-alkenylthio, C₂-C₅-alkinylthio or C₃-C₇-cycloalkylthio, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl,
R⁶ and R⁷ independently of one another represent C₁-C₂₀-alkyl, C₁-C₂₀-alkoxy, C₃-C₈-alkenyl or C₁-C₂₀-alkoxy-C₁-C₂₀-alkyl, each of which is optionally substituted by halogen, phenyl which is optionally substituted by halogen, C₁-C₂₀-halogenoalkyl, C₁-C₂₀-alkyl or C₁-C₂₀-alkoxy, benzyl which is optionally substituted by halogen, C₁-C₂₀-alkyl, C₁-C₂₀-halogenoalkyl or C₁-C₂₀-alkoxy, or together represent a C₄-C₆-alkylene ring which is optionally interrupted by oxygen or sulphur,
and the stereomerically and enantiomerically pure forms of these compounds and their mixtures.

5. 3-Aryl-4-hydroxy-Δ³-dihydrofuranone derivatives of the formula (I) according to Claim 1, in which
X represents C₁-C₆-alkyl, halogen, C₁-C₆-alkoxy or C₁-C₃-halogenoalkyl,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0 to 3,
or in which the radicals X and Z together with the phenyl radical to which they are bonded form the naphthalene radical of the formula 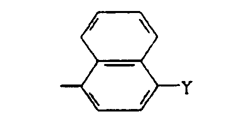 in which Y has the abovementioned meaning,
A and B together with the carbon atom to which they are bonded form a saturated or unsaturated, 5- to 7-membered ring which is substituted by C₁-C₅-alkoxy, C₁-C₃-alkylthio, C₁-C₃-alkylsulphoxyl or C₁-C₃-alkylsulphonyl, or
A and B together with the carbon atom to which they are bonded represent a C₄-C₇-membered ring in which two substituents together with the carbon atoms to which they are bonded represent a saturated or monounsaturated C₅-C₆-ring which is optionally substituted by C₁-C₃-alkyl, C₁-C₃-alkoxy, fluorine or chlorine and which can be interrupted by oxygen or sulphur,
G represents hydrogen (a) or the groups 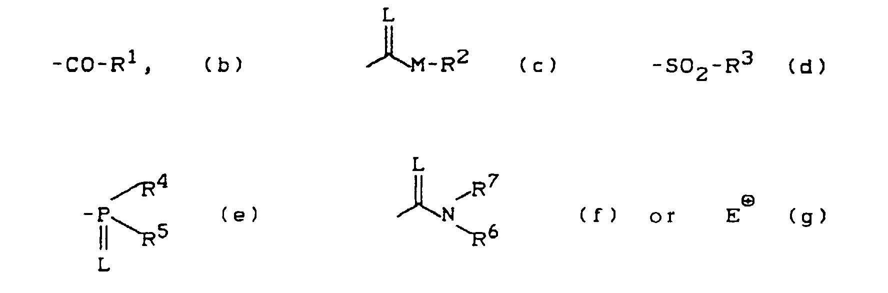 in which
E^{⊕} represents a metal ion equivalent or an ammonium ion,
L and M in each case represent oxygen and/or sulphur,
R¹ represents optionally halogen-substituted C₁-C₁₆-alkyl, C₂-C₁₆-alkenyl, C₁-C₆-alkoxy-C₂-C₆-alkyl, C₁-C₁₆-alkylthio-C₂-C₆-alkyl, C₁-C₆-polyalkoxy-C₂-C₆-alkyl and cycloalkyl having 3-7 ring atoms which can be interrupted by 1-2 oxygen and/or sulphur atoms,
phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl, or C₁-C₃-halogenoalkoxy,
hetaryl which is optionally substituted by halogen and C₁-C₆-alkyl,
phenoxy-C₁-C₅-alkyl which is optionally substituted by halogen and C₁-C₄-alkyl,
hetaryloxy-C₁-C₅-alkyl which is optionally substituted by halogen, amino and C₁-C₄-alkyl,
R² represents optionally halogen-substituted: C₁-C₁₆-alkyl, C₃-C₁₆-alkenyl, C₁-C₁₆-alkoxy-C₂-C_{6-alkyl} or C₁-C₆-polyalkoxy-C₂-C₆-alkyl,
phenyl or benzyl, each of which is optionally substituted by halogen, nitro, C₁-C₄-alkyl, C₁-C₃-alkoxy or C₁-C₃-halogenoalkyl,
R³ , R⁴ and R⁵ independently of one another represent C₁-C₆-alkyl, C₁-C₆-alkoxy, C₁-C₆-alkylamino, di (C₁-C₆)-alkylamino, C₁-C₆-alkylthio, C₃-C₄-alkenylthio, C₂-C₄-alklnylthio or C₃-C₆-cycloalkylthio, each of which is optionally substituted by halogen, or represent phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent C₁-C₂₀-alkyl, C₁-C₂₀-alkoxy, C₃-C₈-alkenyl or C₁-C₂₀-alkoxy-C₁-C₂₀-alkyl, each of which is optionally substituted by halogen, or represent phenyl which is optionally substituted by halogen, C₁-C₅-halogenoalkyl, C₁-C₅-alkyl or C₁-C₅-alkoxy, or represent benzyl which is optionally substituted by halogen, C₁-C₅-alkyl, C₁-C₅-halogenoalkyl or C₁-C₅-alkoxy, or together represent a C₄-C₆-alkylene ring which is optionally interrupted by oxygen or sulphur,
and their stereomerically and enantiomerically pure forms and their mixtures.

6. 3-Aryl-4-hydroxy-Δ³-dihydrofuranone derivatives of the formula (I) according to Claim 1, in which
X represents methyl, ethyl, propyl, i-propyl, fluorine, chlorine, bromine, methoxy, ethoxy and trifluoromethyl,
Y represents hydrogen, methyl, ethyl, propyl, i-propyl, butyl, i-butyl, tert-butyl, fluorine, chlorine, bromine, methoxy, ethoxy and trifluoromethyl,
Z represents methyl, ethyl, i-propyl, butyl, i-butyl, tert-butyl, fluorine, chlorine, bromine, methoxy and ethoxy,
n represents a number from 0 to 3,
or in which the radicals X and Z together with the phenyl radical to which they are bonded form the radical of the formula 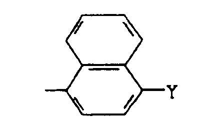 in which Y has the abovementioned meaning,
A and B together with the carbon atom to which they are bonded form a saturated or unsaturated 5- to 6-membered ring which is substituted by C₁-C₄-alkoxy, C₁-C₂-alkylthio, C₁-C₂-alkylsulphoxyl or C₁-C₂-alkylsulphonyl, or
A and B together with the carbon atom to which they are bonded represent a C₄-C₆-membered ring in which two substituents together with the carbon atoms to which they are bonded represent a saturated or monounsaturated C₅-C₆-ring which is optionally substituted by methyl, ethyl, methoxy, ethoxy, fluorine or chlorine and which can be interrupted by oxygen or sulphur,
G represents hydrogen (a) or the groups 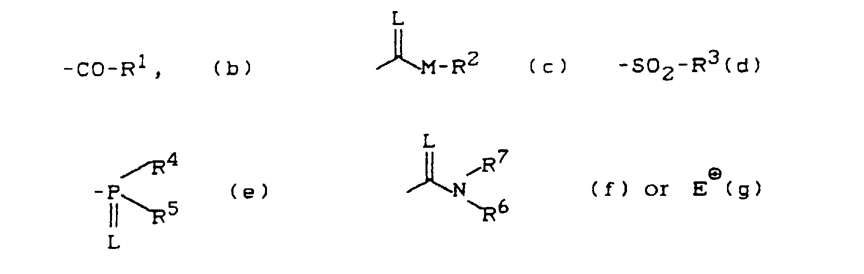 in which
E^{⊕} represents a metal ion equivalent or an ammonium ion,
L and M in each case represent oxygen and/or sulphur,
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl and cycloalkyl having 3-6 ring atoms which can be interrupted by 1-2 oxygen and/or sulphur atoms, or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents pyridyl, pyrimidyl, thiazolyl and pyrazolyl, each of which is optionally substituted by fluorine, chlorine, bromine, methyl or ethyl,
or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxy-C₁-C₄-alkyl, each of which is optionally substituted by fluorine, chlorine, amino, methyl or ethyl,
R² represents C₁-C₁₄-alkyl, C₃-C₁₄-alkenyl, C₁-C₄-alkoxy-C₂-C₆-alkyl or C₁-C₄-polyalkoxy-C₂-C₆-alkyl, each of which is optionally substituted by fluorine or chlorine,
or represents phenyl or benzyl, each of which is optionally substituted by fluorine, chlorine, nitro, methyl, ethyl, propyl, i-propyl, methoxy, ethoxy, or trifluoromethyl,
R³, R⁴ and R⁵ independently of one another represent C₁-C₄-alkyl, C₁-C₄-alkoxy, C₁-C₄-alkylamino, di-(C₁-C₄-alkyl)-amino or C₁-C₄-alkylthio, each of which is optionally substituted by fluorine or chlorine, or represents phenyl, phenoxy or phenylthio, each of which is optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl,
R⁶ and R⁷ independently of one another represent C₁-C₁₀-alkyl, C₃-C₆-alkenyl, C₁-C₁₀-alkoxy or C₁-C₁₀-alkoxy-(C₁-C₁₀)alkyl, each of which is optionally substituted by fluorine, chlorine or bromine, or represent phenyl which is optionally substituted by fluorine, chlorine, bromine, C₁-C₂₀-halogenoalkyl, C₁-C₂₀-alkyl or C₁-C₄-alkoxy, or represent benzyl which is optionally substituted by fluorine, chlorine, bromine, C₁-C₄-alkyl, C₁-C₄-halogenoalkyl or C₁-C₄-alkoxy, or together represent a C₄-C₆-alkylene ring which is optionally interrupted by oxygen or sulphur,
and their stereomerically and enantiomerically pure forms and their mixtures.

7. Compounds of the formula (II) 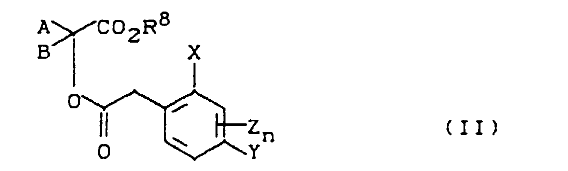 in which
A, B, X, Y, Z, n and R⁸ have the meaning given in Claim 1.

8. Process for the preparation of the O-acyl-α-hydroxy carboxylic esters of the formula (II) according to Claim 7, characterized in that
a) 2-hydroxy carboxylic acid (esters) of the formula (XIII) 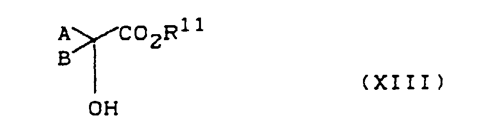 in which
R¹¹ represents hydrogen in the case of compounds of the formula (XIIIa) or alkyl in the case of compounds of the formula (XIIIb) and
A and B have the meaning given in Claim 1,
are acylated with phenylacetyl halides of the formula (XIV) 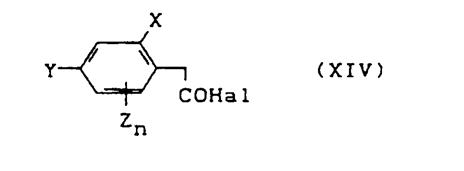 in which
X, Y, Z and n have the abovementioned meaning and
Hal represents chlorine or bromine,
or when hydroxy carboxylic acids of the formula (IIa) 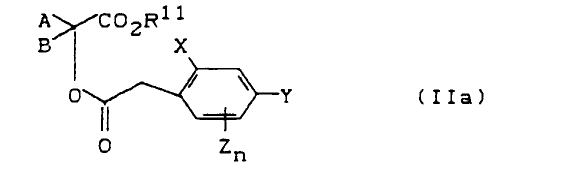 in which
A, B, X, Y, Z and n have the abovementioned meaning
and
R¹¹ represents hydrogen
are esterified.

9. Pesticides, characterized in that they contain at least one 3-aryl-4-hydroxy-Δ³-dihydrofuranone derivative of the formula (I) according to Claim 1.

10. Use of 3-aryl-4-hydroxy-Δ³-dihydrofuranone derivatives of the formula (I) according to Claim 1 for combating pests.

11. Process for the preparation of pesticides, characterized in that 3-aryl-4-hydroxy-Δ³-dihydrofuranone derivatives of the formula (I) according to Claim 1. are mixed with extenders and/or surface-active agents.

## Revendications

1. Dérivés de 3-aryl-4-hydroxy-Δ³-dihydrofurannones de formule générale (I) 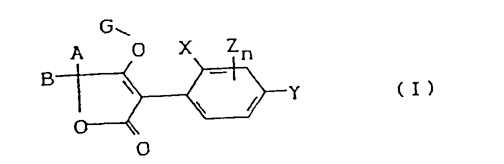 dans laquelle
X représente un groupe alkyle, un halogène, un groupe alkoxy ou halogénalkyle,
Y représente de l'hydrogène, un groupe alkyle, un halogène, un groupe alkoxy, halogénalkyle,
Z représente un groupe alkyle, un halogène, un groupe alkoxy,
n est un nombre de 0 à 3, les restes X et Z formant conjointement avec le reste phényle auquel ils sont liés le reste naphtalène de formule 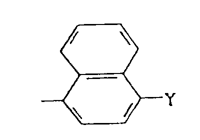 dans laquelle Y a la définition indiquée ci-dessus,
G représente de l'hydrogène (a) ou les groupes 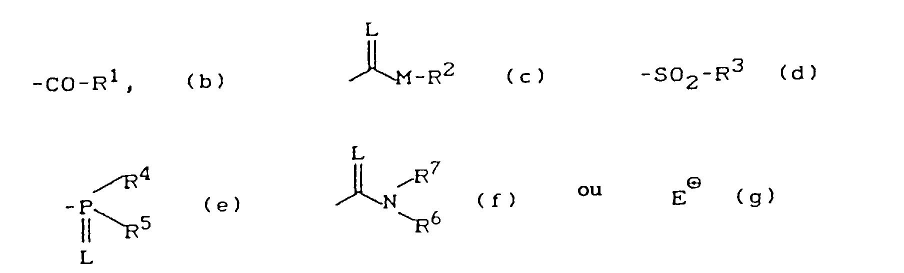
A et B forment, conjointement avec l'atome de carbone auquel ils sont liés, un cycle substitué par un radical alkoxy, alkylthio, alkylsulfoxyle ou alkylsulfonyle, ou bien
A et B forment, conjointement avec l'atome de carbone auquel ils sont liés, un cycle dans lequel deux substituants forment, conjointement avec les atomes de carbone auxquels ils sont liés, un cycle saturé ou mono-insaturé qui est éventuellement substitué par un radical alkyle, alkoxy ou halogéno et qui peut être interrompu par de l'oxygène ou du soufre,
E⁺ désigne un équivalent d'ion métallique ou un ion ammonium,
L et M représentent de l'oxygène et/ou du soufre,
R¹ est un groupe, éventuellement substitué par un halogène, alkyle, alcényle, alkoxyalkyle, alkylthioalkyle, polyalkoxyalkyle, ou cycloalkyle qui peut être interrompu par des hétéroatomes, un groupe phényle éventuellement substitué, phénylalkyle éventuellement substitué, hétaryle éventuellement substitué, phénoxyalkyle éventuellement substitué ou hétaryloxyalkyle éventuellement substitué, et
R² représente un groupe, éventuellement substitué par un halogène, alkyle, alcényle, alkoxyalkyle, polyalkoxyxalkyle ou un groupe phényle ou benzyle éventuellement substitué,
R³, R⁴ et R⁵ représentent indépendamment les uns des autres un groupe, éventuellement substitué par un halogène, alkyle, alkoxy, alkylamino, dialkylamino, alkylthio, alcénylthio, alcynylthio, cycloalkylthio, et un groupe phényle, phénoxy ou phénylthio éventuellemnet substitué,
R⁶ et R⁷ représentent, indépendamment l'un de l'autre, de l'hydrogène, un groupe, éventuellement substitué par un halogène, alkyle, alcényle, alkoxy, alkoxyalkyle, un groupe phényle éventuellement substitué, un groupe benzyle éventuellement substitué. ou bien
R⁶ et R⁷ forment ensemble un reste alkylène éventuellement interrompu par de l'oxygène,
ainsi que les formes stéréo-isomères et énantiomères pures et leurs mélanges.

2. Procédé de production des dérivés de 3-aryl-4-hydroxy-Δ³-dihydrofurannones de formule I suivant la revendication 1 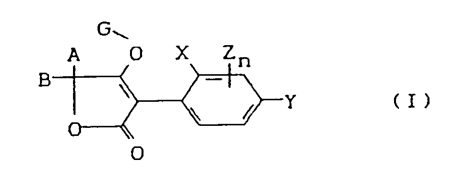 dans laquelle
X représente un groupe alkyle, un halogène, un groupe alkoxy ou halogénalkyle,
Y représente de l'hydrogène, un groupe alkyle, un halogène, un groupe alkoxy, halogénalkyle,
Z représente un groupe alkyle, un halogène, un groupe alkoxy,
n est un nombre de 0 à 3, les restes X et Z formant, conjointement avec le reste phényle auquel ils sont liés, le reste naphtalène de formule 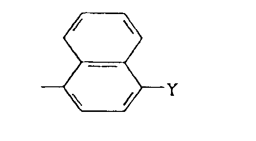 dans laquelle Y a la définition indiquée ci-dessus,
G représente de l'hydrogène (a) ou les groupes 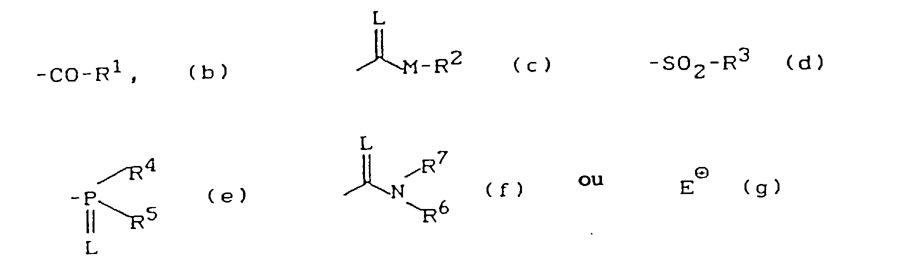
A et B forment, conjointement avec l'atome de carbone auquel ils sont liés, un cycle substitué par un radical alkoxy, alkoxythio ou alkylsulfonyle, ou bien
A et B forment, conjointement avec l'atome de carbone auquel ils sont liés, un cycle dans lequel deux substituants forment, conjointement avec les atomes de carbone auxquels ils sont liés, un cycle saturé ou mono-insaturé qui est éventuellement substitué par un radical alkyle, alkoxy ou halogéno et qui peut être interrompu par de l'oxygène ou du soufre,
E^{⊕} désigne un équivalent d'ion métallique ou un ion ammonium,
L et M représentent de l'oxygène et/ou du soufre,
R¹ est un groupe, éventuellement substitué par un halogène, alkyle, alcényle, alkoxyalkyle, alkylthioalkyle, polyalkoxyalkyle, ou cycloalkyle qui peut être interrompu par des hétéroatomes, un groupe phényle éventuellement substitué, phénylalkyle éventuellement substitué, hétaryle éventuellement substitué, phénoxyalkyle éventuellement substitué ou hétaryloxyalkyle éventuellement substitué, et
R² représente un groupe, éventuellement substitué par un halogène, alkyle, alcényle, alkoxyalkyle, polyalkoxyxalkyle ou un groupe phényle ou benzyle éventuellement substitué,
R³, R⁴ et R⁵ représentent indépendamment les uns des autres un groupe, éventuellement substitué par un halogène, alkyle, alkoxy, alkylamino, dialkylamino, alkylthio, alcénylthio, alcynylthio, cycloalkylthio, et un groupe phényle, phénoxy ou phénylthio éventuellemnet substitué,
R⁶ et R⁷ représentent, indépendamment l'un de l'autre, de l'hydrogène, un groupe, éventuellement substitué par un halogène, alkyle, alcényle, alkoxy, alkoxyalkyle, un groupe phényle éventuellement substitué, un groupe benzyle éventuellement substitué, ou bien
R⁶ et R⁷ forment ensemble un reste alkylène éventuellement interrompu par de l'oxygène,
caractérisé en ce que
(A)
pour la production de composés de formule Ia 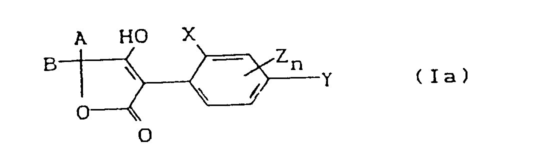 dans laquelle
A, B, X, Y, Z et n ont la définition indiquée ci-dessus,
on effectue la condensation intramoléculaire d'esters d'acides carboxyliques de formule (II) 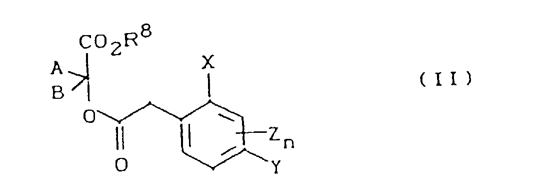 dans laquelle
A, B, X, Y, Z et n ont la définition indiquée ci-dessus,
et
R⁸ représente un groupe alkyle,
en présence d'un diluant et en présence d'une base, ou bien
(B)
pour la production de composés de formule (Ib) 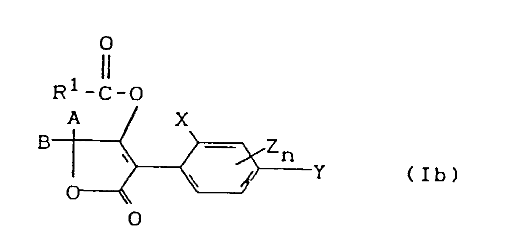 dans laquelle
A, B, X, Y, Z, R¹ et n ont la définition indiquée ci-dessus, on fait réagir des composés de formule (Ia) 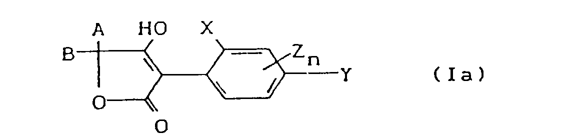 dans laquelle
A, B, X, Y, Z et n ont la définition indiquée ci-dessus,
α) avec des halogénures d'acides de formule générale (III) 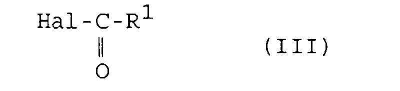 dans laquelle
R¹ a la définition indiquée ci-dessus,
et,
Hal représente un halogène, en particulier le chlore et le brome,
le cas échéant en présence d'un diluant et en la présence éventuelle d'un accepteur d'acide,
ou bien
β) avec des anhydrides d'acides carboxyliques de formule générale (IV)
R¹-CO-O-CO-R¹ (IV)
dans laquelle
R¹ a la définition indiquée ci-dessus,
le cas échéant en présence d'un diluant et en présence éventuelle d'un accepteur d'acide,
ou bien
(C)
pour la production de composés de formule (Ic) 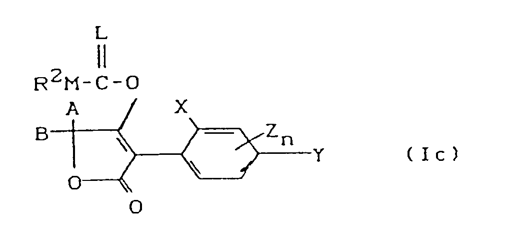 dans laquelle
A, B, X, Y, Z, R² et n ont la définition indiquée ci-dessus,
L est de l'oxygène
et
M est de l'oxygène ou du soufre,
on fait réagir des composés de formule (Ia) 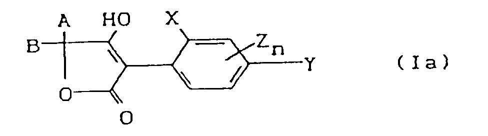 dans laquelle
A, B, X, Y, Z et n ont la définition indiquée ci-dessus,
avec un ester d'acide chloroformique ou un thiolester d'acide chloroformique de formule générale (V)
R²-M-CO-Cl (V)
dans laquelle
R² et m ont la définition indiquée ci-dessus,
le cas échéant en présence d'un diluant et en la présence éventuelle d'un accepteur d'acide, ou bien
(D)
pour la production de composés de formule (Ic) 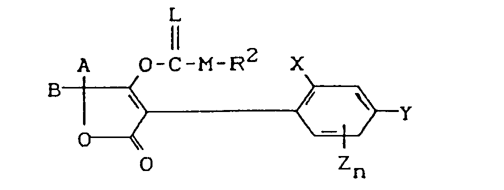 dans laquelle
A, B, R², X, Y, Z et n ont la définition indiquée ci-dessus,
L est du soufre
et
M est de l'oxygène ou du soufre,
on fait réagir des composés de formule (Ia) 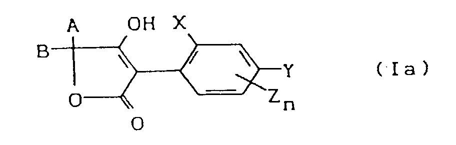 dans laquelle
A, B, X, Y, Z et n ont la définition indiquée ci-dessus
avec des esters d'acide chloromonothioformique ou des esters d'acide chlorodithioformique de formule générale (VI) 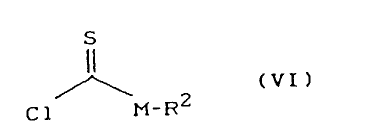 dans laquelle
M et R² ont la définition indiquée ci-dessus
le cas échéant en présence d'un diluant et en la présence éventuelle d'un accepteur d'acide, ou bien
(E)
pour la production de composés de formule (Id) 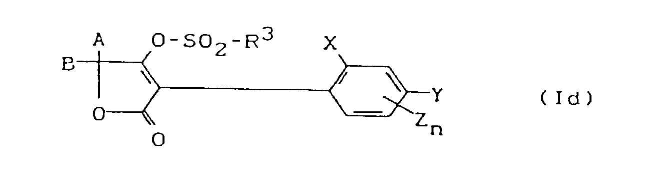 dans laquelle
A, B, X, Y, Z, R³ et n ont la définition indiquée ci-dessus,
on fait réagir des composés de formule (Ia) 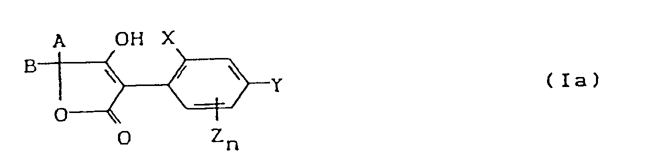 dans laquelle
A, B, X, Y, Z et n ont la définition indiquée ci-dessus,
avec des chlorures d'acide sulfonique de formule générale (VII)
R³-SO₂-Cl (VII)
dans laquelle
R³ a la définition indiquée ci-dessus,
le cas échéant en présence d'un diluant et en la présence éventuelle d'un accepteur d'acide,
(F)
pour la production de composés de formule (Ie) 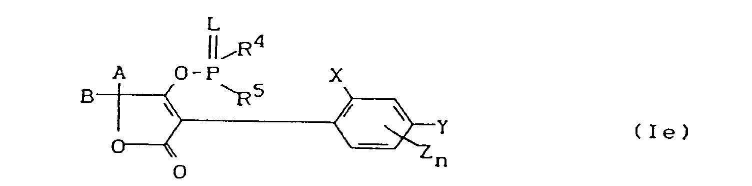 dans laquelle
A, B, L, X, Y, Z, R⁴, R⁵ et n ont la définition indiquée ci-dessus,
on fait réagir des composés de formule (Ia) 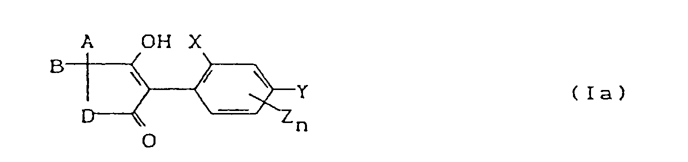 dans laquelle
A, B, X, Y, Z et n ont la définition indiquée ci-dessus
avec des composés de phosphore de formule générale (VIII) 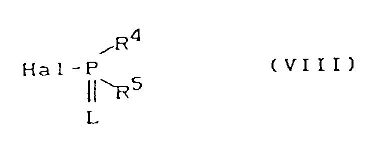 dans laquelle
L, R⁴ et R⁵ ont la définition indiquée ci-dessus,
et
Hal représente un halogène, en particulier le chlore ou le brome,
le cas échéant en présence d'un diluant et en présence éventuelle d'un accepteur d'acide, ou bien
(G)
pour la production de composés de formule (If) 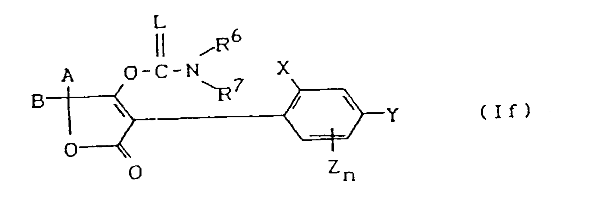 dans laquelle
A, B, L, X, Y, Z, R⁶, R⁷ et n ont la définition indiquée ci-dessus,
on fait réagir des composés de formule (Ia), 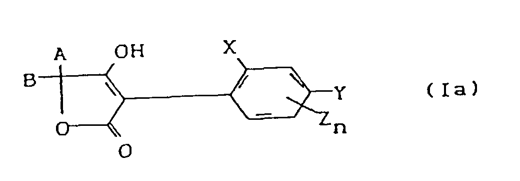 dans laquelle
A, B, X, Y, Z et n ont la définition indiquée ci-dessus
α) avec des isocyanates de formule générale (IX)
R⁶-N=C=L (IX)
dans laquelle
L et R⁶ ont la définition indiquée ci-dessus
le cas échéant en présence d'un diluant et en la présence éventuelle d'un catalyseur,
ou bien
β) avec des chlorures d'acide carbamique ou des chlorures d'acide thiocarbamique de formule générale (X) 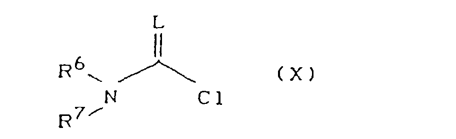 dans laquelle
L, R⁶ et R⁷ ont la définition indiquée ci-dessus,
le cas échéant en présence d'un diluant et en la présence éventuelle d'un accepteur d'acide, ou bien
(H)
pour la production de composés de formule (Ig) 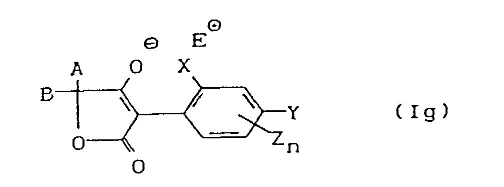 dans laquelle
X, Y, Z, A, B et n ont la définition indiquée ci-dessus,
et E^{⊕} représente un équivalent d'ion métallique ou un ion ammonium,
on fait réagir des composés de formule (Ia) 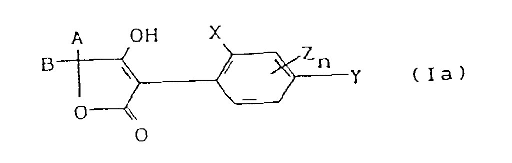 dans laquelle
X, Y, Z, A, B et n ont la définition indiquée ci-dessus, avec des hydroxydes de métaux ou des amines de formules générales (XI) et (XII) 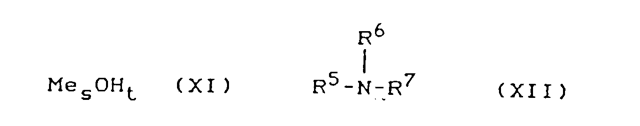 dans lesquelles
Me désigne un ion métallique monovalent ou divalent
s et t représentent le nombre 1 ou 2 et
R⁵, R⁶ et R⁷ représentent, indépendamment les uns des autres, de l'hydrogène et un groupe alkyle,
le cas échéant en présence d'un diluant.

3. Dérivés de 3-aryl-4-hydroxy-Δ³-dihydrofurannones de formule générale (I) suivant la revendication 1 qui possèdent, en faisant intervenir les différentes définitions de (a), (b), (c), (d), (e), (f) et (g) du groupe G, les structures (Ia) à (Ig) suivantes : 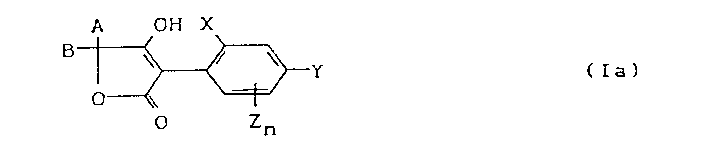 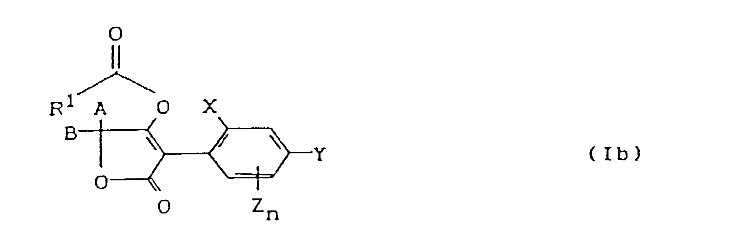 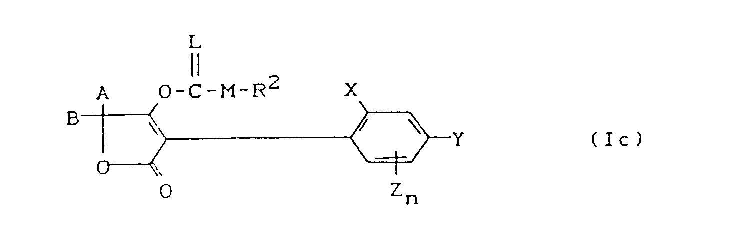 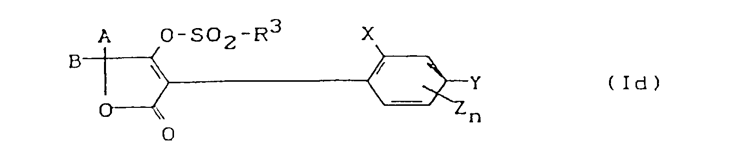 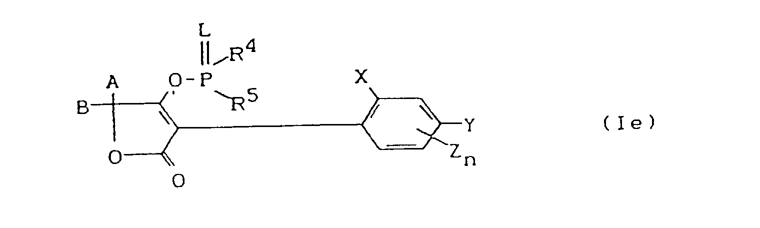 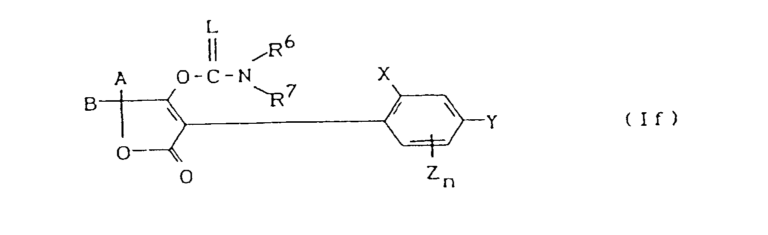 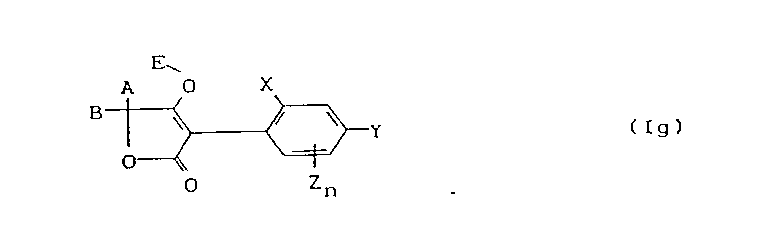 dans lesquelles
A, B, E, L, M, X, Y, Zₙ, R¹, R², R³, R⁴, R⁵, R⁶ et R⁷ possèdent les définitions indiquées dans la revendication 1.

4. Dérivés de 3-aryl-4-hydroxy-Δ³-dihydrofurannones de formule générale (I) suivant la revendication 1, dans laquelle
X représente un groupe alkyle en C₁ à C₆, un halogène, un groupe alkoxy en C₁ à C₆ ou halogénalkyle en C₁ à C₃,
Y est de l'hydrogène, un groupe alkyle en C₁ à C₆, un halogène, un groupe alkoxy en C₁ à C₆ ou halogénalkyle en C₁ à C₃,
Z représente un groupe alkyle en C₁ à C₆, un halogène, un groupe alkoxy en C₁ à C₆,
n représente un nombre de 0 à 3,
ou bien les restes X et Z forment, conjointement avec le reste phényle auquel ils sont liés, le reste naphtalène de formule 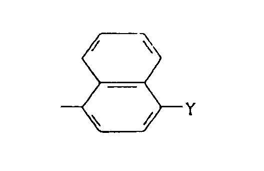 dans laquelle Y a la définition indiquée ci-dessus,
ou dans laquelle
A et B forment, conjointement avec l'atome de carbone auquel ils sont liés, un noyau saturé ou non saturé, de 3 à 8 chaînons, substitué par un radical alkoxy en C₁ à C₆, alkylthio en C₁ à C₄, alkylsulfoxyle en C₁ à C₄ ou alkylsulfonyle en C₁ à C₄, ou bien
A et B forment, conjointement avec l'atome de carbone auquel ils sont liés, un noyau en C₃ à C₈ dans lequel deux substituants forment, conjointement avec les atomes de carbone auxquels ils sont liés, un noyau en C₅ à C₇ saturé ou mono-insaturé qui est éventuellement substitué par un radical alkyle, alkoxy ou halogéno et qui peut être interrompu par de l'oxygène ou du soufre,
G représente de l'hydrogène (a) ou les groupes 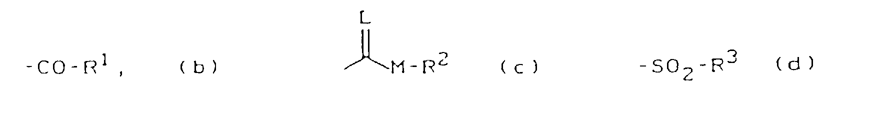 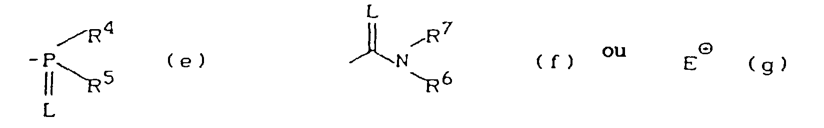 dans lesquels
E ⁺ représente un équivalent d'ion métallique ou un ion ammonium,
L et M représentent de l'oxygène et/ou du soufre,
R¹ est un groupe, éventuellement substitué par un halogène : alkyle en C₁ à C₂₀, alcényle en C₂ à C₂₀, (alkoxy en C₁ à C₈)-(alkyle en C₂ à C₈), (alkylthio en C₁ à C₈)-(alkyle en C₂ à C₈), (polyalkoxy en C₁ à C₈)-(alkyle en C₂ à C₈) ou cycloalkyle à noyau de 3 à 8 atomes, qui peut être interrompu par des atomes d'oxygène et/ou de soufre,
un groupe phényle éventuellement substitué par un halogène, un radical nitro, alkyle en C₁ à C₆, alkoxy en C₁ à C₆, halogénalkyle en C₁ à C₆, halogénalkoxy en C₁ à C₆ ;
un groupe phényl-(alkyle en C₁ à C₆) éventuellement substitué par un halogène, un radical alkyle en C₁ à C₆, alkoxy en C₁ à C₆, halogénalkyle en C₁ à C₆, halogénalkoxy en C₁ à C₆,
un groupe hétaryle éventuellement substitué par un halogène et/ou un radical alkyle en C₁ à C₆, un groupe phénoxy-(alkyle en C₁ à C₆) éventuellement substitué par un halogène et un radical alkyle en C₁ à C₆, un groupe hétaryloxy-(alkyle en C₁ à C₆) éventuellement substitué par un halogène, un radical amino et alkyle en C₁ à C₆,
R² est un groupe, éventuellement substitué par un halogène, alkyle en C₁ à C₂₀, alcényle en C₂ à C₂₀, (alkoxy en C₁ à C₈)-(alkyle en C₂ à C₈₎, (polyalkoxy en C₁ à C₈) - (alkyle en C₂ à C₈), un groupe phényle ou benzyle éventuellement substitué par un halogène, un radical nitro, alkyle en C₁ à C₆, alkoxy en C₁ à C₆, halogénalkyle en C₁ à C₆,
R³, R⁴ et R⁵ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)amino, alkylthio en C₁ à C₈, alcénylthio en C₂ à C₅, alcynylthio en C₂ à C₅, cycloalkylthio en C₃ à C₇, un groupe phényle, phénoxy ou phénylthio éventuellement substitué par un halogène, un radical nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₃ à C₈, (alkoxy en C₁ à C₂₀)-(alkyle en C₁ à C₂₀), un groupe phényle éventuellement substitué par un halogène, un radical halogénalkyle en C₁ à C₂₀, alkyle en C₁ à C₂₀ ou alkoxy en C₁ à C₂₀, un groupe benzyle éventuellement substitué par un halogène, un radical alkyle en C₁ à C₂₀, halogénalkyle en C₁ à C₂₀ ou alkoxy en C₁ à C₂₀, ou forment ensemble un noyau alkylène en C₄ à C₆ éventuellement interrompu par de l'oxygène ou du soufre,
ainsi que les formes stéréo-isomères et énantiomères pures de ces composés et leurs mélanges.

5. Dérivés de 3-aryl-4-hydroxy-Δ³-dihydrofuran-nones de formule (I) suivant la revendication 1, dans laquelle
X représente un groupe alkyle en C₁ à C₆, un halogène, un groupe alkoxy en C₁ à C₆ ou halogénalkyle en C₁ à C₃,
Y est de l'hydrogène, un groupe alkyle en C₁ à C₆, un halogène, un groupe alkoxy en C₁ à C₆, halogénalkyle en C₁ à C₃,
Z est un groupe alkyle en C₁ à C₆, un halogène, un groupe alkoxy en C₁ à C₆,
n représente un nombre de 0 à 3,
ou bien les restes X et Z forment, conjointement
avec le reste phényle auquel ils sont liés, le reste naphtalène de formule 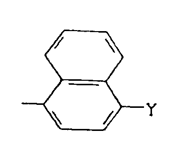 dans laquelle Y a la définition indiquée ci-dessus,
A et B forment, conjointement avec l'atome de carbone auquel ils sont liés, un noyau pentagonal à heptagonal saturé ou non saturé, substitué par un radical alkoxy en C₁ à C₅, alkylthio en C₁ à C₃, alkylsulfoxyle en C₁ à C₃ ou alkylsulfonyle en C₁ à C₃, ou bien
A et B forment, conjointement avec le carbone auquel ils sont liés, un noyau de 4 à 7 chaînons dans lequel deux substituants forment, conjointement avec les atomes de carbone auxquels ils sont liés, un noyau pentagonal ou hexagonal saturé ou mono-insaturé éventuellement substitué par un radical alkyle en C₁ à C₃, alkoxy en C₁ à C₃, du fluor ou du chlore et pouvant être interrompu par de l'oxygène ou du soufre,
G représente de l'hydrogène (a) ou les groupes 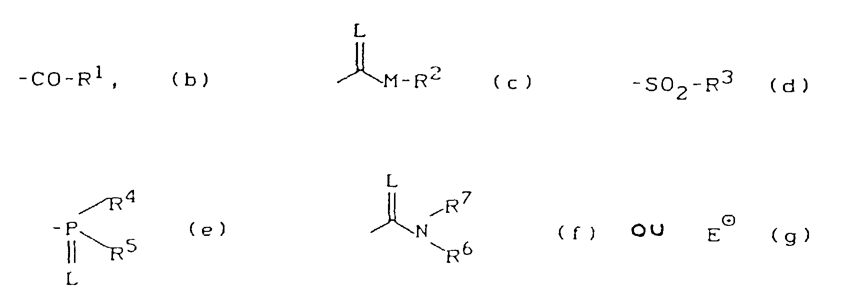 dans lesquels
E^{⊕} désigne un équivalent d'ion métallique ou un ion ammonium
L et M représentent chacun de l'oxygène et/ou du soufre,
R¹ est un groupe, éventuellement substitué par un halogène, alkyle en C₁ à C₁₆, alcényle en C₂ à C₁₆, (alkoxy en C₁ à C₆)-(alkyle en C₂ à C₆), (alkylthio en C₁ à C₁₆)-(alkyle en C₂ à C₆), (polyalkoxy en C₁ à C₆)-(alkyle en C₂ à C₆) et cycloalkyle à noyau de 3 à 7 atomes, qui peut être interrompu par 1 ou 2 atomes d'oxygène et/ou de soufre,
un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃,
un groupe phényl-(alkyle en C₁ à C₄) éventuellement substitué par un halogène, un radical alkyle en C₁ à C₄, alkoxy en C₁ à C₄, halogénalkyle en C₁ à C₃, halogénalkoxy en C₁ à C₃,
un groupe hétaryle éventuellement substitué par un halogène et un radical alkyle en C₁ à C₆,
un groupe phénoxy-(alkyle en C₁ à C₅) éventuellement substitué par un halogène et un radical alkyle en C₁ à C₄,
un groupe hétaryloxy-(alkyle en C₁ à C₅) éventuellement substitué par un halogène, un radical amino et alkyle en C₁ à C₄,
R² est un groupe, éventuellement substitué par un halogène : alkyle en C₁ à C₁₆, alcényle en C₃ à C₁₆, (alkoxy en C₁ à C₁₆)-(alkyle en C₂ à C₆), (polyalkoxy en C₁ à C₆)-(alkyle en C₂ à C₆), un groupe phényle ou benzyle éventuellement substitué par un radical halogéno, nitro, alkyle en C₁ à C₄, alkoxy en C₁ à C₃, halogénalkyle en C₁ à C₃,
R³, R⁴ et R⁵ représentent, indépendamment les uns des autres, un groupe, éventuellement substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, phénoxy ou phénylthio éventuellement substitué par du fluor, du chlore, du brome, un radical nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₃ à C₈, (alkoxy en C₁ à C₂₀)-(alkyle en C₁ à C₂₀), un groupe phényle éventuellement substitué par un halogène, un radical halogénalkyle en C₁ à C₅, alkyle en C₁ à C₅ ou alkoxy en C₁ à C₅, un groupe benzyle éventuellement substitué par un halogène, un radical alkyle en C₁ à C₅, halogénalkyle en C₁ à C₅ ou alkoxy en C₁ à C₅, ou forment ensemble un noyau alkylène en C₄ à C₆ éventuellement interrompu par de l'oxygène ou du soufre,
ainsi que leurs formes stéréo-isomères et énantiomères pures et leurs mélanges.

6. Dérivés de 3-aryl-4-hydroxy-Δ³-dihydrofuran-nones de formule (I) suivant la revendication 1, dans laquelle
X représente les groupes méthyle, éthyle, propyle, isopropyle, du fluor, du chlore, du brome, les groupes méthoxy, éthoxy et trifluorométhyle,
Y représente de l'hydrogène, les groupes méthyle, éthyle, propyle, isopropyle, butyle, isobutyle, tertiobutyle, du fluor, du chlore, du brome, les groupes méthoxy, éthoxy et trifluorométhyle,
Z représente les groupes méthyle, éthyle, isopropyle, butyle, isobutyle, tertiobutyle, le fluor, le chlore, le brome, les groupes méthoxy et éthoxy,
n représente un nombre de 0 à 3,
ou bien les restes X et Z forment, conjointement avec le reste phényle auquel ils sont liés, le reste de formule 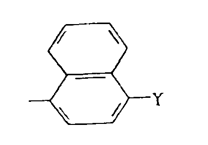 dans laquelle Y a la définition indiquée ci-dessus,
A et B forment, conjointement avec l'atome de carbone auquel ils sont liés, un noyau pentagonal ou hexagonal saturé ou non saturé, substitué par un radical alkoxy en C₁ à C₄, alkylthio en C₁ ou C₂, alkylsulfoxyle en C₁ ou C₂ ou alkylsulfonyle en C₁ ou C₂, ou bien
A et B forment, conjointement avec l'atome de carbone auquel ils sont liés, un noyau de 4 à 6 chaînons, dans lequel deux substituants forment, conjointement avec les atomes de carbone auxquels ils sont liés, un noyau pentagonal ou hexagonal saturé ou mono-insaturé éventuellement substitué par un radical méthyle, éthyle, méthoxy, éthoxy, fluoro ou chloro et pouvant être interrompu par de l'oxygène ou du soufre,
G représente de l'hydrogène (a) ou les groupes 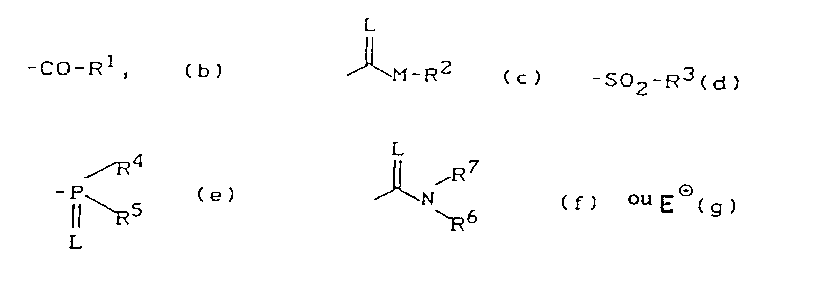 dans lesquels
E^{⊕} représente un équivalent d'ion métallique ou un ion ammonium,
L et M représentent de l'oxygène et/ou du soufre,
R¹ est un groupe, éventuellement substitué par du fluor ou du chlore, alkyle en C₁ à C₁₄, alcényle en C₂ à C₁₄, (alkoxy en C₁ à C₄)-(alkyle en C₂ à C₆), (alkylthio en C₁ à C₄)-(alkyle en C₂ à C₆), (polyalkoxy en C₁ à C₄)-(alkyle en C₂ à C₄) et un groupe cycloalkyle à noyau de 3 à 6 atomes qui peut être interrompu par un ou deux atomes d'oxygène et/ou de soufre, un groupe phényle éventuellement substitué par du fluor, du chlore, du brome, un radical méthyle, éthyle, propyle, isopropyle, méthoxy, éthoxy, trifluorométhyle, trifluorométhoxy, nitro,
un groupe phényle-(alkyle en C₁ à C₃) éventuellement substitué par du fluor, du chlore, du brome, un radical méthyle, éthyle, propyle, isopropyle, méthoxy, éthoxy, trifluorométhyle, trifluorométhoxy,
des groupes pyridyle, pyrimidyle, thiazolyle et pyrazolyle éventuellement substitués par du fluor, du chlore, du brome, un radical méthyle ou éthyle,
un groupe phénoxy-(alkyle en C₁ à C₄) éventuellement substitué par du fluor, du chlore, un radical méthyle ou éthyle,
des groupes pyridyloxy-(alkyle en C₁ à C₄), pyrimidyloxy-(alkyle en C₁ à C₄) et thiazolyloxy-(alkyle en C₁ à C₄) éventuellement substitués par du fluor, du chlore, un radical amino, méthyle, éthyle,
R² représente un groupe, éventuellement substitué par du fluor ou du chlore, alkyle en C₁ à C₁₄, alcényle en C₃ à C₁₄, (alkoxy en C₁ à C₄)-(alkyle en C₂ à C₆), (polyalkoxy en C₁ à C₄)-(alkyle en C₂ à C₆),
ou un groupe phényle ou benzyle éventuellement substitué par du fluor, du chlore, un radical nitro, méthyle, éthyle, propyle, isopropyle, méthoxy, éthoxy, trifluorométhyle,
R³, R⁴ et R⁵ représentent, indépendamment les uns des autres, un groupe, éventuellement substitué par du fluor ou du chlore, alkyle en C₁ à C₄, alkoxy en C₁ à C₄, alkylamino en C₁ à C₄, di-(alkyle en C₁ à C₄)amino, alkylthio en C₁ à C₄, un groupe phényle, phénoxy ou phénylthio éventuellement substitué par du fluor, du chlore, du brome, un radical nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃,
R⁶ et R⁷ représentent, indépendamment l'un de l'autre, un groupe, éventuellement substitué par du fluor, du chlore ou du brome, alkyle en C₁ à C₁₀, alcényle en C₃ à C₆, alkoxy en C₁ à C₁₀, (alkoxy en C₁ à C₁₀)-(alkyle en C₁ à C₁₀), un groupe phényle éventuellement substitué par du fluor, du chlore, du brome, un radical halogénalkyle en C₁ à C₂₀, alkyle en C₁ à C₂₀ ou alkoxy en C₁ à C₄, un groupe benzyle éventuellement substitué par du fluor, du chlore, du brome, un radical alkyle en C₁ à C₄, halogénalkyle en C₁ à C₄ ou alkoxy en C₁ à C₄, ou forment ensemble un noyau alkylène en C₄ à C₆ éventuellement interrompu par de l'oxygène ou du soufre.
ainsi que leurs formes stéréo-isomères et énantiomères pures et leurs mélanges.

7. Composés de formule (II) 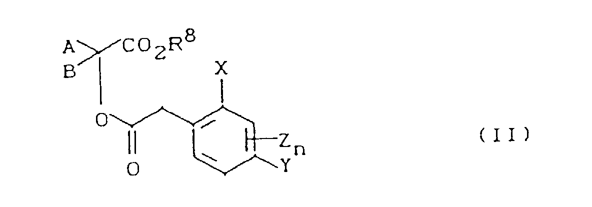 dans laquelle
A, B, X, Y, Z, n et R⁸ ont la définition indiquée dans la revendication 1.

8. Procédé de production d'esters d'acides O-acyl-α-hydroxycarboxyliques de formule (II) suivant la revendication 7, caractérisé en ce que
a) on acyle des acides 2-hydroxycarboxyliques (esters) de formule (XIII) 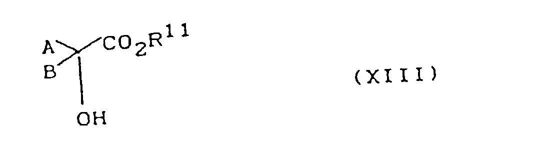 dans laquelle
R¹¹ représente de l'hydrogène dans le cas de composés de formule (XIIIa) ou un groupe alkyle dans le cas de composés de formule (XIIIb)
et
A et B ont la définition indiquée dans la revendication 1,
avec des halogénures d'acide phénylacétique de formule (XIV) 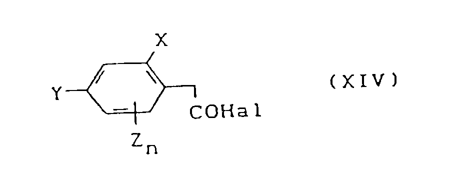 dans laquelle
X, Y, Z et n ont la définition indiquée ci-dessus et
Hal représente du chlore ou du brome,
ou bien on estérifie des acides hydroxycarboxyliques de formule (IIa) 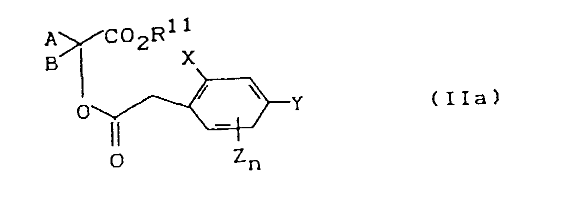 dans laquelle
A, B, X, Y, Z et n ont la définition indiquée ci-dessus
et
R¹¹ est de l'hydrogène.

9. Compositions pesticides, caractérisées par une teneur en au moins un dérivé de 3-aryl-4-hydroxy-Δ³-dihydrofurannone de formule (I) suivant la revendication 1;

10. Utilisation de dérivés de 3-aryl-4-hydroxy-Δ³-dihydrofurannones de formule (I) suivant la revendication 1 pour combattre des parasites.

11. Procédé de préparation de compositions pesticides, caractérisé en ce qu'on mélange des dérivés de 3-aryl-4-hydroxy-Δ³-dihydrofurannones de formule (I) suivant la revendication 1 avec des diluants et/ou des agents tensioactifs.
